# EUROPEAN PATENT SPECIFICATION

(11) **EP 4 469 461 B1**
(45) Date of publication and mention of the grant of the patent: **15.10.2025**
(21) Application number: 23701996.3
(22) Date of filing: 27.01.2023
(51) Int. Cl.: C07D 491/052, C07D 519/00, A61P 31/12, A61P 35/00, A61K 45/06, A61K 31/4162, A61K 31/437

(54) **TETRAHYDROPYRANOPYRAZOLE DERIVATIVES FOR THE TREATMENT OF CANCER AND VIRAL INFECTIONS**
TETRAHYDROPYRANOPYRAZOL-DERIVATE ZUR BEHANDLUNG VON KREBS UND VIRALEN INFEKTEN
DÉRIVÉS DE TETRAHYDROPYRANOPYRAZOLE POUR LE TRAITEMENT DU CANCER ET D'INFECTIONS VIRALES

(30) Priority: 27.01.2022 EP 22153611
(43) Date of publication of application: 04.12.2024
(73) Proprietor: Genase Therapeutics B.V., 3521 AL Utrecht (NL)
(72) Inventor: BARF, Tjeerd, 5371AS Ravenstein (NL); LAIN, Sonia, 11532 Stockholm (SE); BENGTSSON, Christoffer, 16860 Bromma (SE); SANDBERG, Lars, 122 64 Enskede (SE); YNGVE, Ulrika, 757 52 Uppsala (SE)
(74) Representative: Boult Wade Tennant LLP
(86) International application number: PCT/EP2023/052058
(87) International publication number: WO 2023/144332

(56) References cited:
- WO-A1-2017/077280
- LADDS, M.J.G.W. ET AL.: "A DHODH inhibitor increases p53 synthesis and enhances tumor cell killing by p53 degradation blockage", vol. 9, no. 1, 2018, pages 1 - 14, XP055937124, Retrieved from the Internet <URL:https://www.nature.com/articles/s41467-018-03441-3.pdf> DOI: 10.1038/s41467-018-03441-3
- POPOVA, G. ET AL.: "Optimization of Tetrahydroindazoles as Inhibitors of Human Dihydroorotate Dehydrogenase and Evaluation of Their Activity and In Vitro Metabolic Stability", vol. 63, no. 8, 2020, pages 3915 - 3934, XP055937127, ISSN: 0022-2623, Retrieved from the Internet <URL:http://pubs.acs.org/doi/pdf/10.1021/acs.jmedchem.9b01658> DOI: 10.1021/acs.jmedchem.9b01658

## Description

### Field of the invention

The present invention relates to novel compounds, compositions and medical uses thereof. In particular, the present invention relates to certain tetrahydropyranopyrazoles, which are useful in the treatment of cancers and/or the treatment or prevention of viral infections.

### Background of the invention

The listing or discussion of an apparently prior-published document in this specification should not necessarily be taken as an acknowledgement that the document is part of the state of the art or is common general knowledge.

During the last decade a number of small molecules and peptides that activate p53 tumor suppressor functions in a DNA damage independent manner have been identified (see, for example, C. J. Brown et al., Nature reviews: Cancer, 9, 862 (2009)). Some of these compounds impair the interaction of p53 with mdm2 and/or mdmx (also called mdm4), two important negative regulators of p53 (see Hoe, C. S. et al., Nat Rev Drug Discov. 13, 217 (2014)).

There are several chemically distinct classes of mdm2/p53 binding antagonists and of these nutlin-3 is the most easily available and commonly used to protect p53 from degradation (see L. T. Vassilev et al., Science. 303, 844 (2004); I. R. Hardcastle et al., Bioorganic & medicinal chemistry letters, 15, 1515 (2005); K. Ding et al., Journal of medicinal chemistry, 49, 3432 (2006); and C. J. Brown et al., ACS chemical biology, 8, 506 (2013)). A derivative of nutlin-3, RG7112 (Roche), has recently completed Phase I clinical trials (see I. Ray-Coquard et al., The Lancet Oncology, 13, 1133 (2012).

Although mdm2/p53 binding antagonists have cytotoxic effects, they also have a reversible cytostatic effect that is likely to limit their efficacy. To some extent, this cytostatic effect could be due to a strong induction of p21 (waf1/cip1) by these compounds.

In the last few years, a series of reports have demonstrated that the efficacy of nutlin-3 at tumor cell killing is increased when administered in combination with other targeted small molecules such as the ATM kinase inhibitor KU-55933 and the BRAF^{V600E} inhibitor vemurafenib (see K. D. Sullivan et al., Nature Chemical Biology, 8, 646 (2012); Z. Ji et al., Clinical cancer research : an official journal of the American Association for Cancer Research, 19, 4383 (2013); and M. Lu et al., Cancer Cell, 23, 618 (2013)).

Previous disclosures have utilized a series of phenotypic screens searching for novel p53 activators. These screens were carried out using a murine fibroblast cell line (T22 RGCΔFos-LacZ cells) and led to the identification of compounds that, as described for nutlin-3, activate p53 in all *TP53* wild-type cells tested (see S. Lain et al., Cancer Cell, 13, 454 (2008); G. M. Marshall et al., PLoS genetics, 7, e1002135 (2011); H. Yuan et al., Blood, 119, 1904 (2012); A. Menssen et al., Proc Natl Acad Sci USA, 109, E187 (2012)).

Targeted therapeutics such as mdm2/p53 binding antagonists and BCR/ABL tyrosine kinase inhibitors can reduce cancer growth but rarely lead to the complete eradication of malignant cells. Thus, there exists a need to identify compounds capable of increasing pro-apoptotic functions of tumour suppressors that may increase the chance of achieving a cure in cancer patients.

Severe acute respiratory syndrome (SARS), Middle East respiratory syndrome (MERS), Western/Eastern equine encephalitis (WEE/EEE), and Ebola, as well as pandemic influenza (e.g. H1N1) are lethal and transmissible through travellers. The fast spread of these diseases constitute a major threat to public health worldwide and will require broad-spectrum antiviral agents to prevent pandemic scale outbreaks and allow time for the development of effective vaccines, when possible ("Broad-spectrum antiviral agents," Jun-Da Zhu, WenMeng, Xiao-JiaWang and Hwa-Chain, R. Wang, Frontiers in Microbiology, doi: 10.3389/fmicb.2015.00517). Furthermore, inhibition of DHODH or *de novo* pyrimidine biosynthesis was reported as an antiviral approach for *inter alia* Ebola, HIV, HCV, hCMV and influenza (Hoffmann et al., Proc. Natl. Acad. Sci. 2011; 108: 5777; Wang et al., J. Virol. 2011; 85: 6548, Hahn et al., Viruses 2020; 12: 1394) as well as rotaviruses (Chen et al., Antiviral Research 2019; 167: 35)

WO2017/077280 discloses 4,5,6,7-tetrahydroindazoles compounds for use in treating cancer and viral infections.

Ladds, M. J. G. W. et al., Nature Communications, 9, 1-14 (2018) describes tetrahydroindazoles-based human dihydroorotate dehydrogenase (hDHODH) inhibitors that increased p53 synthesis and enhance tumor cell killing via blockade of p53 degradation.

Popova, G. et al., Journal of Medicinal Chemistry, 63(8), 3915-3934 (2020) describes tetrahydroindazoles-based hDHODH inhibitors that were evaluated for their activity and in vitro metabolic stability.

### Description of the Invention

The compounds described by Ladds *et al.* (2018) and Popova *et al.* (2020) are tetrahydroindazoles that suffer from metabolic liabilities and possess certain Cytochrome P450 (CYP) liabilities. The present inventors have surprisingly found that by introducing an oxygen atom in the central ring system the resulting tetrahydropyranopyrazoles are more stable in human liver microsomes and human hepatocytes while displaying comparatively lower inhibition of a range of CYPs in human liver microsomes.

### Compounds

In a first aspect of the invention, there is provided a compound of formula I 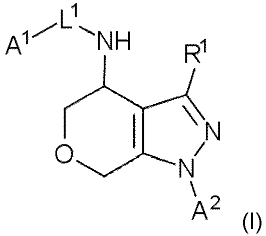 or a pharmaceutically acceptable salt thereof, wherein:
A¹ is a 9-membered bicyclic heteroaryl, 6-membered heteroaryl or 5-membered heteroaryl, optionally substituted with one or more groups independently selected from G²;
A² is phenyl optionally substituted by one or more groups independently selected from G³;
L¹ represents -C(O)-;
R¹ represents H;
wherein:
   each G² independently represents halo, R^{a2}, -CN, -A^{a2}-C(Q^{a2})R^{b2} -A^{b2}-C(Q^{b2})N(R^{c2})R^{d2}, -A^{c2}-C(Q^{c2})OR^{e2}, -A^{d2}-S(O)ₚR^{f2}, -A^{e2}-S(O)_{q}N(R^{g2})R^{h2}, -A^{f2}-S(O)ₚORⁱ², -N₃, -N(R^{j2})R^{k2}, -N(H)CN, -NO₂, -ONO₂, -OR^{l2} or -SR^{m2};
   each Q^{a2} to Q^{c2} independently represents =O, =S, =NRⁿ² or =N(OR^{o2});
   each A^{a2} to A^{f2} independently represents a single bond, -N(R^{p2})- or -O-;
   each G³ independently represents halo, R^{a3}, -CN, -A^{a3}-C(Q^{a3})R^{b3}, -A^{b3}-C(Q^{b3})N(R^{c3})R^{d3}, -A^{c3}-C(Q^{c3})OR^{e3}, -A^{d3}-S(O)ₚR^{f3}, -A^{e3}-S(O)_{q}N(R^{g3})R^{h3}, -A^{f3}-S(O)ₚORⁱ³, -N₃, -N(R^{j3})R^{k3}, -N(H)CN, -NO₂, -ONO₂, -OR^{l3} or -SR^{m3};
   each Q^{a3} to Q^{c3} independently represents =O, =S, =NRⁿ³ or =N(OR^{o3});
   each A^{a3} to A^{f3} independently represents a single bond, -N(R^{p3})- or -O-;
   each R^{a2} and R^{f2} independently represents C₁₋₆ alkyl optionally substituted by one or more groups independently selected from G^{6a}, heterocycloalkyl optionally substituted by one or more groups independently selected from G^{6b}, aryl optionally substituted by one or more groups independently selected from G^{6c}, or heteroaryl optionally substituted by one or more groups independently selected from G^{6d};
   each R^{p2} independently represents H or C₁₋₆ alkyl optionally substituted by one or more F;
   each R^{b2}, R^{c2}, R^{d2}, R^{e2}, R^{g2}, R^{h2}, Rⁱ², R^{j2}, R^{k2}, R^{l2}, R^{m2}, Rⁿ² and R^{o2} independently represents H, C₁₋₆alkyl optionally substituted by one or more groups independently selected from G^{6a}, heterocycloalkyl optionally substituted by one or more groups independently selected from G^{6b}, aryl optionally substituted by one or more groups independently selected from G^{6c}, or heteroaryl optionally substituted by one or more groups independently selected from G^{6d}; or
   alternatively any of R^{c2} and R^{d2}, R^{g2} and R^{h2} and/or R^{j2} and R^{k2} are linked together to form, together with the nitrogen atom to which they are attached, a 3- to 6-membered ring, which ring optionally contains one further heteroatom and which ring optionally is substituted by one or more groups independently selected from halo, C₁₋₃ alkyl optionally substituted by one or more halo, and =O;
   each R^{a3} and R^{f3} independently represents C₁₋₆ alkyl optionally substituted by one or more groups independently selected from G^{7a}, heterocycloalkyl optionally substituted by one or more groups independently selected from G^{7b}, aryl optionally substituted by one or more groups independently selected from G^{7c}, or heteroaryl optionally substituted by one or more groups independently selected from G^{7d};
   each R^{p3} independently represents H or C₁₋₆ alkyl optionally substituted by one or more F;
   each R^{b3}, R^{c3}, R^{d3}, R^{e3}, R^{g3}, R^{h3}, Rⁱ³, R^{j3}, R^{k3}, R^{l3}, R^{m3}, Rⁿ³ and R^{o3} independently represents H, C₁₋₆ alkyl optionally substituted by one or more groups independently selected from G^{7a}, heterocycloalkyl optionally substituted by one or more groups independently selected from G^{7b}, aryl optionally substituted by one or more groups independently selected from G^{7c}, or heteroaryl optionally substituted by one or more groups independently selected from G^{7d}; or
   alternatively any of R^{c3} and R^{d3}, R^{g3} and R^{h3} and/or R^{j3} and R^{k3} are linked together to form, together with the nitrogen atom to which they are attached, a 3- to 6-membered ring, which ring optionally contains one further heteroatom and which ring optionally is substituted by one or more groups independently selected from halo, C₁₋₃ alkyl optionally substituted by one or more halo, and =O;
   each G^{6a}, G^{6b}, G^{7a} and G^{7b} independently represents halo, -CN, -N(R^{b5})R^{c5}, -OR^{d5}, -SR^{e5} or =O;
   each G^{6c}, G^{6d}, G^{7c} and G^{7d} independently represents halo, R^{a5}, -CN, -N(R^{b5})R^{c5}, -OR^{d5}, -SR^{e5} or =O;
   each R^{a5} independently represents C₁₋₆ alkyl optionally substituted by one or more F;
   each R^{b5}, R^{c5}, R^{d5} and R^{e5} independently represents H or C₁₋₆ alkyl optionally substituted by one or more F or =O; and
   or R^{b5} and R^{c5} are linked together to form, along with the nitrogen atom to which they are attached, a 3- to 6-membered ring, which ring optionally contains one further heteroatom and which ring optionally is substituted by one or more groups independently selected from F, C₁₋₃ alkyl optionally substituted by one or more F, and =O;
   and each p and q independently represents 1 or 2,
   which compounds (including pharmaceutically acceptable salts) may be referred to herein as compounds of the first aspect of the invention.

The skilled person will understand that references herein to compounds of particular aspects of the invention will include references to all embodiments and particular forms thereof, which embodiments and particular forms may be taken in combination to form further embodiments .

Unless indicated otherwise, all technical and scientific terms used herein have the same meaning as commonly understood by one of ordinary skill in the art to which this invention pertains.

Pharmaceutically acceptable salts include acid addition salts and base addition salts. Such salts may be formed by conventional means, for example by reaction of a free acid or a free base form of a compound of the invention with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin.

Particular acid addition salts that may be mentioned include carboxylate salts (e.g. formate, acetate, trifluoroacetate, propionate, isobutyrate, heptanoate, decanoate, caprate, caprylate, stearate, acrylate, caproate, propiolate, ascorbate, citrate, glucuronate, glutamate, glycolate, α-hydroxybutyrate, lactate, tartrate, phenylacetate, mandelate, phenylpropionate, phenylbutyrate, benzoate, chlorobenzoate, methylbenzoate, hydroxybenzoate, methoxybenzoate, dinitrobenzoate, *o*-acetoxybenzoate, salicylate, nicotinate, isonicotinate, cinnamate, oxalate, malonate, succinate, suberate, sebacate, fumarate, malate, maleate, hydroxymaleate, hippurate, phthalate or terephthalate salts), halide salts (e.g. chloride, bromide or iodide salts), sulfonate salts (e.g. benzenesulfonate, methyl-, bromo- or chloro-benzenesulfonate, xylenesulfonate, methanesulfonate, ethanesulfonate, propanesulfonate, hydroxyethanesulfonate, 1- or 2- naphthalenesulfonate or 1,5-naphthalenedisulfonate salts) or sulfate, pyrosulfate, bisulfate, sulfite, bisulfite, phosphate, monohydrogenphosphate, dihydrogenphosphate, metaphosphate, pyrophosphate or nitrate salts, and the like.

Particular base addition salts that may be mentioned include salts formed with alkali metals (such as Na and K salts), alkaline earth metals (such as Mg and Ca salts), organic bases (such as ethanolamine, diethanolamine, triethanolamine, tromethamine and lysine) and inorganic bases (such as ammonia and aluminium hydroxide). More particularly, base addition salts that may be mentioned include Mg, Ca and, most particularly, K and Na salts.

For the avoidance of doubt, compounds of the first aspect of the invention may exist as solids, and thus the scope of the invention includes all amorphous, crystalline and part crystalline forms thereof, and may also exist as oils. Where compounds of the first aspect of the invention exist in crystalline and part crystalline forms, such forms may include solvates, which are included in the scope of the invention. Compounds of the first aspect of the invention may also exist in solution.

Compounds of the first aspect of the invention contain double bonds and may thus exist as *E* (*entgegen*) and *Z* (*zusammen*) geometric isomers about each individual double bond. All such geometric isomers and mixtures thereof are included within the scope of the invention.

Compounds of the first aspect of the invention may also exhibit tautomerism. All tautomeric forms and mixtures thereof are included within the scope of the invention.

Compounds of the invention also contain one or more asymmetric carbon atoms and may therefore exhibit optical and/or diastereoisomerism. Diastereoisomers may be separated using conventional techniques, e.g. chromatography or fractional crystallisation. The various stereoisomers (i.e. enantiomers) may be isolated by separation of a racemic or other mixture of the compounds using conventional, e.g. fractional crystallisation or HPLC, techniques. Alternatively, the desired optical isomers may be made by reaction of the appropriate optically active starting materials under conditions which will not cause racemisation or epimerisation (i.e. a 'chiral pool' method), by reaction of the appropriate starting material with a 'chiral auxiliary' which can subsequently be removed at a suitable stage, by derivatisation (i.e. a resolution, including a dynamic resolution); for example, with a homochiral acid followed by separation of the diastereomeric derivatives by conventional means such as chromatography, or by reaction with an appropriate chiral reagent or chiral catalyst all under conditions known to the skilled person. All stereoisomers and mixtures thereof are included within the scope of the invention.

In particular, compounds of the first aspect of the invention may exhibit stereoisomerism at the carbon marked with an asterisk (*) in the compound of formula I below, with compounds of the first aspect of the invention existing in the *R*- and *S*- configurations at that carbon (which configuration may be determined by those skilled in the art).

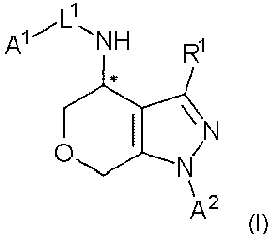

As used herein, references to halo and/or halogen groups will each independently refer to fluoro, chloro, bromo and iodo (for example, fluoro (F) and chloro (CI)).

Unless otherwise specified, C_{1-z} alkyl groups (where z is the upper limit of the range) defined herein may be straight-chain or, when there is a sufficient number (i.e. a minimum of two or three, as appropriate) of carbon atoms, be branched-chain.

As used herein, the term aryl includes references to C₆₋₁₄ (e.g. C₆₋₁₀) aromatic groups. Such groups may be monocyclic or bicyclic and, when bicyclic, be either wholly or partly aromatic. C₆₋₁₀ aryl groups that may be mentioned include phenyl, naphthyl, 1,2,3,4-tetrahydronaphthyl, indanyl, and the like (e.g. phenyl, naphthyl and the like, such as phenyl). For the avoidance of doubt, the point of attachment of substituents on aryl groups may be *via* any carbon atom of the ring system.

As used herein, the term heteroaryl (or heteroaromatic) includes references to 5- to 14-(e.g. 5- to 10-) membered heteroaromatic groups containing one or more heteroatoms selected from oxygen, nitrogen and/or sulfur. Such heteroaryl groups may comprise one, two, or three rings, of which at least one is aromatic (e.g. a heteroaryl group may comprise two rings, one of which is aromatic). Substituents on heteroaryl/heteroaromatic groups may, where appropriate, be located on any atom in the ring system including a heteroatom. The point of attachment of heteroaryl/heteroaromatic groups may be *via* any atom in the ring system including (where appropriate) a heteroatom. In particular, bicyclic heteroaryl/heteroaromatic groups may comprise a benzene ring fused to one or more further aromatic or non-aromatic heterocyclic rings, in which instances, the point of attachment of the polycyclic heteroaryl/heteroaromatic group may be *via* any ring including the benzene ring or the heteroaryl/heteroaromatic or heterocycloalkyl ring. Examples of heteroaryl/heteroaromatic groups that may be mentioned include pyridinyl, pyrrolyl, furanyl, thiophenyl, oxadiazolyl, thiadiazolyl, thiazolyl, oxazolyl, pyrazolyl, triazolyl, tetrazolyl, isoxazolyl, isothiazolyl, imidazolyl, imidazopyrimidinyl, imidazothiazolyl, thienothiophenyl, pyrimidinyl, furopyridinyl, indolyl, azaindolyl, pyrazinyl, pyrazolopyrimidinyl**,** indazolyl, pyrimidinyl, quinolinyl, isoquinolinyl, quinazolinyl, benzofuranyl, benzothiophenyl, benzoimidazolyl, benzoxazolyl, benzothiazolyl, benzotriazolyl and purinyl. The oxides of heteroaryl/heteroaromatic groups are also embraced within the scope of the invention (e.g. the *N*-oxide). As stated above, heteroaryl includes polycyclic (e.g. bicyclic) groups in which one ring is aromatic (and the other(s) may or may not be aromatic). Hence, other heteroaryl groups that may be mentioned include e.g. benzo[1,3]dioxolyl, benzo[1,4]dioxinyl, dihydrobenzo[d]isothiazole, 3,4-dihydrobenz[1,4]oxazinyl, dihydrobenzothiophenyl, indolinyl, *5H,6H,7H*-pyrrolo[1,2-*b*]pyrimidinyl, tetrahydro-1,2-benzisoxazolyl, 1,2,3,4-tetrahydroquinolinyl, thiochromanyl and the like.

As used herein, the term heterocycloalkyl may refer to non-aromatic monocyclic and bicyclic heterocycloalkyl groups (which groups may further be bridged) in which at least one (e.g. one to four) of the atoms in the ring system is other than carbon (i.e. a heteroatom), and in which the total number of atoms in the ring system is between three and twelve (e.g. between five and ten and, most preferably, between three and eight, e.g. a 5- or 6-membered heterocycloalkyl group). Further, such heterocycloalkyl groups may be saturated or unsaturated containing one or more double and/or triple bonds, forming for example a C_{2-z} (e.g. C_{4-z}) heterocycloalkenyl (where z is the upper limit of the range) or a C_{7-z} heterocycloalkynyl group. C_{2-z} heterocycloalkyl groups that may be mentioned include 7-azabicyclo-[2.2.1]heptanyl, 6-azabicyclo[3.1.1]heptanyl, 6-azabicyclo[3.2.1]-octanyl, 8-azabicyclo[3.2.1]octanyl, aziridinyl, azetidinyl, 2,3-dihydroisothiazolyl, dihydropyranyl, dihydropyridyl, dihydropyrrolyl (including 2,5-dihydropyrrolyl), dioxolanyl (including 1,3-dioxolanyl), dioxanyl (including 1,3-dioxanyl and 1,4-dioxanyl), dithianyl (including 1,4-dithianyl), dithiolanyl (including 1,3-dithiolanyl), imidazolidinyl, imidazolinyl, isothiazolidinyl, morpholinyl, 7-oxabicyclo[2.2.1]heptanyl, 6-oxabicyclo[3.2.1]-octanyl, oxetanyl, oxiranyl, piperazinyl, piperidinyl, pyranyl, pyrazolidinyl, pyrrolidinonyl, pyrrolidinyl, pyrrolinyl, quinuclidinyl, sulfolanyl, 3-sulfolenyl, tetrahydropyranyl, tetrahydrofuryl, tetrahydropyridyl (such as 1,2,3,4-tetrahydropyridyl and 1,2,3,6-tetrahydropyridyl), thietanyl, thiiranyl, thiolanyl, tetrahydrothiopyranyl, thiomorpholinyl, trithianyl (including 1,3,5-trithianyl), tropanyl and the like. Substituents on heterocycloalkyl groups may, where appropriate, be located on any atom in the ring system including a heteroatom. Further, in the case where the substituent is another cyclic compound, then the cyclic compound may be attached through a single atom on the heterocycloalkyl group, forming a so-called "spiro"-compound. The point of attachment of heterocycloalkyl groups may be *via* any atom in the ring system including (where appropriate) a further heteroatom (such as a nitrogen atom), or an atom on any fused carbocyclic ring that may be present as part of the ring system. Heterocycloalkyl groups may also be in the N- or *S*- oxidised form.

At each occurrence when mentioned herein, particular heterocycloalkyl groups that may be mentioned include 3- to 8-membered heterocycloalkyl groups (e.g. a 4- to 6-membered heterocycloalkyl group).

For the avoidance of doubt, as used herein, references to heteroatoms will take their normal meaning as understood by one skilled in the art. Particular heteroatoms that may be mentioned include phosphorus, selenium, tellurium, silicon, boron, oxygen, nitrogen and sulfur (e.g. oxygen, nitrogen and sulfur).

For the avoidance of doubt, references to polycyclic (e.g. bicyclic) groups (e.g. when employed in the context of heterocycloalkyl groups) will refer to ring systems wherein more than two scissions would be required to convert such rings into a straight chain, with the minimum number of such scissions corresponding to the number of rings defined (e.g. the term bicyclic may indicate that a minimum of two scissions would be required to convert the rings into a straight chain). For the avoidance of doubt, the term bicyclic (e.g. when employed in the context of heterocycloalkyl groups) may refer to groups in which the second ring of a two-ring system is formed between two adjacent atoms of the first ring, and may also refer to groups in which two non-adjacent atoms are linked by either an alkylene or heteroalkylene chain (as appropriate), which later groups may be referred to as bridged.

For the avoidance of doubt, when an aryl or an heteroaryl group is substituted with a group *via* a double bond, such as =O, it is understood that the aryl or heteroaryl group is partly aromatic, i.e. the aryl or heteroaryl group consists of at least two rings where at least one ring is not aromatic.

The present invention also embraces isotopically-labelled compounds of the present invention which are identical to those recited herein, but for the fact that one or more atoms are replaced by an atom having an atomic mass or mass number different from the atomic mass or mass number usually found in nature (or the most abundant one found in nature). All isotopes of any particular atom or element as specified herein are contemplated within the scope of the compounds of the invention. Hence, the compounds of the invention also include deuterated compounds, i.e. in which one or more hydrogen atoms are replaced by the hydrogen isotope deuterium.

For the avoidance of doubt, in cases in which the identity of two or more substituents in a compound of the invention may be the same, the actual identities of the respective substituents are not in any way interdependent. For example, in the situation in which two or more R³ groups are present, those R³ groups may be the same or different. Likewise, when more than one R^{a1} is present and each independently represents C₁₋₆ alkyl substituted by one or more G^{1a} group, the identities of each G^{1a} are in no way interdependent.

For the avoidance of doubt, when a term such as *"A^{a1} to A^{f1}"* is employed herein, this will be understood by the skilled person to mean A^{a1}, A^{b1}, A^{c1}, A^{d1}, A^{e1} and A^{f1} inclusively. Unless otherwise stated, the same reasoning will apply to other such terms used herein.

The skilled person will appreciate that compounds of the invention that are the subject of this invention include those that are stable. That is, compounds of the invention include those that are sufficiently robust to survive isolation, e.g. from a reaction mixture, to a useful degree of purity.

All embodiments of the invention and particular features mentioned herein may be taken in isolation or in combination with any other embodiments and/or particular features mentioned herein (hence describing more particular embodiments and particular features as disclosed herein) without departing from the disclosure of the invention.

The term 'optionally substituted' as used herein means that the referenced group may be substituted with one or more additional group(s).

Disclosed herein but not part of the claimed invention, A¹ represents phenyl optionally substituted by one or more (such as one, two or three, e.g. one or two) groups independently selected from G¹ or heteroaryl optionally substituted by one or more (such as one, two or three, e.g. one or two) groups independently selected from G².

In more particular instances, A¹ represents heteroaryl optionally substituted by one or more (such as one, two or three, e.g. one or two) groups independently selected from G².

In yet more particular instances, A¹ represents a mono- or bi- cyclic heteroaryl optionally substituted by one or more (e.g. one or two groups) groups (i.e. G² groups) independently selected from halo, R^{a2}, -C(O)OR^{e2}, -OR^{l2} and -SR^{m2}.

In yet more particular instances, A¹ represents a mono- or bi- cyclic heteroaryl optionally substituted by one or more (e.g. one or two) groups (i.e. G² groups) independently selected from halo (e.g. F), C₁₋₃ alkyl optionally substituted by one or more fluoro (such as -CF₃), - C(O)OH, -C(O)OC₁₋₃ alkyl, -OH, -OC₁₋₃ alkyl, -SH and -SC₁₋₃ alkyl (such as F, C₁₋₂ alkyl, - OH and -SCH₃).

In yet more particular instances, A¹ represents a mono- or bi- cyclic heteroaryl optionally substituted by one or more (e.g. one or two) groups (i.e. G² groups) independently selected from halo (e.g. F), C₁₋₃ alkyl, -CF₃, -C(O)OH, -C(O)OC₁₋₃ alkyl, -OH, -OC₁₋₃ alkyl, -SH and -SC₁₋₃ alkyl (such as F, C₁₋₂ alkyl, -OH and -SCH₃).

In yet more particular instances, A¹ represents a mono- or bi- cyclic heteroaryl optionally substituted by one or more (e.g. one or two) groups (i.e. G² groups) independently selected from halo (e.g. F), C₁₋₃ alkyl, -C(O)OH, -C(O)OC₁₋₃ alkyl, -OH, -OC₁₋₃ alkyl, -SH and -SC₁₋₃ alkyl (such as F, C₁₋₂ alkyl, -OH and -SCH₃).

For example, A¹ may represent a bi- cyclic heteroaryl (for example, a 9-membered bi-cyclic heteroaryl, such as tetrahydro-1,2-benzisoxazolyl, e.g. tetrahydro-1,2-benzisoxazol-3-yl) optionally substituted by one or two groups (i.e. G² groups) independently selected from halo, R^{a2}, -C(O)OR^{e2}, -OR^{l2} and -SR^{m2} (such as one or two groups independently selected from halo (e.g. F), C₁₋₃ alkyl, -C(O)OH, -C(O)OC₁₋₃ alkyl, -OH, -OC₁₋₃ alkyl, -SH and -SC₁₋₃ alkyl, e.g. one or two groups (i.e. G² groups) independently selected from F, C₁₋₂ alkyl, -OH and -SCH₃). For the avoidance of doubt, such bi- cyclic heteroaryl (for example, a 9-membered bi-cyclic heteroaryl, such as tetrahydro-1,2-benzisoxazolyl, e.g. tetrahydro-1,2-benzisoxazol-3-yl) groups may be unsubstituted.

Further, A¹ may represent a mono- cyclic heteroaryl (for example, a 6-membered mono-cyclic heteroaryl, such pyridinyl, e.g. pyridine-2-yl) optionally substituted by one or two groups (i.e. G² groups) independently selected from halo, R^{a2}, -C(O)OR^{e2}, -OR^{l2} and -SR^{m2} (such as one or two groups independently selected from halo (e.g. F), C₁₋₃ alkyl, -C(O)OH, -C(O)OC₁₋₃ alkyl, -OH, -OC₁₋₃ alkyl, -SH and -SC₁₋₃ alkyl, e.g. one or two groups selected from F, C₁₋₃ alkyl, -C(O)OH and -C(O)OC₁₋₃ alkyl, for example one -C(O)OC₁₋₃ alkyl or - C(O)OH group). For the avoidance of doubt, such mono- cyclic heteroaryl (for example, a 6-membered mono-cyclic heteroaryl, such pyridinyl, e.g. pyridine-2-yl) groups may be unsubstituted.

In the first aspect of the invention, the mono- or bi- cyclic heteroaryl representing A¹ may be a 5- or 6- membered monocyclic or a 9- membered bicyclic heteroaryl. In particular, the mono- or bi- cyclic heteroaryl may be selected from pyridinyl (e.g. pyridine-2-yl and pyridine-3-yl), pyrazinyl (e.g. pyrazin-2-yl), benzofuranyl (e.g. benzofuran-3-yl), thiazolyl (e.g. thiazol-4-yl), thiophenyl (e.g. thiophen-2-yl), isoxazolyl (e.g. isoxazol-3-yl), 4,5,6,7-tetrahydrobenzo[c]isoxazolyl (e.g. 4,5,6,7-tetrahydrobenzo[c]isoxazol-3-yl), 1,3-benzoxazolyl (e.g. 1,3-benzoxazol-2-yl) and 1,2-benzisoxazolyl (e.g. 1,2-benzisoxazol-3-yl).

In the first aspect of the invention, the mono- or bi- cyclic heteroaryl representing A¹ may be a 5- or 6- membered monocyclic or a 9- membered bicyclic heteroaryl. In particular, the mono- or bi- cyclic heteroaryl may be selected from tetrahydro-2,1-benzisoxazolyl (e.g. tetrahydro-2,1-benzisoxazol-3-yl), benzoxazoyl (e.g. 1,3-benzisoxazol-3-yl or 1,2-benzisoxazol-3-yl), pyrazinyl, indazolyl, quinolinyl, 5H,6H,7H,8H-imidazo[1,5-a]pyridine-3-yl, 5H,6H,7H,8H-imidazo[1,5-a]pyridine-1-yl, imidazo[1,5-a]pyridin-3-yl, pyridinyl (e.g. pyridine-2-yl), thiazolyl (e.g. thiazol-4-yl), isoxazolyl (e.g. isoxazol-3-yl) and tetrahydro-1,2-benzisoxazolyl (e.g. tetrahydro-1,2-benzisoxazol-3-yl). More particularly, the mono- or bi-cyclic heteroaryl may be selected from pyridinyl (e.g. pyridine-2-yl), thiazolyl (e.g. thiazol-4-yl), isoxazolyl (e.g. isoxazol-3-yl) and tetrahydro-1,2-benzisoxazolyl (e.g. tetrahydro-1,2-benzisoxazol-3-yl). In some embodiments, A¹ is 5H,6H,7H,8H-imidazo[1,5-a]pyridine-3-yl or 5H,6H,7H,8H-imidazo[1,5-a]pyridine-1-yl.

Disclosed herein but not part of the claimed invention, A² represents phenyl optionally substituted by one or more (preferably one, two or three, e.g. one or two) groups independently selected from G³ or a 5- or 6-membered heteroaryl optionally substituted by one or more (preferably one, two or three, e.g. one or two) groups independently selected from G⁴.

In more particular instances, A² represents aryl optionally substituted by one or more (preferably one, two or three, e.g. one or two) groups independently selected from G³.

In the first aspect of the invention, A² represents phenyl optionally substituted by one or more (preferably one, two or three, e.g. one or two) groups independently selected from G³.

In more particular embodiments, A² represents phenyl optionally substituted by one or more (e.g. one or two) groups (i.e. G³ groups) independently selected from halo, R^{a3} and -OR^{l3}.

In further embodiments, G³ represents a group selected from halo, -A^{c3}-C(Q^{c3})OR^{e3} (e.g. -C(O)OR^{e3}, such as wherein R^{e3} represents C₁₋₃ alkyl), R^{a3} and -OR^{l3}.

In yet further embodiments, G³ represents a group selected from halo, -A^{a3}-C(Q^{a3})R^{b3} (e.g. -C(O)heteroaryl, for example -C(O)-morpholinyl, such as -C(O)-morpholin-4-yl) or -A^{c3}-C(Q^{c3})OR^{e3} (e.g. -C(O)OR^{e3}, such as wherein R^{e3} represents C₁₋₃ alkyl), R^{a3} and -OR^{l3}.

In alternative embodiments, G³ represents a group selected from halo, -R^{a3} and -OR^{l3}.

More particularly, A² may represent phenyl optionally substituted by one or more (e.g. one or two) groups (i.e. G³ groups) independently selected from halo (e.g. F), -C(O)OC₁₋₃ alkyl (such as -C(O)OCH₂CH₃), and C₁₋₄ alkyl (such as -CH₃ and -C(CH₃)₃). For example, A² may represent phenyl optionally substituted by one or two groups (i.e. G³ groups) independently selected from F, -CH₃, -C(O)OCH₂CH₃ and -C(CH₃)₃, such as phenyl substituted by one or two F.

Alternatively, A² may represent phenyl optionally substituted by one or more (e.g. one or two) groups (i.e. G³ groups) independently selected from halo (e.g. F), -C(O)-morpholinyl (such as -C(O)-morpholin-4-yl), -C(O)OC₁₋₃ alkyl (such as -C(O)OCH₂CH₃), and C₁₋₄ alkyl (such as -CH₃ and -C(CH₃)₃).

In more particular embodiments, A² is unsubstituted in at least the 2-position (i.e. relative to the point of attachment to the essential tetrahydroindazole, as depicted in formula I, wherein the skilled person will understand that the point of attachment is referred to as the 1- position).

Thus, in certain embodiments, the group representing A² may be depicted as 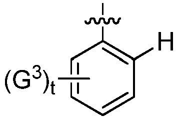 wherein G³ is as defined herein (i.e. as defined in any embodiment of the first aspect of the invention, or any combination thereof, such as where G³ represents a group independently selected from halo, R^{a3} and -OR^{l3}, e.g. a group selected from F, -CH₃ and -C(CH₃)₃ (for example, a F group), t represents 0 to 5 (e.g. 0 to 2) and the bond disected by the wavy line indicates the point of attachment to the essential tetrahydroindazole group.

More particularly, A² may be phenyl substituted with one or two substituents which are present at the 3-, 4-, 5- and/or 6-position(s). For example, A² may be phenyl substituted only in the 6- position (and therefore unsubstituted in the 2-, 3-, 4- and 5- positions), only in the 4- and 6- positions, or only in the 3- and 4- positions.

In other embodiments, A² is substituted in at least the 2-position optionally with halo (such as chloro or fluoro).

Disclosed herein but not part of the claimed invention, L¹ represents -C(O)N(R²)-, -C(O)O-, -S(O)₂N(R³)-, -C(O)- or -S(O)₂- (such as -C(O)- or -S(O)₂-). In the first aspect of the invention, L¹ represents -C(O)-.

Disclosed herein but not part of the claimed invention, R¹ represents H or C₁₋₃ alkyl (e.g. C₁ alkyl) optionally substituted by one or more F (e.g. H or -CH₃). In the first aspect of the invention, R¹ represents H.

Disclosed herein but not part of the claimed invention, R² and R³ each independently represents H or C₁₋₃ alkyl optionally substituted by one or more F. In more particular instances, R³ represents H. Thus, in yet more particular instances, R² and R³ each represent H. In some embodiments, R² represents H.

Disclosed herein but not part of the claimed invention:
each G¹ independently represents -CN, -A^{a1}-C(Q^{a1})R^{b1}, -A^{b1}-C(Q^{b1})N(RC¹)R^{d1}, -A^{c1}-C(Q^{c1})OR^{e1}, -A^{d1}-S(O)ₚR^{f1}, -A^{e1}-S(O)_{q}N(R^{g1})R^{h1}, -A^{f1}-S(O)ₚORⁱ¹, -N₃, -N(R^{j1})R^{k1}, -N(H)CN, -NO₂, -ONO₂, halo, R^{a1}, -OR^{l1} or -SR^{m1} (such as halo, R^{a1}, -C(O)OR^{e1}, -OR^{l1} and -SR^{m1});
each Q^{a1} to Q^{c1} independently represents =O, =S, =NRⁿ¹ or =N(OR^{o1}) (such as =O); and/or (e.g. and)
each A^{a1} to A^{f1} independently represents a single bond, -N(R^{p1})- or -O-.

In the first aspect of the invention:
each G² independently represents -CN, -A^{a2}-C(Q^{a2})R^{b2} -A^{b2}-C(Q^{b2})N(R^{c2})R^{d2}, -A^{c2}-C(Q^{c2})OR^{e2}, -A^{d2}-S(O)ₚR^{f2}, -A^{e2}-S(O)_{q}N(R^{g2})R^{h2}, -A^{f2}-S(O)ₚORⁱ², -N₃, -N(R^{j2})R^{k2}, -N(H)CN, -NO₂, -ONO₂, halo, R^{a2}, -OR^{l2} or -SR^{m2} (such as halo, R^{a2}, -C(O)OR^{e2}, -OR^{l2} or -SR^{m2});
each Q^{a2} to Q^{c2} independently represents =O, =S, =NRⁿ² or =N(OR^{o2}) (such as =O); and
each A^{a2} to A^{f2} independently represents a single bond, -N(R^{p2})- or -O-.

In particular embodiments, each G² independently represents halo, R^{a2}, -C(O)OR^{e2}, -OR^{l2} and -SR^{m2}. In more particular embodiments, each G² independently represents halo (e.g. F), C₁₋₃ alkyl, -C(O)OH, -C(O)OC₁₋₃ alkyl, -OH, -OC₁₋₃ alkyl, -SH and -SC₁₋₃ alkyl (such as F, C₁₋₂ alkyl, -OH and -SCH₃).

In the first aspect of the invention:
each G³ independently represents -CN, -A^{a3}-C(Q^{a3})R^{b3}, **-**A^{b3}-C(Q^{b3})N(R^{c3})R^{d3}, -A^{c3}-C(Q^{c3})OR^{e3}, -A^{d3}-S(O)ₚR^{f3}, -A^{e3}-S(O)_{q}N(R^{g3})R^{h3}, -A^{f3}-S(O)ₚORⁱ³, -N₃, -N(R^{j3})R^{k3}, -N(H)CN, -NO₂, -ONO₂, halo, R^{a3}, -OR^{l3} or -SR^{m3} (such as halo, R^{a3}, -OR^{l3} or -SR^{m3});
each Q^{a3} to Q^{c3} independently represents =O, =S, =NRⁿ³ or =N(OR^{o3}) (such as =O); and
each A^{a3} to A^{f3} independently represents a single bond, -N(R^{p3})- or -O-.

In more particular embodiments, each G³ independently represents halo, R^{a3} or -OR^{l3}.

In yet more particular embodiments, each G³ independently represents halo (e.g. F) or C₁₋₄ alkyl, such as F**,** -CH₃ or -C(CH₃)₃.

In yet more particular embodiments, each G³ independently represents F or -CH₃, such as F.

Disclosed herein but not part of the claimed invention:
each G⁴ independently represents -CN, -A^{a4}-C(Q^{a4})R^{b4}, -A^{b4}-C(Q^{b4})N(R^{c4})R^{d4}, -A^{c4}-C(Q^{c4})OR^{e4}, -A^{d4}-S(O)ₚR^{f4}, -A^{e4}-S(O)_{q}N(R^{g4})R^{h4}, -A^{f4}-S(O)ₚORⁱ⁴, -N₃, -N(R^{j4})R^{k4}, -N(H)CN, -NO₂, -ONO₂, halo, R^{a4}, -OR¹⁴ or -SR^{m4} (such as halo, R^{a4}, -OR^{l4} or -SR^{m4});
each Q^{a4} to Q^{c4} independently represents =O, =S, =NRⁿ⁴ or =N(OR^{o4}) (such as =O); and/or (e.g. and)
each A^{a4} to A^{f4} independently represents a single bond, -N(R^{p4})- or -O-.

In particular embodiments, p represents 2 and/or (e.g. and) q represents 2.

In some embodiments, A¹ represents a 9- membered bicyclic heteroaryl (such as 5H,6H,7H,8H-imidazo[1,5-a]pyridine-3-yl or 5H,6H,7H,8H-imidazo[1,5-a]pyridine-1-yl) optionally substituted by one or more groups independently selected from G²; A² represents phenyl optionally substituted by one or more groups independently selected from G³ (such as A² substituted in at least the 2-position optionally with halo (e.g. chloro or fluoro)); R¹ represents H; and L¹ represents -C(O)-.

In some embodiments, A¹ is a 9-membered bicyclic heteroaryl (such as 5H,6H,7H,8H-imidazo[1,5-a]pyridine-3-yl, 5H,6H,7H,8H-imidazo[1,5-a]pyridine-1-yl, 4H,5H,6H,7H-1,2-benzisoxazol-3-yl, 4H,5H,6H,7H-2,1-benzisoxazol-3-yl or 4H,5H,6H,7H-indazole-3-yl); 6-membered heteroaryl (such as pyridinyl) or 5-membered heteroaryl (such as pyrazolyl or imidazolyl), wherein the 5- or 6- membered heteroaryl is optionally substituted with one or more groups independently selected from G²; A² is phenyl optionally substituted by one or more groups independently selected from G³; R¹ is H; and/or L¹ is-C(O)-.

In some embodiments, each G² independently may represent R^{a2} (such as C₁₋₂ alkyl); and/or each G³ independently represents halo, R^{a3}, -A^{b3}-C(Q^{b3})N(R^{c3})R^{d3}, -A^{c3}-C(Q^{c3})OR^{e3}, -A^{d3}-S(O)ₚR^{f3}, -OR^{l3}; optionally wherein:
halo is fluoro or chloro, R^{a3} is CH₃ or CF₃, A^{b3}, A^{c3} and A^{d3} are a single bond, Q^{b3} and Q^{c3} are =O, p is 2,
R^{c3} and R^{d3} are CH₃ or are linked together to form, together with the nitrogen atom to which they are attached, a 5- or 6-membered ring, which ring optionally contains one further heteroatom (such as oxygen),
R^{e3} is C₁₋₃ alkyl, R^{f3} is CH₃,
R^{l3} is CH₃, CF₃, C₂ alkyl substituted with N(CH₃)₂, OH, OCH₃ or N(C=O)CH₃, and/or
C₁ alkyl substituted with C(=O)N(CH₃)₂.

In particular embodiments of the first aspect of the invention, the compound of formula I is not:

| **Structure** | **Name** |
|---|---|
| 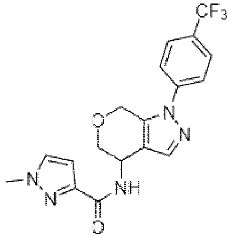 | 1-methyl-*N*-(1-(4-(trifluoromethyl)phenyl)-1,4,5,7-tetrahydropyrano[3,4-*c*]pyrazol-4-yl)-1*H*-pyrazole-3-carboxamide |
| 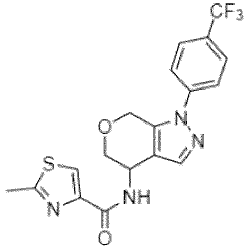 | 2-methyl-*N*-(1-(4-(trifluoromethyl)phenyl)-1,4,5,7-tetrahydropyrano[3,4-c]pyrazol-4-yl)thiazole-4-carboxamide |
| 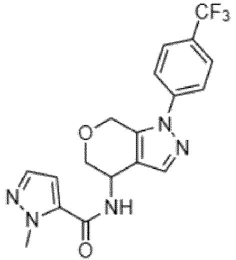 | 1-methyl-*N*-(1-(4-(trifluoromethyl)phenyl)-1,4,5,7-tetrahydropyrano[3,4-c]pyrazol-4-yl)-1*H*-pyrazole-5-carboxamide |

Particular compounds of the first aspect of the invention that may be mentioned include the compounds of the examples provided herein, and pharmaceutically acceptable salts thereof.

Particular compounds of the first aspect of the invention that may be mentioned include:
N-(1-(2-fluorophenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-fluorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-fluorophenyl)-1,4,5,7-tetrahydropyrano[3,4-c]pyrazol-4-yl)-5,6,7,8-tetrahydroimidazo[1,5-a]pyridine-1-carboxamide;
N-(1-(2-fluorophenyl)-1,4,5,7-tetrahydropyrano[3,4-c]pyrazol-4-yl)-4,5,6,7-tetrahydrobenzo[d]isoxazole-3-carboxamide;
(R)-N-(1-(2-fluorophenyl)-1,4,5,7-tetrahydropyrano[3,4-c]pyrazol-4-yl)-4,5,6,7-tetrahydrobenzo[d]isoxazole-3-carboxamide;
(S)-N-(1-(2-fluorophenyl)-1,4,5,7-tetrahydropyrano[3,4-c]pyrazol-4-yl)-4,5,6,7-tetrahydrobenzo[d]isoxazole-3-carboxamide;
N-(1-(2-fluorophenyl)-1,4,5,7-tetrahydropyrano[3,4-c]pyrazol-4-yl)-5,6,7,8-tetrahydroimidazo[1,5-a]pyridine-3-carboxamide;
(R)-N-(1-(2-fluorophenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-fluorophenyl)-1,4,5,7-tetrahydropyrano[3,4-c]pyrazol-4-yl)-5,6,7,8-tetrahydroimidazo[1,5-a]pyridine-3-carboxamide;
N-1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-1-carboxamide;
N-((4S)-1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-1-carboxamide;
N-((4R)-1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-1-carboxamide;
N-1-(2-chloro-6-fluorophenyl)-1,4,5,7-tetrahydropyrano[3,4-c]pyrazol-4-yl)-5,6,7,8-tetrahydroimidazo[1,5-a]pyridine-1-carboxamide;
N-((4S)-1-(2-chloro-6-fluorophenyl)-1,4,5,7-tetrahydropyrano[3,4-c]pyrazol-4-yl)-5,6,7,8-tetrahydroimidazo[1,5-a]pyridine-1-carboxamide;
N-((4R)-1-(2-chloro-6-fluorophenyl)-1,4,5,7-tetrahydropyrano[3,4-c]pyrazol-4-yl)-5,6,7,8-tetrahydroimidazo[1,5-a]pyridine-1-carboxamide;
N-(1-(2,6-dichlorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2,6-dichlorophenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2,6-dichlorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-methylphenyl)-1,4,5,7-tetrahydropyrano[3,4-c]pyrazol-4-yl)-5-ethyl-1-methyl-1H-imidazole-4-carboxamide;
(R)-N-(1-(2-chloro-3-methylphenyl)-1,4,5,7-tetrahydropyrano[3,4-c]pyrazol-4-yl)-5-ethyl-1-methyl-1H-imidazole-4-carboxamide;
(S)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-3-carboxamide;
(R)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-3-methylphenyl)-1,4,5,7-tetrahydropyrano[3,4-c]pyrazol-4-yl)-4,5,6,7-tetrahydro-1H-indazole-3-carboxamide;
(R)-N-(1-(2-chloro-3-methylphenyl)-1,4,5,7-tetrahydropyrano[3,4-c]pyrazol-4-yl)-4,5,6,7-tetrahydro-1H-indazole-3-carboxamide;
(S)-N-(1-(2-chloro-3-methylphenyl)-1,4,5,7-tetrahydropyrano[3,4-c]pyrazol-4-yl)-4,5,6,7-tetrahydrobenzo[d]isoxazole-3-carboxamide;
(R)-N-(1-(2-chloro-3-methylphenyl)-1,4,5,7-tetrahydropyrano[3,4-c]pyrazol-4-yl)-4,5,6,7-tetrahydrobenzo[d]isoxazole-3-carboxamide;
(S)-N-(1-(2-chloro-3-methylphenyl)-1,4,5,7-tetrahydropyrano[3,4-c]pyrazol-4-yl)-4-ethyl-5-methyl-1H-pyrazole-3-carboxamide;
(R)-N-(1-(2-chloro-3-methylphenyl)-1,4,5,7-tetrahydropyrano[3,4-c]pyrazol-4-yl)-4-ethyl-5-methyl-1H-pyrazole-3-carboxamide;
(S)-N-(1-(2-chloro-3-methylphenyl)-1,4,5,7-tetrahydropyrano[3,4-c]pyrazol-4-yl)-4,5-dimethylpicolinamide;
(R)-N-(1-(2-chloro-3-methylphenyl)-1,4,5,7-tetrahydropyrano[3,4-c]pyrazol-4-yl)-4,5-dimethylpicolinamide;
(S)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-methoxyphenyl)-1,4,5,7-tetrahydropyrano[3,4-c]pyrazol-4-yl)-5-ethyl-1-methyl-1H-imidazole-4-carboxamide;
(R)-N-(1-(2-chloro-3-methoxyphenyl)-1,4,5,7-tetrahydropyrano[3,4-c]pyrazol-4-yl)-5-ethyl-1-methyl-1H-imidazole-4-carboxamide;
(S)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-3-carboxamide;
(R)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-3-methoxyphenyl)-1,4,5,7-tetrahydropyrano[3,4-c]pyrazol-4-yl)-4,5,6,7-tetrahydro-1H-indazole-3-carboxamide;
(R)-N-(1-(2-chloro-3-methoxyphenyl)-1,4,5,7-tetrahydropyrano[3,4-c]pyrazol-4-yl)-4,5,6,7-tetrahydro-1H-indazole-3-carboxamide;
(S)-N-(1-(2-chloro-3-methoxyphenyl)-1,4,5,7-tetrahydropyrano[3,4-c]pyrazol-4-yl)-4,5,6,7-tetrahydrobenzo[d]isoxazole-3-carboxamide;
(R)-N-(1-(2-chloro-3-methoxyphenyl)-1,4,5,7-tetrahydropyrano[3,4-c]pyrazol-4-yl)-4,5,6,7-tetrahydrobenzo[d]isoxazole-3-carboxamide;
(S)-N-(1-(2-chloro-3-methoxyphenyl)-1,4,5,7-tetrahydropyrano[3,4-c]pyrazol-4-yl)-4-ethyl-5-methyl-1H-pyrazole-3-carboxamide;
(R)-N-(1-(2-chloro-3-methoxyphenyl)-1,4,5,7-tetrahydropyrano[3,4-c]pyrazol-4-yl)-4-ethyl-5-methyl-1H-pyrazole-3-carboxamide;
(S)-N-(1-(2-chloro-6-fluoro-3-methoxyphenyl)-1,4,5,7-tetrahydropyrano[3,4-c]pyrazol-4-yl)-4,5-dimethylpicolinamide;
(R)-N-(1-(2-chloro-6-fluoro-3-methoxyphenyl)-1,4,5,7-tetrahydropyrano[3,4-c]pyrazol-4-yl)-4,5-dimethylpicolinamide;
(S)-N-(1-(2-chloro-6-fluoro-3-methoxyphenyl)-1,4,5,7-tetrahydropyrano[3,4-c]pyrazol-4-yl)-5-ethyl-1-methyl-1H-imidazole-4-carboxamide;
(R)-N-(1-(2-chloro-6-fluoro-3-methoxyphenyl)-1,4,5,7-tetrahydropyrano[3,4-c]pyrazol-4-yl)-5-ethyl-1-methyl-1H-imidazole-4-carboxamide;
(S)-N-(1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-3-carboxamide;
(R)-N-(1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-6-fluoro-3-methoxyphenyl)-1,4,5,7-tetrahydropyrano[3,4-c]pyrazol-4-yl)-4,5,6,7-tetrahydro-1H-indazole-3-carboxamide;
(R)-N-(1-(2-chloro-6-fluoro-3-methoxyphenyl)-1,4,5,7-tetrahydropyrano[3,4-c]pyrazol-4-yl)-4,5,6,7-tetrahydro-1H-indazole-3-carboxamide;
(S)-N-(1-(2-chloro-6-fluoro-3-methoxyphenyl)-1,4,5,7-tetrahydropyrano[3,4-c]pyrazol-4-yl)-4,5,6,7-tetrahydrobenzo[d]isoxazole-3-carboxamide;
(R)-N-(1-(2-chloro-6-fluoro-3-methoxyphenyl)-1,4,5,7-tetrahydropyrano[3,4-c]pyrazol-4-yl)-4,5,6,7-tetrahydrobenzo[d]isoxazole-3-carboxamide;
(S)-N-(1-(2-chloro-6-fluoro-3-methoxyphenyl)-1,4,5,7-tetrahydropyrano[3,4-c]pyrazol-4-yl)-4-ethyl-5-methyl-1H-pyrazole-3-carboxamide;
(R)-N-(1-(2-chloro-6-fluoro-3-methoxyphenyl)-1,4,5,7-tetrahydropyrano[3,4-c]pyrazol-4-yl)-4-ethyl-5-methyl-1H-pyrazole-3-carboxamide;
(S)-N-(1-(2-chloro-6-fluoro-3-methoxyphenyl)-1,4,5,7-tetrahydropyrano[3,4-c]pyrazol-4-yl)-4,5-dimethylpicolinamide;
(R)-N-(1-(2-chloro-6-fluoro-3-methoxyphenyl)-1,4,5,7-tetrahydropyrano[3,4-c]pyrazol-4-yl)-4,5-dimethylpicolinamide;
(S)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-6-methylphenyl)-1,4,5,7-tetrahydropyrano[3,4-c]pyrazol-4-yl)-5-ethyl-1-methyl-1H-imidazole-4-carboxamide;
(R)-N-(1-(2-chloro-6-methylphenyl)-1,4,5,7-tetrahydropyrano[3,4-c]pyrazol-4-yl)-5-ethyl-1-methyl-1H-imidazole-4-carboxamide;
(S)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-3-carboxamide;
(R)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-6-methylphenyl)-1,4,5,7-tetrahydropyrano[3,4-c]pyrazol-4-yl)-4,5,6,7-tetrahydro-1H-indazole-3-carboxamide;
(R)-N-(1-(2-chloro-6-methylphenyl)-1,4,5,7-tetrahydropyrano[3,4-c]pyrazol-4-yl)-4,5,6,7-tetrahydro-1H-indazole-3-carboxamide;
(S)-N-(1-(2-chloro-6-methylphenyl)-1,4,5,7-tetrahydropyrano[3,4-c]pyrazol-4-yl)-4,5,6,7-tetrahydrobenzo[d]isoxazole-3-carboxamide;
(R)-N-(1-(2-chloro-6-methylphenyl)-1,4,5,7-tetrahydropyrano[3,4-c]pyrazol-4-yl)-4,5,6,7-tetrahydrobenzo[d]isoxazole-3-carboxamide;
(S)-N-(1-(2-chloro-6-methylphenyl)-1,4,5,7-tetrahydropyrano[3,4-c]pyrazol-4-yl)-4-ethyl-5-methyl-1H-pyrazole-3-carboxamide;
(R)-N-(1-(2-chloro-6-methylphenyl)-1,4,5,7-tetrahydropyrano[3,4-c]pyrazol-4-yl)-4-ethyl-5-methyl-1H-pyrazole-3-carboxamide;
(S)-N-(1-(2-chloro-6-methylphenyl)-1,4,5,7-tetrahydropyrano[3,4-c]pyrazol-4-yl)-4,5-dimethylpicolinamide;
(R)-N-(1-(2-chloro-6-methylphenyl)-1,4,5,7-tetrahydropyrano[3,4-c]pyrazol-4-yl)-4,5-dimethylpicolinamide;
(S)-5-ethyl-N-(1-(2-fluorophenyl)-1,4,5,7-tetrahydropyrano[3,4-c]pyrazol-4-yl)-1-methyl-1H-imidazole-4-carboxamide;
(R)-5-ethyl-N-(1-(2-fluorophenyl)-1,4,5,7-tetrahydropyrano[3,4-c]pyrazol-4-yl)-1-methyl-1H-imidazole-4-carboxamide;
(S)-N-(1-(2-fluorophenyl)-1,4,5,7-tetrahydropyrano[3,4-c]pyrazol-4-yl)-4,5,6,7-tetrahydro-1H-indazole-3-carboxamide;
(R)-N-(1-(2-fluorophenyl)-1,4,5,7-tetrahydropyrano[3,4-c]pyrazol-4-yl)-4,5,6,7-tetrahydro-1H-indazole-3-carboxamide;
(S)-N-(1-(2-fluorophenyl)-1,4,5,7-tetrahydropyrano[3,4-c]pyrazol-4-yl)-4,5,6,7-tetrahydrobenzo[d]isoxazole-3-carboxamide;
(R)-N-(1-(2-fluorophenyl)-1,4,5,7-tetrahydropyrano[3,4-c]pyrazol-4-yl)-4,5,6,7-tetrahydrobenzo[d]isoxazole-3-carboxamide;
(S)-4-ethyl-N-(1-(2-fluorophenyl)-1,4,5,7-tetrahydropyrano[3,4-c]pyrazol-4-yl)-5-methyl-1H-pyrazole-3-carboxamide;
(R)-4-ethyl-N-(1-(2-fluorophenyl)-1,4,5,7-tetrahydropyrano[3,4-c]pyrazol-4-yl)-5-methyl-1H-pyrazole-3-carboxamide;
(S)-N-(1-(2-fluorophenyl)-1,4,5,7-tetrahydropyrano[3,4-c]pyrazol-4-yl)-4,5-dimethylpicolinamide;
(R)-N-(1-(2-fluorophenyl)-1,4,5,7-tetrahydropyrano[3,4-c]pyrazol-4-yl)-4,5-dimethylpicolinamide;
(S)-N-(1-(2-chloro-6-fluorophenyl)-1,4,5,7-tetrahydropyrano[3,4-c]pyrazol-4-yl)-4,5,6,7-tetrahydro-1H-indazole-3-carboxamide;
(R)-N-(1-(2-chloro-6-fluorophenyl)-1,4,5,7-tetrahydropyrano[3,4-c]pyrazol-4-yl)-4,5,6,7-tetrahydro-1H-indazole-3-carboxamide;
(S)-N-(1-(2-chloro-6-fluorophenyl)-1,4,5,7-tetrahydropyrano[3,4-c]pyrazol-4-yl)-5,6,7,8-tetrahydroimidazo[1,5-a]pyridine-3-carboxamide;
(R)-N-(1-(2-chloro-6-fluorophenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-6-fluorophenyl)-1,4,5,7-tetrahydropyrano[3,4-c]pyrazol-4-yl)-4,5-dimethylpicolinamide;
(R)-N-(1-(2-chloro-6-fluorophenyl)-1,4,5,7-tetrahydropyrano[3,4-c]pyrazol-4-yl)-4,5-dimethylpicolinamide;
(S)-N-(1-(3-(methylsulfonyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(3-(methylsulfonyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(trifluoromethyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(trifluoromethyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chlorophenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chlorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(o-tolyl)-1,4,5,7-tetrahydropyrano[3,4-c]pyrazol-4-yl)-5,6,7,8-tetrahydroimidazo[1,5-a]pyridine-1-carboxamide;
(R)-N-(1-(o-tolyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-5-methylphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-5-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-methoxyphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-methoxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-(trifluoromethyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-(trifluoromethyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(3-methoxyphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(3-methoxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(3-(trifluoromethoxy)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(3-(trifluoromethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-fluoro-6-methylphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-fluoro-6-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2,6-difluorophenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2,6-difluorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-6-methoxyphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-6-methoxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-methoxy-6-methylphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-methoxy-6-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-hydroxyphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-hydroxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-(dimethylamino)ethoxy)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(2-(dimethylamino)ethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-(dimethylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(2-(dimethylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-(methylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(2-(methylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(3-(2-acetamidoethoxy)-2-chlorophenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(3-(2-acetamidoethoxy)-2-chlorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-methoxyethoxy)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(2-methoxyethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-hydroxyethoxy)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(2-hydroxyethoxy)phenyl)-1,4,5,7-tetrahydropyrano[3,4-c]pyrazol-4-yl)-5,6,7,8-tetrahydroimidazo[1,5-a]pyridine-1-carboxamide;
Isopropyl (S)-2-chloro-3-(4-(5,6,7,8-tetrahydroimidazo[1,5-a]pyridine-1-carboxamido)-4,7-dihydropyrano[3,4-c]pyrazol-1(5H)-yl)benzoate;
Isopropyl (R)-2-chloro-3-(4-(5,6,7,8-tetrahydroimidazo[1,5-a]pyridine-1-carboxamido)-4,7-dihydropyrano[3,4-c]pyrazol-1(5H)-yl)benzoate;
(S)-N-(1-(2-chloro-3-(dimethylcarbamoyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(dimethylcarbamoyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(pyrrolidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(pyrrolidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(piperidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(piperidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(morpholine-4-carbonyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(morpholine-4-carbonyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(diethylcarbamoyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(diethylcarbamoyl)phenyl)-1,4,5,7-tetrahydropyrano[3,4-c]pyrazol-4-yl)-5,6,7,8-tetrahydroimidazo[1,5-a]pyridine-1-carboxamide;
and pharmaceutically acceptable salts thereof.

As described herein, compounds of the first aspect of the invention may exist as stereoisomers. In particular, compounds of the first aspect of the invention may exist as stereoisomers at the position marked with an asterisk in formula I as depicted herein above, at which position the skilled person will understand that compounds of the first aspect of the invention may exist in the *R*- or *S*- configuration.

For the avoidance of doubt, the skilled person will understand that, where the carbon marked with an asterisk in formula I as depicted herein above is the only stereo centre in the compound of formula I, the compound of formula I may exist in the form of two enantiomers having differing configurations at that stereo centre, which enantiomers may be referred to as the *R*- and *S*- enantiomers (indicating the configuration at that position, as understood by one skilled in the art).

In particular, compounds of the first aspect of the invention may exists as mixtures (e.g. about equal mixtures) of stereoisomers, i.e. mixtures of each possible stereoisomer. In particular, where compounds of the invention exist as *R*- and *S*- enantiomers (i.e. in the *R*- and *S*- configurations at the carbon marked with an asterisk in the compound of formula I as depicted above), the compound of the first aspect of the invention may be a mixture of those enantiomers, particularly an about equal mixture (e.g. a racemic mixture).

In other embodiments, the compound of the first aspect of the invention may exist in either the *R*- or *S*- configuration at the marked carbon (e.g. as either the *R*- or *S*- enantiomer), in which case the compound may exist in the substantial absence of compounds of the alternative configuration (e.g. in greater than 60%, such as greater than 70%, greater than 80% or greater than 90% (e.g. greater than 99%, such as greater than or equal to 99.9%) purity relative to the alternative configuration.

For example, where the compound of the first aspect of the invention exists as either the *R*- or *S*- enantiomer as described herein above, that compound may have an enantiomeric excess (e.e.) as understood by one skilled in the art, of greater than greater than 60%, such as greater than 70%, greater than 80% or greater than 90% (e.g. greater than 99%, such as greater than or equal to 99.9%).

In particular, the compound of the first aspect of the invention may exist as the *S-*enantiomer as described herein above.

Thus, in a particular embodiment of the first aspect of the invention, the compound of formula I is a compound of formula la 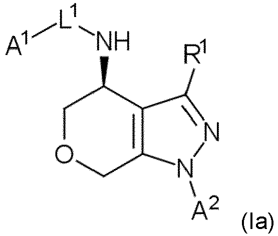 wherein A¹, A², L¹ and R¹ are as defined in the first aspect of the invention (including all embodiments and combinations of embodiments thereof).

In particular, the compound of formula la is provided in the substantial absence of the compound of formula I, or pharmaceutically acceptable salt thereof, in the alternative configuration, e.g. a compound of formula Ib 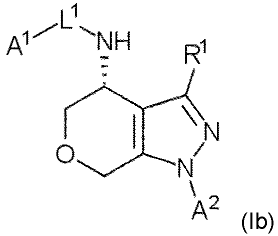 wherein A¹, A², L¹ and R¹ are as defined in the first aspect of the invention (including all embodiments and combinations of embodiments thereof), or a pharmaceutically acceptable salt thereof.

In particular, the compound of formula la, or a pharmaceutically acceptable salt thereof, may be provided in greater than 60%, such as greater than 70%, greater than 80% or greater than 90% (e.g. greater than 99%, such as greater than or equal to 99.9%) purity relative to the compound of Ib or a pharmaceutically acceptable salt thereof (e.g. where present in greater than 90% purity, the compounds of formula la, or a pharmaceutically acceptable salt thereof, will make up greater than 90% of the combined amount of compounds of formula la and Ib, and pharmacetically acceptable salts thereof).

In alternative embodiments, references to the *S*- enantiomer or compound of formula la may be replaced with references to the *R*- enantiomer or compound of formula Ib, and *vice versa.*

For example, the compound of the first aspect of the invention may be provided as a compound of formula Ib, or a pharmaceutically acceptable salt thereof, in greater than 60%, such as greater than 70%, greater than 80% or greater than 90% (e.g. greater than 99%, such as greater than or equal to 99.9%) purity relative to the compound of la or a pharmaceutically acceptable salt thereof.

Further compounds of the first aspect of the invention that may be mentioned include:
(S)-N-(1-(2-fluorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-fluorophenyl)-1,4,5,7-tetrahydropyrano[3,4-c]pyrazol-4-yl)-4,5,6,7-tetrahydrobenzo[d]isoxazole-3-carboxamide;
(S)-N-(1-(2-fluorophenyl)-1,4,5,7-tetrahydropyrano[3,4-c]pyrazol-4-yl)-5,6,7,8-tetrahydroimidazo[1,5-a]pyridine-3-carboxamide;
N-((4S)-1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-1-carboxamide;
N-((4S)-1-(2-chloro-6-fluorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2,6-dichlorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-methylphenyl)-1,4,5,7-tetrahydropyrano[3,4-c]pyrazol-4-yl)-5-ethyl-1-methyl-1H-imidazole-4-carboxamide;
(S)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-3-methylphenyl)-1,4,5,7-tetrahydropyrano[3,4-c]pyrazol-4-yl)-4,5,6,7-tetrahydro-1H-indazole-3-carboxamide;
(S)-N-(1-(2-chloro-3-methylphenyl)-1,4,5,7-tetrahydropyrano[3,4-c]pyrazol-4-yl)-4,5,6,7-tetrahydrobenzo[d]isoxazole-3-carboxamide;
(S)-N-(1-(2-chloro-3-methylphenyl)-1,4,5,7-tetrahydropyrano[3,4-c]pyrazol-4-yl)-4-ethyl-5-methyl-1H-pyrazole-3-carboxamide;
(S)-N-(1-(2-chloro-3-methylphenyl)-1,4,5,7-tetrahydropyrano[3,4-c]pyrazol-4-yl)-4,5-dimethylpicolinamide;
(S)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-methoxyphenyl)-1,4,5,7-tetrahydropyrano[3,4-c]pyrazol-4-yl)-5-ethyl-1-methyl-1H-imidazole-4-carboxamide;
(S)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-3-methoxyphenyl)-1,4,5,7-tetrahydropyrano[3,4-c]pyrazol-4-yl)-4,5,6,7-tetrahydro-1H-indazole-3-carboxamide;
(S)-N-(1-(2-chloro-3-methoxyphenyl)-1,4,5,7-tetrahydropyrano[3,4-c]pyrazol-4-yl)-4,5,6,7-tetrahydrobenzo[d]isoxazole-3-carboxamide;
(S)-N-(1-(2-chloro-3-methoxyphenyl)-1,4,5,7-tetrahydropyrano[3,4-c]pyrazol-4-yl)-4-ethyl-5-methyl-1H-pyrazole-3-carboxamide;
(S)-N-(1-(2-chloro-6-fluoro-3-methoxyphenyl)-1,4,5,7-tetrahydropyrano[3,4-c]pyrazol-4-yl)-4,5-dimethylpicolinamide;
(S)-N-(1-(2-chloro-6-fluoro-3-methoxyphenyl)-1,4,5,7-tetrahydropyrano[3,4-c]pyrazol-4-yl)-5-ethyl-1-methyl-1H-imidazole-4-carboxamide;
(S)-N-(1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-6-fluoro-3-methoxyphenyl)-1,4,5,7-tetrahydropyrano[3,4-c]pyrazol-4-yl)-4,5,6,7-tetrahydro-1H-indazole-3-carboxamide;
(S)-N-(1-(2-chloro-6-fluoro-3-methoxyphenyl)-1,4,5,7-tetrahydropyrano[3,4-c]pyrazol-4-yl)-4,5,6,7-tetrahydrobenzo[d]isoxazole-3-carboxamide;
(S)-N-(1-(2-chloro-6-fluoro-3-methoxyphenyl)-1,4,5,7-tetrahydropyrano[3,4-c]pyrazol-4-yl)-4-ethyl-5-methyl-1H-pyrazole-3-carboxamide;
(S)-N-(1-(2-chloro-6-fluoro-3-methoxyphenyl)-1,4,5,7-tetrahydropyrano[3,4-c]pyrazol-4-yl)-4,5-dimethylpicolinamide;
(S)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-6-methylphenyl)-1,4,5,7-tetrahydropyrano[3,4-c]pyrazol-4-yl)-5-ethyl-1-methyl-1H-imidazole-4-carboxamide;
(S)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-6-methylphenyl)-1,4,5,7-tetrahydropyrano[3,4-c]pyrazol-4-yl)-4,5,6,7-tetrahydro-1H-indazole-3-carboxamide;
(S)-N-(1-(2-chloro-6-methylphenyl)-1,4,5,7-tetrahydropyrano[3,4-c]pyrazol-4-yl)-4,5,6,7-tetrahydrobenzo[d]isoxazole-3-carboxamide;
(S)-N-(1-(2-chloro-6-methylphenyl)-1,4,5,7-tetrahydropyrano[3,4-c]pyrazol-4-yl)-4-ethyl-5-methyl-1H-pyrazole-3-carboxamide;
(S)-N-(1-(2-chloro-6-methylphenyl)-1,4,5,7-tetrahydropyrano[3,4-c]pyrazol-4-yl)-4,5-dimethylpicolinamide;
(S)-5-ethyl-N-(1-(2-fluorophenyl)-1,4,5,7-tetrahydropyrano[3,4-c]pyrazol-4-yl)-1-methyl-1H-imidazole-4-carboxamide;
(S)-N-(1-(2-fluorophenyl)-1,4,5,7-tetrahydropyrano[3,4-c]pyrazol-4-yl)-4,5,6,7-tetrahydro-1H-indazole-3-carboxamide;
(S)-N-(1-(2-fluorophenyl)-1,4,5,7-tetrahydropyrano[3,4-c]pyrazol-4-yl)-4,5,6,7-tetrahydrobenzo[d]isoxazole-3-carboxamide;
(S)-4-ethyl-N-(1-(2-fluorophenyl)-1,4,5,7-tetrahydropyrano[3,4-c]pyrazol-4-yl)-5-methyl-1H-pyrazole-3-carboxamide;
(S)-N-(1-(2-fluorophenyl)-1,4,5,7-tetrahydropyrano[3,4-c]pyrazol-4-yl)-4,5-dimethylpicolinamide;
(S)-N-(1-(2-chloro-6-fluorophenyl)-1,4,5,7-tetrahydropyrano[3,4-c]pyrazol-4-yl)-4,5,6,7-tetrahydro-1H-indazole-3-carboxamide;
(S)-N-(1-(2-chloro-6-fluorophenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-6-fluorophenyl)-1,4,5,7-tetrahydropyrano[3,4-c]pyrazol-4-yl)-4,5-dimethylpicolinamide;
(S)-N-(1-(3-(methylsulfonyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(trifluoromethyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chlorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(o-tolyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-5-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-methoxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-(trifluoromethyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(3-methoxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(3-(trifluoromethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-fluoro-6-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2,6-difluorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-6-methoxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-methoxy-6-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-hydroxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-(dimethylamino)ethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-(dimethylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-(methylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(3-(2-acetamidoethoxy)-2-chlorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-methoxyethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-hydroxyethoxy)phenyl)-1,4,5,7-tetrahydropyrano[3,4-c]pyrazol-4-yl)-5,6,7,8-tetrahydroimidazo[1,5-a]pyridine-1-carboxamide;
Isopropyl (S)-2-chloro-3-(4-(5,6,7,8-tetrahydroimidazo[1,5-a]pyridine-1-carboxamido)-4,7-dihydropyrano[3,4-c]pyrazol-1(5H)-yl)benzoate;
(S)-N-(1-(2-chloro-3-(dimethylcarbamoyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(pyrrolidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(piperidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(morpholine-4-carbonyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(diethylcarbamoyl)phenyl)-1,4,5,7-tetrahydropyrano[3,4-c]pyrazol-4-yl)-5,6,7,8-tetrahydroimidazo[1,5-a]pyridine-1-carboxamide;
and pharmaceutically acceptable salts thereof.

### Methods of treatment and medical uses

The references to methods of treatment in this description are to be interpreted as references to the compounds, pharmaceutical compositions and medicaments of the present invention for use in a method for treatment of the human (or animal) body by therapy.

As described herein, compounds of the first aspect of the invention are therapeutically active.

Accordingly, in a second aspect of the invention, there is provided a compound, or a pharmaceutically acceptable salt thereof, as defined in the first aspect of the invention (including all embodiments and combinations of embodiments thereof) for use in medicine (which may also be referred to as use as a pharmaceutical).

In an alternative second aspect of the invention, there is provided a compound, or a pharmaceutically acceptable salt thereof, as defined in the first aspect of the invention (including all embodiments and combinations of embodiments thereof) for use in a method of treating disease comprising administering to a patient in need thereof a therapeutically effective amount of the compound, or pharmaceutically acceptable salt thereof.

Also described herein is a compound, or a pharmaceutically acceptable salt thereof, as defined in the first aspect of the invention (including all embodiments and combinations of embodiments thereof) for use in the manufacture of a medicament for the treatment of disease.

The compounds of the invention are useful for treating cancers and/or viral infections. Accordingly, in a third aspect of the invention, there is provided a compound, or a pharmaceutically acceptable salt thereof, as defined in the first aspect of the invention (including all embodiments and combinations of embodiments thereof) for use in the treatment of cancer and/or the treatment or prevention of a viral infection.

Also described herein is a method of treating cancer and/or treating or preventing a viral infection comprising administering to a patient in need thereof a therapeutically effective amount of a compound, or a pharmaceutically acceptable salt thereof, as defined in the first aspect of the invention (including all embodiments and combinations of embodiments thereof).

Also described herein is a compound, or a pharmaceutically acceptable salt thereof, as defined in the first aspect of the invention (including all embodiments and combinations of embodiments thereof) for use in the manufacture of a medicament for the treatment of cancer and/or the treatment or prevention of a viral infection.

The skilled person will understand that references to the treatment of a particular condition (or, similarly, to treating that condition) take their normal meaning in the field of medicine. In particular, the terms may refer to achieving a reduction in the severity of one or more clinical symptom associated with the condition. For example, in the case of a cancer, the term may refer to achieving a reduction of the amount of cancerous cells present (e.g. in the case of a cancer forming a solid tumour, indicated by a reduction in tumour volume). Similarly, in the case of a viral infection, the term may refer to achieving a reduction in the viral load (i.e. the number of viral units present in a given amount of plasma in the relevant patient, e.g. the number of viral units per mL of plasma).

The skilled person will understand that references to the prevention of a particular condition (and, similarly, to preventing that condition) take their normal meaning in the field of medicine, and include references to the prophylaxis of the condition (and vice-versa). In particular, the term may refer to achieving a reduction in the likelihood of the patient (or a healthy subject) developing the condition (for example, at least a 10% reduction, such as at least a 20%, 30% or 40% reduction, e.g. at least a 50% reduction).

As used herein, references to patients will refer to a living subject being treated, including mammalian (e.g. human) patients. Thus, in particular embodiments of all aspects of the invention, the treatment is in a mammal (e.g. a human).

As used herein, the term therapeutically effective amount will refer to an amount of a compound that confers a therapeutic effect on the treated patient. The effect may be objective (i.e. measurable by some test or marker) or subjective (i.e. the subject gives an indication of and/or feels an effect).

Although compounds of the first aspect of the invention may possess pharmacological activity as such, certain pharmaceutically-acceptable (e.g. "protected") derivatives of compounds of the invention may exist or be prepared which may not possess such activity, but may be administered parenterally or orally and thereafter be metabolised in the body to form compounds of the invention. Such compounds (which may possess some pharmacological activity, provided that such activity is appreciably lower than that of the active compounds to which they are metabolised) may therefore be described as "prodrugs" of compounds of the invention.

As used herein, references to prodrugs will include compounds that form a compound of the invention, in an experimentally-detectable amount, within a predetermined time, following enteral or parenteral administration (e.g. oral or parenteral administration).

Various forms of prodrugs are known in the art. For examples of such prodrug derivatives, see:
a) Design of Prodrugs, edited by H. Bundgaard, (Elsevier, 1985) and Methods in Enzymology, Vol. 42, p. 309-396, edited by K. Widder, et al. (Academic Press, 1985);
b) A Textbook of Drug Design and Development, edited by Krogsgaard-Larsen and H. Bundgaard, Chapter 5 "Design and Application of Prodrugs", by H. Bundgaard p. 113-191 (1991);
c) H. Bundgaard, Advanced Drug Delivery Reviews, 8, 1-38 (1992);
d) H. Bundgaard, et al., Journal of Pharmaceutical Sciences, 77, 285 (1988); and N. Kakeya, et al., Chem Pharm Bull, 32, 692 (1984).

By way of non-limiting example, hydroxy or carboxylic acid groups may be protected as esters and amines may be protected as carbamates.

For the avoidance of doubt, the compounds of the first aspect of the invention are useful because they possess pharmacological activity, and/or are metabolised in the body following oral or parenteral administration to form compounds that possess pharmacological activity. In particular, as described herein, compounds of the first aspect of the invention are useful in the treatment of cancer, which term will be readily understood by one of skill in the art.

In a particular embodiment of all aspects of the invention, references to the treatment of cancer and/or the treatment or prevention of a viral infection will refer in particular to the treatment of cancer.

In an alternative embodiment of all aspects of the invention, references to the treatment of cancer and/or the treatment or prevention of a viral infection will refer in particular to the treatment or prevention (more particularly, the treatment) of a viral infection.

In particular embodiments of all aspects of the invention, the cancer is a cancer selected from the list consisting of:
soft tissue cancers, such as sarcoma, myxoma, rhabdomyoma, fibroma, lipoma and teratoma;
lung cancers, such as bronchogenic carcinoma, alveolar or bronchiolar carcinoma, bronchial adenoma, sarcoma, lymphoma, chondromatous hamartoma, mesothelioma;
gastrointestinal cancers, such as esophageal cancers, stomach cancers, pancreatic cancers, small bowel cancers, large bowel cancers (e.g. colon cancer);
genitourinary tract cancers, such as cancer of the kidney, bladder and urethra, prostate, testis;
liver cancers, such as hepatoma, cholangiocarcinoma, hepatoblastoma, angiosarcoma, hepatocellular adenoma, hemangioma;
bone cancers, such as osteogenic sarcoma, fibrosarcoma, malignant fibrous histiocytoma, chondrosarcoma, Ewing's sarcoma, malignant lymphoma, multiple myeloma, malignant giant cell tumor chordoma, osteochronfroma, benign chondroma, chondroblastoma, chondromyxofibroma, osteoid osteoma and giant cell tumors;
cancers of the head and/or nervous system, such as cancers of the skull, meninges, brain, spinal cord;
gynecological cancers, such as cancers of the uterus, cervix, ovaries, cancers of the vulva, vagina, fallopian tubes;
haematologic cancers, such as cancers of the blood and bone marrow (e.g. leukemia, such as acute myeloid leukemia, chronic myeloid leukemia, acute lymphocytic leukemia, chronic lymphocytic leukemia), Hodgkin's disease, non-Hodgkin's lymphoma, diffuse large B cell lymphoma, mantle cell lymphoma, marginal zone lymphoma, follicular lymphoma, Waldenstrom's macroglobulinemia Burkitt's lymphoma, multiple myeloma, Richter's syndrome and T cell lymphoma;
skin cancers, such as malignant melanoma, basal cell carcinoma, squamous cell carcinoma, Kaposi's sarcoma, moles dysplastic nevi, lipoma, angioma, dermatofibroma, keloids; neurofibromatosis and adrenal glands; and
neuroblastomas.

As used herein, references to cancerous cells and the like will include references to a cell afflicted by any one of the above-identified conditions.

More particular cancers that may be mentioned include those corresponding to the cell lines used in the examples provided herein. For example, particular cancers that may be mentioned include:
colon cancer;
skin cancer (e.g. as defined herein, such as malignant melanoma);
lymphomas;
leukemia (such as chronic myeloid leukemia); and
sarcomas (such as those described herein).

In more particular embodiments, the cancer is selected from the group consisting of a skin cancer (such as malignant melanoma) and a haematologic cancer (such as chronic myeloid leukemia). For example, the cancer may be a skin cancer as known to those skilled in the art, such as a skin cancer as described herein.

More particularly, the cancer may be chronic myeloid leukemia (CML) or malignant melanoma. Most particularly, the cancer may be malignant melanoma.

In particular embodiments of all aspects of the invention, references to a viral infection will refer to an infection caused by one or more viruses. The virus may cause a human disease such as AIDS, Ebola hemorrhoragic fever, severe acute respiratory syndrome (SARS), Middle East respiratory syndrome (MERS), Western/Eastern equine encephalitis (WEE/EEE), dengue fever, zika fever, influenza, hepatitis, West Nile fever, polio, and measles.

The virus may be an RNA virus, such as a single-stranded RNA (ssRNA) or double-stranded RNA (dsRNA) virus, where the ssRNA virus may be a positive sense or negative sense ssRNA virus. For the purposes of the present invention, an RNA virus is one that is within Group III, IV, V or VI of the Baltimore classification system unless otherwise indicated. Such viruses include but are not limited to the following:
Double-stranded (ds)RNA viruses (Group III) including rotaviruses and picobirnaviruses. Positive-sense ssRNA viruses (Group IV) including coronaviruses, bymoviruses, comoviruses, nepoviruses, nodaviruses, picornaviruses, potyviruses, sobemoviruses, luteoviruses, carmoviruses, dianthoviruses, flaviviruses, pestiviruses, tombusviruses, hepatitis C virus, alphaviruses, carlaviruses, furoviruses, hordeiviruses, potexviruses, rubiviruses, tobraviruses, tricornaviruses, tymoviruses and hepatitis E virus. Negative-sense ssRNA viruses (Group V), including Ebola virus, Marburg virus, Measles virus, Mumps virus, Nipah virus, Hendra virus, RSV, NOV, Rabies virus, Nyavirus, Lassa virus, Hantavirus, Crimean-Congo hemorrhagic fever, influenza viruses, Hepatitis D virus. Retroviruses (Group VI) including lentiviruses, e.g. HIV-1 and HIV-2.

The skilled person will understand that treatment with compounds of the first aspect of the invention may further comprise (i.e. be combined with) further (i.e. additional/other) treatment(s) for the same condition. Those further treatments may be known treatments and may, for example, represent the standard of care treatment for that particular condition.

For example, when used in the treatment of cancers, treatment with compounds of the invention may be combined with other means for the treatment of cancer, such as treatment with one or more other therapeutic agent that is useful in the treatment of cancer (including chemotherapeutic agents and treatment with cell-based therapies, such as immunotherapies) and/or one or more physical method used in the treatment of cancer (such as treatment through surgery and/or radiotherapy), as known to those skilled in the art.

Thus, treatment with compounds of the first aspect of the invention may further comprise (i.e. be combined with) further (i.e. additional/other) treatment(s) for cancer (such treatment with an additional agent for the treatment of a cancer as described herein, e.g. malignant melanoma).

In particular, treatment with compounds of the invention may be performed in combination with (e.g. in a patient who is also being treated with) one or more (e.g. one) additional compounds (i.e. therapeutic agents) that:
(i) are capable of reducing levels of nucleosides and/or nucleotides within a cell (e.g. a cancerous cell); and/or
(ii) are activators of p53 (such as inhibitors of MDM2),
both of which are described herein below.

Similarly, when used in the treatment or prevention of viral infections, treatment or prevention using compounds of the invention may be performed in combination with one or more other therapeutic agent that is useful in the treatment or prevention of a viral infection (such as an anti-viral compound or a vaccine), as known to those skilled in the art.

### Pharmaceutical compositions

As described herein, compounds of the invention are useful as pharmaceuticals. Such compounds may be administered alone or may be administered by way of known pharmaceutical compositions/formulations.

In a fourth aspect of the invention, there is provided a pharmaceutical composition comprising a compound of the (first aspect of the) invention, and optionally one or more pharmaceutically acceptable adjuvant, diluent and/or carrier.

The skilled person will understand that references herein to compounds of the first aspect of the invention being for particular uses (and, similarly, to uses and methods of use relating to compounds of the invention) also apply, *mutatis mutandis,* to pharmaceutical compositions comprising compounds of the invention as described herein.

In a fifth aspect of the invention, there is provided a pharmaceutical composition comprising a compound as defined in the first aspect of the invention, and optionally one or more pharmaceutically acceptable adjuvant, diluent and/or carrier, for use in the treatment of cancer and/or the treatment or prevention of a viral infection (as defined in the first aspect of the invention).

The skilled person will understand that compounds of the invention may act systemically and/or locally (i.e. at a particular site).

The skilled person will understand that compounds and compositions as described in all aspects of the invention will normally be administered orally, intravenously, subcutaneously, buccally, rectally, dermally, nasally, tracheally, bronchially, sublingually, intranasally, topically, by any other parenteral route or *via* inhalation, in a pharmaceutically acceptable dosage form. Pharmaceutical compositions as described herein will include compositions in the form of tablets, capsules or elixirs for oral administration, suppositories for rectal administration, sterile solutions or suspensions for parenteral or intramuscular administration, and the like. Alternatively, particularly where such compounds of the invention act locally, pharmaceutical compositions may be formulated for topical administration.

Thus, in particular embodiments of all relevant aspects of the invention, the pharmaceutical formulation is provided in a pharmaceutically acceptable dosage form, including tablets or capsules, liquid forms to be taken orally or by injection, suppositories, creams, gels, foams, inhalants (e.g. to be applied intranasally), or forms suitable for topical administration. For the avoidance of doubt, in such embodiments, compounds of the invention may be present as a solid (e.g. a solid dispersion), liquid (e.g. in solution) or in other forms, such as in the form of micelles.

For example, in the preparation of pharmaceutical formulations for oral administration, the compound may be mixed with solid, powdered ingredients such as lactose, saccharose, sorbitol, mannitol, starch, amylopectin, cellulose derivatives, gelatin, or another suitable ingredient, as well as with disintegrating agents and lubricating agents such as magnesium stearate, calcium stearate, sodium stearyl fumarate and polyethylene glycol waxes. The mixture may then be processed into granules or compressed into tablets.

Soft gelatine capsules may be prepared with capsules containing one or more active compounds (e.g. compounds of the invention, and optionally additional therapeutic agents), together with, for example, vegetable oil, fat, or other suitable vehicle for soft gelatine capsules. Similarly, hard gelatine capsules may contain such compound(s) in combination with solid powdered ingredients such as lactose, saccharose, sorbitol, mannitol, potato starch, corn starch, amylopectin, cellulose derivatives or gelatine.

Dosage units for rectal administration may be prepared (i) in the form of suppositories which contain the compound(s) mixed with a neutral fat base; (ii) in the form of a gelatine rectal capsule which contains the active substance in a mixture with a vegetable oil, paraffin oil, or other suitable vehicle for gelatine rectal capsules; (iii) in the form of a ready-made micro enema; or (iv) in the form of a dry micro enema formulation to be reconstituted in a suitable solvent just prior to administration.

Liquid preparations for oral administration may be prepared in the form of syrups or suspensions, e.g. solutions or suspensions, containing the compound(s) and the remainder of the formulation consisting of sugar or sugar alcohols, and a mixture of ethanol, water, glycerol, propylene glycol and polyethylene glycol. If desired, such liquid preparations may contain colouring agents, flavouring agents, saccharine and carboxymethyl cellulose or other thickening agent. Liquid preparations for oral administration may also be prepared in the form of a dry powder to be reconstituted with a suitable solvent prior to use.

Solutions for parenteral administration may be prepared as a solution of the compound(s) in a pharmaceutically acceptable solvent. These solutions may also contain stabilizing ingredients and/or buffering ingredients and are dispensed into unit doses in the form of ampoules or vials. Solutions for parenteral administration may also be prepared as a dry preparation to be reconstituted with a suitable solvent extemporaneously before use.

In particular embodiments of the fourth and fifth aspects of the invention, the pharmaceutical composition does not comprise liposomes.

The skilled person will understand that compounds of the invention may be administered (for example, as compositions as described in the fourth and fifth aspects of the invention) at varying doses, with suitable doses being readily determined by one of skill in the art. Oral, pulmonary and topical dosages (and subcutaneous dosages, although these dosages may be relatively lower) may range from between about 0.01 mg/kg of body weight per day (mg/kg/day) to about 200 mg/kg/day, preferably about 0.01 to about 10 mg/kg/day, and more preferably about 0.1 to about 5.0 mg/kg/day. For example, when administered orally, treatment with such compounds and compositions may comprise administration of a compositions typically containing between about 0.01 mg to about 2000 mg, for example between about 0.1 mg to about 500 mg, or between 1 mg to about 100 mg, of the active ingredient. When admixture intravenously, the most preferred doses will range from about 0.001 to about 10 mg/kg/hour during constant rate infusion. Advantageously, treatment may comprise administration of such compounds and compositions in a single daily dose, or the total daily dosage may be administered in divided doses of two, three or four times daily (with reference to the doses described herein).

In any event, the skilled person (e.g. the physician) will be able to determine the actual dosage which will be most suitable for an individual patient, which is likely to vary with the route of administration, the type and severity of the condition that is to be treated, as well as the species, age, weight, sex, renal function, hepatic function and response of the particular patient to be treated. The above-mentioned dosages are exemplary of the average case; there can, of course, be individual instances where higher or lower dosage ranges are merited, and such are within the scope of this invention.

As described herein above, the skilled person will understand that treatment with compounds of the invention may further comprise (i.e. be combined with) further (i.e. additional/other) treatment(s) for the same condition. In particular, treatment with compounds of the invention may be combined with other means for the treatment of cancer, such as treatment with one or more other therapeutic agent that is useful in the treatment of cancer, or other means for the treatment or prevention of a viral infection.

In particular embodiments of the fourth and fifth aspects of the invention, the pharmaceutical composition may further comprise one or more additional (i.e. other) therapeutic agent.

In more particular embodiments, the one or more additional therapeutic agent is an agent for the treatment of cancer (such as an additional agent for the treatment of a cancer as described herein, e.g. malignant melanoma).

In other embodiments, the one or more additional therapeutic agent is an agent for the treatment or prevention of a viral infection (e.g. a viral infection as described herein).

The skilled person will understand that combinations of therapeutic agents may also be described as a combination product and/or provided as a kit-of-parts.

In a sixth aspect of the invention, there is provided a combination product comprising:
(A) a compound as defined in the first aspect of the invention; and
(B) one or more additional therapeutic agent for the treatment of cancer or for the treatment or prevention of a viral infection,
wherein each of components (A) and (B) is formulated in admixture, optionally with one or more a pharmaceutically-acceptable adjuvant, diluent or carrier.

In a seventh aspect of the invention, there is provided a kit-of-parts comprising:
(a) a compound as defined in the first aspect of the invention, or a pharmaceutical composition as defined in the fourth or fifth aspect of the invention; and
(b) one or more additional therapeutic agent for the treatment of cancer or for the treatment or prevention of a viral infection, optionally in admixture with one or more pharmaceutically-acceptable adjuvant, diluent or carrier,
which components (a) and (b) are each provided in a form that is suitable for administration in conjunction with the other.

In particular embodiments of the sixth and seventh aspects of the invention, the additional therapeutic agent is a therapeutic agent for the treatment of cancer (such as an additional agent for the treatment of a cancer as described herein, e.g. malignant melanoma).

In particular embodiments of the sixth and seventh aspects of the invention, the additional therapeutic agent is an agent for the treatment of malignant melanoma.

In particular embodiments of the sixth and seventh aspects of the invention, the additional therapeutic agent is an agent that is:
(i) capable of reducing levels of nucleosides and/or nucleotides within a cell (e.g. a cancerous cell, such as a cell forming part of a cancer as described herein); and/or
(ii) an activator of p53 (such as an inhibitor of MDM2).

For the avoidance of doubt, compounds (i.e. therapeutic agents) that are
(i) capable of reducing levels of nucleosides and/or nucleotides within a cell (e.g. a cancerous cell, such as a cell forming part of a cancer as described herein); and/or
(ii) an activators of p53 (such as an inhibitor of MDM2),
will be readily identified by those skilled in the art and include, in particular, such therapeutic agents that are commercially available (e.g. agents that the subject of a marketing authorization in one or more territory, such as a European or US marketing authorization).

The skilled person will understand that references to therapeutic agents capable of reducing levels of nucleosides and/or nucleotides within a cell (e.g. a cancerous cell, such as a cell forming part of a cancer as described herein) may refer to compounds capable of reducing levels of such nucleosides and/or nucleotides (such as the levels of uridine and/or uridine phosphates, e.g. mono-, di- and tri- phosphates) within such a cell by at least 10% (such as at least 20%, at least 30% or at least 40%, for example at least 50%, at least 60%, at least 70% or at least 80%, e.g. at least 90%) when compared to the levels of such nucleosides and/or nucleotides in such a cell prior to treatment with the relevant compound.

The skilled person will further understand that references to therapeutic agents capable of reducing levels of nucleosides and/or nucleotides within a cell (e.g. a cancerous cell, such as a cell forming part of a cancer as described herein) may include references to compounds that act by inhibiting uptake of such nucleosides and/or nucleotides by such cells. For example, such therapeutic agents may include inhibitors of uridine uptake, as known to those skilled in the art (such as the therapeutic agent nilotinib or dipyridamole).

Particular therapeutic agents capable of reducing levels of nucleosides and/or nucleotides within a cell include nilotinib, dipyridamole, imatinib, gefitinib, ibrutinib, varlitinib, volitinib and vemurafenib (e.g. nilotinib and imatinib).

For example, therapeutic agents capable of reducing levels of nucleosides and/or nucleotides within a cell include nilotinib, dipyridamole, imatinib, ibrutinib and vemurafenib.

More particular therapeutic agents capable of reducing levels of nucleosides and/or nucleotides within a cell include nilotinib, ibrutinib and dipyridamole.

Yet more particular therapeutic agents capable of reducing levels of nucleosides and/or nucleotides within a cell include imatinib.

Yet more particular therapeutic agents capable of reducing levels of nucleosides and/or nucleotides within a cell include vemurafenib.

For example, therapeutic agents capable of reducing levels of nucleosides and/or nucleotides within a cell include ASLAN001 (varlitinib), ibrutinib, rapamycin, vemurafenib, gefitinib, antimycin A, BI-2536, VX-680, roscovitine, olomoucine, imatinib (STI-571), AG1879, AG1517, AG1478, AG825, AG18, AG490, vinblastine, etoposide, genistein, U0126, Raf-1 inhibitor 1, H89, KN93, staurosporine, ZM336372 and indirubin-3'-monoxime.

In particular, therapeutic agents capable of reducing levels of nucleosides and/or nucleotides within a cell include ASLAN001 (varlitinib).

In particular, therapeutic agents capable of reducing levels of nucleosides and/or nucleotides within a cell include ibrutinib.

Other compounds that could reduce the levels of nucleosides and/or nucleotides in cells include BIBW2992, AG013736, PF-02341066 / PF-2341066, BMS-354825, 5-aza-2'-deoxycytidine, LY317615, CP-358774 / OSI-774, RAD001 / SDZ-RAD / Certican, GW572016 / GW2016, AMN-107, GW786034 / Armala, AP24534, BAY73-4506, INCB018424, Bay 43-9006 / Nexaxar, Sutent / SU-11248, Torisel / CCI-779, tasocitinib / CP-690550, GSK1120212, ZD6474, PLX4032 / RG7204 / RO5185426, SAHA / Zolinza / MK-0683, AT-877 / HA-1077 and quizartinib.

The skilled person will understand that references to activators of p53 may refer to therapeutic agents that are capable of: increasing p53 synthesis and/or stability (e.g. by increasing thermo stability or through inhibition of degradation); and/or increasing transcription factor function, within a cell (e.g. a cancerous cell, such as a cell forming part of a cancer as described herein).

In particular, it may refer to increasing p53 synthesis and/or stability such that the levels of p53 within a cell (e.g. a cancerous cell, such as a cell forming part of a cancer as described herein) are increased by at least by at least 10% (such as at least 20%, at least 30% or at least 40%, for example at least 50%, at least 60%, at least 70% or at least 80%, e.g. at least 90% or at least 100%), when compared to the levels of p53 in such a cell prior to treatment with the relevant compound. Further, it may refer to increasing the transcription factor function of p53 in a cell (e.g. a cancerous cell, such as a cell forming part of a cancer as described herein) by at least 10% (such as at least 20%, at least 30% or at least 40%, for example at least 50%, at least 60%, at least 70% or at least 80%, e.g. at least 90% or at least 100%), when compared to the levels of p53 in such a cell prior to treatment with the relevant compound.

The skilled person will further understand that references to activators of p53 may include references to therapeutic agents that act by inhibiting MDM2 (such as the therapeutic agent nutlin-3).

The skilled person will understand that references to therapeutic agents that act by inhibiting MDM2 may refer to compounds capable of inhibiting the activity of MDM2 within a cell (e.g. a cancerous cell, such as a cell forming part of a cancer as described herein) by at least 10% (such as at least 20%, at least 30% or at least 40%, for example at least 50%, at least 60%, at least 70% or at least 80%, e.g. at least 90%) when compared to the levels of such activity in such a cell prior to treatment with the relevant compound.

Particular therapeutic agents capable of activating p53 (e.g. by inhibiting MDM2) include nutlin-3, RO5045337, idasanutlin, AMG-232, DS3032b, serdemetan, ALRN-6924, SAR-405838, CGM-097, RO-5503781, RO-6839921 and HDM-201.

More particular therapeutic agents capable of activating p53 (e.g. by inhibiting MDM2) include nutlin-3.

Further therapeutic agents capable of activating p53 (e.g. by increasing p53 levels) include PS-341 / MS-341, Ara-C, adriamycin, 5-FU, Camptosar and mithramycin A.

In alternative embodiments of the sixth and seventh aspects of the invention, the additional therapeutic agent is a therapeutic agent for the treatment or prevention of a viral infection, such as a viral infection as described herein.

### Preparation of compounds/compositions

Pharmaceutical compositions/formulations, combination products and kits as described herein may be prepared in accordance with standard and/or accepted pharmaceutical practice.

Thus, disclosed herein but not part of the claimed invention is a process for the preparation of a pharmaceutical composition/formulation, as hereinbefore defined, which process comprises bringing into association a compound of the invention, as hereinbefore defined, with one or more pharmaceutically-acceptable adjuvant, diluent or carrier.

Also disclosed herein is a process for the preparation of a combination product or kit-of-parts as hereinbefore defined, which process comprises bringing into association a compound of the invention, as hereinbefore defined, or a pharmaceutically acceptable salt thereof with the other therapeutic agent that is useful in the treatment of cancer and/or the treatment or prevention of a viral infection, and at least one pharmaceutically-acceptable adjuvant, diluent or carrier.

As used herein, references to bringing into association will mean that the two components are rendered suitable for administration in conjunction with each other.

Thus, in relation to the process for the preparation of a kit of parts as hereinbefore defined, by bringing the two components "into association with" each other, we include that the two components of the kit of parts may be:
(i) provided as separate formulations (i.e. independently of one another), which are subsequently brought together for use in conjunction with each other in combination therapy; or
(ii) packaged and presented together as separate components of a "combination pack" for use in conjunction with each other in combination therapy.

Compounds of the invention as defined herein may be prepared in accordance with techniques that are well known to those skilled in the art, such as those described in the examples provided hereinafter.

In an eighth aspect of the invention, there is provided a process for the preparation of a compound of formula I, or a pharmaceutically acceptable salt thereof, as defined in the first aspect of the invention, which process comprises:
(i) reaction of a compound of formula II 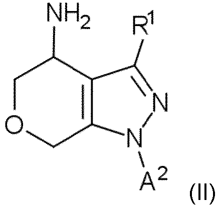 wherein R¹, and A² are as defined in the first aspect of the invention, with a compound of formula III

   LG₁-L¹-A¹ (III)

   wherein A¹ and L¹ are as defined in the first aspect of the invention and LG₁ represents a suitable leaving group, in the presence of a suitable solvent and optionally in the presence of a suitable base and/or a suitable catalyst, under conditions known to those skilled in the art (for example, where LG₁ represents -OH, the reaction may be performed in the presence of reagents suitable for performing peptide coupling reactions as known to those skilled in the art, such as in the presence of HOBt and EDC.HCl, or alternatively HATU or HBTU, and optionally in the presence of a suitable base, e.g. Et₃N);
(ii) where L¹ represents -C(O)N(R²)- wherein R² represents H, reaction of a compound of formula II with a compound of formula IV

   A¹-N=C=O (IV)

   wherein A' is as defined in the first aspect of the invention in the presence of a suitable solvent and optionally in the presence of a suitable base and/or a suitable catalyst, under conditions known to those skilled in the art; or
(iii) reaction of a compound of formula (V) 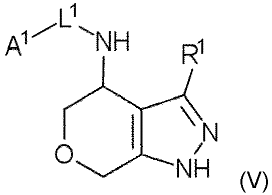 wherein R¹, A¹ and L¹ are as defined in the first aspect of the invention, with a compound of formula VI

   LG₂-A² (VI)

   wherein A² is as defined in the first aspect of the invention and LG₂ represents a suitable leaving group, in the presence of a suitable solvent and optionally in the presence of a suitable base and/or a suitable catalyst, under conditions known to those skilled in the art (for example, under Buchwald-Hartwig conditions as known to those skilled in the art, e.g. where LG₂ represents a suitable leaving group such as halo and in the presence of a suitable catalyst such as a suitable Pd catalyst (e.g. Pd₂(dba)₃) and a suitable base, or under Chan-Lam conditions as known to those skilled in the art, e.g. where LG₂ represents a suitable leaving group such as -B(OH)₂ and in the presence of a suitable catalyst such as a suitable Cu catalyst (e.g. Cu(OAc)₂) and a suitable base).

Compounds of formulae II, III, IV, V and VI are either commercially available, are known in the literature, or may be obtained either by analogy with the processes described herein, or by conventional synthetic procedures, in accordance with standard techniques, from available starting materials using appropriate reagents and reaction conditions. In this respect, the skilled person may refer to *inter alia "*Comprehensive Organic Synthesis" by B. M. Trost and I. Fleming, Pergamon Press, 1991. Further references that may be employed include *"*Heterocyclic Chemistry" by J. A. Joule, K. Mills and G. F. Smith, 3rd edition, published by Chapman & Hall, *"*Comprehensive Heterocyclic Chemistry II" by A. R. Katritzky, C. W. Rees and E. F. V. Scriven, Pergamon Press, 1996 and *"*Science of Synthesis", Volumes 9-17 (Hetarenes and Related Ring Systems), Georg Thieme Verlag, 2006.

For example, compounds of formula II may be prepared by reaction of a compound of formula VII 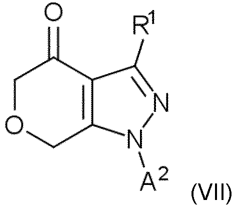 wherein R¹ and A² are as defined herein for compounds of formula I (and formula la), with a suitable chiral amide (such as (*S_{S}*)-2-methyl-2-propanesulfinamide) under conditions known to those skilled in the art (for example, in the presence of a suitable catalyst (such as Ti(OEt)₄) and a suitable reducing agent (such as L-selectride), and in the presence of a suitable solvent), followed by cleavage of the amide, under conditions known to those skilled in the art (i.e. to provide the free amine of the compound of formula II).

Further, compounds of formula VII may be prepared by reaction of a compound of formula VIII 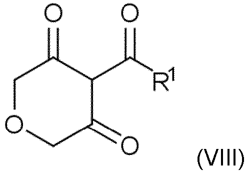 wherein R¹ is as defined herein for compounds of formula VII, with a compound of formula IX 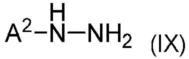 wherein A² is as defined herein for compounds of formula I (and formula la), under conditions known to those skilled in the art.

Alternatively, compounds of formula VII wherein R¹ represents H may be prepared by reaction of a compound of formula X 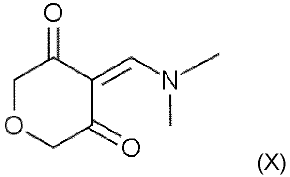 with a compound of formula IX as defined herein, under conditions known to those skilled in the art.

The skilled person will understand that compounds of formula III may be commercially available, known in the literature, or may be prepared by analogy with the synthesis of a compound of formula I but wherein A² represents H or a suitable protecting group as known to those skilled in the art, wherein the latter case the preparation will require removal of the protecting group, under conditions known to those skilled in the art, to yield the compound of formula III.

Compounds of formulae VII, VIII, IX and X are either commercially available, are known in the literature, or may be obtained either by analogy with the processes described herein, or by conventional synthetic procedures, in accordance with standard techniques, from available starting materials using appropriate reagents and reaction conditions.

The skilled person will understand that compounds of formula la and lb, and pharmaceutically acceptable salts thereof, as defined herein may be prepared by analogy with the synthesis of compounds of formula I, and pharmaceutically acceptable salts thereof, as described in the eighth aspect of the invention. In particular, such processes may be performed in accordance with the eighth aspect of the invention wherein the compounds of formula II and V are replaced by:

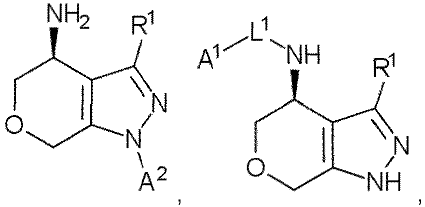

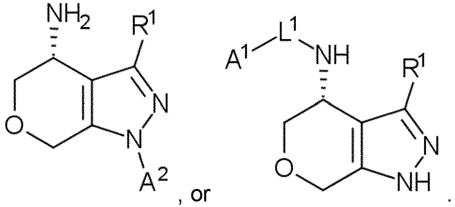

For example, compounds of formula II, but wherein the stereochemistry shown is reversed (i.e. the opposite enantiomer is provided) may be prepared by reaction of a compound of formula VII, as defined herein, with a suitable chiral amide (such as (*S_{S}*)-2-methyl-2-propanesulfinamide) under conditions known to those skilled in the art (for example, in the presence of a suitable catalyst (such as Ti(OEt)₄) and a suitable reducing agent (such as NaBH₄), and in the presence of a suitable solvent), followed by cleavage of the amide, under conditions known to those skilled in the art (i.e. to provide the free amine of the compound corresponding to the compound of formula II).

The substituents on A', A², L¹ and R¹ groups, as hereinbefore defined, may be modified one or more times, after or during the processes described above for preparation of compounds of formula la (and, similarly, compounds of formula lb) by way of methods that are well known to those skilled in the art. Examples of such methods include substitutions, reductions, oxidations, dehydrogenations, alkylations, dealkylations, acylations, hydrolyses, esterifications, etherifications, halogenations and nitrations. The precursor groups can be changed to a different such group, or to the groups defined in formula I, at any time during the reaction sequence. The skilled person may also refer to *"*Comprehensive Organic Functional Group Transformations" by A. R. Katritzky, O. Meth-Cohn and C. W. Rees, Pergamon Press, 1995 and/or *"*Comprehensive Organic Transformations" by R. C. Larock, Wiley-VCH, 1999.

Such compounds may be isolated from their reaction mixtures and, if necessary, purified using conventional techniques as known to those skilled in the art. Thus, processes for preparation of compounds of the invention as described herein may include, as a final step, isolation and optionally purification of the compound of the invention (e.g. isolation and optionally purification of the compound of formula I).

It will be appreciated by those skilled in the art that, in the processes described above and hereinafter, the functional groups of intermediate compounds may need to be protected by protecting groups. The protection and deprotection of functional groups may take place before or after a reaction in the above-mentioned schemes.

Protecting groups may be applied and removed in accordance with techniques that are well known to those skilled in the art and as described hereinafter. For example, protected compounds/intermediates described herein may be converted chemically to unprotected compounds using standard deprotection techniques. The type of chemistry involved will dictate the need, and type, of protecting groups as well as the sequence for accomplishing the synthesis. The use of protecting groups is fully described in *"*Protective Groups in Organic Synthesis", 3rd edition, T.W. Greene & P.G.M. Wutz, Wiley-Interscience (1999).

Compounds as described herein (in particular, compounds as defined in the first aspect of the invention) may have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties over, compounds known in the prior art, whether for use in the above-stated indications or otherwise. In particular, such compounds may have the advantage that they are more efficacious and/or exhibit advantageous properties *in vivo.*

Without wishing to be bound by theory, compounds as described herein are thought to be potent inhibitors of DHODH, which leads to activity as inhibitors of *de novo* pyrimidine nucleotide synthesis. Cancer cells are known to have greater susceptibility to nucleotide level modulation, due to their defective cell cycle check points. This allows for potent and selective inhibition of cancer cell growth and promotion of cancer cell death. Further, combining compounds as described herein with therapeutic agents that are able to decrease the level of nucleotides and nucleosides, such as pyrimidine nucleotides and nucleosides, in the cell (for example, by inhibiting uptake or preventing retention within the cell; such as the therapeutic agent nilotinib) are thought to provide an effective and synergistic combination therapy.

Surprisingly, despite their activity in reducing levels of nucleotides and nucleosides in such cells, it has also been found that compounds described herein are able to increase the synthesis of p53, which is a known tumour suppressor. Further, it has been found that combining compounds described herein with other activators of p53 (such as inhibitors of MDM2, e.g. nutlin-3) also provides an effective and synergistic combination therapy.

Again without wishing to be bound by theory, it is thought that the observed synergy between the DHODH inhibitors described herein and inhibitors of p53 degradation, such as nutlin-3, results from the DHODH inhibitors ability to increase p53 synthesis. This increase in p53 synthesis together with the inhibition of p53 degradation by nutlin-3 (an MDM2 inhibitor) leads to a stronger increase in p53 levels, which serves to induce p53 pro-apoptotic functions.

Further, compounds described herein are thought to be potent inhibitors of viral replication, which renders them of particular use in the treatment or prevention of viral infections.

### Description of the Figures

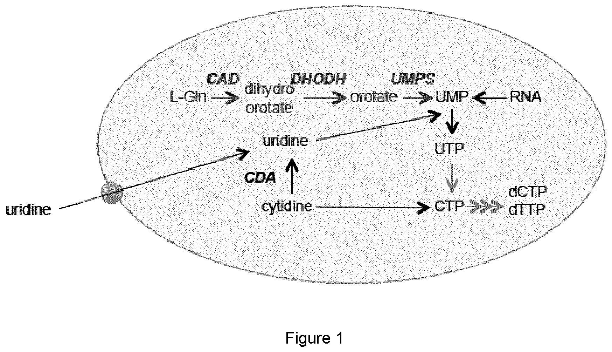

Figure 1 shows a simplified description of the pyrimidine nucleotide *de novo* synthesis (blue) and salvage (black) pathways.
Figure 2 shows results obtained from Biological Example 2, in which the compounds of synthetic Examples 6, 9, 11 and 13 reduce MOLM13 cell growth and/or viability, and that this effect is largely prevented by the addition of excess uridine.

### Examples

Chemicals and reagents were obtained from commercial suppliers and were used as received unless otherwise stated. All reactions involving moisture sensitive reagents were performed in oven or flame dried glassware under a positive pressure of nitrogen. Thin-layer chromatography was performed using aluminium plates coated with silica gel (with fluorescent indicator UV254). Developed plates were air-dried and analysed under a UV lamp or by KMnO₄ dip staining. Flash column chromatography was performed using silica gel (60-120 µm).Low resolution (LR) electrospray mass spectral (LC-MS) analyses were acquired by electrospray ionisation (ESI) on Shimadzu LCMS(SQD)- 2010EV or Agilent LCMS(SQD)-1200 series LC/ G6125B-MS. Nuclear Magnetic Resonance (NMR) spectra were recorded on a Varian/Bruker spectrometer at 400 MHz for proton (1H NMR), 100 MHz for carbon (13C NMR) and fluorine (19F NMR); chemical shifts are reported in ppm (δ) relative to residual protons in deuterated solvent peaks. Coupling constants (J) are quoted in Hz and are recorded to the nearest 0.1 Hz. The following abbreviations are used; s, singlet; d, doublet; dd, doublet of doublets; t, triplet; m, multiplet; q, quartet; and br, broad. Assignments of signals are proposed based on the values observed. HPLC analyses were obtained on a Shimadzu HPLC 2010CHT HPLC consisting of a LC 20 AD prominence pump, DGU-20 A3 prominence degasser, SPD-M20A prominence DAD detector, SIL-HTC autosampler. Separation was achieved using a Waters X-Select CSH C18 (4.6x150) mm; 5u column.

Analytical RPLC-MS was performed using a HPLC-MS Shimadzu HPLC 2010CHT coupled with a Shimadzu LCMS(SQD)- 2010EV, under the following conditions: Column: Waters X-Select C18 CSH (3.0*50) mm 2.5µ; Mobile phases: ACN/water gradients (0.05% HCOOH); Flowrate, 1.2 mL/min; Detection, UV (214, 254, 280 nm) and MS (ESI, pos,& neg polarity) or Agilent LCMS(SQD)-1200 series LC/ G6125B-MS mass spectrometer with electrospray ionization (ESI+). HPLC-MS methods were the following: Method 1 - Waters X-Select C18 CSH (3.0*50) mm 2.5µ, 4 min gradient mobile phase ACN /[5 mM NH₄HCO₃/H₂O]; Method 2 - Column: Waters X-Select C18 CSH (3.0*50) mm 2.5µ; Mobile phases, ACN/water gradients (0.05% HCOOH) using positive and negative electrospray ionization.

Preparative RP-LC was performed using a Shimadzu preparative HPLC system composed of the following: CBM-20A system controller, LC-8A binary gradient pump, SPD-M20A photodiode array detector, FRC-10A fraction collector equipped with a Xselect CSH ^{®} Prep C18 5mm OBD^{™} 30x250 mm, 5 µm column, using ACN/H₂O (0.05% HCOOH) gradients at a flow rate of 30 mL/min with UV (220 or 254 nm) detection. Xselect CSH ^{®} Prep C18 5mm OBD^{™} 30x250 mm, 5µm column, on a GILSON 333/334 Prep-Scale system with a flow rate of 30mL/min, detection at 254 nm, using ACN/H₂O (0.05% HCOOH) gradients system.

Preparative and analytical chiral HPLC was performed using a variety of columns and conditions.

### UPLC Analysis Methods

| **S.No.** | **Method** | **Column Name and Mobile phases** |
|---|---|---|
| 1. | **A** | **Column Name:** SunFire C18 (4.6 X 250 mm) 5µ |
| | | **Mobile phase:** A: 0.1% Trifluoroacetic Acid in water C: 100% ACN |
| 2. | **B** | **Column Name:** XBridge Shield RP18 (4.6 X 250 mm)5 µ |
| | | **Mobile phase:** D: 5mM Ammonium Acetate in water C: 100% ACN |
| 3. | **C** | **Column Name:** XBridge Shield RP18 (4.6 X 250 mm)5 µm |
| | | **Mobile phase:** A: 0.1% Trifluoroacetic Acid in water C: 100% ACN |
| 4. | **D** | **Column Name:** XBridge C8 (4.6 X 250 mm) 5µ |
| | | **Mobile phase:** A: 0.1% Trifluoroacetic Acid in water C: 100% ACN |
| 5. | **E** | **Column Name:** SUNFIRE C18 (4.6 X 250 mm)5 µm |
| | | **Mobile phase:** B: 5mM Ammonium Acetate in water C: 100% ACN |
| 6. | **F** | **Column Name:** XBridge C18 (4.6 X 250 mm) 5µ |
| | | **Mobile phase:** B: 5mM Ammonium Acetate in water C: 100% ACN |
| 7. | **G** | **Column Name:** Xselect Phenyl-Hexyl (4.6 X 250 mm) 5µ |
| | | **Mobile phase:** A: 0.1% Trifluoroacetic Acid in water C: 100% ACN |
| 8. | **H** | **Column Name:** XTIMATE C18 |
| | | **Mobile Phase:** A: 0.1% TFA , Mobile Phase -C: ACN |
| 9. | **I** | **Column Name:** XBridge C8 (4.6 X 250 mm) 5µ |
| | | **Mobile phase:** B: 5mM Ammonium Acetate in water C: 100% ACN |
| 10. | **J** | **Column Name:** XBridge Shield RP18 (4.6 X 250 mm) 5µ |
| | | **Mobile phase:** A: 0.1% Trifluoroacetic Acid in Water , C: 100% ACN |
| 11. | **K** | **Column Name:** Atlantis T3 (4.6 X 250 mm) 5µ |
| | | **Mobile phase:** A: 0.1% Trifluoroacetic Acid in water C: 100% ACN |
| 12. | **L** | **Column Name:** XSelect Phenyl-hexyl (4.6 X 250 mm) 5µ |
| | | **Mobile phase:** B: 5mM Ammonium Acetate in water C: 100% ACN |

### Example compounds

The invention is illustrated by way of the following example compounds.

In the event that there is a discrepancy between nomenclature and the structure of compounds as depicted graphically, it is the latter that presides (unless contradicted by any experimental details that may be given and/or unless it is clear from the context).

The following compounds were prepared in the manner described below.

### Example 1: N-(1-(2-fluorophenyl)-1,4,5,7-tetrahydropyrano[3,4-c]pyrazol-4-yl)-5,6,7,8-tetrahydroimidazo[1,5-a]pyridine-1-carboxamide

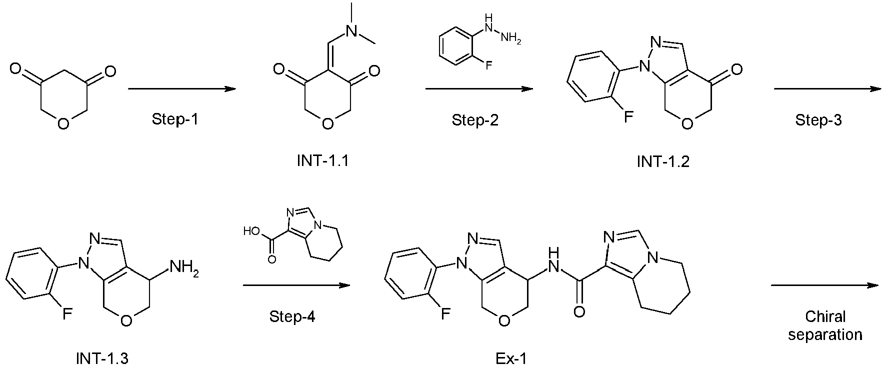

Step-1: Synthesis of 4-((dimethylamino)methylene)-2H-pyran-3,5(4H,6H)-dione (INT-1.1): A solution of tetrahydropyran-3,5-dione (5.00 g, 43.85 mmol) in toluene (50 mL) was added DMF-DMA (10.45 g, 87.71 mmol) was heated at 90 °C for 6 h. After completion of the reaction (monitored by TLC), the solvent was evaporated *in vacuo* and the subsequent residue was triturated with n-pentane to afford INT-1.2 (5.1 g, crude) as an off white solid; 1H NMR (400 MHz, DMSO-d6): δ 8.08 (s, 1H), 4.02 (s, 4H), 3.45 (s, 3H), 3.16 (s, 3H).

Step-2: Synthesis of 1-(2-fluorophenyl)-1,7-dihydropyrano[3,4-c]pyrazol-4(5H)-one (INT-1.2): A solution of INT-1.1 (4.10 g, 24.23 mmol), 1-(2-fluorophenyl)hydrazine (3.94 g, 24.23 mmol) in AcOH (3.6 mL, 63.0 mmol) and ethanol (40 mL) was refluxed for 16 h. After completion (monitored by TLC), the reaction mixture was diluted with water and extracted with DCM (3 X 100 mL). The combined organic layers were dried over sodium sulfate, filtered and concentrated *in vacuo.* The resulting crude solid was purified with column chromatography on silica gel (100-200 mesh), eluting with 5-10 % methanol in DCM to afford INT-1.2 (2.85 g, 50.7 %) as a white solid; LCMS (ESI) m/z = 233.1 [M+H]+.

Step-3: Synthesis of 1-(2-fluorophenyl)-1,4,5,7-tetrahydropyrano[3,4-c]pyrazol-4-amine (INT-1.3): To a solution of INT-1.2 (2.00 g, 8.61 mmol) in 2-propanol (50 mL) was added ammonium acetate (6.63 g, 86.13 mmol) and molecular sieves (4 Å). After vigorous stirring NaBH₃CN (2.70 g, 43.06 mmol) was cautiously added. The reaction was heated to 70 °C for 48 h. After completion (monitored by TLC), the reaction mixture was filtered and concentrated *in vacuo.* The residue was purified with column chromatography on silica gel (100-200 mesh), eluting with 5-10 % methanol in DCM to afford INT-1.3 (1.40 g, 70 %) as a white solid; LCMS (ESI) m/z = 234.2 [M+H]+.

Step-4: Synthesis of N-(1-(2-fluorophenyl)-1,4,5,7-tetrahydropyrano[3,4-c]pyrazol-4-yl)-5,6,7,8-tetrahydroimidazo[1,5-a]pyridine-1-carboxamide (Ex-1): A solution of INT-1.3 (0.200 g, 0.861 mmol) in DCM (5 mL) was cooled to 0 °C. 5,6,7,8-tetrahydroimidazo[1,5-a]pyridine-1-carboxylic acid (0.157 g, 0.947 mmol) and triethylamine (0.3 mL, 2.584 mmol) were added. The resulting mixture was stirred for 5 min after which time T₃P (50 % ethyl acetate, 0.4 mL, 1.29 mmol) was added. The reaction mixture was stirred at room temperature for 16 h. After completion (monitored by TLC), the reaction mixture was diluted with water and extracted with ethyl acetate (3 x 30 mL). The combined organic layers were dried over sodium sulfate, filtered and concentrated *in vacuo* to afford crude title compound. The crude solid material was purified with column chromatography on silica gel (100-200 mesh) eluting with 5-10% MeOH in DCM to afford pure Ex-1; LCMS (ESI) m/z = 381.9 [M+H]+.

Ex-1 was subjected for separation and purification by chiral HPLC to afford Ex-2 (22 mg, 8.5 %) and Ex-3 (22 mg, 7.8 %) as off-white solids, respectively (DAICEL CHIRALPAK-IG (250x30 mm, 5µ) / Mobile phase: A. n-hexane+0.1% DEA; B. DCM:MEOH (50:50) isocratic 50% B / Flow rate: 30 mL min⁻¹) *t*_{R} (peak 1) 9.1, *t*_{R} (peak 2) 10.4.

### Example 2: (R)-N-(1-(2-fluorophenyl)-1,4,5,7-tetrahydropyrano[3,4-c]pyrazol-4-yl)-5,6,7,8-tetrahydroimidazo[1,5-a]pyridine-1-carboxamide (Peak 1)

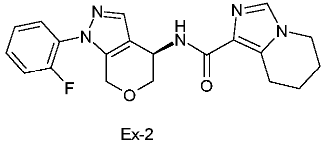

LCMS (ESI) m/z = 381.9 [M+H]+; HPLC: 98.7 % (*t*_{R} = 5.920 min); 1H NMR (400 MHz, DMSO-d6): δ 7.71 (s, 1H), 7.62-7.43 (m, 5H), 7.42-7.32 (m, 1H), 5.10-4.99 (m, 1H), 4.77-4.51 (m, 2H), 3.99 (br t, J = 5.9 Hz, 2H), 3.85 (d, J = 3.9 Hz, 2H), 2.99 (br t, J = 6.6 Hz, 2H), 1.90 - 1.83 (m, 2H), 1.80 - 1.72 (m, 2H).

### Example 3: (S)-N-(1-(2-fluorophenyl)-1,4,5,7-tetrahydropyrano[3,4-c]pyrazol-4-yl)-5,6,7,8-tetrahydroimidazo[1,5-a]pyridine-1-carboxamide (Peak 2)

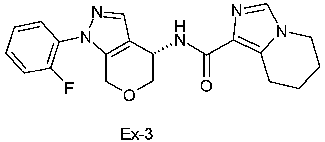

LCMS (ESI) m/z = 381.9 [M+H]+; HPLC: 99.8 % (*t*_{R} = 5.926 min); 1H NMR (400 MHz, DMSO-d6): δ 7.71 (s, 1H), 7.61 - 7.44 (m, 5H), 7.41-7.34 (m, 1H), 5.17-4.98 (m, 1H), 4.75-4.53 (m, 2H), 4.01-3.98 (m, 2H), 3.86-3.82 (m, 2H), 3.01-2.98 (m, 2H), 1.89-1.81 (m, 2H), 1.81-1.72 (m, 2H).

### Example 4: N-(1-(2-fluorophenyl)-1,4,5,7-tetrahydropyrano[3,4-c]pyrazol-4-yl)-5,6,7,8-tetrahydroimidazo[1,5-a]pyridine-3-carboxamide

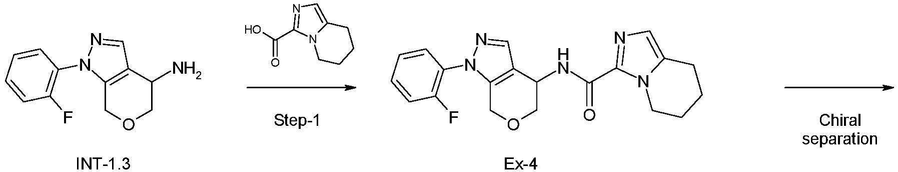

Step-1: Synthesis of N-(1-(2-fluorophenyl)-1,4,5,7-tetrahydropyrano[3,4-c]pyrazol-4-yl)-5,6,7,8-tetrahydroimidazo[1,5-a]pyridine-3-carboxamide (Ex-4): To a solution of INT-1.3 (0.100 g, 0.430 mmol) at 0 °C in DCM (5 mL) was added 5,6,7,8-Tetrahydroimidazo[1,5-a]pyridine-3-carboxylic acid (0.768 g, 0.473 mmol) and triethylamine (0.3 mL, 1.29 mmol). This mixture was stirred for 5 min after which time T₃P (50 % ethyl acetate) was added. The reaction was stirred at room temperature for 16 h. After completion (monitored by TLC), the reaction mixture was diluted with water and extracted with ethyl acetate (3 x 30 mL). The combined organic layers were dried over sodium sulfate, filtered and concentrated *in vacuo* to afford a solid residue. The crude title compound was was purified with column chromatography on silica gel (100-200 mesh), eluting with with 5-10% MeOH in DCM to afford pure Ex-4; LCMS (ESI) m/z = 381.8 [M+H]+.

Ex-4 was subjected for separation and purification by chiral HPLC to afford Ex-5 (17 mg, 7.8 %) and Ex-6 (18 mg, 8.5 %) as off-white solids, respectively (DAICEL CHIRALPAK-IG (250x30 mm, 5µ) / Mobile phase: A. n-hexane+0.1% DEA; B. DCM:MEOH (50:50) isocratic 50% B / Flow rate: 30 mL min-1) *t*_{R} (peak 1) 8.9, *t*_{R} (peak 2) 11.5.

### Example 5: (R)-N-(1-(2-fluorophenyl)-1,4,5,7-tetrahydropyrano[3,4-c]pyrazol-4-yl)-5,6,7,8-tetrahydroimidazo[1,5-a]pyridine-3-carboxamide (Peak 1)

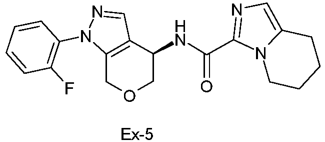

LCMS (ESI) m/z = 381.8 [M+H]+; HPLC: 98.9% (*t*_{R} = 7.011 min); 1H NMR (400 MHz, CDCl₃): δ 7.75 (s, 1H), 7.61 (d, J = 8.8 Hz, 1H), 7.51 (t, J = 7.1 Hz, 1H), 7.47-7.36 (m, 1H), 7.30-7.28 (m, 1H), 7.25-7.21 (m, 1H), 6.75 (s, 1H), 5.22-5.20 (m, 1H), 4.80 - 4.63 (m, 2H), 4.55 - 4.35 (m, 2H), 4.09-4.05 (m, 1H), 3.92-3.88 (m, 1H), 2.81 (br t, J = 6.4 Hz, 2H), 2.04-1.94 (m, 2H), 1.85-1.79 (m, 2H).

### Example 6: (S)-N-(1-(2-fluorophenyl)-1,4,5,7-tetrahydropyrano[3,4-c]pyrazol-4-yl)-5,6,7,8-tetrahydroimidazo[1,5-a]pyridine-3-carboxamide (Peak 2)

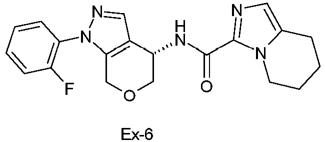

LCMS (ESI) m/z = 380.8 [M+H]+; HPLC: 97.7% (*t*_{R} = 7.023 min); 1H NMR (400 MHz, CDCl₃): δ 7.76-7.72 (m, 1H), 7.61 (d, J = 8.3 Hz, 1H), 7.55-7.48 (m, 1H), 7.45-7.37 (m, 1H), 7.33-7.27 (m, 1H), 7.23-7.20 (m, 1H), 6.75 (s, 1H), 5.29-5.08 (m, 1H), 4.69-4.65 (m, 2H), 4.52-4.49 (m, 2H), 4.09-4.05 (m, 1H), 3.92-3.88 (m, 1H), 2.82 (br t, J = 6.1 Hz, 2H), 2.05 - 1.95 (m, 2H), 1.91 - 1.75 (m, 2H).

### Example 7: N-1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-1-carboxamide

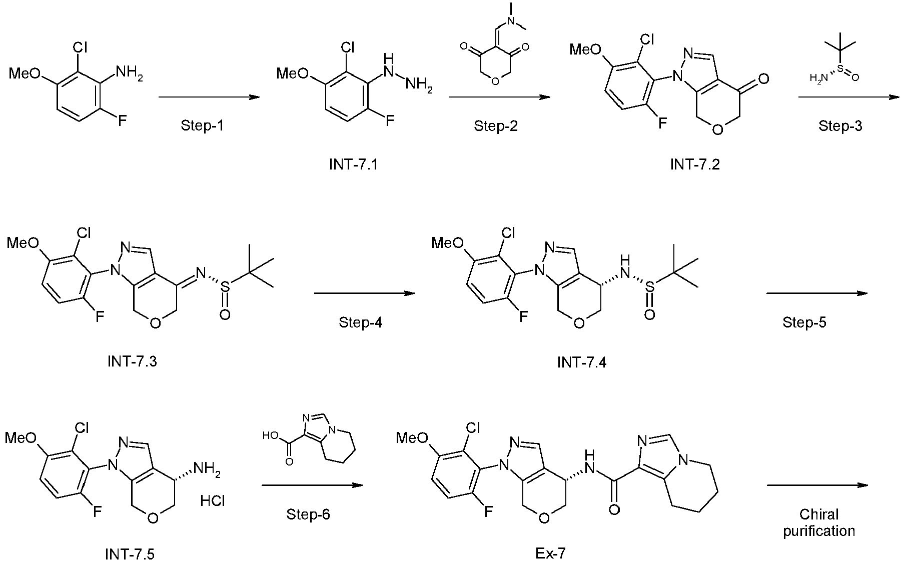

Step-1: Synthesis of (2-chloro-6-fluoro-3-methoxyphenyl)hydrazine hydrochloride (INT-7.1): To a stirred solution of 2-chloro-6-fluoro-3-methoxyaniline (4,00 g, 22.8 mmol) in conc. HCl (20 mL) was added NaNO₂ (1.91 g, 27.33 mmol) at 0 °C. Reaction was stirred at 0 °C for 1 h. After 1 h, SnCl₂ (12.84 g, 56.95 mmol) in conc. HCl (20 mL) was added dropwise at 0 °C. The resulting reaction mixture was stirred at room temperature for 16 h. After completion (monitored by TLC), the formed precipitate was filtered off and dried under vacuum to afford compound INT-7-1 (6.03 g, crude) as a white solid. LCMS (ESI) m/z = 190.9 [M+H]+

Step-2: Synthesis of 1-(2-chloro-6-fluoro-3-methoxyphenyl)-1,7-dihydropyrano[3,4-c]pyrazol-4(5H)-one (INT 7.2): A solution of INT-1.1 (5.00 g, 29.55 mmol) and INT-7.1 (5.60 g, 29.55 mmol) in 4M HCl in dioxane (10 mL, 29.55 mmol) was heated at 100 °C for 16 h. After completion (monitored by TLC) reaction mixture was diluted with water and extracted with DCM (3 X 100 mL). The combined organic layers were dried over sodium sulfate, filtered and concentrated *in vacuo.* The resulting crude material was purified with silica gel column chromatography eluting with 5-10% MeOH in DCM to afford INT-7.2 (2.5 g, 57%) as a white solid. LCMS (ESI) m/z = 296.9 [M+H]+

Step-3: Synthesis of (S,Z)-N-(1-(2-chloro-6-fluoro-3-methoxyphenyl)-1,7-dihydropyrano[3,4-c]pyrazol-4(5H)-ylidene)-2-methylpropane-2-sulfinamide (INT-7.3): To a solution of ketone INT-7.2 (3.5 g, 11.8 mmol) and (S)-2-methylpropane-2-sulfinamide (0.77 g, 34.54 mmol) in THF (10 mL) at room temperature was added Ti(OiPr)₄ (3.61 g, 70.8 mmol). The reaction mixture was heated to 100 °C for 4 h. After completion (monitored by TLC), the reaction mixture was diluted with water and extracted with DCM (3 X 100 mL). The combined organic layers were dried over sodium sulfate, filtered and concentrated *in vacuo.* The crude material was purified with silica gel column chromatography eluting with 5-10% MeOH in DCM to afford compound 6 (2.3 g, 18%) as a white solid. LCMS (ESI) m/z = 400.1 [M+H]+

Step-4: Synthesis of (S)-N-((S)-1-(2,6-dichlorophenyl)-1,4,5,7-tetrahydropyrano[3,4-c]pyrazol-4-yl)-2-methylpropane-2-sulfinamide (INT-7.4): To a solution of INT-7.3 (2.3 g, 0.775 mmol) at 0 °C in anhydrous THF (5 mL), was added L-Selectride (1 M in THF, 1.55 mL, 1.55 mmol). The reaction mixture was stirred at room temperature for 3 h, and after completion (monitored by TLC), the reaction mixture was quenched with saturated aq. NH₄Cl and extracted with DCM (3 X 100 mL). The combined organic layers were dried over sodium sulfate, filtered and concentrated *in vacuo* to afford INT-7.4 (2.5 g, crude) as an off white solid. This material was directly used in the next step without further purification.

Step-5: Synthesis of (S)-1-(2-chloro-6-fluoro-3-methoxyphenyl)-1,4,5,7-tetrahydropyrano[3,4-c]pyrazol-4-amine hydrochloride (INT-7.5): A solution of INT-7.4 (0.110 g, 0.284 mmol) in DCM (2 mL) at 0 °C was added 4M HCl in dioxane (5 mL). The reaction mixture was stirred at room temperature for 4 h. After completion (monitored by TLC), the volatiles were removed *in vacuo* to afford an oily residue that was triturated with diethyl ether to afford INT-7.5 (0.100 g, crude) as an off-white solid; LCMS (ESI) m/z = 283.90 [M+H]+

Step-6: Synthesis of N-((4R)- and N-((4S)-1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-1-carboxamide (Ex-8 and Ex-9): To a solution of INT-7.5 (0.250 g, 0.823 mmol) and 5,6,7,8-Tetrahydroimidazo[1,5-a]pyridine-1-carboxylic acid (0.137 g, 0.823 mmol) at 0 °C in DMF (5 mL), was added DIPEA (0.437 mL, 2.469 mmol) followed by the addition of HATU (0.470 g, 1.234 mmol). The resulting reaction mixture was stirred at room temperature for 16 h. After completion (monitored by TLC), the reaction mixture was diluted with water (15 mL) and extracted with ethyl acetate (3 X 100 mL). The combined organic layers were dried over sodium sulfate, filtered and concentrated *in vacuo* to afford crude material. The crude compound was purified with silica gel column chromatography eluting with 5-10% MeOH in DCM. The column purified compound (mixture of Ex-8 and Ex-9) was subjected to separation and purification by chiral HPLC to afford Ex-8 (0.010 g, 3 %) and Ex-9 (0.050 g, 15 %) as off-white solids.

### Example 8: N-((4R)-1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-1-carboxamide (Peak 1)

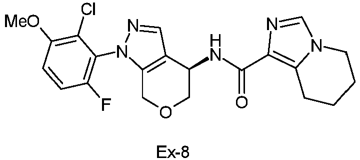

LCMS (ESI) m/z = 446.0 [M+H]+; HPLC: 99.1% (*t*_{R} = 5.483 min); 1H NMR (400 MHz, DMSO-d6): δ 7.73 (d, J = 3.5 Hz, 1H), 7.59-7.36 (m, 4H), 5.14-4.98 (m, 1H), 4.64-4.36 (m, 2H), 4.00 (t, J = 5.9 Hz, 2H), 3.92 (s, 3H), 3.85-3.82 (m, 2H), 3.00 (t, J = 6.5 Hz, 2H), 1.90-1.70 (m, 4H).

### Example 9: N-((4S)-1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-1-carboxamide (Peak 2)

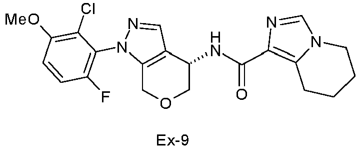

LCMS (ESI) m/z = 446.05 [M+H]+; HPLC: 99.8% (*t*_{R} = 5.481 min); 1H NMR (400 MHz, DMSO-d6): δ 7.73 (d, J = 3.5 Hz, 1H), 7.56 (d, J = 2.7 Hz, 1H), 7.53-7.35 (m, 3H), 5.19-4.95 (m, 1H), 4.64-4.29 (m, 2H), 4.00 (t, J = 5.9 Hz, 2H), 3.92 (s, 3H), 3.85-3.82 (m, 2H), 3.00 (t, J = 6.5 Hz, 2H), 1.98-1.66 (m, 4H).

In addition, the following compounds were also prepared in analogy to the methods described above:

### Example 10: N-((4R)-1-(2-chloro-6-fluorophenyl)-1,4,5,7-tetrahydropyrano[3,4-c]pyrazol-4-yl)-5,6,7,8-tetrahydroimidazo[1,5-a]pyridine-1-carboxamide (Peak 1)

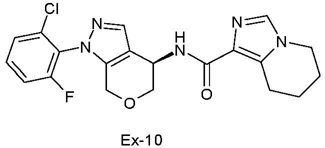

LCMS (ESI) m/z = 416.0 [M+H]+; HPLC: 99.1% (*t*_{R} = 5.326 min); 1H NMR (400 MHz, DMSO-d6): δ 7.74 (d, J = 3.1 Hz, 1H), 7.69-7.35 (m, 5H), 5.22-4.90 (m, 1H), 4.62-4.34 (m, 2H), 4.00 (t, J = 5.9 Hz, 2H), 3.85-3.83 (m, 2H), 3.00 (t, J = 6.4 Hz, 2H), 1.95-1.69 (m, 3H), 1.20-1.13 (m, 1H).

### Example 11: N-((4S)-1-(2-chloro-6-fluorophenyl)-1,4,5,7-tetrahydropyrano[3,4-c]pyrazol-4-yl)-5,6,7,8-tetrahydroimidazo[1,5-a]pyridine-1-carboxamide (Peak 2)

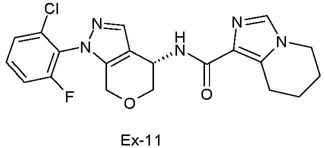

LCMS (ESI) m/z = 416.0 [M+H]+; HPLC: 99.8% (*t*_{R} = 5.319 min); 1H NMR (400 MHz, DMSO-d6) δ 7.01 (s, 1H), 6.84-6.77 (m, 1H), 6.73-6.64 (m, 2H), 6.60-6.53 (m, 1H), 4.36 (br s, 1H), 3.89-3.58 (m, 2H), 3.26 (t, J = 5.9 Hz, 2H), 3.22-3.06 (m, 2H), 2.31 (t, J = 6.4 Hz, 2H), 1.24 - 1.01 (m, 4H), 0.55-0.37 (m, 1H).

### Example 12: (S)-N-(1-(2,6-dichlorophenyl)-1,4,5,7-tetrahydropyrano[3,4-c]pyrazol-4-yl)-5,6,7,8-tetrahydroimidazo[1,5-a]pyridine-1-carboxamide (Peak 1)

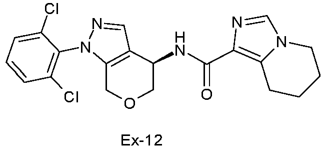

LCMS (ESI) m/z = 416.0 [M+H]+; HPLC: 99.1% (*t*_{R} = 5.326 min); 1H NMR (400 MHz, DMSO-d6): δ 7.74 (d, J = 3.1 Hz, 1H), 7.69-7.35 (m, 5H), 5.22-4.90 (m, 1H), 4.62-4.34 (m, 2H), 4.00 (t, J = 5.9 Hz, 2H), 3.85-3.83 (m, 2H), 3.00 (t, J = 6.4 Hz, 2H), 1.95-1.69 (m, 3H), 1.20-1.13 (m, 1H).

### Example 13: (S)-N-(1-(2,6-dichlorophenyl)-1,4,5,7-tetrahydropyrano[3,4-c]pyrazol-4-yl)-5,6,7,8-tetrahydroimidazo[1,5-a]pyridine-1-carboxamide (Peak 2)

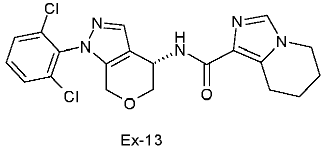

LCMS (ESI) m/z = 416.0 [M+H]+; HPLC: 99.8% (*t*_{R} = 5.319 min); 1H NMR (400 MHz, DMSO-d6) δ 7.01 (s, 1H), 6.84-6.77 (m, 1H), 6.73-6.64 (m, 2H), 6.60-6.53 (m, 1H), 4.36 (br s, 1H), 3.89-3.58 (m, 2H), 3.26 (t, J = 5.9 Hz, 2H), 3.22-3.06 (m, 2H), 2.31 (t, J = 6.4 Hz, 2H), 1.24 - 1.01 (m, 4H), 0.55-0.37 (m, 1H).

### Example 14: (S)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-1-carboxamide

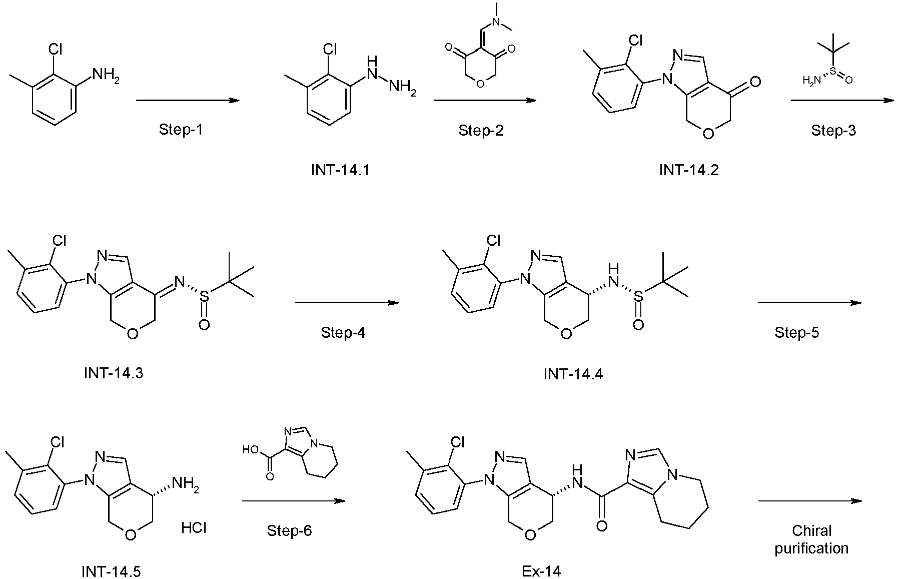

Step-1: Synthesis of hydrazine (INT-14.1): To a stirred solution of 2-chloro-3-methylaniline (5.0 g, 35.5 mmol) in conc. HCl (25 mL) was added sodium nitrite (8.53 g, 124.0 mmol) at 0 °C. The reaction was stirred at 0 °C for 1 h. After 1 h, stannous chloride dihydrate (31.9 g, 141.0 mmol) dissolved in hydrogen chloride (25 mL) was added dropwise at 0 °C. The reaction was allowed to stir at rt and the progress of reaction was monitored by TLC. After 16 h, the precipitate was filtered and washed with diethyl ether and n-pentane and dried under vacuum to afford pure compound INT-1.1 (5.2 g, 94%) as white solid. LCMS (ESI) m/z = 157.06 [M+H]⁺.

Step-2: Synthesis of 1-(2-chloro-3-methylphenyl)-1,7-dihydropyrano[3,4-c]pyrazol-4(5H)-one (INT 14.2): To a stirred solution of INT-14.1 (5.0 g, 31.9 mmol) in acetic acid (15 mL) was added INT-1.1 (3.78 g, 22.3 mmol) at rt. The reaction mixture was heated at 100 °C for 16 h. After completion, the reaction mixture was diluted with water and extracted with EtOAc. The combined organic layers were dried over sodium sulfate and concentrated in vacuum. The resulting crude material was purified with silica gel column chromatography using a gradient of 50-60% EtOAc in hexanes to afford INT-1.3 (2.52 g, 30%) as a yellow solid. LCMS (ESI) m/z = 263.12 [M+H]⁺.

Step-3: Synthesis of (S,Z)-N-(1-(2-chloro-3-methylphenyl)-1,7-dihydropyrano[3,4-c]pyrazol-4(5H)-ylidene)-2-methylpropane-2-sulfinamide (INT-14.3): To a stirred solution of INT-14.2 (2.0 g, 7.61 mmol) in THF (20 mL) were added titanium tetraisopropoxide (13.9 mL, 45.7 mmol) and (S)-2-methylpropane-2-sulfinamide (2.77 g, 22.8 mmol) at rt under N₂ atmosphere. The reaction was allowed to stir at 80 °C for 7 h. After completion, the reaction was cooled to rt, quenched with brine water. The solid precipitate was filtered through celite bed and washed with EtOAc. The collective filtrate was extracted with EtOAc, dried over sodium sulphate, and concentrated in vacuum to get crude. The crude was purified by silica gel column chromatography using gradient of 50-60% EtOAc in hexanes to get INT-1.4 (1.8 gm, 65%) as a yellow solid. LCMS (ESI) m/z = 366.27 [M+H]⁺.

Step-4: Synthesis of (S)-N-((S)-1-(2-chloro-3-methylphenyl)-1,4,5,7-tetrahydropyrano[3,4-c]pyrazol-4-yl)-2-methylpropane-2-sulfinamide (INT-14.4): To a stirred solution of INT-14.3 (1.8 g, 4.92 mmol) in THF (18 mL) was added L-selectride (1 M in THF) (6.55 g, 34.4 mmol) at -78 °C under N₂ atmosphere. The reaction was allowed to stir at rt for 4 h. After completion, the reaction mixture was quenched with saturated brine solution and extracted with EtOAc. The combined organic layers were dried over sodium sulfate, concentrated in vacuum and purified with flash column chromatography using gradient of 60-70% EtOAc in hexanes to get INT-1.5 (1.8 g, 99%) as a yellow solid. LCMS (ESI) m/z = 368.28 [M+H]⁺.

Step-5: Synthesis of (S)-1-(2-chloro-3-methylphenyl)-1,4,5,7-tetrahydropyrano[3,4-c]pyrazol-4-amine hydrochloride (INT-14.5): To a stirred solution of INT-14.4 (1.8 g, 4.89 mmol) in DCM (36 mL) was added 4M HCl in 1,4 dioxane (21 mL) at 0 °C. The reaction mixture was allowed to stir at rt for 4 h. After completion of reaction, solvent was evaporated to get the crude. The crude was dissolved in DCM and re-precipitated by n-pentane to afford INT-14.5 (1.25 g, 97%) as an off white solid. LCMS (ESI) m/z = 264.29 [M+H]⁺.

Step-6: Synthesis of (S)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-1-carboxamide (Ex-14): To a stirred solution of 5,6,7,8-tetrahydroimidazo[1,5-a]pyridine-1-carboxylic acid (0.075 g, 0.46 mmol) and INT-14.6 (0.12 g, 0.46 mmol) in dry DMF (1.2 mL) were added HATU (0.21 g, 0.55 mmol) and DIPEA (0.10 mL, 0.55 mmol) at 0 °C. The reaction was then allowed to stir at rt for 16 h. After completion, the reaction mass was poured on ice water and extracted with EtOAc. The collective organic layer was washed with brine, dried over sodium sulphate and evaporated in vacuum to get the crude compound. The crude product was purified with the silica gel column chromatography using gradient of 2-10% MeOH in DCM to get enantiomeric mixture [74:26] of Ex-14 (120 mg) as an off white solid compound. Further, Ex-14 was subjected for separation and purification by chiral HPLC to afford pure **Ex-14** (70 mg, 36.8%) as a white solid [Column Name: CHIRALCEL-OJH (4.6*250) mm,5u; Mobile Phase: CO₂/0.1% AH in MeOH (80:20); Flow rate: 3.0 mL/min; Flow mode: Isocratic; Column Temperature: 35°C; ABPR Pressure: 1500psi, *t*_{R} (peak-1) 3.19 min. (74%), *t*_{R} (peak-2) 3.59 min. (26%)]. LCMS (ESI) m/z = 412.28 [M+H]⁺; Chiral HPLC (peak-1): 99.8% (*t*_{R} = 3.18 min); UPLC (Method E): 96.6% (*t*_{R} 9.27 min); 1H NMR (400 MHz, DMSO-d6): δ 7.66 (s, 1H), 7.56 (s, 1H), 7.53 (dd, J = 6.8 Hz, J = 2.4 Hz, 1H), 7.42-7.39 (m, 3H), 5.07-5.03 (m, 1H), 4.53 (dd, J = 14.8 Hz, J = 11.6 Hz, 2H), 3.99 (t, J = 6.0 Hz, 2H), 3.83 (td, J = 11.6 Hz, J = 4.0 Hz, 2H), 2.99 (t, J = 6.4 Hz, 2H), 2.43 (s, 3H), 1.86-1.84 (m, 2H), 1.79-1.76 (m, 2H).

### Example 15: (S)-N-(1-(2-chloro-3-methylphenyl)-1,4,5,7-tetrahydropyrano[3,4-c]pyrazol-4-yl)-5-ethyl-1-methyl-1H-imidazole-4-carboxamide

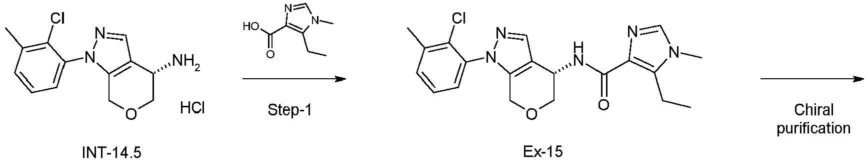

To a stirred solution of 5-ethyl-1-methyl-1H-imidazole-4-carboxylic acid (0.25 g, 1.59 mmol) and INT-14.5 (0.35 g, 1.33 mmol) in dry DMF (3.5 mL) were added HATU (0.61 g, 1.59 mmol) and DIPEA (0.694 mL, 3.98 mmol) at 0 °C. The reaction was then allowed to stir at rt for 16 h. After completion, the reaction mass was poured on ice water and extracted with EtOAc. The collective organic layer was washed with brine, dried over sodium sulphate and evaporated in vacuum to get the crude compound. The crude was purified with the silica gel column chromatography using gradient of 2-10% MeOH in DCM to get enantiomeric mixture [28:78] of Ex-01 (250 mg) as an off white solid compound. Further, Ex-15 was subjected for separation and purification by chiral HPLC to afford pure Ex-15 (30 mg, 5.6%) as a white solid [Column Name: Chiralpak-IG (4.6 X 250) mm, 5um, Mobile phase: 0.1% TEA in IPA/ACN = 70:30 (v/v), Flow Rate: 1.0 ml/min, temp: 25°C, Flow mode: Isocratic, *t*_{R} (peak-1) 4.82 min. (28%), *t*_{R} (peak-2) 5.78 min. (78%)]. LCMS (ESI) m/z = 400.11 [M+H]⁺; Chiral HPLC (peak-2): 99.5% (*t*_{R} 5.76 min); UPLC (Method B): 99.9% (*t*_{R} 8.18 min); 1H NMR (400 MHz, DMSO-d6): δ 7.67 (s, 1H), 7.56 (s, 1H), 7.55-7.52 (m, 1H), 7.48 (d, J = 8.4 Hz, 1H), 7.42-7.37 (m, 2H), 5.07-5.03 (m, 1H), 4.53 (dd, J = 14.8 Hz, J = 11.2 Hz, 2H), 3.83 (qd, J = 11.6 Hz, J = 3.6 Hz, 2H), 3.60 (s, 3H), 2.98 (q, J = 7.6 Hz, 2H), 3.43 (s, 3H), 1.22 (t, J = 7.6 Hz, 3H).

### Example 16: (S)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-3-carboxamide

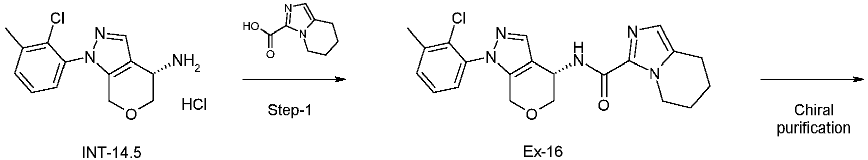

To a stirred solution of 5,6,7,8-tetrahydroimidazo[1,5-a]pyridine-3-carboxylic acid (0.38 g, 2.28 mmol) and INT-14.5 (0.4 g, 1.52 mmol) in dry DMF (4.0 mL) were added HATU (0.87 g, 2.28 mmol) and DIPEA (0.80 mL, 2.28 mmol) at 0 °C. The reaction was then allowed to stir at rt for 16 h. After completion, the reaction mass was poured on ice water and extracted with EtOAc. The collective organic layer was washed with brine, dried over sodium sulphate and evaporated in vacuum to get the crude compound. The crude product was purified with the silica gel column chromatography using gradient of 2-10% MeOH in DCM to get enantiomeric mixture [12:88] of Ex-16 (160 mg) as an off white solid compound. Further, Ex-16 was subjected for separation and purification by chiral HPLC to afford pure Ex-16 (80 mg, 12.8%) as a white solids [Column Name: CHIRALPAK IA (4.6*250) mm,5u; Mobile Phase: CO2/0.2% TEA in IPA:ACN (50:50) = (60:40) (v/v); Flow rate: 3.0 mL/min; Flow mode: Isocratic; Column Temperature: 35°C; ABPR Pressure : 1500psi, *t*_{R} (peak-1) 2.93 min. (12%), *t*_{R} (peak-2) 3.69 min. (88%)]. LCMS (ESI) m/z = 412.09 [M+H]⁺; Chiral HPLC (peak-2): 99.8% (*t*_{R} 3.77 min); UPLC (Method A): 99.5% (*t*_{R} 11.08 min); 1H NMR (400 MHz, DMSO-d6): δ 7.89 (d, J = 8.4 Hz, 1H), 7.67 (s, 1H), 7.54 (dd, J = 7.6 Hz, J = 1.6 Hz, 1H), 7.42-7.36 (m, 2H), 6.76 (s, 1H), 5.09-5.05 (m, 1H), 4.52 (dd, J = 14.8 Hz, J = 14.4 Hz, 2H), 4.44-4.33 (m, 2H), 3.85 (td, J = 11.6 Hz, J = 4.0 Hz, 2H), 2.76 (t, J = 6.4 Hz, 2H), 2.43 (s, 3H), 1.93-1.87 (m, 2H), 1.76-1.70 (m, 2H).

### Example 17: (S)-N-(1-(2-chloro-3-methylphenyl)-1,4,5,7-tetrahydropyrano[3,4-c]pyrazol-4-yl)-4,5,6,7-tetrahydro-1H-indazole-3-carboxamide

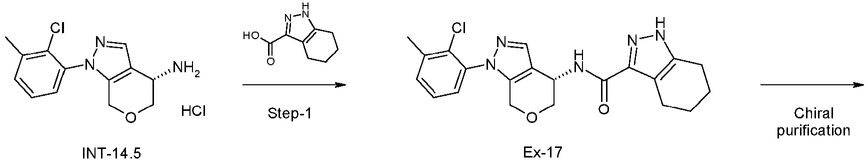

To a stirred solution of 4,5,6,7-tetrahydro-1H-indazole-3-carboxylic acid (0.33 g, 1.99 mmol) and INT-14.5 (0.35 g, 1.33 mmol) in dry DMF (3.5 mL) were added HATU (0.76 g, 1.99 mmol) and DIPEA (0.70 mL, 3.98 mmol) at 0 °C. The reaction was then allowed to stir at rt for 16 h. After completion, the reaction mass was poured on ice water and extracted with EtOAc. The collective organic layer was washed with brine and dried over sodium sulfate and evaporated in vacuum to get the crude compound. The crude product was purified with the silica gel column chromatography using gradient of 2-10% MeOH in DCM to get enantiomeric mixture [24:76] of Ex-17 (250 mg) as an off white solid compound. Further, Ex-17 was subjected for separation and purification by chiral HPLC to afford pure Ex-17 (66 mg, 12.1%) as a white solid [Column Name: Chiralpak-IA (4.6*250) mm,5u; Mobile Phase: CO2/0.2% TEA in IPA = (60:40) (v/v); Flow rate: 3 mL/min; Flow mode: Isocratic; Column Temperature: 35°C; ABPR Pressure: 1500psi, *t*_{R} (peak-1) 2.79 min. (24%), *t*_{R} (peak-2) 3.67 min. (76%)]. LCMS (ESI) m/z = 412.19 [M+H]⁺; Chiral HPLC (peak-2): 99.3% (*t*_{R} 3.90 min); UPLC (Method B): 97.2% (*t*_{R} 7.32 min); 1H NMR (400 MHz, DMSO-d6): δ 12.82 (br, s, 1H), 7.67 (s, 1H), 7.59 (br, s, 1H), 7.53 (dd, J = 7.6 Hz, J = 1.6 Hz, 1H), 7.40 (t, J = 7.6 Hz, 1H), 7.36 (dd, J = 7.6 Hz, J = 1.6 Hz, 1H), 5.10-5.06 (m, 1H), 4.49 (dd, J = 14.8 Hz, J = 14.4 Hz, 2H), 3.83 (d, J = 4.0 Hz, 2H), 2.69-2.66 (m, 2H), 2.59 (t, J = 6.0 Hz, 2H), 2.43 (s, 3H), 1.74-1.64 (m, 4H).

### Example 18: (S)-N-(1-(2-chloro-3-methylphenyl)-1,4,5,7-tetrahydropyrano[3,4-c]pyrazol-4-yl)-4,5,6,7-tetrahydrobenzo[d]isoxazole-3-carboxamide

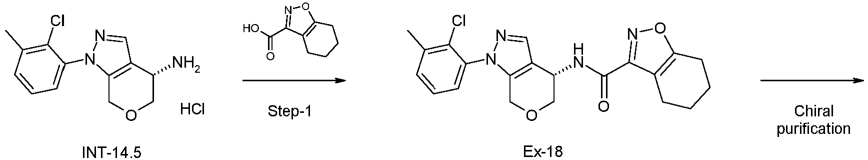

To a stirred solution of 4,5,6,7-tetrahydrobenzo[d]isoxazole-3-carboxylic acid (0.38 g, 2.28 mmol) and INT-14.5 (0.40 g, 1.52 mmol) in dry DMF (4.0 mL) were added HATU (0.87 g, 2.28 mmol) and DIPEA (0.80 mL, 4.55 mmol) at 0 °C. The reaction was then allowed to stir at rt for 16 h. After completion, the reaction mass was poured on ice water and extracted with EtOAc. The collective organic layer was washed with brine, dried over sodium sulphate and evaporated in vacuum to get the crude compound. The crude product was purified with the silica gel column chromatography using gradient of 2-10% MeOH in DCM to get enantiomeric mixture [75:25] of Ex-18 (350 mg) as an off white solid compound. Further, Ex-18 was subjected for separation and purification by chiral HPLC to afford pure Ex-18 (110 mg, 17.5%) as a white solid [Column Name: CHIRALCEL OJ-H (4.6*250) mm,5u; Mobile Phase: CO₂/0.2% TEA in MEOH = (60:40) (v/v); Flow rate: 3.0 mL/min; Flow mode: Isocratic; Column Temperature: 35°C; ABPR Pressure: 1500psi, *t*_{R} (peak-1) 2.08 min. (75%), *t*_{R} (peak-2) 3.32 min. (25%)]. LCMS (ESI) m/z = 413.16 [M+H]⁺; Chiral HPLC (peak-1): 99.9% (*t*_{R} 1.94 min); UPLC (Method A): 99.4% (*t*_{R} 11.41 min); 1H NMR (400 MHz, DMSO-d6): δ 8.75 (d, J = 7.6 Hz, 1H), 7.67 (s, 1H), 7.53 (dd, J = 7.6 Hz, J = 0.8 Hz, 1H), 7.41 (t, J = 8.0 Hz, 1H), 7.33 (dd, J = 7.6 Hz, J = 1.2 Hz, 1H), 5.14-5.10 (m, 1H), 4.51 (dd, J = 14.8 Hz, J = 6.0 Hz, 2H), 3.88 (dd, J = 11.6 Hz, J = 4.0 Hz, 1H), 3.81 (dd, J = 11.6 Hz, J = 4.8 Hz, 1H), 2.72 (t, J = 6.0 Hz, 2H), 2.63-2.55 (m, 2H), 2.43 (s, 3H), 1.82-1.76 (m, 2H), 1.72-1.66 (m, 2H).

### Example 19: (S)-N-(1-(2-chloro-3-methylphenyl)-1,4,5,7-tetrahydropyrano[3,4-c]pyrazol-4-yl)-4-ethyl-5-methyl-1H-pyrazole-3-carboxamide

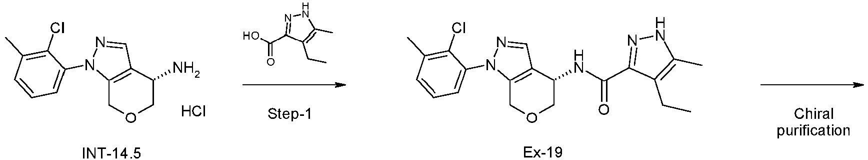

To a stirred solution of 4-ethyl-5-methyl-1H-pyrazole-3-carboxylic acid (0.35 g, 2.28 mmol) and INT-14.5 (0.40 g, 1.52 mmol) in dry DMF (4.0 mL) was added HATU (0.87 g, 2.28 mmol) and DIPEA (0.8 mL, 4.58 mmol) at 0 °C. The reaction was then allowed to stir at rt for 16 h. After completion, the reaction mass was poured on ice water and extracted with EtOAc. The collective organic layer was washed with brine, dried over sodium sulphate and evaporated in vacuum to get the crude compound. The crude product was purified with the silica gel column chromatography using gradient of 2-10% MeOH in DCM to get enantiomeric mixture [18:82] of Ex-19 (300 mg) as an off white solid compound. Further, Ex-19 was subjected for separation and purification by chiral HPLC to afford pure Ex-19 (75 mg, 12.4%) as a white solid [Column Name: Chiralpak-IA (4.6*250) mm,5u; Mobile Phase: CO2/0.2% TEA in IPA= (60:40) (v/v); Flow rate: 3.0 mL/min; Flow mode: Isocratic; Column Temperature: 35°C; ABPR Pressure: 1500psi, *t*_{R} (peak-1) 2.11 min. (18%), *t*_{R} (peak-2) 2.56 min. (82%)]. LCMS (ESI) m/z = 400.21 [M+H]⁺; Chiral HPLC (peak-2): 99.1% (*t*_{R} 2.60 min); UPLC (Method A): 99.5% (*t*_{R} 7.79 min); 1H NMR (400 MHz, DMSO-d6): δ 12.7 (s, 1H), 7.67 (s, 1H), 7.57 (d, J = 8.0 Hz, 1H), 7.54 (dd, J = 7.2 Hz, J = 1.6 Hz, 1H), 7.42-7.36 (m, 2H), 5.10-5.06 (m, 1H), 4.52 (dd, J = 14.8 Hz, J = 10.0 Hz, 2H), 3.85 (dd, J = 16.0 Hz, J = 12.4 Hz, 2H), 2.65 (q, J = 7.2 Hz, 2H), 2.43 (s, 3H), 2.17 (s, 3H), 1.07 (t, J = 7.6 Hz, 3H).

### Example 20: (S)-N-(1-(2-chloro-3-methylphenyl)-1,4,5,7-tetrahydropyrano[3,4-c]pyrazol-4-yl)-4,5-dimethylpicolinamide

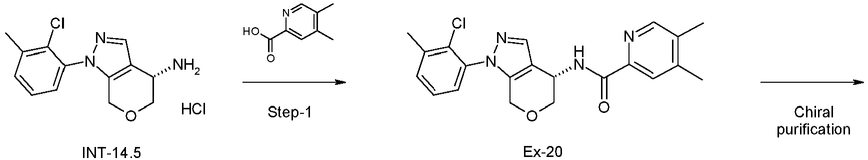

To a stirred solution of 4,5-dimethylpicolinic acid (0.17 g, 1.14 mmol) and INT-14.5 (0.2 g, 0.76 mmol) in dry DMF (2.0 mL) were added HATU (0.72 g, 1.14 mmol) and DIPEA (0.41 mL, 2.28 mmol) at 0 °C. The reaction was then allowed to stir at rt for 16 h. After completion, the reaction mass was poured on ice water and extracted with EtOAc. The collective organic layer was washed with brine, dried over sodium sulphate and evaporated in vacuum to get the crude compound. The crude product was purified with the silica gel column chromatography using gradient of 2-10% MeOH in DCM to get enantiomeric mixture [78:22] of Ex-20 (200 mg) as an off white solid compound. Further, Ex-20 was subjected for separation and purification by chiral HPLC to afford pure **Ex-20** (70 mg, 23.2%) as a white solid [Column Name: Lux-Amylose-1 (4.6*250) mm, 5u; Mobile Phase: CO₂/0.2% TEA in IPA= (60:40) (v/v); Flow rate: 3.0 mL/min; Flow mode: Isocratic; Column Temperature: 35°C; ABPR Pressure: 1500psi, *t*_{R} (peak-1) 2.91 min. (78%), *t*_{R} (peak-2) 3.60 min. (22%)]. LCMS (ESI) m/z = 397.04 [M+H]⁺; Chiral HPLC (peak-1): 99.3% (*t*_{R} 2.90 min); UPLC (Method A): 99.9% (*t*_{R} 6.67 min); 1H NMR (400 MHz, DMSO-d6): δ 8.41 (d, J = 8.4 Hz, 1H), 8.34 (s, 1H), 7.90 (s, 1H), 7.70 (s, 1H), 7.53 (dd, J = 6.8 Hz, J = 2.8 Hz, 1H), 7.43-7.37 (m, 2H), 5.14-5.11 (m, 1H), 4.55 (dd, J = 14.8 Hz, J = 12.4 Hz, 2H), 3.93 (dd, J = 11.6 Hz, J = 4.0 Hz, 1H), 3.85 (dd, J = 7.6 Hz, J = 4.0 Hz, 1H), 2.43 (s, 3H), 2.35 (s, 3H), 2.29 (s, 3H).

### Example 21: (S)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-1-carboxamide

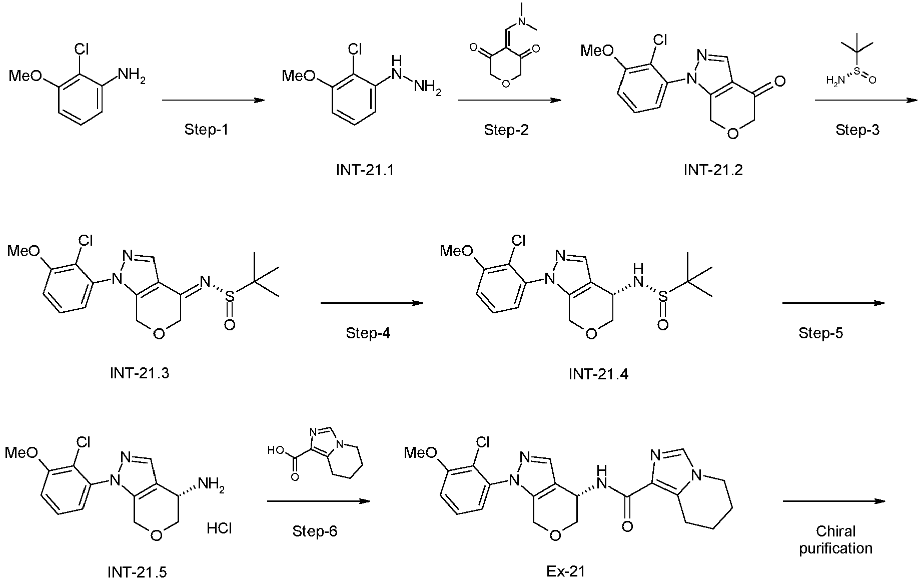

Step-1: Synthesis of (2-chloro-3-methoxyphenyl)hydrazine hydrochloride (INT-21.1): Following the experiment procedure of **INT-14.1,** the reaction was performed on 5.0 g scale to get INT-21.1 (4.8 g, 87%) as white solid. LCMS (ESI) m/z = 173.01 [M+H]⁺.

Step-2: Synthesis of 1-(2-chloro-3-methoxyphenyl)-1,7-dihydropyrano[3,4-c]pyrazol-4(5H)-one (INT 21.2): Following the experiment procedure of **INT-14.2,** the reaction was performed on 2.5 g scale to get INT-21.2 (0.75 g, 18%) as a yellow solid. LCMS (ESI) m/z = 279.23 [M+H]⁺.

Step-3: Synthesis of (S,E)-N-(1-(2-chloro-3-methoxyphenyl)-1,7-dihydropyrano[3,4-c]pyrazol-4(5H)-ylidene)-2-methylpropane-2-sulfinamide (INT-21.3): Following the experiment procedure of **INT-14.3,** the reaction was performed on 2.0 g scale to get INT-213 (1.70 gm, 62%) as a yellow solid. LCMS (ESI) m/z = 382.29 [M+H]⁺.

Step-4: Synthesis of (S)-N-((S)-1-(2-chloro-3-methoxyphenyl)-1,4,5,7-tetrahydropyrano[3,4-c]pyrazol-4-yl)-2-methylpropane-2-sulfinamide (INT-21.4): Following the experiment procedure of **INT-14.4,** the reaction was performed on 1.7 g scale to get INT-21.4 (1.4 g, 82%) as a yellow solid. LCMS (ESI) m/z = 384.27 [M+H]⁺.

Step-5: Synthesis of (S)-1-(2-chloro-3-methoxyphenyl)-1,4,5,7-tetrahydropyrano[3,4-c]pyrazol-4-amine hydrochloride (INT-21.5): Following the experiment procedure of **INT-**14.5, the reaction was performed on 2.0 g scale to get INT-2.5 (1.4 g, 96%) as an off white solid. LCMS (ESI) m/z = 280.09 [M+H]⁺.

Step-6: Synthesis of (S)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-1-carboxamide (Ex-21): Following the experiment procedure of **Ex-14,** the reaction was performed on 0.15 g scale with 5,6,7,8-tetrahydroimidazo[1,5-a]pyridine-1-carboxylic acid to get enantiomeric mixture [82:18] of Ex-21 (120 mg) as an off white solid compound. Further, Ex-21 was subjected for separation and purification by chiral HPLC to afford pure **Ex-21** (25 mg, 11%) as a white solid [Column Name: Lux_Amylose_1 (4.6*250) mm,5u; Mobile Phase: CO₂/0.1% AH in IPA (60:40); Flow rate: 3.0 mL/min; Flow mode: Isocratic; Column Temperature: 35°C; ABPR Pressure: 1500psi, *t*_{R} (peak-1) 5.64 min. (82%), *t*_{R} (peak-2) 7.63 min. (18%)]. LCMS (ESI) m/z = 428.23 [M+H]⁺; Chiral HPLC (peak-1): 99.9% (*t*_{R} 5.64 min); UPLC (Method A): 99.4% (*t*_{R} 9.50 min); 1H NMR (400 MHz, DMSO-d6): δ 7.66 (s, 1H), 7.56 (s, 1H), 7.46 (t, J = 8.4 Hz, 1H), 7.41 (d, J = 8.4 Hz, 1H), 7.32 (d, J = 8.0 Hz, 1H), 7.12 (d, J = 8.0 Hz, 1H), 5.07-5.03 (m, 1H), 4.53 (dd, J = 14.8 Hz, J = 11.2 Hz, 2H), 3.99 (t, J = 5.6 Hz, 2H), 3.93 (s, 3H), 3.83 (t, J = 3.2 Hz, 2H), 2.99 (t, J = 6.4 Hz, 2H), 1.86-1.84 (m, 2H), 1.78-1.77 (m, 2H).

### Example 22: (S)-N-(1-(2-chloro-3-methoxyphenyl)-1,4,5,7-tetrahydropyrano[3,4-c]pyrazol-4-yl)-5-ethyl-1-methyl-1H-imidazole-4-carboxamide

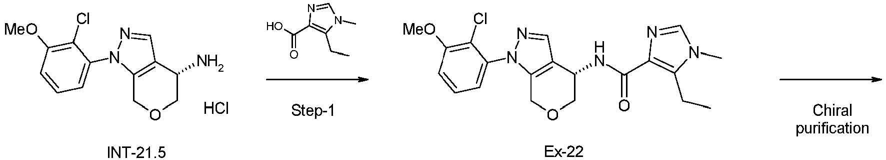

Following the experiment procedure of **Ex-15,** the reaction was performed on 0.25 g scale to get enantiomeric mixture [83:17] of Ex-22 (140 mg) as an off white solid compound. Further, Ex-22 was subjected for separation and purification by chiral HPLC to afford pure **Ex-22** (60 mg, 16.1%) as a white solid [Column Name: Lux Amylose-1 (4.6*250) mm,5u; Mobile Phase: CO₂/0.2%AH in IPA = (60:40) (v/v); Flow rate: 3.0 mL/min; Flow mode: Isocratic; Column Temperature: 35°C; ABPR Pressure: 1500psi, *t*_{R} (peak-1) 3.39 min. (83%), *t*_{R} (peak-2) 4.19 min. (17%)]. LCMS (ESI) m/z = 416.22 [M+H]⁺; Chiral HPLC (peak-1): 99.8% (*t*_{R} 3.38 min); UPLC (Method A): 99.9% (*t*_{R} 13.67 min); 1H NMR (400 MHz, DMSO-d6): δ 7.67 (s, 1H), 7.56 (s, 1H), 7.50-7.48 (m, 1H), 7.45 (d, J = 8.0 Hz, 1H), 7.32 (d, J = 8.0 Hz, 1H), 7.12 (dd, J = 7.6 Hz, J = 0.8 Hz, 1H), 5.07-5.03 (m, 1H), 4.55 (dd, J = 14.8 Hz, J = 10.4 Hz, 2H), 3.92 (s, 3H), 3.87-3.79 (m, 2H), 3.60 (s, 3H), 2.97 (q, J = 7.6 Hz, 2H), 1.12 (t, J = 7.6 Hz, 3H).

### Example 23: (S)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-3-carboxamide

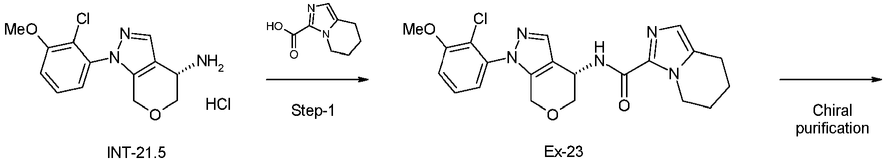

Following the experiment procedure of **Ex-16,** the reaction was performed on 0.20 g scale to get enantiomeric mixture [77:23] of Ex-23 (135 mg) as an off white solid compound. Further, Ex-23 was subjected for separation and purification by chiral HPLC to afford pure Ex-23 (27 mg, 8.83%) as a white solid [Column Name: Chiralcel OJ-H (4.6*250) mm, 5u; Mobile Phase: CO₂/0.2% TEA in IPA = (80:20) (v/v); Flow rate: 3 mL/min; Flow mode: Isocratic; Column Temperature: 35°C; ABPR Pressure: 1500psi, *t*_{R} (peak 1) 4.07 min. *(77%), t*_{R} (peak 2) 5.02 min. (23%)]. LCMS (ESI) m/z = 428.38 [M+H]⁺; Chiral HPLC (peak-1): 99.5% (*t*_{R} 4.10 min); UPLC (Method A): 95.3% (*t*_{R} 10.78 min); 1H NMR (400 MHz, DMSO-d6): δ 7.90 (d, J = 8.0 Hz, 1H), 7.67 (s, 1H), 7.46 (t, J = 8.0 Hz, 1H), 7.32 (dd, J = 8.4 Hz, J = 0.8 Hz, 1H), 7.11 (dd, J = 6.8 Hz, J = 1.2 Hz, 1H), 6.76 (s, 1H), 5.08-5.04 (m, 1H), 4.53 (dd, J = 14.8 Hz, J = 10.4 Hz, 2H), 4.48-4.32 (m, 2H), 3.93 (s, 3H), 3.89-3.81 (m, 2H), 2.76 (t, J = 6.4 Hz, 2H), 2.07-1.87 (m, 2H), 1.76-1.70 (m, 2H).

### Example 24: (S)-N-(1-(2-chloro-3-methoxyphenyl)-1,4,5,7-tetrahydropyrano[3,4-c]pyrazol-4-yl)-4,5,6,7-tetrahydro-1H-indazole-3-carboxamide

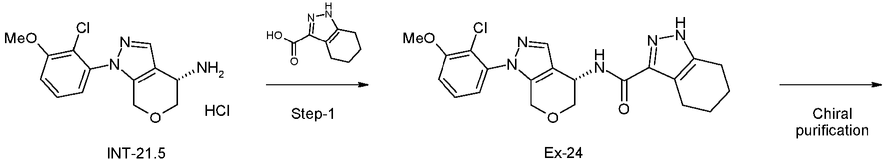

Following the experiment procedure of **Ex-17,** the reaction was performed on 0.30 g scale to get enantiomeric mixture [74:26] of Ex-24 (230 mg) as an off white solid compound. Further, Ex-24 was subjected for separation and purification by chiral HPLC to afford pure Ex-24 (113 mg, 24.6%) as a white solid [Column Name: Chiralcel OJH (4.6*250) mm,5u; Mobile Phase: CO₂/0.2% TEA in MeOH= (70:30) (v/v); Flow rate: 3 mL/min; Flow mode: Isocratic; Column Temperature: 35°C; ABPR Pressure: 1500psi, *t*_{R} (peak-1) 2.25 min. (74%), *t*_{R} (peak-2) 2.93 min. (26%)]. LCMS (ESI) m/z = 428.23 [M+H]⁺; Chiral HPLC (peak-1): 99.3% (*t*_{R} 2.26 min); UPLC (Method A): 99.0% (*t*_{R} 11.08 min); 1H NMR (400 MHz, DMSO-d6): δ 12.77 (s, 1H), 7.67 (s, 1H), 7.57 (br, s, 1H), 7.46 (t, J = 8.4 Hz, 1H), 7.32 (dd, J = 8.8 Hz, J = 1.2 Hz, 1H), 7.11 (dd, J = 8.0 Hz, J = 1.2 Hz, 1H), 5.10-5.06 (m, 1H), 4.52 (dd, J = 14.8 Hz, J = 10.0 Hz, 2H), 3.92 (s, 3H), 3.83 (d, J = 4.0 Hz, 2H), 2.69-2.66 (m, 2H), 2.58 (t, J = 5.6 Hz, 2H), 1.72-1.66 (m, 4H).

### Example 25: (S)-N-(1-(2-chloro-3-methoxyphenyl)-1,4,5,7-tetrahydropyrano[3,4-c]pyrazol-4-yl)-4,5,6,7-tetrahydrobenzo[d]isoxazole-3-carboxamide

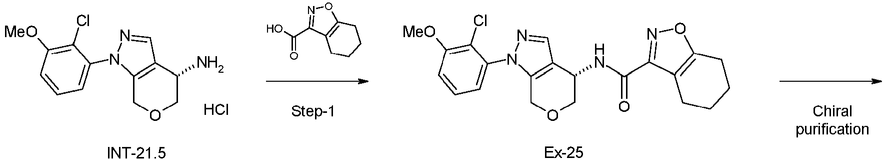

Following the experiment procedure of Ex-18, the reaction was performed on 0.30 g scale to get enantiomeric mixture [25:75] of Ex-25 (300 mg) as an off white solid compound. Further, Ex-25 was subjected for separation and purification by chiral HPLC to afford pure Ex-25 (138 mg, 30.0%) as a white solid [Column Name: Chiralcel OJH (4.6*250) mm,5u; Mobile Phase: CO₂/0.2% TEA in MeOH= (70:30) (v/v); Flow rate: 3 mL/min; Flow mode: Isocratic; Column Temperature: 35°C; ABPR Pressure: 1500psi, *t*_{R} (peak-1) 3.18 min. (25%), *t*_{R} (peak-2) 4.16 min. (75%)]. LCMS (ESI) m/z = 429.17 [M+H]⁺; Chiral HPLC (peak-2): 99.4% (*t*_{R} 4.18 min); UPLC (Method D): 99.9% (*t*_{R} 7.51 min); 1H NMR (400 MHz, DMSO-d6): δ 8.77 (d, J = 7.6 Hz, 1H), 7.67 (s, 1H), 7.47 (t, J = 8.8 Hz, 1H), 7.32 (dd, J = 8.8 Hz, J = 1.2 Hz, 1H), 7.07 (dd, J = 8.0 Hz, J = 1.2 Hz, 1H), 5.13-5.09 (m, 1H), 4.52 (dd, J = 14.8 Hz, J = 20.8 Hz, 2H), 3.93 (s, 3H), 3.90 (dd, J = 11.6 Hz, J = 4.4 Hz, 1H), 3.80 (dd, J = 11.6 Hz, J = 4.8 Hz, 1H), 2.72 (t, J = 6.0 Hz, 2H), 2.61-2.55 (m, 2H), 1.83-1.77 (m, 2H), 1.72-1.68 (m, 2H)

### Example 26: (S)-N-(1-(2-chloro-3-methoxyphenyl)-1,4,5,7-tetrahydropyrano[3,4-c]pyrazol-4-yl)-4-ethyl-5-methyl-1H-pyrazole-3-carboxamide

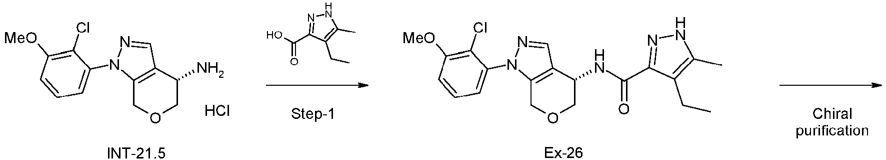

Following the experiment procedure of **Ex-19,** the reaction was performed on 0.40 g scale to get enantiomeric mixture [81:19] of Ex-26 (150 mg) as an off white solid compound. Further, Ex-26 was subjected for separation and purification by chiral HPLC to afford pure **Ex-26** (60 mg, 10.1%) as a white solid [Column Name: Chiralcel OJH (4.6*250) mm,5u; Mobile Phase: CO₂/0.2% TEA in IPA= (70:30) (v/v); Flow rate: 3 mL/min; Flow mode: Isocratic; Column Temperature: 35°C; ABPR Pressure: 1500psi, *t*_{R} (peak-1) 2.12 min. (81%), *t*_{R} (peak-2) 3.07 min. (19%)]. LCMS (ESI) m/z = 416.19 [M+H]⁺; Chiral HPLC (peak-1): 99.5% (*t*_{R} 2.12 min); UPLC (Method A): 99.9% (*t*_{R} 11.20 min); 1H NMR (400 MHz, DMSO-d6): δ 12.76 (s, 1H), 7.67 (s, 1H), 7.58 (d, J = 8.0 Hz, 1H), 7.46 (t, J = 8.0 Hz, 1H), 7.32 (dd, J = 8.4 Hz, J = 0.8 Hz, 1H), 7.12 (dd, J = 8.0 Hz, J = 1.6 Hz, 1H), 5.10-5.06 (m, 1H), 4.53 (dd, J = 14.8 Hz, J = 24.4 Hz, 2H), 3.93 (s, 3H), 3.85 (d, J = 4.0 Hz, 2H), 2.68-2.62 (m, 2H), 2.18 (s, 3H), 1.07 (t, J = 7.2 Hz, 3H).

### Example 27: (S)-N-(1-(2-chloro-6-fluoro-3-methoxyphenyl)-1,4,5,7-tetrahydropyrano[3,4-c]pyrazol-4-yl)-4,5-dimethylpicolinamide

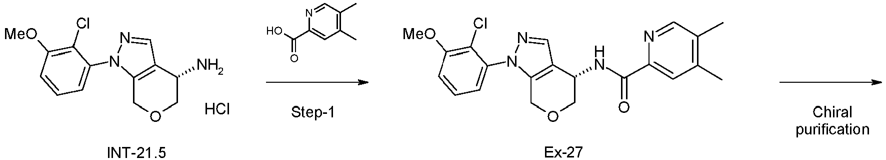

Following the experiment procedure of **Ex-20,** the reaction was performed on 0.20 g scale to get enantiomeric mixture [79:21] of Ex-27 (210 mg) as an off white solid compound. Further, Ex-27 was subjected for separation and purification by chiral HPLC to afford pure Ex-27 (90 mg, 30.5%) as a white solid [Column Name: Lux-Amylose-1 (4.6*250) mm,5u; Mobile Phase: CO₂/0.2% TEA in IPA = (60:40) (v/v); Flow rate: 3.0 mL/min; Flow mode: Isocratic; Column Temperature: 35°C; ABPR Pressure: 1500psi, *t*_{R} (peak-1) 3.35 min. (79%), *t*_{R} (peak-2) 4.30 min. (21%)]. LCMS (ESI) m/z = 413.23 [M+H]⁺; Chiral HPLC (peak-1): 99.1% (*t*_{R} 3.36 min); UPLC (Method A): 99.8% (*t*_{R} 11.30 min); 1H NMR (400 MHz, DMSO-d6): δ 8.41 (d, J = 8.0 Hz, 1H), 8.34 (s, 1H), 7.90 (s, 1H), 7.70 (s, 1H), 7.47 (t, J = 8.4 Hz, 1H), 7.33 (dd, J = 8.4 Hz, J = 1.2 Hz, 1H), 7.12 (dd, J = 8.0 Hz, J = 1.6 Hz, 1H), 5.14-5.10 (m, 1H), 4.55 (dd, J = 14.8 Hz, J = 12.0 Hz, 2H), 3.95 (s, 3H), 3.93-3.83 (m, 2H), 2.34 (s, 3H), 2.28 (s, 3H).

### Example 28: (S)-N-(1-(2-chloro-6-fluoro-3-methoxyphenyl)-1,4,5,7-tetrahydropyrano[3,4-c]pyrazol-4-yl)-5-ethyl-1-methyl-1H-imidazole-4-carboxamide

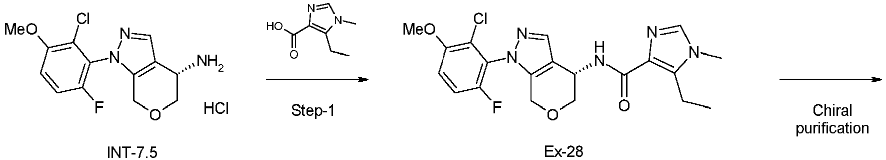

Following the experiment procedure of **Ex-15,** the reaction was performed on 0.20 g scale to get enantiomeric mixture [22:78] of Ex-28 (150 mg) as an off white solid compound. Further, Ex-28 was subjected for separation and purification by chiral HPLC to afford pure **Ex-28** (50 mg, 17.1%) as a white solid [Column Name: Column Name: Chiralpak IG (4.6*250) mm,5u; Mobile Phase: CO₂/0.2% TEA in ACN/IPA = (60:40) (v/v); Flow rate: 3.0 mL/min; Flow mode: Isocratic; Column Temperature: 35°C; ABPR Pressure: 1500psi, *t*_{R} (peak-1) 3.85 min. (22%), *t*_{R} (peak-2) 5.69 min. (78%)]. LCMS (ESI) m/z = 434.33 [M+H]⁺; Chiral HPLC (peak-2): 96.4% (*t*_{R} 5.58 min); UPLC (Method A): 99.5% (*t*_{R} 10.51 min); 1H NMR (DMSO-d6) δ 7.74 (d, J = 3.6 Hz, 1H), 7.56 (d, J = 2.4 Hz, 1H), 7.54-7.47 (m, 2H), 7.42-7.38 (m, 1H), 5.08-5.04 (m, 1H), 4.55 (dd, J = 14.8 Hz, J = 2.8 Hz, 1H), 4.45 (d, J = 14.8 Hz, 1H), 3.92 (s, 3H), 3.89-3.80 (m, 2H), 3.60 (s, 3H), 2.97 (q, J = 7.6 Hz, 2H), 1.22 (t, J = 7.6 Hz, 3H).

### Example 29: (S)-N-(1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-3-carboxamide

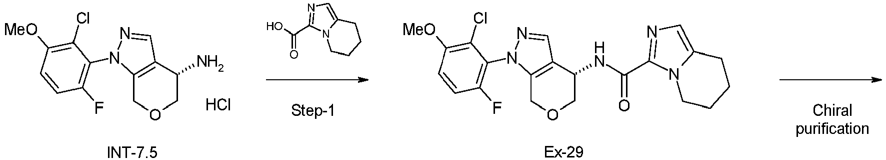

Following the experiment procedure of **Ex-16,** the reaction was performed on 0.20 g scale to get enantiomeric mixture [22:78] of Ex-29 (150 mg) as an off white solid compound. Further, Ex-29 was subjected for separation and purification by chiral HPLC to afford pure **Ex-29** (50 mg, 16.7%) as a white solid [Column Name: CHIRALPAK-IA (4.6*250) mm,5u; Mobile Phase: CO₂/100% MeOH (50:50); Flow rate: 4.0 mL/min; Flow mode: Isocratic; Column Temperature: 35°C; ABPR Pressure: 1500psi, *t*_{R} (peak-1) 2.01 min. (22%), *t*_{R} (peak-2) 2.57 min. (78%)]. LCMS (ESI) m/z = 446.26 [M+H]⁺; Chiral HPLC (peak-2): 99.8% (*t*_{R} 2.62 min); UPLC (Method C): 97.8% (*t*_{R} 6.92 min); 1H NMR (400 MHz, DMSO-d6): δ 7.95 (dd, J = 8.4 Hz, J = 8.4 Hz, 1H), 7.74 (d, J = 2.8 Hz, 1H), 7.50 (td, J = 9.2 Hz, J = 2.8, 1H), 7.40 (dd, J = 4.8 Hz, J = 4.8 Hz, 1H), 6.76 (d, J = 1.6 Hz, 1H), 5.09-5.05 (m, 1H), 4.54 (d, J = 14.8 Hz, 1H), 4.46 (d, J = 19.6 Hz, 1H), 4.42-4.34 (m, 2H), 3.92 (s, 3H), 3.86 (d, J = 4.4 Hz, 2H), 2.76 (t, J = 6.4 Hz, 2H), 1.93-1.87 (m, 2H), 1.76-1.70 (m, 2H).

### Example 30: (S)-N-(1-(2-chloro-6-fluoro-3-methoxyphenyl)-1,4,5,7-tetrahydropyrano[3,4-c]pyrazol-4-yl)-4,5,6,7-tetrahydro-1H-indazole-3-carboxamide

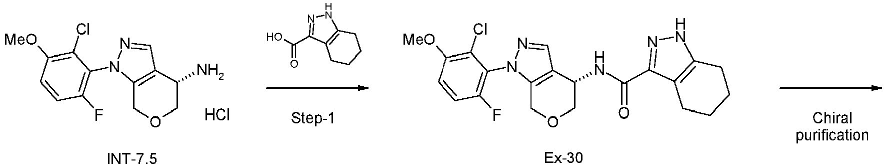

Following the experiment procedure of **Ex-17,** the reaction was performed on 0.15 g scale to get enantiomeric mixture [22:78] of Ex-30 (170 mg) as an off white solid compound. Further, Ex-30 was subjected for separation and purification by chiral HPLC to afford pure **Ex-30** (60 mg, 26.7%) as a white solid [Column Name: CHIRALPAK-IG (4.6*250) mm,5u; Mobile phase: CO₂/0.2% TEA in MeOH (50:50); Flow rate: 3 mL/min; Flow mode: Isocratic; Column Temperature: 35°C; ABPR Pressure: 1500psi, *t*_{R} (peak-1) 4.39 min. (22%), *t*_{R} (peak-2) 6.38 min. (78%)]. LCMS (ESI) m/z = 446.19 [M+H]⁺; Chiral HPLC (peak-2): 99.8% (*t*_{R} 6.38 min); UPLC (Method B): 98.4% (*t*_{R} 10.44 min); 1H NMR (400 MHz, DMSO-d6): δ 12.77 (s, 1H), 7.72 (d, J = 2.8 Hz, 1H), 7.60 (dd, J = 8.0 Hz, J = 8.4 Hz, 1H), 7.50 (t, J=8.8 Hz, 1H), 7.42-7.38 (m, 1H), 5.10-5.08 (m, 1H), 4.49 (dd, J = 14.8 Hz, J = 18.4 Hz, 2H), 3.92 (s, 3H), 3.84 (s, 2H), 2.60 (s, 2H), 2.59 (s, 2H), 1.72-1.66 (m, 4H).

### Example 31: (S)-N-(1-(2-chloro-6-fluoro-3-methoxyphenyl)-1,4,5,7-tetrahydropyrano[3,4-c]pyrazol-4-yl)-4,5,6,7-tetrahydrobenzo[d]isoxazole-3-carboxamide

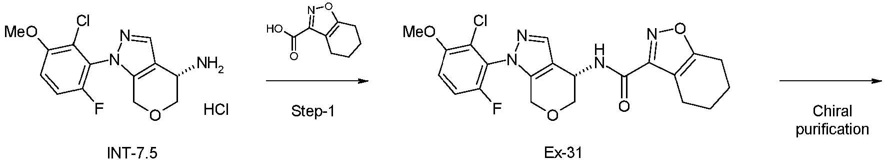

Following the experiment procedure of **Ex-18,** the reaction was performed on 0.15 g scale to get enantiomeric mixture [75:25] of Ex-31 (190 mg) as an off white solid compound. Further, Ex-31 was subjected for separation and purification by chiral HPLC to afford pure **Ex-31** (57 mg, 25.3%) as a white solid [Column Name: CHIRALCEL-OJH (4.6*250) mm,5u; Mobile Phase: CO2/0.1% AH in MeOH (60:40); Flow rate: 3.0 mL/min; Flow mode: Isocratic; Column Temperature: 35°C; ABPR Pressure: 1500psi, *t*_{R} (peak-1) 1.95 min. (75%), *t*_{R} (peak-2) 2.39 min. (25%)]. LCMS (ESI) m/z = 447.17 [M+H]⁺; Chiral HPLC (peak-1): 99.9% (*t*_{R} 1.95 min); UPLC (Method C): 96.7% (*t*_{R} 12.72 min); 1H NMR (DMSO-d6) δ 8.89-8.84 (m, 1H), 7.74 (s, 1H), 7.50 (t, J = 9.2 Hz, 1H), 7.42-7.38 (m, 1H), 5.14-5.10 (m, 1H), 4.49 (dd, J = 14.8 Hz, J = 12.8 Hz, 2H), 3.92 (s, 3H), 3.89-3.86 (m, 1H), 3.82-3.78 (m, 1H), 2.72 (t, J = 5.6 Hz, 2H), 2.57 (s, 2H), 1.83-1.77 (m, 2H), 1.72-1.66 (m, 2H).

### Example 32: (S)-N-(1-(2-chloro-6-fluoro-3-methoxyphenyl)-1,4,5,7-tetrahydropyrano[3,4-c]pyrazol-4-yl)-4-ethyl-5-methyl-1H-pyrazole-3-carboxamide

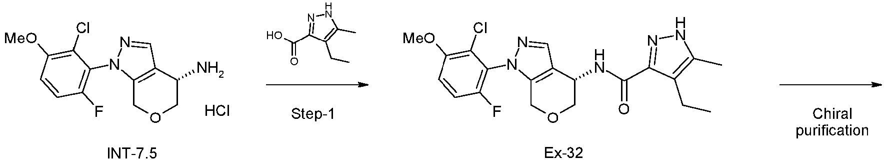

Following the experiment procedure of **Ex-19,** the reaction was performed on 0.15 g scale to get enantiomeric mixture [75:15] of Ex-32 (220 mg) as an off white solid compound. Further, Ex-32 was subjected for separation and purification by chiral HPLC to afford pure **Ex-32** (75 mg, 34.3%) as a white solid [Column Name: CHIRALCEL-OJH (4.6*250) mm,5u; Mobile Phase: CO₂/0.2% Isopropylamine in MeOH (80:20); Flow rate: 3.0 mL/min; Flow mode: Isocratic; Column Temperature: 35°C; ABPR Pressure: 1500psi, *t*_{R} (peak-1) 2.17 min. (75%), *t*_{R} (peak-2) 2.72 min. (15%)]. LCMS (ESI) m/z = 434.24 [M+H]⁺; Chiral HPLC (peak-1): 99.9% (*t*_{R} 2.18 min); UPLC (Method C): 99.3% (*t*_{R} 9.29 min); 1H NMR (400 MHz, DMSO-d6): δ 12.7 (s, 1H), 7.74 (d, J = 2.4 Hz, 1H), 7.65 (broad, s, 1H), 7.50 (t, J = 9.2 Hz, 1H), 7.42-7.38 (m, 1H), 5.09-5.08 (m, 1H), 4.50 (dd, J = 14.8 Hz, J = 19.2 Hz, 2H), 3.92 (s, 3H), 3.85 (d, J = 3.6 Hz, 2H), 2.66-2.65 (m, 2H), 2.17 (s, 3H), 1.08 (t, J = 8.8 Hz, 3H).

### Example 33: (S)-N-(1-(2-chloro-6-fluoro-3-methoxyphenyl)-1,4,5,7-tetrahydropyrano[3,4-c]pyrazol-4-yl)-4,5-dimethylpicolinamide

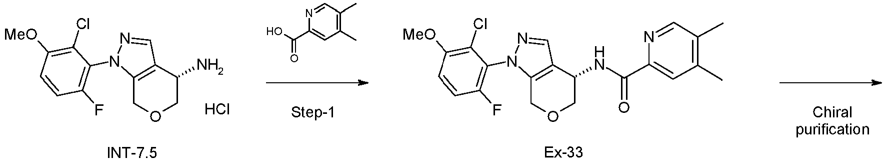

Following the experiment procedure of **Ex-20,** the reaction was performed on 0.50 g scale to get enantiomeric mixture [18:82] of Ex-33 (210 mg) as an off white solid compound. Further, Ex-33 was subjected for separation and purification by chiral HPLC to afford pure **Ex-33** (44 mg, 6.08%) as a white solid [Column Name: CHIRALPAK-IG (4.6*250) mm,5u; Mobile Phase: CO₂/0.2% AH in MeOH (50:50); Flow rate: 4.0 mL/min; Flow mode: Isocratic; Column Temperature: 35°C; ABPR Pressure: 1500psi, *t*_{R} (peak-1) 4.51 min. (18%), *t*_{R} (peak-2) 5.83 min. (82%)]. LCMS (ESI) m/z = 431.24 [M+H]⁺; Chiral HPLC (peak-2): 99.8% (*t*_{R} 5.83 min); UPLC (Method C): 98.7% (*t*_{R} 8.61 min); 1H NMR (400 MHz, DMSO-d6): δ 8.44 (dd, J = 8.4 Hz, J = 8.8 Hz, 1H), 8.35 (s, 1H), 7.90 (s, 1H), 7.76 (d, J = 2.0 Hz, 1H), 7.50 (td, J = 2.8 Hz, J = 9.2 Hz, 1H), 7.40 (dd, J = 4.8 Hz, J = 4.8 Hz, 1H), 5.14-5.12 (m, 1H), 4.60 (dd, J = 15.2 Hz, J = 12.8 Hz, 1H), 4.48 (d, J = 14.8 Hz, 1H), 3.98 (s, 3H), 3.89-3.85 (m, 2H), 2.35 (s, 3H), 2.29 (s, 3H).

### Example 34: (S)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-1-carboxamide

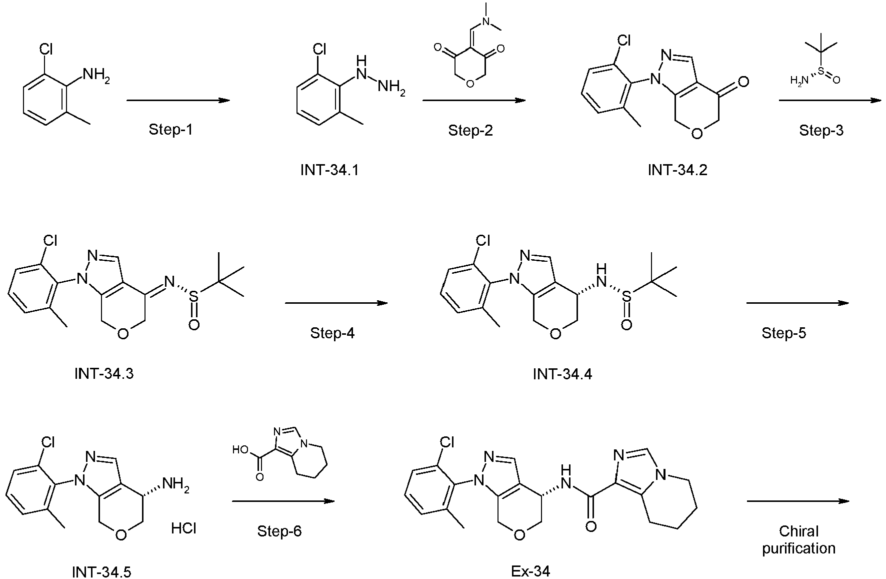

Step-1: Synthesis of (2-chloro-6-methylphenyl)hydrazine hydrochloride (INT-34.1): Following the experiment procedure of **INT-14.1,** the reaction was performed on 2.0 g scale to get INT-4.1 (1.9 g, 85%) as white solid. LCMS (ESI) m/z = 157.06 [M+H]⁺.

Step-2: Synthesis of 1-(2-chloro-6-methylphenyl)-1,7-dihydropyrano[3,4-c]pyrazol-4(5H)-one (INT 34.2): Following the experiment procedure of **INT-14.2,** the reaction was performed on 2.0 g scale to get INT-4.2 (1.0 g, 29%) as a yellow solid. LCMS (ESI) m/z = 263.06 [M+H]⁺.

Step-3: Synthesis of (S,Z)-N-(1-(2-chloro-6-methylphenyl)-1,7-dihydropyrano[3,4-c]pyrazol-4(5H)-ylidene)-2-methylpropane-2-sulfinamide (INT-34.3): Following the experiment procedure of **INT-14.3,** the reaction was performed on 2.0 g scale to get INT-4.3 (1.7 gm, 61%) as a yellow solid. LCMS (ESI) m/z = 365.09 [M+H]⁺.

Step-4: Synthesis of (S)-N-((S)-1-(2-chloro-6-methylphenyl)-1,4,5,7-tetrahydropyrano[3,4-c]pyrazol-4-yl)-2-methylpropane-2-sulfinamide (INT-34.4): Following the experiment procedure of **INT-14.4,** the reaction was performed on 1.7 g scale to get INT-34.4 (1.6 g, 93%) as a yellow solid. LCMS (ESI) m/z = 368.14 [M+H]⁺.

Step-5: Synthesis of (S)-1-(2-chloro-6-methylphenyl)-1,4,5,7-tetrahydropyrano[3,4-c]pyrazol-4-amine hydrochloride (INT-34.5): Following the experiment procedure of **INT-**14.5, the reaction was performed on 1.6 g scale to get INT-34.5 (1.30 g, 99%) as an off white solid. LCMS (ESI) m/z = 265.14 [M+H]⁺.

Step-6: Synthesis of ((S)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-1-carboxamide (Ex-34): Following the experiment procedure of **Ex-14,** the reaction was performed on 0.10 g scale with 5,6,7,8-tetrahydroimidazo[1,5-a]pyridine-1-carboxylic acid to get enantiomeric mixture [30:69] of Ex-34 (110 mg) as an off white solid compound. Further, Ex-34 was subjected for separation and purification by chiral HPLC to afford pure **Ex-34** (20 mg, 12.8%) as a white solid [Column Name: Chiralpak-IG (4.6 X 250) mm, 5um; Mobile Phase: 0.1% Ammonium Hydroxide in MeOH = 100% (v/v); Flow Rate: 1.0 ml/min, Temp: 25°C; Flow mode: Isocratic, *t*_{R} (peak-1) 21.6 min. (30%), *t*_{R} (peak-2) 25.7 min. (69%)]. Ex-34 was isolated and characterized as a mixture of atropisomers. LCMS (ESI) m/z = 412.23 [M+H]⁺; Chiral HPLC (peak-1): 80.7% (*t*_{R} 22.12 min), (peak-2): 19.2% (*t*_{R} 28.27 min); UPLC (Method D): (peak-1) 18.4% (*t*_{R} 13.08 min), (peak-2) 79.3% (*t*_{R} 13.42 min); Two *rotameric peaks* observed in 1H NMR. 1H NMR (400 MHz, DMSO-d6): δ 7.89 (br, s, 1H), 7.68 (d, J = 4.0 Hz, 1H), 7.56 (s, 1H), 7.56-7.45 (m, 2H), 7.40 (d, J = 6.4 Hz, 1H), 5.08-5.05 (m, 1H), 4.49-4.37 (m, 2H), 4.00 (t, J = 5.6 Hz, 2H), 3.84-3.77 (m, 2H), 2.99 (t, J = 6.4 Hz, 2H), 2.02 (s, 2.4H), 1.98 (s, 0.6H), 1.79-1.78 (m, 2H), 1.77-1.75 (m, 2H).

### Example 35: (S)-N-(1-(2-chloro-6-methylphenyl)-1,4,5,7-tetrahydropyrano[3,4-c]pyrazol-4-yl)-5-ethyl-1-methyl-1H-imidazole-4-carboxamide

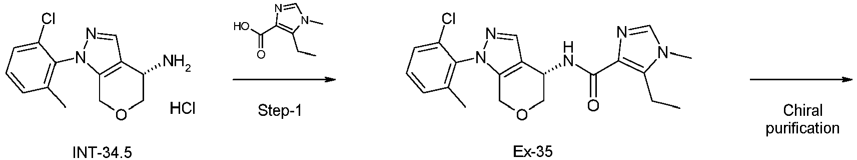

Following the experiment procedure of **Ex-15,** the reaction was performed on 0.40 g scale to get isomeric mixture [6:9:8:37:36] of Ex-35 (400 mg) as an off white solid compound. Further, Ex-35 was subjected for separation and purification by chiral HPLC to afford pure **Ex-35** (135 mg, 22.2%) as a white solid [Column Name: Chiralpak-IG (4.6 X 250) mm, 5um; Mobile Phase: 0.1% Ammonium Hydroxide in MeOH = 100%; Flow Rate: 1.0 ml/min, Temp: 25°C; Flow mode: Isocratic, *t*_{R} (peak-1) 8.22 min. (6%), *t*_{R} (peak-2) 9.48 min. (9%), *t*_{R} (peak-3) 11.01 min. (8%), *t*_{R} (peak-4) 13.36 min. (37%), *t*_{R} (peak-5) 16.8 min. (36%)]. Ex-35 was isolated and characterized as a mixture of atropisomers. LCMS (ESI) m/z = 400.18 [M+H]⁺; Chiral HPLC (peak-4): 52.1% (*t*_{R} 13.38 min), (peak-5): 47.5% (*t*_{R} 16.8 min); UPLC (Method C): (peak-1) 46.9% (*t*_{R} 9.44 min), (peak-2) 52.3% (*t*_{R} 9.59 min); Two *rotameric peaks* observed in 1H NMR. 1H NMR (400 MHz, DMSO-d6): δ 7.70 (d, J = 4.0 Hz, 1H), 7.56 (d, J = 1.6 Hz, 1H), 7.55-7.45 (m, 3H), 7.41 (d, J = 7.2 Hz, 1H), 5.08-5.05 (m, 1H), 4.55-4.37 (m, 2H), 3.86-3.78 (m, 2H), 3.60 (s, 3H), 2.98 (q, J = 7.6 Hz, 2H), 2.01 (s, 1.5H), 1.98 (s, 1.5H), 1.12 (t, J = 7.2 Hz, 3H).

### Example 36: (S)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-3-carboxamide

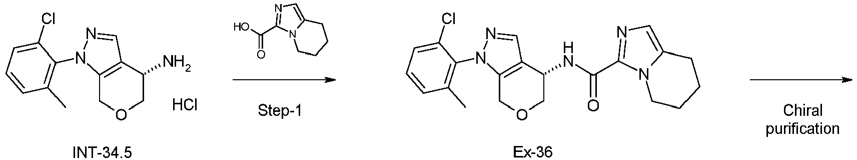

Following the experiment procedure of **Ex-16,** the reaction was performed on 0.40 g scale to get enantiomeric mixture [18:82] of Ex-36 (350 mg) as an off white solid compound. Further, Ex-36 was subjected for separation and purification by chiral HPLC to afford pure **Ex-36** (55 mg, 8.8%) as a white solid [Column Name: Chiralpak-IA (4.6 X 250) mm, 5um; Mobile Phase: 0.1% Ammonium Hydroxide in MeOH= 100%; Flow Rate: 1.0 ml/min, Temp: 25°C; Flow mode: Isocratic, *t*_{R} (peak-1 & 2) 6.10 min. (18%), *t*_{R} (peak-3 & 4) 7.50 min. (82%)]. Ex-36 was isolated and characterized as a mixture of atropisomers. LCMS (ESI) m/z = 412.19 [M+H]⁺; Chiral HPLC (peak-3): 52.0% (*t*_{R} 7.60 min), (peak-4): 47.0% (*t*_{R} 7.64 min); UPLC (Method G): (peak-1) 47.1% (*t*_{R} 9.34 min), (peak-2) 52.8% (*t*_{R} 9.49 min); Two *rotameric peaks* observed in 1H NMR. 1H NMR (400 MHz, DMSO-d6): 8.03 (d, J = 8.4 Hz, 0.5H), 7.89 (d, J = 8.4 Hz, 0.5H), 7.69 (d, J = 5.2 Hz, 1H), 7.53-7.50 (m, 1H), 7.47 (dd, J = 8.8 Hz, J = 1.2 Hz, 1H), 7.40 (d, J = 7.2 Hz, 1H), 6.76 (s, 1H), 5.09-5.07 (m, 1H), 4.49-4.35 (m, 4H), 3.86-3.83 (m, 2H), 2.76 (t, J = 6.4 Hz, 2H), 2.02 (s, 1.5H), 1.98 (s, 1.5H), 1.93-1.87 (m, 2H), 1.76-1.70 (m, 2H).

### Example 37: (S)-N-(1-(2-chloro-6-methylphenyl)-1,4,5,7-tetrahydropyrano[3,4-c]pyrazol-4-yl)-4,5,6,7-tetrahydro-1H-indazole-3-carboxamide

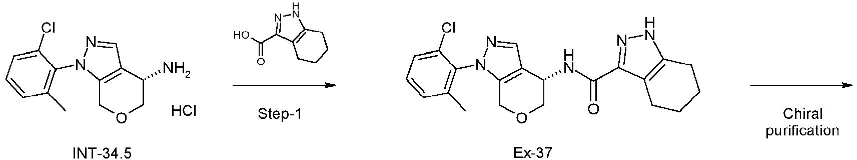

Following the experiment procedure of **Ex-17,** the reaction was performed on 0.40 g scale to get enantiomeric mixture [6:9:6:35:41] of Ex-37 (250 mg) as an off white solid compound. Further, Ex-37 was subjected for separation and purification by chiral HPLC to afford pure **Ex-37** (40 mg, 6.4%) as a white solid [Column Name: Chiralpak IG (4.6*250) mm,5u; Mobile Phase: CO₂/0.2% TEA in IPA =(60:40) (v/v); Flow rate: 3.0 mL/min; Flow mode: Isocratic; Column Temperature: 35°C; ABPR Pressure : 1500psi, *t*_{R} (peak-1) 4.34 min. (6%), *t*_{R} (peak-2) 5.27 min. (9%), *t*_{R} (peak-3) 5.88 min. (6%), *t*_{R} (peak-4) 6.54 min. (35%), *t*_{R} (peak-5) 7.46 min. (41%)]. Ex-37 was isolated and characterized as a mixture of atropisomers. LCMS (ESI) m/z = 412.19 [M+H]⁺; Chiral HPLC (peak-4): 54.0% (*t*_{R} 6.65 min), (peak-5): 46.0% (*t*_{R} 7.61 min); UPLC (Method G): (peak-1) 53.6% (*t*_{R} 11.77 min), (peak-2) 44.6% (*t*_{R} 11.88 min); Two *rotameric peaks* observed in 1H NMR.1H NMR (400 MHz, DMSO-d6): δ 12.76 (s, 1H), 7.68 (d, J = 4.8 Hz, 1H), 7.66-7.49 (m, 2H), 7.46 (d, J = 8.0 Hz, 1H), 7.40 (d, J = 7.2 Hz, 1H), 5.10-5.08 (m, 1H), 4.48-4.37 (m, 2H), 3.87-3.81 (m, 2H), 2.69 (s, 2H), 2.59 (t, J = 5.6 Hz, 2H), 2.02 (s, 1.5H), 1.98 (s, 1.5H), 1.71-1.68 (m, 4H).

### Example 38: (S)-N-(1-(2-chloro-6-methylphenyl)-1,4,5,7-tetrahydropyrano[3,4-c]pyrazol-4-yl)-4,5,6,7-tetrahydrobenzo[d]isoxazole-3-carboxamide

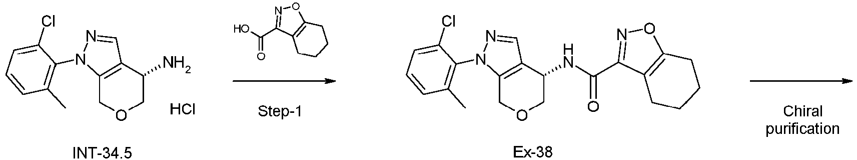

Following the experiment procedure of **Ex-18,** the reaction was performed on 0.40 g scale to get enantiomeric mixture [12:10:33:45] of Ex-38 (350 mg) as an off white solid compound. Further, Ex-38 was subjected for separation and purification by chiral HPLC to afford pure **Ex-38** (100 mg, 16.0%) as a white solid [Column Name: Chiralpak IG (4.6 x 250 mm),5µ; Mobile Phase: CO₂/0.2% AH in IPA = (65:35) (v/v); Flow rate: 3.0 mL/min; Flow mode: Isocratic; Column Temperature: 35°C; ABPR Pressure: 1500psi; *t*_{R} (peak-1) 4.83 min. (12%), *t*_{R} (peak-2) 6.46 min. (10%), *t*_{R} (peak-3) 8.08 min. (33%), *t*_{R} (peak-4) 9.90 min. (45%)]. Ex-38 was isolated and characterized as a mixture of atropisomers. LCMS (ESI) m/z = 413.18 [M+H]⁺; Chiral HPLC (peak-3): 44.0% (*t*_{R} 8.34 min), (peak-4): 55.0% (*t*_{R} 10.36 min); UPLC (Method G): (peak-1) 42.9% (*t*_{R} 11.20 min), (peak-2) 56.6% (*t*_{R} 11.34 min); Two *rotameric peaks* observed in 1H NMR. 1H NMR (400 MHz, DMSO-d6): δ 8.86 (d, J = 19.2 Hz, 0.5H), 8.79 (d, J = 7.6 Hz, 0.5H), 7.69 (s, 1H), 7.53-7.37 (m, 3H), 5.15-5.11 (m, 1H), 4.42 (td, J = 14.8 Hz, J = 6.8 Hz, 2H), 3.97-3.77 (m, 2H), 2.72 (t, J = 6.0 Hz, 2H), 2.57 (s, 2H), 2.02 (s, 1.5H), 1.97 (s, 1.5H), 1.81-1.79 (m, 2H), 1.71-1.68 (m, 2H).

### Example 39: (S)-N-(1-(2-chloro-6-methylphenyl)-1,4,5,7-tetrahydropyrano[3,4-c]pyrazol-4-yl)-4-ethyl-5-methyl-1H-pyrazole-3-carboxamide

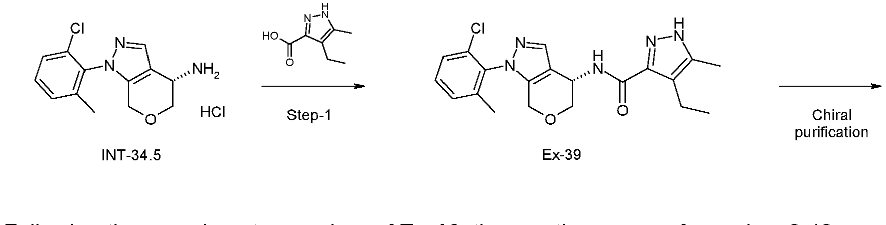

Following the experiment procedure of **Ex-19,** the reaction was performed on 0.40 g scale to get enantiomeric mixture [7:7:49:46] of Ex-25 (350 mg) as an off white solid compound. Further, Ex-25 was subjected for separation and purification by chiral HPLC to afford pure **Ex-25** (45 mg, 7.4%) as white solids, respectively [Column Name: Chiralpak-lG (4.6 X 250) mm, 5um; Mobile Phase: 0.1% Ammonium Hydroxide in MeOH/ACN = 50:50; Flow Rate: 1.0 ml/min, Temp: 25°C; Flow mode: Isocratic, *t*_{R} (peak-1) 3.05 min. (7%), *t*_{R} (peak-2) 3.3 min. (7%), *t*_{R} (peak-3) 4.38 min. (49%), *t*_{R} (peak-4) 5.02 min. (46%)]. Ex-39 was isolated and characterized as a mixture of atropisomers. LCMS (ESI) m/z = 400.18 [M+H]⁺; Chiral HPLC (peak-3): 51.0% (*t*_{R} 4.38 min), (peak-4): 45.0% (*t*_{R} 5.02 min); UPLC (Method L): (peak-1) 54.8% (*t*_{R} 12.39 min), (peak-2) 44.7% (*t*_{R} 12.56 min); Two *rotameric peaks* observed in 1H NMR. 1H NMR (400 MHz, DMSO-d6): δ 12.7 (br, s, 1H), 7.70 (d, J = 4.0 Hz, 1H), 7.60 (br, s, 1H), 7.53-7.51 (m, 1H), 7.47 (t, J = 7.6 Hz, 1H), 7.40 (d, J = 7.2 Hz, 1H), 5.10-5.08 (m, 1H), 4.48-4.37 (m, 2H), 3.87-3.82 (m, 2H), 2.65 (q, J = 6.8 Hz, 2H), 2.18 (s, 3H), 2.02 (s, 1.5H), 1.97 (s, 1.5H), 1.07 (t, J = 7.6 Hz, 3H).

### Example 40: (S)-N-(1-(2-chloro-6-methylphenyl)-1,4,5,7-tetrahydropyrano[3,4-c]pyrazol-4-yl)-4,5-dimethylpicolinamide

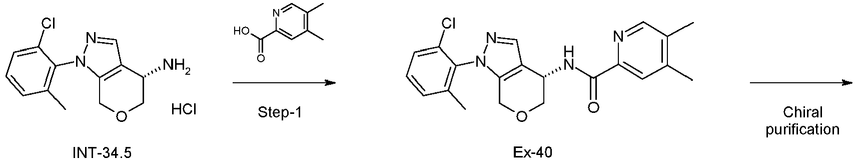

Following the experiment procedure of **Ex-20,** the reaction was performed on 0.20 g scale to get enantiomeric mixture [8:12:39:37] of Ex-40 (110 mg) as an off white solid compound. Further, Ex-40 was subjected for separation and purification by chiral HPLC to afford pure **Ex-40** (45 mg, 14.9%) as a white solid [Column Name: Chiralcel-OX-H (4.6 X 250) mm, 5um; Mobile Phase: 0.1% Ammonium Hydroxide in MeOH = 100%; Flow Rate: 1.0 ml/min, Temp: 25°C; Flow mode: Isocratic, *t*_{R} (peak-1) 7.13 min. (8%), *t*_{R} (peak-2) 7.30 min. (12%), *t*_{R} (peak-3) 8.03 min. (39%), *t*_{R} (peak-4) 8.27 min. (37%)]. Ex-40 was isolated and characterized as a mixture of atropisomers. LCMS (ESI) m/z = 397.17 [M+H]⁺; Chiral HPLC (peak-3): 48.0% (*t*_{R} 8.04 min), (peak-4): 51.5% (*t*_{R} 8.28 min); UPLC (Method C): 99.8% (*t*_{R} 11.19 min); Two *rotameric peaks* observed in 1H NMR. 1H NMR (400 MHz, DMSO-d6): δ 8.50 (d, J = 8.4 Hz, 0.5H), 8.40 (d, J = 8.0 Hz, 0.5H), 8.35 (s, 1H), 7.90 (s, 1H), 7.72 (d, J = 5.2 Hz, 1H), 7.53-7.51 (m, 1H), 7.47 (dd, J = 7.6 Hz, J = 0.4 Hz, 1H), 7.41 (d, J = 7.2 Hz, 1H), 5.16-5.13 (m, 1H), 4.46 (m, 2H), 3.94-3.83 (m, 2H), 2.35 (s, 3H), 2.29 (s, 3H), 2.03 (s, 1.5H), 1.98 (s, 1.5H).

### Example 41: (S)-5-ethyl-N-(1-(2-fluorophenyl)-1,4,5,7-tetrahydropyrano[3,4-c]pyrazol-4-yl)-1-methyl-1H-imidazole-4-carboxamide

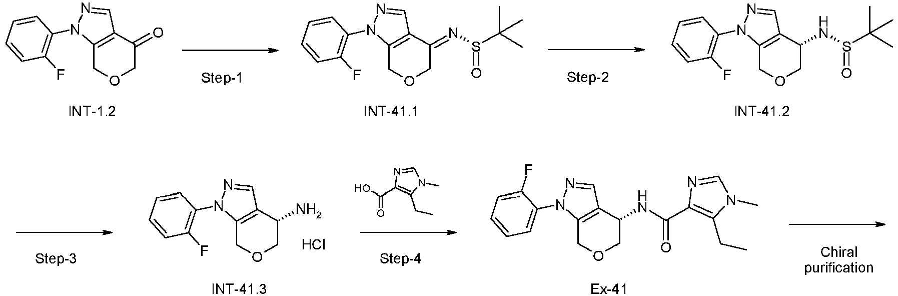

Step-1: Synthesis of (S,Z)-N-(1-(2-fluorophenyl)-1,7-dihydropyrano[3,4-c]pyrazol-4(5H)-ylidene)-2-methylpropane-2-sulfinamide (INT-41.1): Following the experiment procedure of **INT-14.3,** the reaction was performed on 3.0 g scale to get INT-41.1 (1.1 g, 25%) as a yellow solid. LCMS (ESI) m/z = 336.08 [M+H]⁺.

Step-2: Synthesis of (S)-N-((S)-1-(2-fluorophenyl)-1,4,5,7-tetrahydropyrano[3,4-c]pyrazol-4-yl)-2-methylpropane-2-sulfinamide (INT-41.2): Following the experiment procedure of **INT-14.4,** the reaction was performed on 1.1 g scale to get INT-20.4 (0.8 g, 72%) as a yellow solid. LCMS (ESI) m/z = 338.0 [M+H]⁺.

Step-3: Synthesis of (S)-1-(2-fluorophenyl)-1,4,5,7-tetrahydropyrano[3,4-c]pyrazol-4-amine hydrochloride (INT-41.3): Following the experiment procedure of **INT-14.5,** the reaction was performed on 0.8 g scale to get INT-20.5 (0.5 g, 78%) as an off white solid. LCMS (ESI) m/z = 234.06 [M+H]⁺.

Step-4: Synthesis of (S)-5-ethyl-N-(1-(2-fluorophenyl)-1,4,5,7-tetrahydropyrano[3,4-c]pyrazol-4-yl)-1-methyl-1H-imidazole-4-carboxamide (Ex-41): Following the experiment procedure of **Ex-14,** the reaction was performed on 0.10 g scale to get enantiomeric mixture [80:20] of Ex-41 (90 mg) as an off white solid compound. Further, Ex-41 was subjected for separation and purification by chiral HPLC to afford pure **Ex-41** (41 mg, 25.9%) as a white solid [Column Name: Chiralpak IE (250*4.6) mm, 5um; Mobile Phase: CO₂:0.1%lPamine in MEOH (60:40); Flow Rate: 4.0 mL/min; Column Temperature: 40°C; *t*_{R} (peak-1) 5.90 min. (80%), *t*_{R} (peak-2) 9.18 min. (20%)]. LCMS (ESI) m/z = 370.20 [M+H]⁺; Chiral HPLC (peak-1): 99.6% (*t*_{R} 5.90 min); UPLC (Method H): 97.1% (*t*_{R} 8.22 min); 1H NMR (400 MHz, DMSO-d6): δ 7.72 (s, 1H), 7.61-7.46 (m, 5H), 7.37 (td, J = 7.6 Hz, J = 1.2 Hz, 1H), 5.07-5.04 (m, 1H), 4.65 (dd, J = 14.8 Hz, J = 12.4 Hz, 2H), 3.86 (td, J = 8.0 Hz, J = 4.0 Hz, 2H), 3.59 (s, 3H), 2.97 (q, J = 7.6 Hz, 2H), 1.12 (t, J = 7.6 Hz, 3H); 19 F NMR (DMSO-d6) δ -123.88.

### Example 42: (S)-N-(1-(2-fluorophenyl)-1,4,5,7-tetrahydropyrano[3,4-c]pyrazol-4-yl)-4,5,6,7-tetrahydro-1H-indazole-3-carboxamide

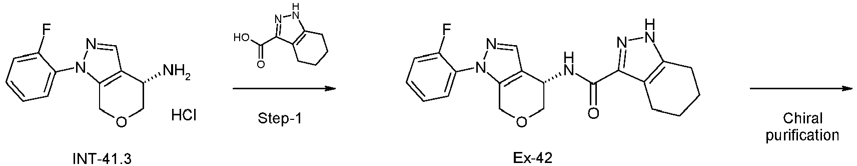

Following the experiment procedure of **Ex-17,** the reaction was performed on 0.30 g scale to get enantiomeric mixture [76:24] of Ex-42 (140 mg) as an off white solid compound. Further, Ex-42 was subjected for separation and purification by chiral HPLC to afford pure **Ex-42** (67 mg, 13.6%) as a white solid [Column Name: Chiralcel AS-H (250*4.6) mm, 5um; Mobile Phase: CO₂:0.1%lPamine in MeOH (60:40); Flow Rate: 3.0 mL/min; Column Temperature: 40°C; *t*_{R} (peak-1) 1.83 min. (76%), *t*_{R} (peak-2) 2.14 min. (24%)]. LCMS (ESI) m/z = 382.20 [M+H]⁺; Chiral HPLC (peak-1): 99.8% (*t*_{R} 1.81 min); UPLC (Method H): (peak-1) 99.8% (*t*_{R} 11.45 min); 1H NMR (400 MHz, DMSO-d6): δ 12.75 (s, 1H), 7.72 (s, 1H), 7.67 (br, s, 1H), 7.57 (t, J = 7.6 Hz, 1H), 7.56-7.46 (m, 2H), 7.37 (t, J = 7.6 Hz, 1H), 5.11-5.07 (m, 1H), 4.65 (dd, J = 14.8 Hz, J = 8.0 Hz, 2H), 3.85 (td, J = 11.6 Hz, J = 4.0 Hz, 2H), 2.68 (d, J = 6.4 Hz, 2H), 2.58 (t, J = 5.2 Hz, 2H), 1.72-1.67 (m, 4H); 19 F NMR (DMSO-d6) δ - 123.87.

### Example 43: (S)-N-(1-(2-fluorophenyl)-1,4,5,7-tetrahydropyrano[3,4-c]pyrazol-4-yl)-4,5,6,7-tetrahydrobenzo[d]isoxazole-3-carboxamide

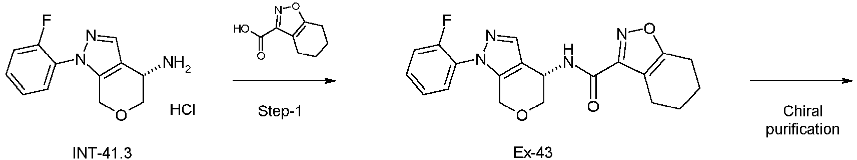

Following the experiment procedure of **Ex-18,** the reaction was performed on 0.30 g scale to get enantiomeric mixture [78:22] of Ex-43 (25 mg) as an off white solid compound. Further, Ex-43 was subjected for separation and purification by chiral HPLC to afford pure **Ex-43** (7 mg, 1.4%) as a white solid [Column Name: Chiralcel AS-H (250*4.6) mm, 5um; Mobile Phase: CO₂:0.1%lPamine in MeOH (60:40); Flow Rate: 3.0 mL/min; Column Temperature: 40°C; *t*_{R} (peak-1) 2.26 min. (78%), *t*_{R} (peak-2) 2.64 min. (22%)]. LCMS (ESI) m/z = 383.25 [M+H]⁺; Chiral HPLC (peak-1): 99.9% (*t*_{R} 2.26 min); UPLC (Method H): (peak-1) 99.6% (*t*_{R} 11.41 min); 1H NMR (400 MHz, DMSO-d6): δ 8.81 (d, J = 7.6 Hz, 1H), 7.73 (s, 1H), 7.57-7.46 (m, 3H), 7.37 (t, J = 7.2 Hz, 1H), 5.13-5.09 (m, 1H), 4.68-4.61 (m, 2H), 3.90 (dd, J = 11.6 Hz, J = 4.4 Hz, 1H), 3.82 (dd, J = 11.6 Hz, J = 4.8 Hz, 1H), 2.72 (t, J = 5.6 Hz, 2H), 2.57-2.55 (m, 2H), 1.81-1.78 (m, 2H), 1.71-1.68 (m, 2H); 19 F NMR (DMSO-d6) δ -123.87.

### Example 44: (S)-4-ethyl-N-(1-(2-fluorophenyl)-1,4,5,7-tetrahydropyrano[3,4-c]pyrazol-4-yl)-5-methyl-1H-pyrazole-3-carboxamide

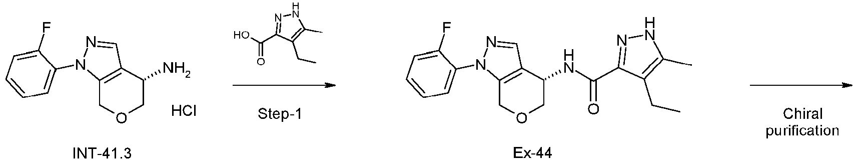

Following the experiment procedure of **Ex-19,** the reaction was performed on 0.30 g scale to get enantiomeric mixture [82:18] of Ex-44 (190 mg) as an off white solid compound. Further, Ex-44 was subjected for separation and purification by chiral HPLC to afford pure **Ex-44** (36 mg, 7.5%) as a white solid [Column Name: Chiralpak IE (250*4.6) mm, 5um; Mobile Phase: CO₂:0.1%IPamine in MEOH (60:40); Flow Rate: 3.0 mL/min; Column Temperature: 40°C; *t*_{R} (peak-1) 4.00 min. (82%), *t*_{R} (peak-2) 4.85 min. (18%)]. LCMS (ESI) m/z = 370.3 [M+H]⁺; Chiral HPLC (peak-3): 99.9% (*t*_{R} 4.00 min); UPLC (Method H): (peak-1) 99.8% (*t*_{R} 10.23 min); 1H NMR (400 MHz, DMSO-d6): δ 12.76 (br, s, 1H), 7.72 (s, 1H), 7.69 (d, J = 8.0 Hz, 1H), 7.58 (td, J = 8.0 Hz, J = 1.6 Hz, 1H), 7.55-7.46 (m, 2H), 7.37 (td, J = 7.6 Hz, J = 1.2 Hz, 1H), 5.10-5.08 (m, 1H), 4.65 (dd, J = 14.8 Hz, J = 8.4 Hz, 2H), 3.85 (td, J = 12.8 Hz, J = 4.4 Hz, 2H), 2.66 (q, J = 7.2 Hz, 2H), 2.17 (s, 3H), 1.06 (t, J = 7.6 Hz, 3H); 19 F NMR (DMSO-d6) δ -123.87.

### Example 45: (S)-N-(1-(2-fluorophenyl)-1,4,5,7-tetrahydropyrano[3,4-c]pyrazol-4-yl)-4,5-dimethylpicolinamide

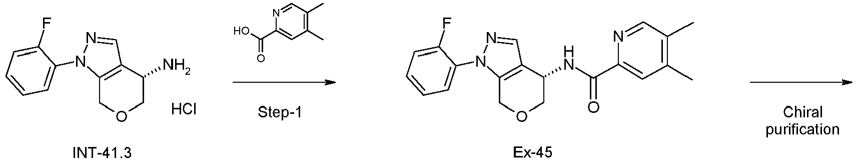

Following the experiment procedure of **Ex-20,** the reaction was performed on 0.10 g scale to get enantiomeric mixture [26:74] of Ex-45 (80 mg) as an off white solid compound. Further, Ex-45 was subjected for separation and purification by chiral HPLC to afford pure **Ex-45** (40 mg, 25.5%) as a white solid [Column Name: Chiralpak IA (250*4.6) mm, 5um; Mobile Phase: CO₂:0.1%lPamine in MeOH (60:40); Flow Rate: 3.0 mL/min; Column Temperature: 40°C; *t*_{R} (peak-1) 2.61 min. (26%), *t*_{R} (peak-2) 3.19 min. (74%)]. LCMS (ESI) m/z = 367.25 [M+H]⁺; Chiral HPLC (peak-2): 98.6% (*t*_{R} 3.10 min); UPLC (Method H): 98.9% (*t*_{R} 10.02 min); 1H NMR (400 MHz, DMSO-d6): δ 8.45 (d, J = 8.4 Hz, 1H), 8.33 (s, 1H), 7.90 (s, 1H), 7.75 (s, 1H), 7.59 (t, J = 8.0 Hz, 1H), 7.55-7.47 (m, 2H), 7.37 (t, J = 8.0 Hz, 1H), 5.14-5.12 (m, 1H), 4.68 (dd, J = 14.8 Hz, J = 12.8 Hz, 2H), 3.91 (qd, J = 11.6 Hz, J = 3.6 Hz, 2H), 2.34 (s, 3H), 2.28 (s, 3H); 19 F NMR (DMSO-d6) δ -123.87.

In addition, the following compounds were also prepared in analogy to the methods described above:

### Example 46: (S)-N-(1-(2-chloro-6-fluorophenyl)-1,4,5,7-tetrahydropyrano[3,4-c]pyrazol-4-yl)-4,5,6,7-tetrahydro-1H-indazole-3-carboxamide

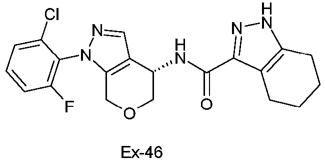

LCMS (ESI) m/z = 416.2 [M+H]+; Chiral HPLC (peak-1): 99.5% (*t*_{R} 2.48 min); UPLC (Method H): 99.6% (*t*_{R} = 10.43 min); 1H NMR (400 MHz, DMSO-d6): δ 12.78 (s, 1H), 7.74 (d, J = 3.2 Hz, 1H), 7.69-7.50 (m, 4H), 5.10-5.08 (m, 1H), 4.52 (d, J = 15.2 Hz, J = 14.8 Hz, 2H), 3.84 (s, 2H), 2.67 (d, J = 1.6 Hz, 2H), 2.57 (t, J = 3.2 Hz, 2H), 1.71-1.66 (m, 4H); Two *rotameric peaks* observed in 19F NMR. 19F NMR (DMSO-d6) δ -118.17, -118.46.

### Example 47: (S)-N-(1-(2-chloro-6-fluorophenyl)-1,4,5,7-tetrahydropyrano[3,4-c]pyrazol-4-yl)-5,6,7,8-tetrahydroimidazo[1,5-a]pyridine-3-carboxamide

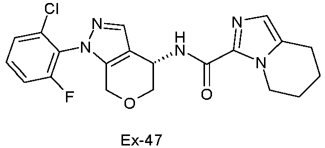

LCMS (ESI) m/z = 416.15 [M+H]+; Chiral HPLC (peak-1): 99.9% (*t*_{R} 8.30 min); UPLC (Method H): 99.8% (*t*_{R} = 8.76 min); Two *rotameric peaks* observed in 1H and 19F NMR. 1H NMR (400 MHz, DMSO-d6): δ 8.01 (d, J = 8.4 Hz, 0.5H), 7.94 (d, J = 8.0 Hz, 0.5H), 7.74 (d, J = 3.2 Hz, 1H), 7.68-7.58 (m, 1H), 7.59 (d, J = 8.4 Hz, 1H), 7.53 (t, J = 8.8 Hz, 1H), 6.75 (s, 1H), 5.10-5.06 (m, 1H), 4.57 (dd, J = 14.8 Hz, J = 3.6 Hz, 1H), 4.48 (d, J = 14.8 Hz, 1H), 4.42-4.34 (m, 2H), 3.85 (d, J = 4.0 Hz, 2H), 2.76 (t, J = 6.4 Hz, 2H), 1.92-1.86 (m, 2H), 1.76-1.71(m, 2H); 19F NMR (DMSO-d6) δ-118.18, -118.49.

### Example 48: (S)-N-(1-(2-chloro-6-fluorophenyl)-1,4,5,7-tetrahydropyrano[3,4-c]pyrazol-4-yl)-4,5-dimethylpicolinamide

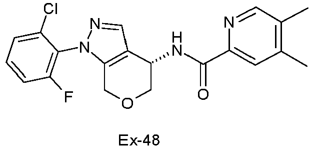

LCMS (ESI) m/z = 401.25 [M+H]+; Chiral HPLC (peak-1): 99.9% (*t*_{R} 7.26 min); UPLC (Method H): 99.9% (*t*_{R} = 10.27 min); Two *rotameric peaks* observed in 1H and 19F NMR. 1H NMR (400 MHz, DMSO-d6): δ 8.48 (d, J = 8.0 Hz, 0.5H), 8.43 (d, J = 8.0 Hz, 0.5H), 8.34 (s, 1H), 7.90 (s, 1H), 7.77 (d, J = 2.4 Hz, 1H), 7.68-7.63 (m, 1H), 7.59 (d, J = 8.4 Hz, 1H), 7.53 (t, J = 8.4 Hz, 1H), 5.14-5.12 (m, 1H), 4.60 (dd, J = 14.8 Hz, J = 5.6 Hz, 1H), 4.50 (d, J = 15.2 Hz, 1H), 3.95-3.85 (m, 2H), 2.35 (s, 3H), 2.29 (s, 3H); 19 F NMR (DMSO-d6) δ -118.15, -118.49.

### Example 49: (S)-N-(1-(3-(methylsulfonyl)phenyl)-1,4,5,7-tetrahydropyrano[3,4-c]pyrazol-4-yl)-5,6,7,8-tetrahydroimidazo[1,5-a]pyridine-1-carboxamide

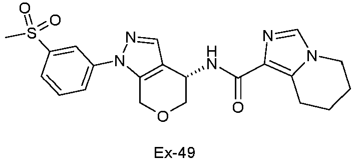

LCMS (ESI) m/z = 442.22 [M+H]+; Chiral HPLC (peak-1): 99.8% (*t*_{R} 5.63 min); UPLC (Method A): 99.4% (*t*_{R} = 11.22 min); 1H NMR (400 MHz, DMSO-d6): 8.06-8.05 (m, 1H), 7.90 (td, J = 3.6 Hz, J = 1.6 Hz, 1H), 7.84-7.77 (m, 2H), 7.76 (s, 1H), 7.54 (s, 1H), 7.48 (d, J = 8.8 Hz, 1H), 5.09-5.05 (m, 1H), 5.01 (s, 2H), 3.99 (t, J = 5.6 Hz, 2H), 3.86 (d, J = 4.0 Hz, 2H), 3.32 (s, 3H), 2.99 (t, J = 6.8 Hz, 2H), 1.88-1.83 (m, 2H), 1.79-1.75 (m, 2H).

### Example 50: (S)-N-(1-(2-chloro-3-(trifluoromethyl)phenyl)-1,4,5,7-tetrahydropyrano[3,4-c]pyrazol-4-yl)-5,6,7,8-tetrahydroimidazo[1,5-a]pyridine-1-carboxamide

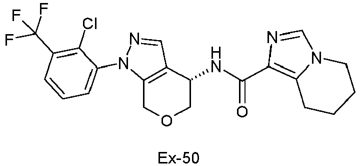

LCMS (ESI) m/z = 466.23 [M+H]+; Chiral HPLC (Method AR) (peak-1): 99.7% (*t*_{R} 2.14 min); UPLC (Method A): 98.8% (*t*_{R} = 8.80 min); 1H NMR (400 MHz, DMSO-d6): 8.05 (d, J = 7.2 Hz, 1H), 7.91 (d, J = 7.2 Hz, 1H), 7.74 (t, J = 6.8 Hz, 2H), 7.56 (s, 1H), 7.44 (d, J = 8.4 Hz, 1H), 5.09-5.05 (m, 1H), 4.58 (dd, J = 15.6 Hz, J = 14.8 Hz, 2H), 3.99 (t, J = 6.0 Hz, 2H), 3.83 (t, J = 3.6 Hz, 2H), 2.99 (t, J = 6.4 Hz, 2H), 1.88-1.85 (m, 2H), 1.79-1.75 (m, 2H).

### Example 51: (S)-N-(1-(2-chlorophenyl)-1,4,5,7-tetrahydropyrano[3,4-c]pyrazol-4-yl)-5,6,7,8-tetrahydroimidazo[1,5-a]pyridine-1-carboxamide

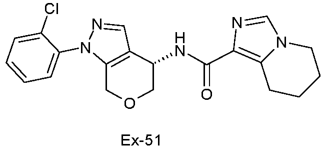

LCMS (ESI) m/z = 398.16 [M+H]+; Chiral HPLC (peak-1): 99.9% (*t*_{R} 19.3 min); UPLC (Method D): 99.6% (*t*_{R} = 11.06 min); 1H NMR (400 MHz, DMSO-d6): 7.72-7.69 (m, 2H), 7.59-7.50 (m, 4H), 7.43 (d, J = 8.4 Hz, 1H), 5.07-5.05 (m, 1H), 4.57 (dd, J = 15.6 Hz, J = 14.8 Hz, 2H), 4.00 (t, J = 5.6 Hz, 2H), 3.85 (t, J = 3.6 Hz, 2H), 3.00 (t, J = 6.4 Hz, 2H), 1.87-1.85 (m, 2H), 1.80-1.76 (m, 2H).

### Example 52: (S)-N-(1-(o-tolyl)-1,4,5,7-tetrahydropyrano[3,4-c]pyrazol-4-yl)-5,6,7,8-tetrahydroimidazo[1,5-a]pyridine-1-carboxamide

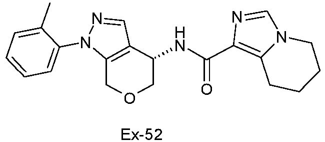

LCMS (ESI) m/z = 378.21 [M+H]+; Chiral HPLC (peak-2): 99.9% (*t*_{R} 21.04 min); UPLC (Method A): 99.4% (*t*_{R} = 10.43 min); 1H NMR (400 MHz, DMSO-d6): 7.63 (s, 1H), 7.56 (s, 1H), 7.44 (d, J = 8.4 Hz, 1H), 7.42-7.40 (m, 2H), 7.35-7.30 (m, 2H), 5.07-5.03 (m, 1H), 4.53 (dd, J = 14.8 Hz, J = 4.8 Hz, 2H), 4.00 (t, J = 5.6 Hz, 2H), 3.82 (d, J = 3.6 Hz, 2H), 3.00 (t, J = 6.4 Hz, 2H), 2.08 (s, 3H), 1.89-1.83 (m, 2H), 1.79-1.73 (m, 2H).

### Example 53: (S)-N-(1-(2-chloro-5-methylphenyl)-1,4,5,7-tetrahydropyrano[3,4-c]pyrazol-4-yl)-5,6,7,8-tetrahydroimidazo[1,5-a]pyridine-1-carboxamide

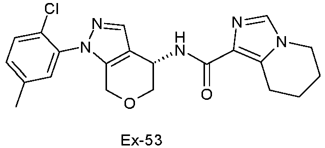

LCMS (ESI) m/z = 412.19 [M+H]+; Chiral HPLC (peak-2): 99.6% (*t*_{R} 14.27 min); UPLC (Method F): 99.8% (*t*_{R} = 7.30 min); 1H NMR (400 MHz, DMSO-d6): 7.68 (s, 1H), 7.57 (t, J = 4.4 Hz, 2H), 7.44-7.38 (m, 3H), 5.08-5.06 (m, 1H), 4.58 (dd, J = 14.8 Hz, J = 10.8 Hz, 2H), 4.01 (t, J = 5.2 Hz, 2H), 3.83 (td, J = 11.6 Hz, J = 3.2 Hz, 2H), 3.01 (t, J = 5.6 Hz, 2H), 2.37 (s, 3H), 1.88-1.86 (m, 2H), 1.79-1.78 (m, 2H).

### Example 54: (S)-N-(1-(2-methoxyphenyl)-1,4,5,7-tetrahydropyrano[3,4-c]pyrazol-4-yl)-5,6,7,8-tetrahydroimidazo[1,5-a]pyridine-1-carboxamide

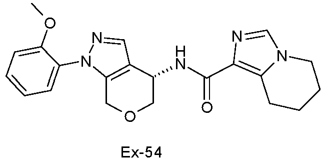

LCMS (ESI) m/z = 394.22 [M+H]+; Chiral HPLC (peak-1): 99.9% (*t*_{R} 5.40 min); UPLC (Method C): 99.8% (*t*_{R} = 8.67 min); 1H NMR (400 MHz, DMSO-d6): 7.61 (s, 1H), 7.55 (s, 1H), 7.45 (t, J = 8.8 Hz, 1H), 7.36 (t, J = 6.4 Hz, 2H), 7.21 (d, J = 8.0 Hz, 1H), 7.05 (t, J = 7.2 Hz, 1H), 5.06-5.02 (m, 1H), 4.52 (dd, J = 14.8 Hz, J = 6.8 Hz, 2H), 3.99 (t, J = 5.6 Hz, 2H), 3.83 (s, 5H), 2.99 (t, J = 6.4 Hz, 2H), 1.88-1.83 (m, 2H), 1.79-1.75 (m, 2H).

### Example 55: (S)-N-(1-(2-(trifluoromethyl)phenyl)-1,4,5,7-tetrahydropyrano[3,4-c]pyrazol-4-yl)-5,6,7,8-tetrahydroimidazo[1,5-a]pyridine-1-carboxamide

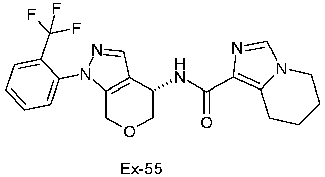

LCMS (ESI) m/z = 432.20 [M+H]+; Chiral HPLC (peak-2): 99.9% (*t*_{R} 7.72 min); UPLC (Method C): 99.3% (*t*_{R} = 9.30 min); 1H NMR (400 MHz, DMSO-d6): 7.96 (d, J = 7.6 Hz, 1H), 7.85 (t, J = 7.2 Hz, 1H), 7.77 (t, J = 7.6 Hz, 1H), 7.66 (s, 1H), 7.64 (d, J = 10.4 Hz, 1H), 7.56 (s, 1H), 7.41 (d, J = 8.4 Hz, 1H), 5.06-5.04 (m, 1H), 4.55 (dd, J = 15.6 Hz, J = 14.8 Hz, 2H), 4.00 (t, J = 6.0 Hz, 2H), 3.82 (dd, J = 11.6 Hz, J = 3.6 Hz, 2H), 3.00 (t, J = 6.4 Hz, 2H), 1.92-1.85 (m, 2H), 1.80-1.76 (m, 2H); 19F NMR (DMSO-d6) -58.62.

### Example 56: (S)-N-(1-(3-methoxyphenyl)-1,4,5,7-tetrahydropyrano[3,4-c]pyrazol-4-yl)-5,6,7,8-tetrahydroimidazo[1,5-a]pyridine-1-carboxamide

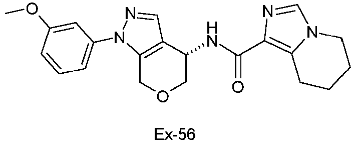

LCMS (ESI) m/z = 394.22 [M+H]+; Chiral HPLC (peak-1): 99.7% (*t*_{R} 5.52 min); UPLC (Method F): 99.1% (*t*_{R} = 6.03 min); 1H NMR (400 MHz, DMSO-d6): 7.66 (s, 1H), 7.54 (s, 1H), 7.45-7.39 (m, 2H), 7.07-7.04 (m, 2H), 6.94 (dd, J = 8.0 Hz, J = 2.0 Hz, 1H), 5.06-5.04 (m, 1H), 4.93 (s, 2H), 3.99 (t, J = 5.6 Hz, 2H), 3.84 (d, J = 3.6 Hz, 2H), 3.81 (s, 3H), 2.99 (t, J = 6.4 Hz, 2H), 1.86-1.85 (m, 2H), 1.78-1.75 (m, 2H).

### Example 57: (S)-N-(1-(3-(trifluoromethoxy)phenyl)-1,4,5,7-tetrahydropyrano[3,4-c]pyrazol-4-yl)-5,6,7,8-tetrahydroimidazo[1,5-a]pyridine-1-carboxamide

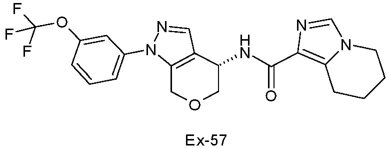

LCMS (ESI) m/z = 448.20 [M+H]+; Chiral HPLC (peak-1): 99.7% (*t*_{R} 7.41 min); UPLC (Method F): 95.6% (*t*_{R}= 7.35 min); 1H NMR (400 MHz, DMSO-d6): 7.73 (s, 1H), 7.66 (t, J = 8.4 Hz, 1H), 7.56-7.51 (m, 3H), 7.47 (d, J = 8.8 Hz, 1H), 7.38 (d, J = 8.0 Hz, 1H), 5.08-5.04 (m, 1H), 4.98 (s, 2H), 3.99 (t, J = 5.6 Hz, 2H), 3.84 (d, J = 3.6 Hz, 2H), 2.99 (t, J = 6.4 Hz, 2H), 1.88-1.83 (m, 2H), 1.79-1.74 (m, 2H).

### Example 58: (S)-N-(1-(2-fluoro-6-methylphenyl)-1,4,5,7-tetrahydropyrano[3,4-c]pyrazol-4-yl)-5,6,7,8-tetrahydroimidazo[1,5-a]pyridine-1-carboxamide

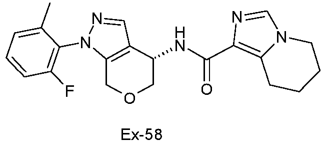

LCMS (ESI) m/z = 396.21 [M+H]+; Chiral HPLC (peak-2): 99.9% (*t*_{R} 18.15 min); UPLC (Method D): 99.4% (*t*_{R} = 11.30 min); 1H NMR (400 MHz, DMSO-d6): 7.69 (s, 1H), 7.56 (s, 1H), 7.56-7.45 (m, 2H), 7.31-7.26 (m, 2H), 5.08-5.04 (m, 1H), 4.57 (dd, J = 16.0 Hz, J = 14.8 Hz, 2H), 4.00 (t, J = 5.6 Hz, 2H), 3.82 (d, J = 3.6 Hz, 2H), 3.00 (t, J = 6.8 Hz, 2H), 2.06 (s, 3H), 1.87-1.85 (m, 2H), 1.80-1.74 (m, 2H).

### Example 59: (S)-N-(1-(2,6-difluorophenyl)-1,4,5,7-tetrahydropyrano[3,4-c]pyrazol-4-yl)-5,6,7,8-tetrahydroimidazo[1,5-a]pyridine-1-carboxamide

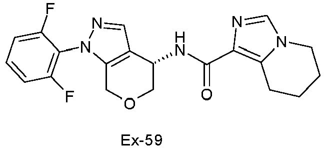

LCMS (ESI) m/z = 400.25 [M+H]+; Chiral HPLC (peak-2): 99.9% (*t*_{R} 6.72 min); UPLC (Method A): 99.8% (*t*_{R} = 8.83 min); 1H NMR (400 MHz, DMSO-d6): 7.75 (s, 1H), 7.67-7.63 (m, 1H), 7.55 (s, 1H), 7.48 (d, J = 8.4 Hz, 1H), 7.40 (t, J = 8.8 Hz, 2H), 5.08-5.04 (m, 1H), 4.63 (d, J = 14.8 Hz, 1H), 4.53 (d, J = 14.8 Hz, 1H), 4.00 (t, J = 5.6 Hz, 2H), 3.83 (d, J = 4.0 Hz, 2H), 3.00 (t, J = 6.4 Hz, 2H), 1.89-1.83 (m, 2H), 1.79-1.73 (m, 2H); 19F NMR (DMSO-d6) -120.09.

### Example 60: (S)-N-(1-(2-chloro-6-methoxyphenyl)-1,4,5,7-tetrahydropyrano[3,4-c]pyrazol-4-yl)-5,6,7,8-tetrahydroimidazo[1,5-a]pyridine-1-carboxamide

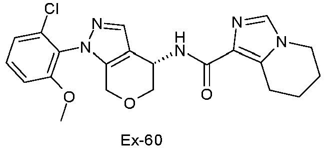

LCMS (ESI) m/z = 428.19 [M+H]+; Chiral HPLC (peak-2): 99.7% (*t*_{R} 2.63 min); UPLC (Method A): 98.8% (*t*_{R} = 9.57 min); 1H NMR (400 MHz, DMSO-d6): 7.64 (s, 1H), 7.56 (d, J = 4.0 Hz, 1H), 7.53 (t, J = 7.6 Hz, 1H), 7.38-7.33 (m, 1H), 7.26-7.22 (m, 2H), 5.04-5.02 (m, 1H), 4.48-4.33 (m, 2H), 4.00 (t, J = 5.2 Hz, 2H), 3.83-3.78 (m, 5H), 3.00 (t, J = 6.4 Hz, 2H), 1.89-1.84 (m, 2H), 1.80-1.76 (m, 2H).

### Example 61: (S)-N-(1-(2-methoxy-6-methylphenyl)-1,4,5,7-tetrahydropyrano[3,4-c]pyrazol-4-yl)-5,6,7,8-tetrahydroimidazo[1,5-a]pyridine-1-carboxamide

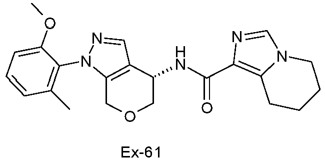

LCMS (ESI) m/z = 408.4 [M+H]+; Chiral HPLC (peak-1): 99.9% (*t*_{R} 10.77 min); UPLC (Method H): 99.9% (*t*_{R} = 8.33 min); 1H NMR (400 MHz, DMSO-d6): 7.60 (d, J = 2.4 Hz, 1H), 7.56 (d, J = 6.0 Hz, 1H), 7.42-7.35 (m, 2H), 7.04 (t, J = 7.2 Hz, 1H), 6.95 (d, J = 7.6 Hz, 1H), 5.06-5.02 (m, 1H), 4.46-4.30 (m, 2H), 4.00 (t, J = 5.2 Hz, 2H), 3.82-3.79 (m, 2H), 3.76 (s, 1.5H), 3.73 (s, 1.5H), 3.00 (t, J = 6.4 Hz, 2H), 1.97 (s, 1.5H), 1.91 (s, 1.5H), 1.86-1.85 (m, 2H), 1.80-1.74 (m, 2H).

### Example 62: (S)-N-(1-(2-chloro-3-hydroxyphenyl)-1,4,5,7-tetrahydropyrano[3,4-c]pyrazol-4-yl)-5,6,7,8-tetrahydroimidazo[1,5-a]pyridine-1-carboxamide

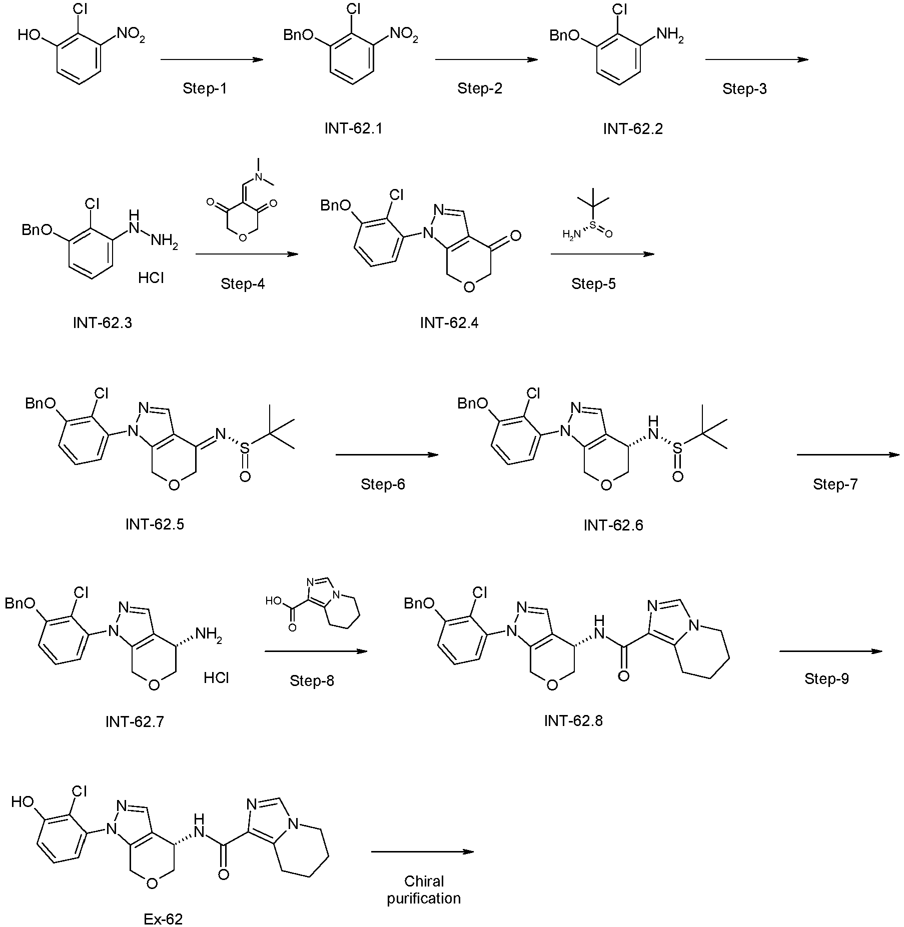

Step-1: Synthesis of 1-(benzyloxy)-2-chloro-3-nitrobenzene (INT-62.1): To a stirred solution of 2-chloro-3-nitrophenol (10.0 g, 57.6 mmol) in DMF (100 mL) were added K₂CO₃ (23.9 g, 3 eq., 173 mmol) and dropwise benzyl bromide (8.21 mL, 69.1 mmol) at rt. The reaction was stirred at 80 °C for 12 h. After completion, the reaction mixture was diluted with ice water and stir for 1 h. The reaction mass filtered through sinter funnel and washed with pentane and dried under vacuum to afford the crude compound INT-62.1 (13.5 g, 89%) as brown solid. LCMS (ESI) m/z = 262.14 (M-H)⁺.

Step-2: Synthesis of 3-(benzyloxy)-2-chloroaniline (INT 62.2): To a stirred solution of INT-62.1 (9.0 g, 34.1 mmol) in EtOH (50 mL) and H₂O (15 mL) was added NH₄Cl (9.13 g, 171 mmol) and Fe-powder (9.53 g, 171 mmol) at rt. The reaction mixture was heated at 70 °C for 4 h. After completion, the reaction mass was filtered through celite bed and washed with DCM. The filtrate was washed with cold water, brine and the organic layer was dried over sodium sulfate, filtered and concentrated to get pure INT-62.2 (7.0 g, 88%) as blackish sticky oil. LCMS (ESI) m/z = 234.0 [M+H]⁺.

Step-3: Synthesis of (3-(benzyloxy)-2-chlorophenyl)hydrazine hydrochloride (INT-62.3): Following the experiment procedure of **INT-14.1,** the reaction was performed on 8.0 g scale to get INT-62.3 (8.0 g, 94%) as white solid. LCMS (ESI) m/z = 249.12 [M+H]⁺.

Step-4: Synthesis of 1-(3-(benzyloxy)-2-chlorophenyl)-1,7-dihydropyrano[3,4-c]pyrazol-4(5H)-one (INT-62.4): Following the experiment procedure of **INT-14.2,** the reaction was performed on 2.0 g scale to get INT-62.4 (0.65 g, 23%) as a yellow solid. LCMS (ESI) m/z = 355.18 [M+H]⁺.

Step-5: Synthesis of (S,Z)-N-(1-(3-(benzyloxy)-2-chlorophenyl)-1,7-dihydropyrano[3,4-c]pyrazol-4(5H)-ylidene)-2-methylpropane-2-sulfinamide (INT-62.5): Following the experiment procedure of **INT-14.3,** the reaction was performed on 2.0 g scale to get INT-62.5 (0.9 g, 35%) as a yellow solid. LCMS (ESI) m/z = 458.28 [M+H]⁺.

Step-6: Synthesis of (S)-N-((S)-1-(3-(benzyloxy)-2-chlorophenyl)-1,4,5,7-tetrahydropyrano[3,4-c]pyrazol-4-yl)-2-methylpropane-2-sulfinamide (INT-62.6): Following the experiment procedure of **INT-14.4,** the reaction was performed on 0.80 g scale to get INT-62.6 (0.78 g, 97%) as a yellow solid. LCMS (ESI) m/z = 459.40 [M+H]⁺.

Step-7: Synthesis of (S)-1-(3-(benzyloxy)-2-chlorophenyl)-1,4,5,7-tetrahydropyrano[3,4-c]pyrazol-4-amine hydrochloride (INT-62.7): Following the experiment procedure of **INT-14.5,** the reaction was performed on 0.78 g scale to get INT-62.7 (0.60 g, 99%) as an off white solid. LCMS (ESI) m/z = 356.80 [M+H]⁺.

Step-8: Synthesis of (S)-N-(1-(3-(benzyloxy)-2-chlorophenyl)-1,4,5,7-tetrahydropyrano[3,4-c]pyrazol-4-yl)-5,6,7,8-tetrahydroimidazo[1,5-a]pyridine-1-carboxamide (INT-62.8): Following the experiment procedure of **Ex-14,** the reaction was performed on 0.40 g scale with 5,6,7,8-tetrahydroimidazo[1,5-a]pyridine-1-carboxylic acid to get an enantiomeric mixture INT-62.8 (0.40 g, 71%) as off white solid compound. LCMS (ESI) m/z = 504.13 [M+H]⁺.

Step-9: Synthesis of *(S)-N-(1-(2-chloro-3-hydroxyphenyl)-1,4,5,7-tetrahydropyrano[3,4-c]pyrazol-4-yl)-5,6,7,8-tetrahydroimidazo[1,5-a]pyridine-1-carboxamide* (Ex-62): To a stirred solution of INT-62.8 (0.18 g, 0.36 mmol) in EtOH (18 mL) was added 10% Pd/C (0.095 g, 50% w/w) at rt and added 1-2 drops of conc. HCI at rt. The reaction mass was flushed with N₂ and refilled with H₂ atmosphere of bladder pressure at rt. The reaction was stirred at rt for 16 h. After completion, the H₂ was discharged and the reaction mass was diluted by DCM and filtered through celite bed. The celite bed was washed with DCM and collective organic layer was dried over sodium sulfate, filtered and concentrated under reduced pressure to get crude product. The resulting crude material was purified with silica gel column chromatography using a gradient of 1-5% DCM in MeOH to get enantiomeric mixture [26:74] of EX-62 (85 mg) as an off white solid compound. Further, Ex-62 was subjected for separation and purification by chiral HPLC to afford pure Ex-62 (12 mg, 8.1%) as a white solid [Column Name: LUX AMYLOSE-1 (4.6*250) mm,5u; Mobile Phase: CO₂/0.2% TEA in IPA= (60:40) (v/v); Flow rate: 3 mL/min; Flow mode: Isocratic; Column Temperature: 35°C; ABPR Pressure: 1500psi, *t*_{R} (peak-1) 2.85 min. (26%), *t*_{R} (peak-2) 3.3 min. (74%)]. LCMS (ESI) m/z = 414.33 [M+H]⁺; Chiral HPLC (peak-2): 99.8% (*t*_{R} 3.33 min); UPLC (Method A): 99.9% (*t*_{R} 9.38 min); 1H NMR (400 MHz, DMSO-d6): 10.71 (br, s, 1H), 7.64 (s, 1H), 7.55 (s, 1H), 7.40 (d, J = 8.4 Hz, 1H), 7.28 (t, J = 8.4 Hz, 1H), 7.11 (dd, J = 8.4 Hz, J = 0.4 Hz, 1H), 6.95 (dd, J = 8.0 Hz, J = 0.3 Hz, 1H), 5.06-5.02 (m, 1H), 4.52 (dd, J = 14.4 Hz, J = 11.2 Hz, 2H), 3.99 (t, J = 5.6 Hz, 2H), 3.82 (td, J = 11.6 Hz, J = 3.6 Hz, 2H), 2.99 (t, J = 6.4 Hz, 2H), 1.88-1.83 (m, 2H), 1.79-1.75 (m, 2H).

### Example 63: (S)-N-(1-(2-chloro-3-(2-(dimethylamino)ethoxy)phenyl)-1,4,5,7-tetrahydropyrano[3,4-c]pyrazol-4-yl)-5,6,7,8-tetrahydroimidazo[1,5-a]pyridine-1-carboxamide

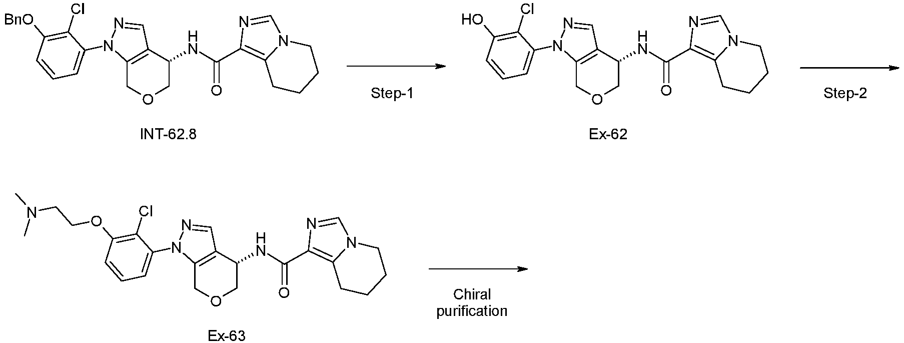

Step-1: Synthesis of (S)-N-(1-(2-chloro-3-hydroxyphenyl)-1,4,5,7-tetrahydropyrano[3,4-c]pyrazol-4-yl)-5,6,7,8-tetrahydroimidazo[1,5-a]pyridine-1-carboxamide (Ex-62): To a stirred solution of INT-62.8 (1.2 g, 2.38 mmol) in DCM (24 mL) was added dropwise BBr₃ (0.23 mL, 2.38 mmol) in DCM (4 mL) at 0 °C. The reaction was stirred at 0 °C for 30 mins. After completion, the reaction mass was quenched with MeOH at 0 °C and stir for 30 mins at rt. Further, the reaction mixture was concentrated under vacuum to afford the crude material. The crude material was purified with silica gel column chromatography using gradient 5-10% MeOH in DCM to get Ex-62 (0.5 g, 51%) as a brown solid. LCMS (ESI) m/z = 414.28 [M+H]⁺

Step-2: Synthesis of (S)-N-(1-(2-chloro-3-(2-(dimethylamino)ethoxy)phenyl)-1,4,5,7-tetrahydropyrano[3,4-c]pyrazol-4-yl)-5,6,7,8-tetrahydroimidazo[1,5-a]pyridine-1-carboxamide (Ex-63): To a stirred solution of Ex-62 (0.20 g, 0.48 mmol) and 2-chloro-N,N-dimethylethan-1-amine hydrochloride (0.13 g, 1.21 mmol) in dry DMF (2 mL) were added K₂CO₃ (0.27 g, 1.93 mmol) in a screw-capped vial at rt. The vial was closed with a cap and the solution was stirred at 65 °C for 16 h. After completion, the reaction mass distilled under vacuum and purified with silica gel column chromatography using gradient 5-20% MeOH in DCM using 1% trimethylamine in DCM to get enantiomeric mixture [25:75] of EX-63 (160 mg) as an off white solid compound. Further, Ex-463 was subjected for separation and purification by chiral HPLC to afford pure **Ex-63** (53 mg, 22.6%) as a white solid [Column Name: Chiralpak-IG (4.6 X 250)5um; Mobile Phase: 0.1% AH in MeOH / ACN = 50:50 (v/v); Flow Rate: 1.0 ml/min, Temp: 25°C; Flow mode: Isocratic; *t*_{R} (peak-1) 7.7 min. (25%), *t*_{R} (peak-2) 10.2 min. (75%)]. LCMS (ESI) m/z = 485.29 [M+H]⁺; Chiral HPLC (peak-2): 99.9% (*t*_{R} 10.27 min); UPLC (Method I): 95.0% (*t*_{R} 9.40 min); 1H NMR (400 MHz, DMSO-d6): 7.67 (s, 1H), 7.56 (s, 1H), 7.47-7.39 (m, 2H), 7.35 (dd, J = 8.4 Hz, J = 0.8 Hz, 1H), 7.12 (dd, J = 7.6 Hz, J = 1.2 Hz, 1H), 5.07-5.03 (m, 1H), 4.53 (dd, J = 14.8 Hz, J = 10.8 Hz, 2H), 4.22 (t, J = 6.0 Hz, 2H), 3.99 (t, J = 5.6 Hz, 2H), 3.83 (td, J = 11.6 Hz, J = 4.4 Hz, 2H), 3.00 (t, J = 6.4 Hz, 2H), 2.71 (t, J = 5.6 Hz, 2H), 2.26 (s, 6H), 1.91-1.83 (m, 2H), 1.80-1.76 (m, 2H).

### Example 64: (S)-N-(1-(2-chloro-3-(2-(dimethylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide

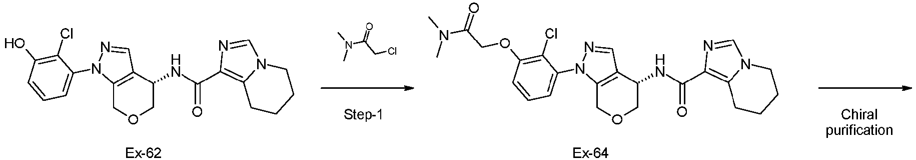

Step-1: Synthesis of (S)-N-(1-(2-chloro-3-(2-(dimethylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide (Ex-64): To a stirred solution of Ex-62 (0.25 g, 0.60 mmol) and 2-chloro-N,N-dimethylacetamide (0.15 mL, 1.51 mmol) in dry DMF (2 mL) were added NaH 60%w/w (0.06 g, 1.51 mmol) in a screw-capped vial at 0 °C. The vial was closed with a cap and the solution was stirred at rt for 16 h. After completion, the reaction mass quenched in MeOH and distilled under vacuum to get the crude product. The crude product was purified with silica gel column chromatography using gradient 2-5% MeOH in DCM to get enantiomeric mixture [23:69] of EX-64 (53 mg) as an off white solid compound. Further, Ex-64 was subjected for separation and purification by chiral HPLC to afford pure Ex-64 (21 mg, 6.97%) as a white solid [Column Name: Chiralpak-lG (4.6 X 250)5um; Mobile Phase: 0.1% Ammonium Hydroxide in MeOH:ACN = 50:50 (v/v); Flow Rate: 1.0 ml/min, Temp: 25°C; Flow mode: Isocratic; *t*_{R} (peak-1) 7.76 min. (23%), *t*_{R} (peak-2) 9.48 min. (69%)]. LCMS (ESI) m/z = 499.29 [M+H]⁺; Chiral HPLC (peak-2): 99.4% (*t*_{R} 9.50 min); UPLC (Method J): 99.5% (*t*_{R} 8.21 min); 1H NMR (400 MHz, DMSO-d6): 7.67 (s, 1H), 7.56 (s, 1H), 7.43-7.37 (m, 2H), 7.16 (d, J = 8.4 Hz, 1H), 7.11 (dd, J = 7.6 Hz, J = 1.2 Hz, 1H), 5.07-5.03 (m, 3H), 4.53 (dd, J = 14.8 Hz, J = 10.8 Hz, 2H), 3.99 (t, J = 5.6 Hz, 2H), 3.83 (td, J = 11.6 Hz, J = 3.6 Hz, 2H), 3.01-2.98 (m, 5H), 2.86 (s, 3H), 1.96-1.83 (m, 2H), 1.80-1.76 (m, 2H).

### Example 65: (S)-N-(1-(2-chloro-3-(2-(methylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide

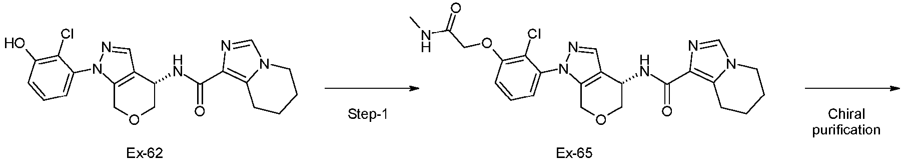

Step-1: Synthesis of ((S)-N-(1-(2-chloro-3-(2-(methylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide (Ex-65): To a stirred solution of Ex-62 (0.2 g, 0.48 mmol) in dry DMF (2.0 mL) were added Cs₂CO₃ (0.47 g, 1.45 mmol), KI (0.02 g, 0.12 mmol) and 2-chloro-N-methylacetamide (0.10 g, 0.97 mmol) at rt. The reaction was stirred at 100 °C for 6 h. After completion, the reaction mixture was concentrated under vacuum and purified with silica gel column chromatography using 2-6% MeOH in DCM to get the crude product. The crude product was purified with silica gel column chromatography using gradient 2-5% MeOH in DCM to get enantiomeric mixture [25:75] of EX-65 (120 mg) as an off white solid compound. Further, Ex-65 was subjected for separation and purification by chiral HPLC to afford pure **Ex-65** (55 mg, 23.4%) as a white solid [Column Name: Chiralpak AD-H (250*4.6) mm, 5um; Mobile Phase: CO₂:0.1%IPamine in MeOH (60:40); Flow Rate: 3.0 mL/min; Column Temperature: 40 °C; *t*_{R} (peak-1) 4.24 min. (25%), *t*_{R} (peak-2) 8.01 min. (75%)]. LCMS (ESI) m/z = 485.25 [M+H]⁺; Chiral HPLC (peak-2): 99.3% (*t*_{R} 8.02 min); UPLC (Method H): 95.7% (*t*_{R} 7.56 min); 1H NMR (400 MHz, DMSO-d6): 7.97 (q, J = 3.6 Hz, 1H), 7.67 (s, 1H), 7.56 (s, 1H), 7.44 (t, J = 8.0 Hz, 2H), 7.17 (t, J = 9.6 Hz, 2H), 5.07-5.04 (m, 1H), 4.68 (s, 2H), 4.54 (dd, J = 14.4 Hz, J = 11.6 Hz, 2H), 3.99 (t, J = 5.6 Hz, 2H), 3.83 (td, J = 12.0 Hz, J = 4.0 Hz, 2H), 2.98 (t, J = 6.4 Hz, 2H), 2.66 (d, J = 8.4 Hz, 3H), 1.88-1.83 (m, 2H), 1.78-1.76 (m, 2H).

### Example 66: (S)-N-(1-(3-(2-acetamidoethoxy)-2-chlorophenyl)-1,4,5,7-tetrahydropyrano[3,4-c]pyrazol-4-yl)-5,6,7,8-tetrahydroimidazo[1,5-a]pyridine-1-carboxamide

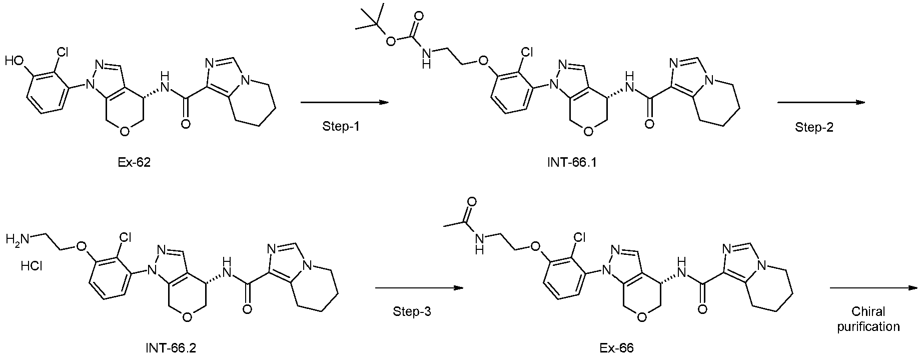

Step-1: Synthesis of tert-butyl (S)-(2-(2-chloro-3-(4-(5,6,7,8-tetrahydroimidazo[1,5-a]pyridine-1-carboxamido)-4,7-dihydropyrano [3,4-c]pyrazol-1(5H)-yl)phenoxy)ethyl)carbamate (INT-66.1): To a stirred solution of Ex-62 (0.4 g, 0.97 mmol) in dry DMF (4 mL) were added Cs₂CO₃ (0.95 g, 2.9 mmol), KI (4 mg, 0.024 mmol) and tert-butyl (2-chloroethyl)carbamate (0.35 g, 1.93 mmol) at rt. The reaction was stirred at 80 °C for 16 h. After completion, the reaction mixture was concentrated under vacuum and purified with silica gel column chromatography using 2-6% MeOH in DCM to get pure INT-18.10 (0.35 g, 65%) as a yellow sticky mass. LCMS (ESI) m/z = 557.21 [M+H]⁺.

Step-2: Synthesis of (S)-N-(1-(3-(2-aminoethoxy)-2-chlorophenyl)-1,4,5,7-tetrahydropyrano[3,4-c]pyrazol-4-yl)-5,6,7,8-tetrahydroimidazo[1,5-a]pyridine-1-carboxamide hydrochloride (INT-66.2): To a stirred solution of INT-66.1 (0.350 g, 0.63 mmol) in dry DCM (7 mL) was added 4 M HCI in dioxane (3.5 mL) at rt. The reaction was stirred at rt for 2 h. After completion, the reaction mixture was concentrated under vacuum to get INT-66.2 (0.300 g, 97%) as a yellow solid. LCMS (ESI) m/z = 457.14 [M+H]⁺.

Step-3: Synthesis of (S)-N-(1-(3-(2-acetamidoethoxy)-2-chlorophenyl)-1,4,5,7-tetrahydropyrano[3,4-c]pyrazol-4-yl)-5,6,7,8-tetrahydroimidazo[1,5-a]pyridine-1-carboxamide (Ex-66): To a stirred solution of INT-66.2 (0.30 g, 0.66 mmol) in MeOH (3 mL) and H₂O (3 mL) were added TEA (0.17 g, 1.64 mmol) and dropwise acetic anhydride (0.10 g, 0.99 mmol) (dissolved in 2.0 ml MeOH) at rt. The reaction mass was stirred for 2 h at rt. After completion, the reaction mixture was concentrated under vacuum and purified with silica gel column chromatography using 2-6% MeOH in DCM to get enantiomeric mixture [30:70] of EX-66 (220 mg) as an off white solid compound. Further, Ex-66 was subjected for separation and purification by chiral HPLC to afford pure Ex-66 (100 mg, 30.5%) as a white solid [Column Name: Chiralpak-IG (4.6 X 250)5um; Mobile Phase: 0.1% AH in MeOH/ACN = 70:30 (v:v); Flow Rate: 1.0 ml/min, Temp: 25°C; Flow mode: Isocratic; *t*_{R} (peak-1) 6.57 min. (30%), *t*_{R} (peak-2) 7.71 min. (70%)]. LCMS (ESI) m/z = 499.20 [M+H]⁺; Chiral HPLC (peak-2): 99.9% (*t*_{R} 7.73 min); UPLC (Method J): 99.6% (*t*_{R} 8.22 min); 1H NMR (400 MHz, DMSO-d6): 8.11 (t, J = 4.8 Hz, 1H), 7.66 (s, 1H), 7.56 (s, 1H), 7.45-7.40 (m, 2H), 7.34 (d, J = 7.6 Hz, 1H), 7.12 (d, J = 7.2 Hz, 1H), 5.07-5.03 (m, 1H), 4.53 (dd, J = 14.8 Hz, J = 10.8 Hz, 2H), 4.15 (t, J = 5.6 Hz, 2H), 3.99 (t, J = 5.6 Hz, 2H), 3.83 (td, J = 12.0 Hz, J = 4.0 Hz, 2H), 3.44 (q, J = 5.6 Hz, 2H), 2.99 (t, J = 6.4 Hz, 2H), 1.86-1.83 (m, 2H), 1.83 (s, 3H), 1.79-1.76 (m, 2H).

### Example 67: (S)-N-(1-(2-chloro-3-(2-methoxyethoxy)phenyl)-1,4,5,7-tetrahydropyrano[3,4-c]pyrazol-4-yl)-5,6,7,8-tetrahydroimidazo[1,5-a]pyridine-1-carboxamide

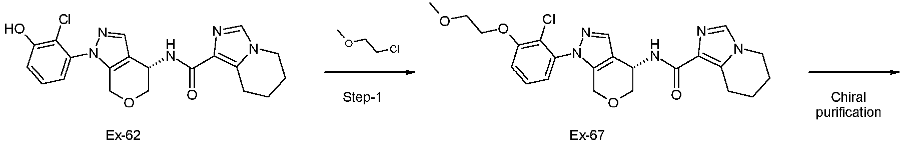

Step-1: Synthesis of (S)-N-(1-(2-chloro-3-(2-methoxyethoxy)phenyl)-1,4,5,7-tetrahydropyrano[3,4-c]pyrazol-4-yl)-5,6,7,8-tetrahydroimidazo[1,5-a]pyridine-1-carboxamide (Ex-67): Following the experiment procedure of **Ex-64,** the reaction was performed on 0.25 g scale to get enantiomeric mixture [20:80] of Ex-67 (150 mg) as an off white solid compound. Further, Ex-67 was subjected for separation and purification by chiral HPLC to afford pure **Ex-67** (60 mg, 21.0%) as a white solid [Column Name: Chiralpak-lG (4.6 X 250), 5um; Mobile Phase: 0.1% Ammonium Hydroxide in MeOH/ACN = 50:50; Flow Rate: 1.0 ml/min, Temp: 25°C; Flow mode: Isocratic; *t*_{R} (peak-1) 7.12 min. (20%), *t*_{R} (peak-2) 9.18 min. (80%)]. LCMS (ESI) m/z = 472.25 [M+H]⁺; Chiral HPLC (peak-2): 99.8% (*t*_{R} 9.22 min); UPLC (Method J): 99.7% (*t*_{R} 9.27 min); 1H NMR (400 MHz, DMSO-d6): 7.66 (s, 1H), 7.56 (s, 1H), 7.45-7.40 (m, 2H), 7.33 (d, J = 8.4 Hz, 1H), 7.11 (dd, J = 4.0 Hz, J = 0.8 Hz, 1H), 5.07-5.03 (m, 1H), 4.53 (dd, J = 14.8 Hz, J = 10.8 Hz, 2H), 4.27 (t, J = 4.4 Hz, 2H), 3.99 (t, J = 5.6 Hz, 2H), 3.83 (td, J = 11.6 Hz, J = 0.4 Hz, 2H), 3.72 (t, J = 4.4 Hz, 2H), 3.33 (d, J = 8.0 Hz, 3H), 2.99 (t, J = 6.4 Hz, 2H), 1.88-1.83 (m, 2H), 1.79-1.74 (m, 2H).

### Example 68: (S)-N-(1-(2-chloro-3-(2-hydroxyethoxy)phenyl)-1,4,5,7-tetrahydropyrano[3,4-c]pyrazol-4-yl)-5,6,7,8-tetrahydroimidazo[1,5-a]pyridine-1-carboxamide

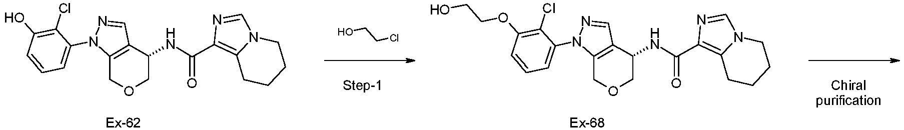

Step-1: Synthesis of (S)-N-(1-(2-chloro-3-(2-hydroxyethoxy)phenyl)-1,4,5,7-tetrahydropyrano[3,4-c]pyrazol-4-yl)-5,6,7,8-tetrahydroimidazo[1,5-a]pyridine-1-carboxamide (Ex-68): Following the experiment procedure of **Ex-64,** the reaction was performed on 0.25 g scale to get enantiomeric mixture [26:74] of Ex-68 (180 mg) as an off white solid compound. Further, Ex-68 was subjected for separation and purification by chiral HPLC to afford pure **Ex-68** (48 mg, 17.3%) as a white solid [Column Name: Chiralpak-lG (4.6 X 250) mm, 5um; Mobile Phase: 0.1% Ammonium Hydroxide in MeOH/ACN = 50:50; Flow Rate: 1.0 ml/min, Temp: 25°C; Flow mode: Isocratic; *t*_{R} (peak-1) 5.48 min. (26%), *t*_{R} (peak-2) 6.54 min. (74%)]. LCMS (ESI) m/z = 458.23 [M+H]⁺; Chiral HPLC (peak-2): 99.9% (*t*_{R} 6.54 min); UPLC (Method K): 97.9% (*t*_{R} 8.99 min); 1H NMR (400 MHz, DMSO-d6): 7.66 (s, 1H), 7.56 (s, 1H), 7.45-7.40 (m, 2H), 7.32 (d, J = 8.4 Hz, 1H), 7.11 (dd, J = 4.0 Hz, J = 0.8 Hz, 1H), 5.07-5.03 (m, 1H), 4.93 (t, J = 5.2 Hz, 1H), 4.53 (dd, J = 14.8 Hz, J = 10.8 Hz, 2H), 4.15 (t, J = 4.8 Hz, 2H), 3.99 (t, J = 5.6 Hz, 2H), 3.83 (t, J = 3.2 Hz, 2H), 3.77 (q, J = 5.2 Hz, 2H), 2.99 (t, J = 6.8 Hz, 2H), 1.87-1.83 (m, 2H), 1.79-1.76 (m, 2H).

### Example 69: Isopropyl (S)-2-chloro-3-(4-(5,6,7,8-tetrahydroimidazo[1,5-a]pyridine-1-carboxamido)-4, 7-dihydropyrano[3, 4-c]pyrazol-1(5H)-yl)benzoate

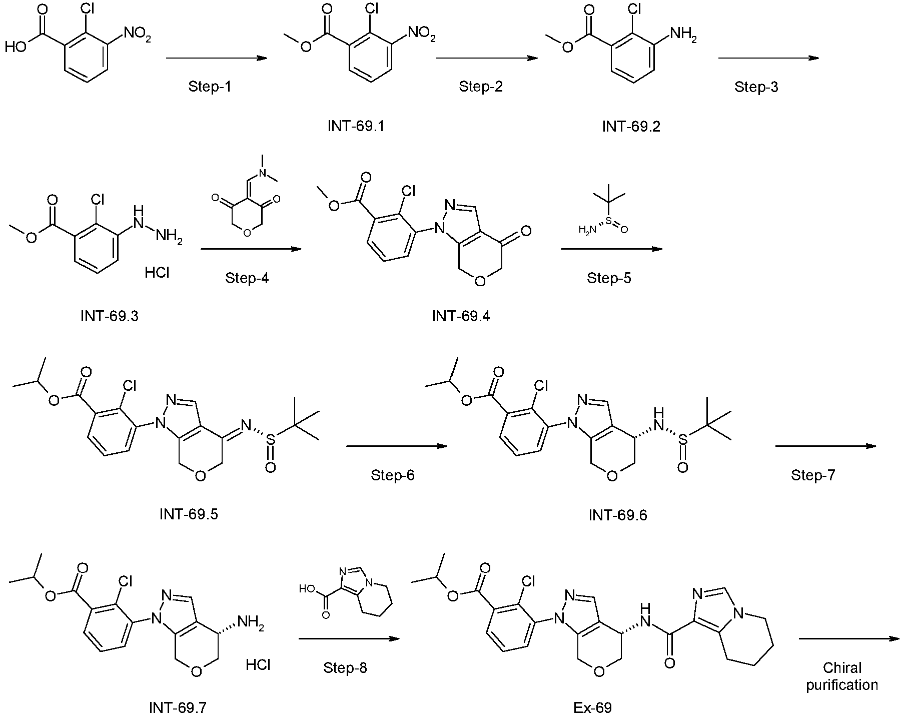

Step-1: Synthesis of Methyl 2-chloro-3-nitrobenzoate (INT-69.1): To a solution of 2-chloro-3-nitrobenzoic acid (10.0 g, 49.6 mmol) in MeOH (100 mL) was added conc. H₂SO₄ (1.35 mL, 24.8 mmol) at rt. The reaction mass was heated at 80 °C for 16 h. After completion, the reaction mass was evaporated under reduced pressure and the crude product was diluted with ice cold water. The organic layer was extracted by EtOAc, collective organic layer was washed with brine solution and dried over sodium sulphate, evaporated and purified with silica gel column chromatography using 10-30% EtOAc in hexanes to get INT-69.1 (10.2 g, 95%) as a white solid. LCMS was not consistent but confirmed by 1H NMR. 1H NMR (400 MHz, DMSO-d6): 8.22 (dd, J = 8.0 Hz, J = 5.6 Hz, 1H), 8.06 (dd, J = 8.0 Hz, J = 1.6 Hz, 1H), 7.66 (t, J = 7.6 Hz, 1H), 3.91 (s, 3H).

Step-2: Synthesis of methyl 3-amino-2-chlorobenzoate (INT 69.2): To a stirred solution of INT-69.1 (5.0 g, 23.2 mmol) in EtOH (50 mL) and water (15 mL) were added Fe-powder (6.48 g, 116.0 mmol) and ammonium chloride (6.2 g, 116.0 mmol) at rt. The resulting reaction mixture was stirred at 70 °C for 4 h. After completion, the precipitate was filtered off and filtrate was concentrated under vacuum to get crude product. The crude product was dissolved in DCM and water and separated organic layer. The organic layer was dried over sodium sulphate and distilled under vacuum to get the INT-69.2 (3.5 g, 81%) as a color less oil. LCMS was not consistent but confirmed by 1H NMR. 1H NMR (400 MHz, DMSO-d6): 7.09 (t, J = 8.0 Hz, 1H), 6.94 (d, J = 8.0 Hz, 1H), 6.86 (d, J = 7.2 Hz, 1H), 5.62 (s, 2H), 3.81 (s, 3H).

Step-3: Synthesis of Methyl 2-chloro-3-hydrazineylbenzoate hydrochloride (INT-69.3): Following the experiment procedure of **INT-14.1,** the reaction was performed on 4.0 g scale to get INT-69.3 (4.0 g, 90%) as white solid. LCMS (ESI) m/z = 201.22 [M+H]⁺.

Step-4: Synthesis of Methyl 2-chloro-3-(4-oxo-4,7-dihydropyrano[3,4-c]pyrazol-1(5H)-yl)benzoate (INT-69.4): Following the experiment procedure of **INT-14.2,** the reaction was performed on 5.0 g scale to get INT-69.4 (0.72 g, 11%) as a yellow solid. LCMS (ESI) m/z = 307.08 [M+H]⁺.

Step-5: Synthesis of isopropyl (S,Z)-3-(4-((tert-butylsulfinyl)imino)-4,7-dihydropyrano[3,4-c]pyrazol-1(5H)-yl)-2-chlorobenzoate (INT-69.5): Following the experiment procedure of **INT-14.3,** the reaction was performed on 1.5 g scale to get INT-69.5 (1.2 g, 56%) as a yellow solid. LCMS (ESI) m/z = 438.07 [M+H]⁺.

Step-6: Synthesis of isopropyl 3-(S)-(4-((S)-tert-butylsulfinyl)amino)-4,7-dihydropyrano[3,4-c]pyrazol-1(5H)-yl)-2-chlorobenzoate (INT-69.6): Following the experiment procedure of **INT-14.4,** the reaction was performed on 1.2 g scale to get INT-69.6 (1.2 g, 99%) as a yellow solid. LCMS (ESI) m/z = 440.11 [M+H]⁺.

Step-7: Synthesis of isopropyl (S)-3-(4-amino-4,7-dihydropyrano[3,4-c]pyrazol-1(5H)-yl)-2-chlorobenzoate hydrochloride (INT-69.7): Following the experiment procedure of **INT-14.5,** the reaction was performed on 1.2 g scale to get INT-69.7 (0.90 g, 98%) as an off white solid. LCMS (ESI) m/z = 337.05 [M+H]⁺.

Step-8: Synthesis of isopropyl (S)-2-chloro-3-(4-(5,6,7,8-tetrahydroimidazo[1,5-a]pyridine-1-carboxamido)-4,7-dihydropyrano[3,4-c]pyrazol-1(5H)-yl)benzoate (Ex-69): Following the experiment procedure of **Ex-14,** the reaction was performed on 0.40 g scale with 5,6,7,8-tetrahydroimidazo[1,5-a]pyridine-1-carboxylic acid to get enantiomeric mixture [18:22] of EX-69 (70 mg) as an off white solid compound. Further, Ex-69 was subjected for separation and purification by chiral HPLC to afford pure **Ex-69** (24 mg, 4.1%) as a white solid [Column Name: Chiralpak-IG (4.6 X 250)5um; Mobile Phase: 0.1% TEA in IPA/ACN = 70:30 (v/v); Flow Rate: 1.0 ml/min, Temp: 25°C; Flow mode: Isocratic; *t*_{R} (peak-1) 5.6 min. (18%), *t*_{R} (peak-2) 7.2 min. (82%)]. LCMS (ESI) m/z = 484.25 [M+H]⁺; Chiral HPLC (peak-2): 99.9% (*t*_{R} 7.25 min); UPLC (Method D): 99.9% (*t*_{R} 8.95 min); 1H NMR (400 MHz, DMSO-d6): 7.88 (dd, J = 11.6 Hz, J = 1.6 Hz, 1H), 7.75 (dd, J = 8.0 Hz, J = 1.6 Hz, 1H), 7.70 (s, 1H), 7.61 (t, J = 8.0 Hz, 1H), 7.55 (s, 1H), 7.43 (d, J = 7.6 Hz, 1H), 5.21-5.15 (m, 1H), 5.07-5.04 (m, 1H), 4.57 (dd, J = 14.8 Hz, J = 14.4 Hz, 2H), 3.99 (t, J = 5.6 Hz, 2H), 3.83 (td, J = 11.6 Hz, J = 3.6 Hz, 2H), 2.99 (t, J = 6.4 Hz, 2H), 1.88-1.83 (m, 2H), 1.78-1.76 (m, 2H), 1.33 (d, J = 6.4 Hz, 6H).

### Example 70: (S)-N-(1-(2-chloro-3-(dimethylcarbamoyl)phenyl)-1,4,5,7-tetrahydropyrano[3,4-c]pyrazol-4-yl)-5,6,7,8-tetrahydroimidazo[1,5-a]pyridine-1-carboxamide

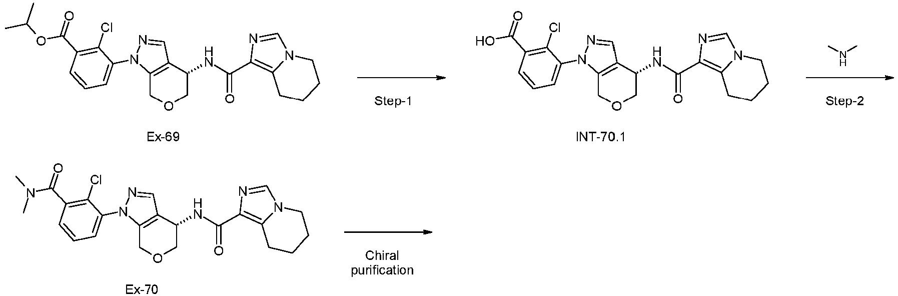

Step-1: Synthesis of (S)-2-chloro-3-(4-(5,6,7,8-tetrahydroimidazo[1,5-a]pyridine-1-carboxamido)-4,7-dihydropyrano[3,4-c]pyrazol-1(5H)-yl)benzoic acid (INT-70.1): To a stirred solution of EX-69 (1.2 g, 2.48 mmol) [NOTE: used enantiomeric enriched mixture of EX-69 for hydrolysis] in MeOH (6 mL) and THF (6 mL) was added LiOH.H₂O (0.42 g, 9.92 mmol) (solution in DM water (1.2 mL)) at rt. The reaction was stirred at rt for 6 h. After completion, the reaction mass was evaporated under reduced pressure and aq. layer was washed with EtOAc. Adjust pH =1-2 with 1 N HCI solution of aq. layer at rt and distilled the reaction mass completely to get INT-70.1 (1.0 g, 91%) as a white solid. LCMS (ESI) m/z = 442.07 [M+H].

Step-2: Synthesis of (S)-N-(1-(2-chloro-3-(dimethylcarbamoyl)phenyl)-1,4,5,7-tetrahydropyrano[3,4-c]pyrazol-4-yl)-5,6,7,8-tetrahydroimidazo[1,5-a]pyridine-1-carboxamide (Ex-70): To a stirred solution of INT-70.1 (0.2 g, 0.45 mmol) and dimethylamine in 2M THF solution (0.08 g, 1.81 mmol) in dry DMF (2 mL) were added HATU (0.26 mg, 0.68 mmol) and DIPEA (0.24 mL, 1.36 mmol) at 0 °C. The reaction was then allowed to stir at rt for 16 h. After completion, the reaction mass was poured on ice water and extracted with EtOAc. The collective organic layer was washed with brine and dried over sodium sulfate and evaporated in vacuum to get the crude compound. The crude product was purified with the silica gel column chromatography using gradient of 2-10% MeOH in DCM to get enantiomeric mixture [22:78] of Ex-70 (170 mg) as an off white solid compound. Further, Ex-70 was subjected for separation and purification by chiral HPLC to afford pure **Ex-70** (90 mg, 42.4%) as a white solid [Column Name: Chiralcel-OX-H (4.6 X 250), 5um; Mobile Phase: 0.1% Ammonium Hydroxide in MeOH = 100%; Flow Rate: 1.0 ml/min, Temp: 25°C; Flow mode: Isocratic; *t*_{R} (peak-1) 11.8 min. (22%), *t*_{R} (peak-2) 13.5 min. (78%)]. LCMS (ESI) m/z = 469.23 [M+H]⁺; Chiral HPLC (peak-2): 98.9% (*t*_{R} 13.50 min); UPLC (Method C): 99.8% (*t*_{R} 9.30 min); 1H NMR (400 MHz, DMSO-d6): 7.69 (s, 1H), 7.62-7.58 (m, 2H), 7.56 (s, 1H), 7.52 (dd, J = 7.2 Hz, J = 2.0 Hz, 1H), 7.43 (d, J = 8.4 Hz, 1H), 5.07-5.04 (m, 1H), 4.57 (dd, J = 14.8 Hz, J = 13.6 Hz, 2H), 3.99 (t, J = 5.6 Hz, 2H), 3.83 (td, J = 11.6 Hz, J = 3.6 Hz, 2H), 3.02 (s, 3H), 2.99 (t, J = 6.4 Hz, 2H), 2.81 (s, 3H), 1.86-1.79 (m, 2H), 1.78-1.76 (m, 2H).

### Example 71: (S)-N-(1-(2-chloro-3-(pyrrolidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide

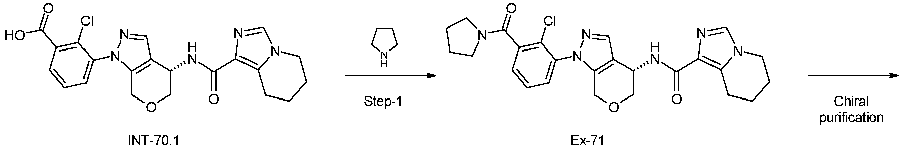

Step-1: Synthesis of (S)-N-(1-(2-chloro-3-(pyrrolidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide (Ex-71): Following the experiment procedure of **Ex-70,** the reaction was performed on 0.15 g scale with pyrrolidine (0.1 g, 4.0 eq.) to get enantiomeric mixture [22:78] of EX-71 (60 mg) as an off white solid compound. Further, Ex-71 was subjected for separation and purification by chiral HPLC to afford pure **Ex-71** (10 mg, 8.3%) as white solid [Column Name: Chiralcel-OX-H (4.6 X 250), 5um; Mobile Phase: 0.1% Ammonium Hydroxide in MeOH = 100%; Flow Rate: 1.0 ml/min, Temp: 25°C; Flow mode: Isocratic; *t*_{R} (peak-1) 15.6 min. (22%), *t*_{R} (peak-2) 18.3 min. (78%)]. LCMS (ESI) m/z = 495.25 [M+H]⁺; Chiral HPLC (peak-2): 99.8% (*t*_{R} 18.42 min); UPLC (Method L): 96.4% (*t*_{R} 8.50 min); 1H NMR (400 MHz, DMSO-d6): 7.69 (s, 1H), 7.62-7.59 (m, 1H), 7.57-7.54 (m, 3H), 7.42 (d, J = 8.4 Hz, 1H), 5.07-5.04 (m, 1H), 4.57 (dd, J = 14.8 Hz, J = 11.6 Hz, 2H), 3.99 (t, J = 5.6 Hz, 2H), 3.83 (td, J = 8.0 Hz, J = 0.4 Hz, 2H), 3.49 (t, J = 6.4 Hz, 2H), 3.12 (t, J = 6.4 Hz, 2H), 2.99 (t, J = 6.4 Hz, 2H), 1.90-1.83 (m, 6H), 1.79-1.76 (m, 2H).

### Example 72: (S)-N-(1-(2-chloro-3-(piperidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide

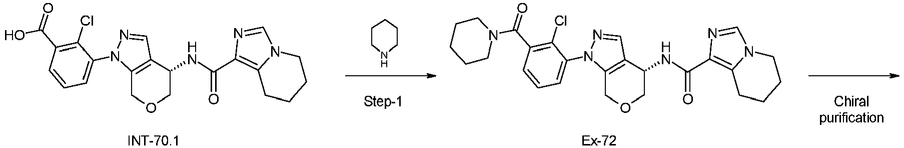

Step-1: Synthesis of ((S)-N-(1-(2-chloro-3-(piperidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide (Ex-72): Following the experiment procedure of **Ex-70,** the reaction was performed on 0.15 g scale with piperidine (0.12 g, 4.0 eq.) to get enantiomeric mixture [22:78] of EX-72 (100 mg) as an off white solid compound. Further, Ex-72 was subjected for separation and purification by chiral HPLC to afford pure **Ex-72** (26 mg, 15.0%) as a white solid [Column Name: Chiralcel-OX-H (4.6 X 250), 5um; Mobile Phase: 0.1% Ammonium Hydroxide in MeOH = 100%; Flow Rate: 1.0 ml/min, Temp: 25°C; Flow mode: Isocratic; *t*_{R} (peak-1) 15.9 min. (22%), *t*_{R} (peak-2) 18.9 min. (78%)]. LCMS (ESI) m/z = 509.28 [M+H]⁺; Chiral HPLC (peak-2): 99.5% (*t*_{R} 19.24 min); UPLC (Method C): 99.5% (*t*_{R} 9.05 min); 1H NMR (400 MHz, DMSO-d6): 7.70 (s, 1H), 7.61-7.56 (m, 3H), 7.51 (dd, J = 7.2 Hz, J = 2.0 Hz, 1H), 7.43 (d, J = 8.4 Hz, 1H), 5.07-5.04 (m, 1H), 4.62 (d, J = 14.8 Hz, 1H), 4.54 (dd, J = 14.4 Hz, J = 4.0 Hz, 1H), 3.99 (t, J = 5.6 Hz, 2H), 3.84 (td, J = 15.2 Hz, J = 3.6 Hz, 2H), 3.71-3.67 (m, 1H), 3.59-3.55 (m, 1H), 3.13 (t, J = 5.6 Hz, 2H), 2.99 (t, J = 6.4 Hz, 2H), 1.88-1.84 (m, 2H), 1.79-1.76 (m, 2H), 1.60-1.58 (m, 4H), 1.47-1.46 (m, 2H).

### Example 73: (S)-N-(1-(2-chloro-3-(morpholine-4-carbonyl)phenyl)-1,4,5,7-tetrahydropyrano[3,4-c]pyrazol-4-yl)-5,6,7,8-tetrahydroimidazo[1,5-a]pyridine-1-carboxamide

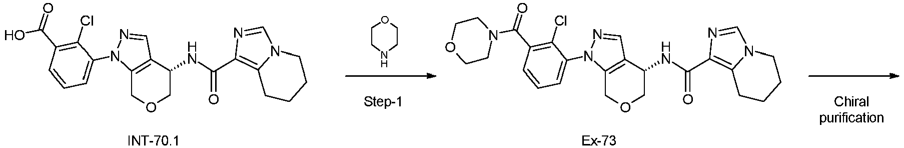

Step-1: Synthesis of (S)-N-(1-(2-chloro-3-(morpholine-4-carbonyl)phenyl)-1,4,5,7-tetrahydropyrano[3,4-c]pyrazol-4-yl)-5,6,7,8-tetrahydroimidazo[1,5-a]pyridine-1-carboxamide (Ex-73): Following the experiment procedure of **Ex-70,** the reaction was performed on 0.15 g scale with morpholine (0.12 g, 4.0 eq.) to get enantiomeric mixture [22:78] of Ex-73 (140 mg) as an off white solid compound. Further, Ex-73 was subjected for separation and purification by chiral HPLC to afford pure **Ex-73** (37 mg, 21.3%) as a white solid [Column Name: Chiralcel-OX-H (4.6 X 250), 5um; Mobile Phase: 0.1% Ammonium Hydroxide in MeOH = 100%; Flow Rate: 1.0 ml/min, Temp: 25°C; Flow mode: Isocratic; *t*_{R} (peak-1) 17.3 min. (22%), *t*_{R} (peak-2) 20.3 min. (78%)]. LCMS (ESI) m/z = 511.26 [M+H]⁺; Chiral HPLC (peak-2): 99.5% (*t*_{R} 20.53 min); UPLC (Method C): 99.6% (*t*_{R} 9.65 min); 1H NMR (400 MHz, DMSO-d6): 7.70 (s, 1H), 7.63-7.56 (m, 4H), 7.42 (dd, J = 4.8 Hz, J = 3.6 Hz, 1H), 5.07-5.05 (m, 1H), 4.58 (dd, J = 14.8 Hz, J = 14.4 Hz, 2H), 3.99 (t, J = 5.6 Hz, 2H), 3.84 (td, J = 16.0 Hz, J = 3.6 Hz, 2H), 3.71-3.60 (m, 4H), 3.55 (t, J = 4.0 Hz, 2H), 3.17 (s, 2H), 2.99 (t, J = 6.4 Hz, 2H), 1.88-1.83 (m, 2H), 1.79-1.74 (m, 2H).

### Example 74: (S)-N-(1-(2-chloro-3-(diethylcarbamoyl)phenyl)-1,4,5,7-tetrahydropyrano[3,4-c]pyrazol-4-yl)-5,6,7,8-tetrahydroimidazo[1,5-a]pyridine-1-carboxamide

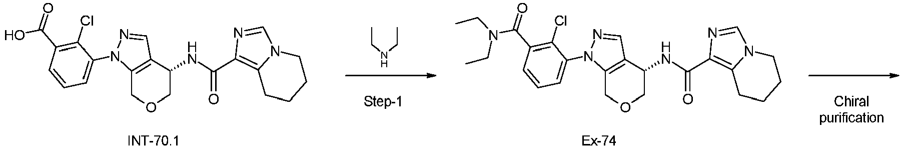

Step-1: Synthesis of (S)-N-(1-(2-chloro-3-(diethylcarbamoyl)phenyl)-1,4,5,7-tetrahydropyrano[3,4-c]pyrazol-4-yl)-5,6,7,8-tetrahydroimidazo[1,5-a]pyridine-1-carboxamide (Ex-74): Following the experiment procedure of Ex-70, the reaction was performed on 0.15 g scale with diethylamine (0.10 g, 4.0 eq.) to get enantiomeric mixture [20:75] of Ex-74 (90 mg) as an off white solid compound. Further, Ex-74 was subjected for separation and purification by chiral HPLC to afford pure **Ex-74** (12 mg, 7.11%) as white solids, respectively [Column Name: Chiralcel-OX-H (4.6 X 250), 5um; Mobile Phase: 0.1% Ammonium Hydroxide in MeOH = 100% Flow Rate: 1.0 ml/min, Temp: 25°C; Flow mode: Isocratic; *t*_{R} (peak-1) 12.5 min. (20%), *t*_{R} (peak-2) 14.2 min. (75%)]. LCMS (ESI) m/z = 497.27 [M+H]⁺; Chiral HPLC (peak-2): 98.1% (*t*_{R} 14.23 min); UPLC (Method K): 99.9% (*t*_{R} 9.48 min); 1H NMR (400 MHz, DMSO-d6): 7.70 (s, 1H), 7.61-7.56 (m, 3H), 7.52 (dd, J = 7.2 Hz, J = 2.4 Hz, 1H), 7.43 (d, J = 8.4 Hz, 1H), 5.07-5.04 (m, 1H), 4.62 (d, J = 15.2 Hz, 1H), 4.54 (dd, J = 14.4 Hz, J = 5.6 Hz, 1H), 3.99 (t, J = 5.6 Hz, 2H), 3.84 (td, J = 11.6 Hz, J = 3.6 Hz, 2H), 3.66-3.57 (m, 1H), 3.39-3.35 (m, 1H), 3.19-3.11 (m, 1H), 3.08-3.03 (m, 1H), 2.99 (t, J = 6.4 Hz, 2H), 1.88-1.84 (m, 2H), 1.79-1.75 (m, 2H), 1.61 (t, J = 7.2 Hz, 3H), 1.02 (t, J = 7.2 Hz, 3H).

### Biological Examples

### Biological Example 1: Inhibition of human dihydroorotate dehydrogenase (DHODH)

Inhibition of human dihydroorotate dehydrogenase (DHODH) by example compounds as described herein was determined using one of two assays as described below.

### Assay A

DHODH enzyme assays were performed with 6 nM recombinant human DHODH (purified essentially as described by Walse et al., Biochemistry, 47, 8929-8936 (2008)). The reaction mixture consisted of 1 mM DL-dihydroorotic acid (#D7003, Sigma-Aldrich), 100 µM 3,4-dimethoxy-5-methyl-p-benzoquinone (#D9150, Sigma-Aldrich), and 100 µM 2,6-dichlorophenolindophenol sodium salt (DCIP) (#D1878, Sigma-Aldrich) in enzyme buffer (50 mM Tris-HCI pH 8.0, 0.1% Triton X-100, 150 mM KCI). A stock solution of 20 mM DCIP was prepared in enzyme buffer and filtered through Whatman paper just before use. Reactions were performed in a total volume of 100 µL, consisting of 1µL of compound, 49 µL of enzyme and 50µL of reaction mixture. Loss in absorbance by chromogen DCIP was measured at 595 nm following a 70 minute incubation at 32 °C.

| **Table 1.IC50 DHODH inhibition activity values** | |
|---|---|
| **IC50** | **Example** |
| ≥1 µM - <10 µM | Ex-2, Ex-5, Ex-49 |
| ≥100 nM - <1 µM | Ex-8, Ex-10, Ex-12, Ex-20, Ex-27, Ex-40, Ex-42, Ex-43, Ex-44, Ex-45, Ex-56, Ex-57 |
| ≥10 nM - <100 nM | Ex-3, Ex-6, Ex-17, Ex-19, Ex-24, Ex-25, Ex-30, Ex-32, Ex-33, Ex-37, Ex-39, Ex-41, Ex-46, Ex-48, Ex-50, Ex-51, Ex-52, Ex-53, Ex-54, Ex-55, Ex-58, Ex-59, Ex-60, Ex-61, Ex-62, Ex-63, Ex-64, Ex-65, Ex-66, Ex-68, Ex-70, Ex-71, Ex-73, Ex-74 |
| <10 nM | Ex-9, Ex-11, Ex-13, Ex-14, Ex-15, Ex-16, Ex-18, Ex-21, Ex-22, Ex-23, Ex-26, Ex-28, Ex-29, Ex-31, Ex-34, Ex-35, Ex-36, Ex-38, Ex-47, Ex-67, Ex-69, Ex-72 |

### Biological Example 2: Inhibition of MOLM13

### Assay B

MOLM-13 cells (an acute myeloid leukemia cell line) were plated in RPMI supplied with 10% FBS and 1% Pen/Strep in 384-well plates at 1,000 cells per well on day 0. On day 1, treatment of the cells with compound (concentration range 10E-5 and 6E-12 M) was initiated in the presence or absence of 100 µM uridine. After 72 hours incubation of the cells with or without test compound, the number of viable cells per well was assessed by Cell Titer-Glo Luminescent Cell Viability Assay (Promega, Madison, WI, USA). IC50 was calculated using DMSO control as 100% cell viability and wells without cells (culture medium only) as 0% cell viability. Each condition was tested in duplicate and the potency of test compounds was determined from two independent experiments.

Using the assays described in Biological Example 2, the following IC₅₀ values were obtained.

| **Table 2. IC50 MOLM13, inhibition activity values** | |
|---|---|
| **IC50** | **Example** |
| ≥100 nM - <1 µM | Ex-8, Ex-10, Ex-20, Ex-42, Ex-43, Ex-44, Ex-56, Ex-57, Ex-63, Ex-65, Ex-66 |
| ≥10 nM - <100 nM | Ex-3, Ex-6, Ex-12, Ex-17, Ex-19, Ex-27, Ex-33, Ex-37, Ex-39, Ex-40, Ex-41, Ex-46, Ex-48, Ex-52, Ex-53, Ex-54, Ex-55, Ex-58, Ex-59, Ex-60, Ex-61, Ex-62, Ex-64, Ex-68, Ex-70, Ex-71, Ex-73, Ex-74 |
| <10 nM | Ex-9, Ex-11, Ex-13, Ex-14, Ex-15, Ex-16, Ex-18, Ex-21, Ex-22, Ex-23, Ex-24, Ex-25, Ex-26, Ex-28, Ex-29, Ex-30, Ex-31, Ex-32, Ex-34, Ex-35, Ex-36, Ex-38, Ex-47, Ex-50, Ex-51, Ex-67, Ex-69, Ex-72 |

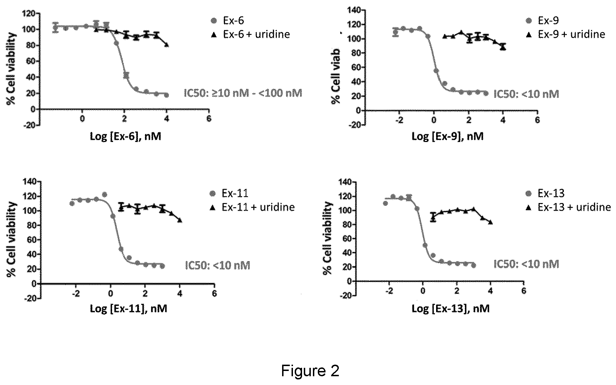

Figure 2 shows the data obtained for Ex-6, Ex-9, Ex-11 and Ex-13 on viability of MOLM13 cells in the presence and absence of uridine.

### Biological Example 3: Anti-viral activity

### MATERIALS AND METHODS

### Cells

The lung cancer epithelial cell line A549 was obtained from the American Type Culture Collection (ATCC; Rockville, MD, USA) and cultured in MEM medium (Gibco BRL, Gaithersburg, MD, USA) with 10% fetal calf serum (FCS; Gibco BRL), 2 mM L-glutamine (Gibco BRL) and 0.075% sodium bicarbonate (Gibco BRL).

### Viruses

The ZIKV MR766 prototype strain and the DENV-2 laboratory-adapted New Guinea C strain were obtained from ATCC and stocks were grown in the C6/36 mosquito cell line (ATCC). Viral stock titers were determined using plaque assay in BHK-21 cells (ATCC).

### Antiviral assay

The anti-ZIKV and anti-DENV-2 activity in A549 cells was determined using a tetrazolium-based colorimetric cytopathic effect (CPE) reduction assay. This assay has been described in detail before (Virology. 2021; doi.org/10.1016/j.virol.2021.07.003). A549 cells (1.5 x 10⁴ cells in 100 µL cell culture medium) were seeded in 96-well flat bottom plates (TPP, Trasadingen, Switzerland) and incubated overnight to allow the cells to grow to confluency. Drug dilutions were prepared in culture medium where FCS concentration was decreased to 2% and threefold diluted in the plates (in duplicate). Finally, 100 µL virus was added, with ZIKV at MOI 1 and DENV-2 at MOI 5, resulting in a final volume of 200 µL. The cytopathic effect induced by the virus was checked regularly microscopically. After 3 d of infection for ZIKV, and 4 d for DENV-2, when a strong cytopathic effect was observed in the positive control (i.e., untreated, infected cells), the cell viability was assessed spectrophotometrically via the in situ reduction of the tetrazolium compound 3-(4,5-dimethylthiazol-2-yl)-5-(3-carboxymethoxyphenyl)-2-(4-sulfophenyl)-2H-tetrazolium inner salt (MTS/PES), using the CellTiter 96 AQueous One Solution Cell Proliferation Assay (Promega, Fitchburg, WI, USA). Three hours after MTS incubation, virus was inactivated using 0.5% Triton X-100 (Thermo Fisher Scientific, Waltham, MA, USA) and the absorbance was then recorded at 490 nm with a 96-well plate reader and compared with four cell control replicates (cells without virus and drugs) and four virus control wells (cells with virus but without drug). Each assay was performed two times. The median inhibitory concentration (IC₅₀), or the concentration that inhibited virus-induced cell death by 50%, was calculated from each dose-response curve by performing a non-linear regression.

Absorbance was recorded using the VersaMax ELISA^{™} microplate reader (Molecular Devices) and analyzed with the Softmax Pro^{®} software (Molecular Devices, Version 4.0). Using mock-infected cells, the cytotoxic concentration 50 (CC₅₀) of the compound was investigated in parallel.

### RESULTS AND DISCUSSION

The compound of Ex-9 was evaluated for antiviral activity against ZIKV and DENV-2. In parallel, the cytotoxicity of the compound for the A549 cells was also determined. The results are shown in the table below with the compound demonstrating antiviral activity against ZIKV and DENV-2.

| **A549** | **CC₅₀ (µM)** | **IC₅₀ (µM)** | |
|---|---|---|---|
| | | **ZIKV MTS** | **DENV-2 MTS** |
| **Ex-9** | 3,65 ± 0,1 | 0,0093 ± 0,002 | 0,0016 ± 0,001 |

| | | | |
|---|---|---|---|
| IC₅₀ = concentration required to inhibit 50% of ZIKV-/DENV- induced CPE in A549 cells. CC₅₀= concentration required to reduce the viability of mock-infected A549 cells by 50%. | | | |

### Head-to-head Example 1: Better stability of O-analogues (Examples 3 - 69) compared to C-analogues (Compounds A - X)

Human Liver Microsomal metabolic stability: The microsomal metabolic stability assay utilized pooled human liver microsomes (mixed gender, BD Gentest), with supplemented cofactors NADPH and UDPGA (Solarbio S&T Co., LTD). Two separate experiments were performed as follows:
a) With cofactors (NADPH & UDPGA): 25 µL of 10 mM NADPH and 25 µL of 20 mM UDPGA were added to 197.5 µL of reaction solution (phosphate buffer (final concentration in assay 100 mM), MgCl₂ (final concentration in assay (5 mM)), alamethacin (final concentration in assay 0.025 mg/mL) and microsomes (final concentration in assay 0.5 mg/mL).
b) Without cofactors (NADPH & UDPGA): 50 µL of H₂O was added to 197.5 µL of reaction solution.

The mixture was pre-warmed at 37 °C for 10 minutes. The reaction was started with the addition of 2.5 µL of 100 µM control compound or test compound solutions. Diclofenac was used as positive control in this study. The final concentration of control compound and test compounds was 1 µM. Aliquots of 25 µL were taken from the reaction solution at 0.5, 5, 15, 30 and 60 minutes. The reaction was stopped by the addition of 5 volumes of cold acetonitrile containing internal standards. Samples were centrifuged at 3,220 g for 40 minutes. An aliquot of 100 µL of the supernatant was mixed with 100 µL of ultra-pure H₂O and then used for LC-MS/MS analysis. Peak areas were determined from extracted ion chromatograms. Percent parent remaining was calculated from the area ratio of test compound. The slope value was determined by linear regression of the natural logarithm of percent parent remaining versus incubation time curve.

Human Hepatocyte metabolic stability: Human hepatocytes (X008000, BiolVT) were freshly thawed prior to the incubations with test compound. Hepatocytes were transferred to 50 mL conical tubes and centrifuged 10 min at 100g. Thawing medium was removed and replaced by preheated (37 °C) incubation medium (William's E Medium supplemented with GlutaMAX), to yield a hepatocyte density ~1.5×10⁶ cells/mL. Cell viability was determined using AO/PI staining and hepatocytes were diluted using incubation medium to a working density of 0.5×10⁶ viable cells/mL. 198 µL of hepatocytes were added into each well of a 96-well plate and placed in the incubator to allow the hepatocytes to warm for 10 minutes. 2 µL test compound or positive control was placed into respective wells of the 96-well plate (final concentration test compound 1 µM) to start the reaction. The plate was returned to the incubator and 25 µL aliquots of the well contents were removed at time points of 0, 15, 30, 60, 90 and 120 minutes. The aliquots were mixed with 6 volumes (150 µL) of acetonitrile containing internal standards (100 nM alprazolam, 200 nM caffeine, and 100 nM tolbutamide) to terminate the reaction. The plate was centrifuged for 20 minutes at 3,200 g. Aliquots of 150 µL of the supernatants were used for LC/MS/MS analysis. All incubations were performed in duplicate.

Peak areas were determined from extracted ion chromatograms to determine the *in vitro* half-life (t½) of parent compound by regression analysis of the percent parent disappearance vs. time curve. The *in vitro* half-life (in vitro t½) was determined from the slope value (in vitro t½ = -0.693/k). All calculations were carried out using Microsoft Excel. Table 3 below summarizes the results and demonstrates improved stability of the compounds of the invention when compared head to head with the corresponding C-analogue.

| **Table 3.Human microsomal and hepatocyte stability** | | |
|---|---|---|
| | **O-analogue** | **C-analogue^{#}** |
| | **Ex-3** | **Compound A** |
| HLM (t½ (min) / % remaining* | > 365 / 92 | 107 / 66 |
| hHEPs (t½ (min) / % remaining** | 598 / 87 | 297 / 77 |
| | | |

| | **Ex-6** | **Compound B** |
|---|---|---|
| HLM (t½ (min) / % remaining | 285 / 85 | 60 / 50 |
| hHEPs (t½ (min) / % remaining | 294 / 87 | 95 / 39 |
| | | |

| | **Ex-9** | **Compound C** |
|---|---|---|
| HLM (t½ (min) / % remaining | > 365 / 100 | 102/69 |
| hHEPs (t½ (min) / % remaining | > 600 / 87 | > 600 / 100 |
| | | |

| | **Ex-10** | **Compound D** |
|---|---|---|
| HLM (t½ (min) / % remaining | > 365 / 100 | > 365 / 100 |
| hHEPs (t½ (min) / % remaining | > 600 / 100 | > 600 / 96 |
| | | |

| | **Ex-11** | **Compound E** |
|---|---|---|
| HLM (t½ (min) / % remaining | > 365 / 93 | 117 / 69 |
| hHEPs (t½ (min) / % remaining | > 600 / 90 | 123 / 50 |
| | | |

| | **Ex-13** | **Compound F** |
|---|---|---|
| HLM (t½ (min) / % remaining | > 365 / 88 | 38 / 34 |
| hHEPs (t½ (min) / % remaining | > 600 / 95 | 35 / 8,3 |
| | | |

| | **Ex-34** | **Compound G** |
|---|---|---|
| HLM (t½ (min) / % remaining | 337 / 100 | 28 / 23 |
| hHEPs (t½ (min) / % remaining | > 600 / 100 | > 600 / 91 |
| | | |

| | **Ex-41** | **Compound H** |
|---|---|---|
| HLM (t½ (min) / % remaining | > 365 / 100 | > 365 / 83 |
| hHEPs (t½ (min) / % remaining | > 600 / 100 | 392 / 83 |
| | | |

| | **Ex-42** | **Compound I** |
|---|---|---|
| HLM (t½ (min) / % remaining | 236 / 85 | 67 / 52 |
| hHEPs (t½ (min) / % remaining | 326 / 76 | 62/13 |
| | | |

| | **Ex-43** | **Compound J** |
|---|---|---|
| HLM (t½ (min) / % remaining | > 365 / 92 | > 365 / 92 |
| hHEPs (t½ (min) / % remaining | > 600 / 93 | 257 / 69 |
| | | |

| | **Ex-44** | **Compound K** |
|---|---|---|
| HLM (t½ (min) / % remaining | > 365 / 92 | 83 / 59 |
| hHEPs (t½ (min) / % remaining | 561 / 89 | 87 / 40 |
| | | |

| | **Ex-45** | **Compound L** |
|---|---|---|
| HLM (t½ (min) / % remaining | 274 / 88 | 52 / 46 |
| hHEPs (t½ (min) / % remaining | 286 / 72 | 36/10 |
| | | |

| | **Ex-46** | **Compound M** |
|---|---|---|
| HLM (t½ (min) / % remaining | > 365 / 90 | 57 / 48 |
| hHEPs (t½ (min) / % remaining | > 600 / 89 | 91 / 41 |
| | | |

| | **Ex-47** | **Compound N** |
|---|---|---|
| HLM (t½ (min) / % remaining | 133 / 75 | 22 / 15 |
| hHEPs (t½ (min) / % remaining | 540 / 84 | 37 / 11 |
| | | |

| | **Ex-48** | **Compound O** |
|---|---|---|
| HLM (t½ (min) / % remaining | > 365 / 96 | 135 / 73 |
| hHEPs (t½ (min) / % remaining | 358 / 82 | 103 / 44 |
| | | |

| | **Ex-51** | **Compound P** |
|---|---|---|
| HLM (t½ (min) / % remaining | 356 / 85 | 129 / 71 |
| hHEPs (t½ (min) / % remaining | > 600 / 100 | 342 / 78 |
| | | |

| | **Ex-52** | **Compound Q** |
|---|---|---|
| HLM (t½ (min) / % remaining | 271 / 84 | 83 / 58 |
| hHEPs (t½ (min) / % remaining | > 600 / 100 | 256 / 73 |
| | | |

| | **Ex-53** | **Compound R** |
|---|---|---|
| HLM (t½ (min) / % remaining | > 365 / 96 | 65 / 54 |
| hHEPs (t½ (min) / % remaining | > 600 / 99 | 205 / 66 |
| | | |

| | **Ex-54** | **Compound S** |
|---|---|---|
| HLM (t½ (min) / % remaining | > 365 / 92 | 120 / 70 |
| hHEPs (t½ (min) / % remaining | > 600 / 100 | > 600 / 84 |
| | | |

| | **Ex-55** | **Compound T** |
|---|---|---|
| HLM (t½ (min) / % remaining | > 365 / 94 | 66 / 53 |
| hHEPs (t½ (min) / % remaining | > 600 / 100 | > 600 / 100 |
| | | |

| | **Ex-58** | **Compound U** |
|---|---|---|
| HLM (t½ (min) / % remaining | 275 / 87 | 100 / 65 |
| hHEPs (t½ (min) / % remaining | > 600 / 100 | 62 / 26 |
| | | |

| | **Ex-59** | **Compound V** |
|---|---|---|
| HLM (t½ (min) / % remaining | > 365 / 91 | > 365 / 91 |
| hHEPs (t½ (min) / % remaining | > 600 / 100 | 360 / 79 |
| | | |

| | **Ex-61** | **Compound W** |
|---|---|---|
| HLM (t½ (min) / % remaining | > 365 / **94** | > 365 / 92 |
| hHEPs (t½ (min) / % remaining | > 600 / 100 | 569 / 85 |
| | | |

| | **Ex-69** | **Compound X** |
|---|---|---|
| HLM (t½ (min) / % remaining | 7,3 / 0,14 | 1,5 / 0,10 |
| hHEPs (t½ (min) / % remaining | 4,6 / BLOD@ | 2,5 / BLOD |
| * % remaining at 60 min ** % remaining at 120 min | | |
| ^{#}All C-analogues were essentially prepared as described in WO2017/077280. In each C-analogue, the oxygen atom in the tetrahydropyran ring of the O-analogue is replaced by CH₂. | | |
| Otherwise the structure is identical. | | |
| @BLOD = Below limit of detection | | |

### Head-to-head Example 2: No inhibition of human CYPs of O-analogue Ex-9 compared to Compound C

CYP inhibition: Co-incubation of compounds with P450 enzyme-selective substrates was performed using pooled human liver microsomes (Cat. No. H0610, Xenotech), to determine their potential to cause drug-drug interactions. The assay was performed in duplicate. The formation of acetaminophen, 7'-hydoxycoumarin, hydrobupropion, N-desethylamodiaquine, 4'-hydroxydiclofenac, 4'-hydroxymephenytoin, 1'-hydroxybufuralol, 6'-hydroxychlorzoxazone were quantified as markers for CYP1A2, CYP2A6, CYP2B6, CYP2C8, CYP2C9, CYP2C19, CYP2D6, CYP2E1, respectively. CYP3A4 inhibition was measured using 2 substrates, measuring the formation of 6β-hydroxytestosterone (3A4-T), and 1'-hydroxymidazolam (3A4-M). The incubation was carried out in 96 deep well plates each well containing: 179 µL of the master solution (0.2 mg/mL microsomes plus CYP substrate (see below for final substrate concentration and incubation time for the different CYP assays)) in 100 mM phosphate buffer and 1 µL of the compound working solution or vehicle. The final concentrations of the compounds in the assay was 30 µM when using a single dose or 0.1, 0.3, 1, 3, 10 and 30 µM for a dose response.

| **Table 4. Substrate final concentrations and incubation times** | | | |
|---|---|---|---|
| **CYP** | **Substrate** | **Final concentration (µM)** | **Incubation time (min)** |
| CYP1A2 | Phenacetin | 40 | 20 |
| CYP2A6 | Coumarin | 2 | 5 |
| CYP2B6 | Bupropion | 40 | 20 |
| CYP2C8 | Amodiaquine | 1 | 5 |
| CYP2C9 | Diclofenac Sodium | 6 | 5 |
| CYP2C19 | Mephenytoin | 50 | 20 |
| CYP2D6 | Dextromethorphan | 2 | 20 |
| CYP2E1 | Chlorzoxazone | 40 | 20 |
| CYP3A4-M | Midozolam | 1 | 5 |
| CYP3A4-T | Testosterone | 40 | 10 |

The incubation plate was placed into a water bath and pre-warmed at 37 °C for 5 minutes before the reactions were started by the addition of 20 µL of 10 mM NADPH solution and incubated at 37 °C for the time listed above. The reaction was stopped by addition of 1.5 volumes (300 µL) of cold ACN containing 3% formic acid and 200 nM tolbutamide, 200 nM aprozolam and 200 nM labalol. The plate was centrifuged at 3,220 g for 50 minutes and then 200 µL of the supernatant was transferred to the analysis plate for LC/MS/MS analysis. The respective metabolite formation from each probe substrate was compared with that observed for the solvent control incubations.

| **Table 5. IC50 values for individual human Cytochrome P450's** | | |
|---|---|---|
| | 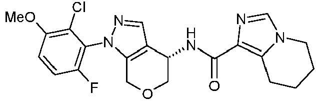 | 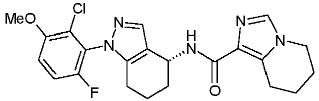 |
| **CYP** | **Ex-9** | **Compound C*** |
| CYP1A2 (µM) | > 30 | > 30 |
| CYP2A6 (µM) | > 30 | > 30 |
| CYP2B6 (µM) | > 30 | > 30 |
| CYP2C8 (µM) | > 30 | > 30 |
| CYP2C9 (µM) | > 30 | 9,2 |
| CYP2C19 (µM) | > 30 | 18 |
| CYP2D6 (µM) | > 30 | > 30 |
| CYP2E1 (µM) | > 30 | > 30 |
| CYP3A4-M (µM) | > 30 | 16 |
| CYP3A4-T (µM) | > 30 | > 30 |
| * Compound C was essentially prepared as described in WO2017/077280 | | |

### Abbreviations

Abbreviations as used herein will be known to those skilled in the art. In particular, the following abbreviations may be used herein.
- Aq.: aqueous
- ACN: acetonitrile
- AcOH: acetic acid
- DCM: dichloromethane
- DEA: diethylamine
- DIPEA: N,N-diisopropylethylamine
- DMF: dimethylformamide
- DMF-DMA: dimethylformamide dimethyl acetal
- DMSO: dimethyl sulfoxide
- EDC: 1-ethyl-3-(3-dimethylaminopropyl)carbodiimide
- EtOH: ethanol
- HATU: 1-[bis(dimethylamino)methylene]-1H-1,2,3-triazolo[4,5-b]pyridinium 3-oxid hexafluorophosphate
- HBTU: N,N,N',N'-tetramethyl-O-(1H-benzotriazol-1-yl)uronium hexafluorophosphate
- HCl: hydrochloric acid
- HOBt: hydroxybenzotriazole
- HPLC: high performance liquid chromatography
- HRMS: high resolution mass spectrometry
- MeOH: methanol
- NaBH₃CN: sodium borohydride cyanide
- NaNO₂: sodium nitrite
- NH₄Cl: ammonium chloride
- NMR: nuclear magnetic resonance
- SnCl₂: tin(II)chloride
- T₃P: Propanephosphonic acid anhydride
- TBTU: O-(benzotriazol-1-yl)-N,N,N',N'-tetramethyluronium tetrafluoroborate
- TFA: trifluoroacetic acid
- THF: tetrahydrofuran
- Ti(OiPr)₄: titanium(IV)tetraisopropoxide
- TLC: thin-layer chromatography
- UPLC: ultra-high performance liquid chromatography

## Claims

1. A compound of formula I 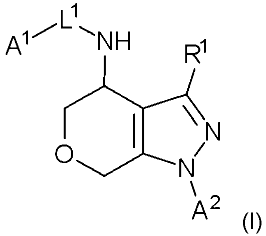 or a pharmaceutically acceptable salt thereof, wherein:
A' is a 9-membered bicyclic heteroaryl, 6-membered heteroaryl or 5-membered heteroaryl, optionally substituted with one or more groups independently selected from G²;
A² is phenyl optionally substituted by one or more groups independently selected from G³;
L¹ represents -C(O)-;
R¹ represents H;
wherein:
each G² independently represents halo, R^{a2}, -CN, -A^{a2}-C(Q^{a2})R^{b2} -A^{b2}-C(Q^{b2})N(R^{c2})R^{d2}, -A^{c2}-C(Q^{c2})OR^{e2}, A^{d2}-S(O)ₚR^{f2}, -A^{e2}-S(O)_{q}N(R^{g2})R^{h2}, -A^{f2}-S(O)ₚOR -N₃, -N(R^{j2})R^{k2}, -N(H)CN, -NO₂, -ONO₂, -OR^{I2} or -SR^{m2};
each Q^{a2} to Q^{c2} independently represents =O, =S, =NRⁿ² or =N(OR^{o2});
each A^{a2} to A^{f2} independently represents a single bond, -N(R^{p2})- or -O-;
each G³ independently represents halo, R^{a3}, -CN, -A^{a3}-C(Q^{a3})R^{b3}, -A^{b3}-C(Q^{b3})N(R^{c3})R^{d3}, -A^{c3}-C(Q^{c3})OR^{e3}, -A^{d3}-S(O)ₚR^{f3}, -A^{e3}-S(O)_{q}N(R^{g3})R^{h3}, -A^{f3}-S(O)ₚORⁱ³, -N₃, -N(R^{j3})R^{k3}, -N(H)CN, -NO₂, -ONO₂, -OR¹³ or -SR^{m3};
each Q^{a3} to Q^{c3} independently represents =O, =S, =NRⁿ³ or =N(OR^{o3});
each A^{a3} to A^{f3} independently represents a single bond, -N(R^{p3})- or -O-;
each R^{a2} and R^{f2} independently represents C₁₋₆ alkyl optionally substituted by one or more groups independently selected from G^{6a}, heterocycloalkyl optionally substituted by one or more groups independently selected from G^{6b}, aryl optionally substituted by one or more groups independently selected from G^{6c}, or heteroaryl optionally substituted by one or more groups independently selected from G^{6d};
each R^{p2} independently represents H or C₁₋₆ alkyl optionally substituted by one or more F;
each R^{b2}, R^{c2}, R^{d2}, R^{e2}, R^{g2}, R^{h2}, Rⁱ², R^{j2}, R^{k2}, R^{l2}, R^{m2}, Rⁿ² and R^{o2} independently represents H, C₁₋₆ alkyl optionally substituted by one or more groups independently selected from G^{6a}, heterocycloalkyl optionally substituted by one or more groups independently selected from G^{6b}, aryl optionally substituted by one or more groups independently selected from G^{6c}, or heteroaryl optionally substituted by one or more groups independently selected from G^{6d}; or
alternatively any of R^{c2} and R^{d2}, R^{g2} and R^{h2} and/or R^{j2} and R^{k2} are linked together to form, together with the nitrogen atom to which they are attached, a 3- to 6-membered ring, which ring optionally contains one further heteroatom and which ring optionally is substituted by one or more groups independently selected from halo, C₁₋₃ alkyl optionally substituted by one or more halo, and =O;
each R^{a3} and R^{f3} independently represents C₁₋₆ alkyl optionally substituted by one or more groups independently selected from G^{7a}, heterocycloalkyl optionally substituted by one or more groups independently selected from G^{7b}, aryl optionally substituted by one or more groups independently selected from G^{7c}, or heteroaryl optionally substituted by one or more groups independently selected from G^{7d};
each R^{p3} independently represents H or C₁₋₆ alkyl optionally substituted by one or more F;
each R^{b3}, R^{c3}, R^{d3}, R^{e3}, R^{g3}, R^{h3}, Rⁱ³, R^{j3}, R^{k3}, R^{l3}, R^{m3}, Rⁿ³ and R^{o3} independently represents H, C₁₋₆ alkyl optionally substituted by one or more groups independently selected from G^{7a}, heterocycloalkyl optionally substituted by one or more groups independently selected from G^{7b}, aryl optionally substituted by one or more groups independently selected from G^{7c}, or heteroaryl optionally substituted by one or more groups independently selected from G^{7d}; or
alternatively any of R^{c3} and R^{d3}, R^{g3} and R^{h3} and/or R^{j3} and R^{k3} are linked together to form, together with the nitrogen atom to which they are attached, a 3- to 6-membered ring, which ring optionally contains one further heteroatom and which ring optionally is substituted by one or more groups independently selected from halo, C₁₋₃ alkyl optionally substituted by one or more halo, and =O;
each G^{6a}, G^{6b}, G^{7a} and G^{7b} independently represents halo, -CN, -N(R^{b5})R^{c5}, -OR^{d5}, -SR^{e5} or =O;
each G^{6c}, G^{6d}, G^{7c} and G^{7d} independently represents halo, R^{a5}, -CN, -N(R^{b5})R^{e5}, -OR^{d5}, -SR^{e5} or =O;
each R^{a5} independently represents C₁₋₆ alkyl optionally substituted by one or more F;
each R^{b5}, R^{c5} , R^{d5} and R^{e5} independently represents H or C₁₋₆ alkyl optionally substituted by one or more F or =O; and
or R^{b5} and R^{c5} are linked together to form, along with the nitrogen atom to which they are attached, a 3- to 6-membered ring, which ring optionally contains one further heteroatom and which ring optionally is substituted by one or more groups independently selected from F, C₁₋₃ alkyl optionally substituted by one or more F, and =O;
and each p and q independently represents 1 or 2.

2. The compound of claim 1, wherein:
each G² independently represents -CN, -A^{a2}-C(Q^{a2})R^{b2} -A^{b2}-C(Q^{b2})N(R^{c2})R^{d2}, -A^{c2}-C(Q^{c2})OR^{e2}, -A^{d2}-S(O)ₚR^{f2} -A^{e2}-S(O)_{q}N(R^{g2})R^{h2}, -A^{f2}-S(O)ₚORⁱ², -N₃, -N(R^{j2})R^{k2}, -N(H)CN, -NO₂, -ONO₂, halo, R^{a2}, -OR^{I2} or -SR^{m2}; each Q^{a2} to Q^{c2} independently represents =O, =S, =NRⁿ² or =N(OR^{o2}); and/or each A^{a2} to A^{f2} independently represents a single bond, -N(R^{p2})- or -O-; and/or
each G³ independently represents -CN, -A^{a3}-C(Q^{a3})R^{b3}, -A^{b3}-C(Q^{b3})N(R^{c3})R^{d3}, -A^{c3}-C(Q^{c3})OR^{e3}, -A^{d3}-S(O)ₚR^{f3}, -A^{e3}-S(O)_{q}N(R^{g3})R^{h3}, -A^{f3}-S(O)ₚORⁱ³, -N₃, -N(R^{j3})R^{k3}, -N(H)CN, -NO₂, -ONO₂, halo, R^{a3}, -OR¹³ or -SR^{m3}; each Q^{a3} to Q^{c3} independently represents =O, =S, =NRⁿ³ or =N(OR^{o3}); and/or each A^{a3} to A^{f3} independently represents a single bond, -N(R^{p3})- or -O-.

3. The compound of claim 1 or 2, wherein:
A¹ is substituted by one or two groups independently selected from halo, R^{a2}, - C(O)OR^{e2}, -OR^{l2} and -SR^{m2}; or C₁₋₃ alkyl optionally substituted by one or more fluoro, - C(O)OH, -C(O)OC₁₋₃ alkyl, -OH, -OC₁₋₃ alkyl, -SH and -SC₁₋₃ alkyl;

4. The compound of any one of claims 1 to 3, wherein
A¹ is selected from tetrahydro-2,1-benzisoxazolyl, benzoxazoyl, pyrazinyl, indazolyl, 5H,6H,7H,8H-imidazo[1,5-a]pyridine-3-yl, 5H,6H,7H,8H-imidazo[1,5-a]pyridine-1-yl, imidazo[1,5-a]pyridin-3-yl, pyridinyl, thiazolyl, isoxazolyl and tetrahydro-1,2-benzisoxazolyl.

5. The compound of any one of claims 1 to 4, wherein:
A² is substituted by one or more groups independently selected from halo, -A^{a3}-C(Q^{a3})R^{b3}, -A^{c3}-C(Q^{c3})OR^{e3}, R^{a3} and -OR^{l3}; or
A² is substituted by one or more groups independently selected from halo, -C(O)-morpholinyl, -C(O)OC₁₋₃ alkyl, and C₁₋₄ alkyl.

6. The compound of any one of claims 1 to 3 or 5, wherein
A¹ is 5H,6H,7H,8H-imidazo[1,5-a]pyridine-3-yl, 5H,6H,7H,8H-imidazo[1,5-a]pyridine-1-yl, 4H,5H,6H,7H-1,2-benzisoxazol-3-yl, 4H,5H,6H,7H-2,1-benzisoxazol-3-yl or 4H,5H,6H,7H-indazole-3-yl; pyridinyl, pyrazolyl or imidazolyl.

7. The compound of any one of claims 1 to 6, wherein:
each G² independently represents R^{a2}; and/or
each G³ independently represents halo, R^{a3}, -A^{b3}-C(Q^{b3})N(R^{c3})R^{d3}, -A^{c3}-C(Q^{c3})OR^{e3}, -A^{d3}-S(O)pR^{f3}, -OR^{l3};
optionally wherein:
halo is fluoro or chloro, R^{a3} is CH₃ or CF₃, A^{b3}, A^{c3} and A^{d3} are a single bond, Q^{b3} and Q^{c3} are =O, p is 2,
R^{c3} and R^{d3} are CH₃ or are linked together to form, together with the nitrogen atom to which they are attached, a 5- or 6-membered ring, which ring optionally contains one further heteroatom,
R^{e3} is C₁₋₃ alkyl, R^{f3} is CH₃,
R^{l3} is CH₃, CF₃, C₂ alkyl substituted with N(CH₃)₂,OH, OCH₃ or N(C=O)CH₃, and/or C₁ alkyl substituted with C(=O)N(CH₃)₂.

8. The compound of any one of claims 1 to 7, wherein the compound is a compound of formula la 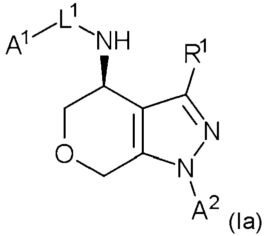 or a pharmaceutically acceptable salt thereof, wherein A¹, A², L¹ and R¹ are as defined in any one of claims 1 to 7.

9. The compound of claim 1, wherein the compound is:
N-(1-(2-fluorophenyl)-1,4,5,7-tetrahydropyrano[3,4-c]pyrazol-4-yl)-5,6,7,8-tetrahydroimidazo[1,5-a]pyridine-1-carboxamide
(R)-N-(1-(2-fluorophenyl)-1,4,5,7-tetrahydropyrano[3,4-c]pyrazol-4-yl)-5,6,7,8-tetrahydroimidazo[1,5-a]pyridine-1-carboxamide
(S)-N-(1-(2-fluorophenyl)-1,4,5,7-tetrahydropyrano[3,4-c]pyrazol-4-yl)-5,6,7,8-tetrahydroimidazo[1,5-a]pyridine-1-carboxamide
N-(1-(2-fluorophenyl)-1,4,5,7-tetrahydropyrano[3,4-c]pyrazol-4-yl)-4,5,6,7-tetrahydrobenzo[d]isoxazole-3-carboxamide
(R)-N-(1-(2-fluorophenyl)-1,4,5,7-tetrahydropyrano[3,4-c]pyrazol-4-yl)-4,5,6,7-tetrahydrobenzo[d]isoxazole-3-carboxamide
(S)-N-(1-(2-fluorophenyl)-1,4,5,7-tetrahydropyrano[3,4-c]pyrazol-4-yl)-4,5,6,7-tetrahydrobenzo[d]isoxazole-3-carboxamide
N-(1-(2-fluorophenyl)-1,4,5,7-tetrahydropyrano[3,4-c]pyrazol-4-yl)-5,6,7,8-tetrahydroimidazo[1,5-a]pyridine-3-carboxamide
(R)-N-(1-(2-fluorophenyl)-1,4,5,7-tetrahydropyrano[3,4-c]pyrazol-4-yl)-5,6,7,8-tetrahydroimidazo[1,5-a]pyridine-3-carboxamide
(S)-N-(1-(2-fluorophenyl)-1,4,5,7-tetrahydropyrano[3,4-c]pyrazol-4-yl)-5,6,7,8-tetrahydroimidazo[1,5-a]pyridine-3-carboxamide
N-1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-1-carboxamide
N-((4S)-1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-1-carboxamide
N-((4R)-1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-1-carboxamide
N-1-(2-chloro-6-fluorophenyl)-1,4,5,7-tetrahydropyrano[3,4-c]pyrazol-4-yl)-5,6,7,8-tetrahydroimidazo[1,5-a]pyridine-1-carboxamide
N-((4S)-1-(2-chloro-6-fluorophenyl)-1,4,5,7-tetrahydropyrano[3,4-c]pyrazol-4-yl)-5,6,7,8-tetrahydroimidazo[1,5-a]pyridine-1-carboxamide
N-((4R)-1-(2-chloro-6-fluorophenyl)-1,4,5,7-tetrahydropyrano[3,4-c]pyrazol-4-yl)-5,6,7,8-tetrahydroimidazo[1,5-a]pyridine-1-carboxamide
N-(1-(2,6-dichlorophenyl)-1,4,5,7-tetrahydropyrano[3,4-c]pyrazol-4-yl)-5,6,7,8-tetrahydroimidazo[1,5-a]pyridine-1-carboxamide
(S)-N-(1-(2,6-dichlorophenyl)-1,4,5,7-tetrahydropyrano[3,4-c]pyrazol-4-yl)-5,6,7,8-tetrahydroimidazo[1,5-a]pyridine-1-carboxamide
(R)-N-(1-(2,6-dichlorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-methylphenyl)-1,4,5,7-tetrahydropyrano[3,4-c]pyrazol-4-yl)-5-ethyl-1-methyl-1H-imidazole-4-carboxamide;
(R)-N-(1-(2-chloro-3-methylphenyl)-1,4,5,7-tetrahydropyrano[3,4-c]pyrazol-4-yl)-5-ethyl-1-methyl-1H-imidazole-4-carboxamide;
(S)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-3-carboxamide;
(R)-N-(1-(2-chloro-3-methylphenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-3-methylphenyl)-1,4,5,7-tetrahydropyrano[3,4-c]pyrazol-4-yl)-4,5,6,7-tetrahydro-1H-indazole-3-carboxamide;
(R)-N-(1-(2-chloro-3-methylphenyl)-1,4,5,7-tetrahydropyrano[3,4-c]pyrazol-4-yl)-4,5,6,7-tetrahydro-1H-indazole-3-carboxamide;
(S)-N-(1-(2-chloro-3-methylphenyl)-1,4,5,7-tetrahydropyrano[3,4-c]pyrazol-4-yl)-4,5,6,7-tetrahydrobenzo[d]isoxazole-3-carboxamide;
(R)-N-(1-(2-chloro-3-methylphenyl)-1,4,5,7-tetrahydropyrano[3,4-c]pyrazol-4-yl)-4,5,6,7-tetrahydrobenzo[d]isoxazole-3-carboxamide;
(S)-N-(1-(2-chloro-3-methylphenyl)-1,4,5,7-tetrahydropyrano[3,4-c]pyrazol-4-yl)-4-ethyl-5-methyl-1H-pyrazole-3-carboxamide;
(R)-N-(1-(2-chloro-3-methylphenyl)-1,4,5,7-tetrahydropyrano[3,4-c]pyrazol-4-yl)-4-ethyl-5-methyl-1H-pyrazole-3-carboxamide;
(S)-N-(1-(2-chloro-3-methylphenyl)-1,4,5,7-tetrahydropyrano[3,4-c]pyrazol-4-yl)-4,5-dimethylpicolinamide;
(R)-N-(1-(2-chloro-3-methylphenyl)-1,4,5,7-tetrahydropyrano[3,4-c]pyrazol-4-yl)-4,5-dimethylpicolinamide;
(S)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-methoxyphenyl)-1,4,5,7-tetrahydropyrano[3,4-c]pyrazol-4-yl)-5-ethyl-1-methyl-1H-imidazole-4-carboxamide;
(R)-N-(1-(2-chloro-3-methoxyphenyl)-1,4,5,7-tetrahydropyrano[3,4-c]pyrazol-4-yl)-5-ethyl-1-methyl-1H-imidazole-4-carboxamide;
(S)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-3-carboxamide;
(R)-N-(1-(2-chloro-3-methoxyphenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-3-methoxyphenyl)-1,4,5,7-tetrahydropyrano[3,4-c]pyrazol-4-yl)-4,5,6,7-tetrahydro-1H-indazole-3-carboxamide;
(R)-N-(1-(2-chloro-3-methoxyphenyl)-1,4,5,7-tetrahydropyrano[3,4-c]pyrazol-4-yl)-4,5,6,7-tetrahydro-1H-indazole-3-carboxamide;
(S)-N-(1-(2-chloro-3-methoxyphenyl)-1,4,5,7-tetrahydropyrano[3,4-c]pyrazol-4-yl)-4,5,6,7-tetrahydrobenzo[d]isoxazole-3-carboxamide;
(R)-N-(1-(2-chloro-3-methoxyphenyl)-1,4,5,7-tetrahydropyrano[3,4-c]pyrazol-4-yl)-4,5,6,7-tetrahydrobenzo[d]isoxazole-3-carboxamide;
(S)-N-(1-(2-chloro-3-methoxyphenyl)-1,4,5,7-tetrahydropyrano[3,4-c]pyrazol-4-yl)-4-ethyl-5-methyl-1H-pyrazole-3-carboxamide;
(R)-N-(1-(2-chloro-3-methoxyphenyl)-1,4,5,7-tetrahydropyrano[3,4-c]pyrazol-4-yl)-4-ethyl-5-methyl-1H-pyrazole-3-carboxamide;
(S)-N-(1-(2-chloro-6-fluoro-3-methoxyphenyl)-1,4,5,7-tetrahydropyrano[3,4-c]pyrazol-4-yl)-4,5-dimethylpicolinamide;
(R)-N-(1-(2-chloro-6-fluoro-3-methoxyphenyl)-1,4,5,7-tetrahydropyrano[3,4-c]pyrazol-4-yl)-4,5-dimethylpicolinamide;
(S)-N-(1-(2-chloro-6-fluoro-3-methoxyphenyl)-1,4,5,7-tetrahydropyrano[3,4-c]pyrazol-4-yl)-5-ethyl-1-methyl-1H-imidazole-4-carboxamide;
(R)-N-(1-(2-chloro-6-fluoro-3-methoxyphenyl)-1,4,5,7-tetrahydropyrano[3,4-c]pyrazol-4-yl)-5-ethyl-1-methyl-1H-imidazole-4-carboxamide;
(S)-N-(1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-3-carboxamide;
(R)-N-(1-(2-chloro-6-fluoro-3-methoxyphenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-6-fluoro-3-methoxyphenyl)-1,4,5,7-tetrahydropyrano[3,4-c]pyrazol-4-yl)-4,5,6,7-tetrahydro-1H-indazole-3-carboxamide;
(R)-N-(1-(2-chloro-6-fluoro-3-methoxyphenyl)-1,4,5,7-tetrahydropyrano[3,4-c]pyrazol-4-yl)-4,5,6,7-tetrahydro-1H-indazole-3-carboxamide;
(S)-N-(1-(2-chloro-6-fluoro-3-methoxyphenyl)-1,4,5,7-tetrahydropyrano[3,4-c]pyrazol-4-yl)-4,5,6,7-tetrahydrobenzo[d]isoxazole-3-carboxamide;
(R)-N-(1-(2-chloro-6-fluoro-3-methoxyphenyl)-1,4,5,7-tetrahydropyrano[3,4-c]pyrazol-4-yl)-4,5,6,7-tetrahydrobenzo[d]isoxazole-3-carboxamide;
(S)-N-(1-(2-chloro-6-fluoro-3-methoxyphenyl)-1,4,5,7-tetrahydropyrano[3,4-c]pyrazol-4-yl)-4-ethyl-5-methyl-1H-pyrazole-3-carboxamide;
(R)-N-(1-(2-chloro-6-fluoro-3-methoxyphenyl)-1,4,5,7-tetrahydropyrano[3,4-c]pyrazol-4-yl)-4-ethyl-5-methyl-1H-pyrazole-3-carboxamide;
(S)-N-(1-(2-chloro-6-fluoro-3-methoxyphenyl)-1,4,5,7-tetrahydropyrano[3,4-c]pyrazol-4-yl)-4,5-dimethylpicolinamide;
(R)-N-(1-(2-chloro-6-fluoro-3-methoxyphenyl)-1,4,5,7-tetrahydropyrano[3,4-c]pyrazol-4-yl)-4,5-dimethylpicolinamide;
(S)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-6-methylphenyl)-1,4,5,7-tetrahydropyrano[3,4-c]pyrazol-4-yl)-5-ethyl-1-methyl-1H-imidazole-4-carboxamide;
(R)-N-(1-(2-chloro-6-methylphenyl)-1,4,5,7-tetrahydropyrano[3,4-c]pyrazol-4-yl)-5-ethyl-1-methyl-1H-imidazole-4-carboxamide;
(S)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-3-carboxamide;
(R)-N-(1-(2-chloro-6-methylphenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-6-methylphenyl)-1,4,5,7-tetrahydropyrano[3,4-c]pyrazol-4-yl)-4,5,6,7-tetrahydro-1H-indazole-3-carboxamide;
(R)-N-(1-(2-chloro-6-methylphenyl)-1,4,5,7-tetrahydropyrano[3,4-c]pyrazol-4-yl)-4,5,6,7-tetrahydro-1H-indazole-3-carboxamide;
(S)-N-(1-(2-chloro-6-methylphenyl)-1,4,5,7-tetrahydropyrano[3,4-c]pyrazol-4-yl)-4,5,6,7-tetrahydrobenzo[d]isoxazole-3-carboxamide;
(R)-N-(1-(2-chloro-6-methylphenyl)-1,4,5,7-tetrahydropyrano[3,4-c]pyrazol-4-yl)-4,5,6,7-tetrahydrobenzo[d]isoxazole-3-carboxamide;
(S)-N-(1-(2-chloro-6-methylphenyl)-1,4,5,7-tetrahydropyrano[3,4-c]pyrazol-4-yl)-4-ethyl-5-methyl-1H-pyrazole-3-carboxamide;
(R)-N-(1-(2-chloro-6-methylphenyl)-1,4,5,7-tetrahydropyrano[3,4-c]pyrazol-4-yl)-4-ethyl-5-methyl-1H-pyrazole-3-carboxamide;
(S)-N-(1-(2-chloro-6-methylphenyl)-1,4,5,7-tetrahydropyrano[3,4-c]pyrazol-4-yl)-4,5-dimethylpicolinamide;
(R)-N-(1-(2-chloro-6-methylphenyl)-1,4,5,7-tetrahydropyrano[3,4-c]pyrazol-4-yl)-4,5-dimethylpicolinamide;
(S)-5-ethyl-N-(1-(2-fluorophenyl)-1,4,5,7-tetrahydropyrano[3,4-c]pyrazol-4-yl)-1-methyl-1H-imidazole-4-carboxamide;
(R)-5-ethyl-N-(1-(2-fluorophenyl)-1,4,5,7-tetrahydropyrano[3,4-c]pyrazol-4-yl)-1-methyl-1H-imidazole-4-carboxamide;
(S)-N-(1-(2-fluorophenyl)-1,4,5,7-tetrahydropyrano[3,4-c]pyrazol-4-yl)-4,5,6,7-tetrahydro-1H-indazole-3-carboxamide;
(R)-N-(1-(2-fluorophenyl)-1,4,5,7-tetrahydropyrano[3,4-c]pyrazol-4-yl)-4,5,6,7-tetrahydro-1H-indazole-3-carboxamide;
(S)-N-(1-(2-fluorophenyl)-1,4,5,7-tetrahydropyrano[3,4-c]pyrazol-4-yl)-4,5,6,7-tetrahydrobenzo[d]isoxazole-3-carboxamide;
(R)-N-(1-(2-fluorophenyl)-1,4,5,7-tetrahydropyrano[3,4-c]pyrazol-4-yl)-4,5,6,7-tetrahydrobenzo[d]isoxazole-3-carboxamide;
(S)-4-ethyl-N-(1-(2-fluorophenyl)-1,4,5,7-tetrahydropyrano[3,4-c]pyrazol-4-yl)-5-methyl-1H-pyrazole-3-carboxamide;
(R)-4-ethyl-N-(1-(2-fluorophenyl)-1,4,5,7-tetrahydropyrano[3,4-c]pyrazol-4-yl)-5-methyl-1H-pyrazole-3-carboxamide;
(S)-N-(1-(2-fluorophenyl)-1,4,5,7-tetrahydropyrano[3,4-c]pyrazol-4-yl)-4,5-dimethylpicolinamide;
(R)-N-(1-(2-fluorophenyl)-1,4,5,7-tetrahydropyrano[3,4-c]pyrazol-4-yl)-4,5-dimethylpicolinamide;
(S)-N-(1-(2-chloro-6-fluorophenyl)-1,4,5,7-tetrahydropyrano[3,4-c]pyrazol-4-yl)-4,5,6,7-tetrahydro-1H-indazole-3-carboxamide;
(R)-N-(1-(2-chloro-6-fluorophenyl)-1,4,5,7-tetrahydropyrano[3,4-c]pyrazol-4-yl)-4,5,6,7-tetrahydro-1H-indazole-3-carboxamide;
(S)-N-(1-(2-chloro-6-fluorophenyl)-1,4,5,7-tetrahydropyrano[3,4-c]pyrazol-4-yl)-5,6,7,8-tetrahydroimidazo[1,5-a]pyridine-3-carboxamide;
(R)-N-(1-(2-chloro-6-fluorophenyl)-1,4,5,7-tetrahydropyrano[3,4-c]pyrazol-4-yl)-5,6,7,8-tetrahydroimidazo[1,5-a]pyridine-3-carboxamide;
(S)-N-(1-(2-chloro-6-fluorophenyl)-1,4,5,7-tetrahydropyrano[3,4-c]pyrazol-4-yl)-4,5-dimethylpicolinamide;
(R)-N-(1-(2-chloro-6-fluorophenyl)-1,4,5,7-tetrahydropyrano[3,4-c]pyrazol-4-yl)-4,5-dimethylpicolinamide;
(S)-N-(1-(3-(methylsulfonyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(3-(methylsulfonyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(trifluoromethyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(trifluoromethyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chlorophenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chlorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(o-tolyl)-1,4,5,7-tetrahydropyrano[3,4-c]pyrazol-4-yl)-5,6,7,8-tetrahydroimidazo[1,5-a]pyridine-1-carboxamide;
(R)-N-(1-(o-tolyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-5-methylphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-5-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-methoxyphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-methoxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-(trifluoromethyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-(trifluoromethyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(3-methoxyphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(3-methoxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(3-(trifluoromethoxy)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(3-(trifluoromethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-fluoro-6-methylphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-fluoro-6-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2,6-difluorophenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2,6-difluorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-6-methoxyphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-6-methoxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-methoxy-6-methylphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-methoxy-6-methylphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-hydroxyphenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-hydroxyphenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-(dimethylamino)ethoxy)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(2-(dimethylamino)ethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-(dimethylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(2-(dimethylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-(methylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(2-(methylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(3-(2-acetamidoethoxy)-2-chlorophenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(3-(2-acetamidoethoxy)-2-chlorophenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-methoxyethoxy)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(2-methoxyethoxy)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(2-hydroxyethoxy)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(2-hydroxyethoxy)phenyl)-1,4,5,7-tetrahydropyrano[3,4-c]pyrazol-4-yl)-5,6,7,8-tetrahydroimidazo[1,5-a]pyridine-1-carboxamide;
Isopropyl (S)-2-chloro-3-(4-(5,6,7,8-tetrahydroimidazo[1,5-a]pyridine-1-carboxamido)-4,7-dihydropyrano[3,4-c]pyrazol-1(5H)-yl)benzoate;
Isopropyl (R)-2-chloro-3-(4-(5,6,7,8-tetrahydroimidazo[1,5-a]pyridine-1-carboxamido)-4,7-dihydropyrano[3,4-c]pyrazol-1(5H)-yl)benzoate;
(S)-N-(1-(2-chloro-3-(dimethylcarbamoyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(dimethylcarbamoyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(pyrrolidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(pyrrolidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(piperidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(piperidine-1-carbonyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(morpholine-4-carbonyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(morpholine-4-carbonyl)phenyl)-1,4,5,7-tetrahydropyrano[3,4-c]pyrazol-4-yl)-5,6,7,8-tetrahydroimidazo[1,5-a]pyridine-1-carboxamide;
(S)-N-(1-(2-chloro-3-(diethylcarbamoyl)phenyl)-1,4,5,7-tetrahydropyrano[3,4-c]pyrazol-4-yl)-5,6,7,8-tetrahydroimidazo[1,5-a]pyridine-1-carboxamide;
(R)-N-(1-(2-chloro-3-(diethylcarbamoyl)phenyl)-1,4,5,7-tetrahydropyrano[3,4-c]pyrazol-4-yl)-5,6,7,8-tetrahydroimidazo[1,5-a]pyridine-1-carboxamide;
or a pharmaceutically acceptable salts thereof.

10. A pharmaceutical composition comprising a compound as defined in any one of claims 1 to 9, and optionally one or more pharmaceutically acceptable adjuvant, diluent and/or carrier.

11. A compound as defined in any one of claims 1 to 9 or pharmaceutical composition as defined in claim 10 for use in medicine.

12. A compound as defined in any one of claims 1 to 9 or pharmaceutical composition as defined in claim 10 for use in the treatment of cancer and/or the treatment or prevention of a viral infection.

13. A combination product comprising:
(A) a compound as defined in any one of claims 1 to 9; and
(B) one or more additional therapeutic agent for the treatment of cancer or for the treatment or prevention of a viral infection,
wherein each of components (A) and (B) is formulated in admixture, optionally with one or more a pharmaceutically-acceptable adjuvant, diluent or carrier.

14. A kit-of-parts comprising:
(a) a compound as defined in any one of claims 1 to 9, or a pharmaceutical composition as defined in claim 10; and
(b) one or more additional therapeutic agent for the treatment of cancer or for the treatment or prevention of a viral infection, optionally in admixture with one or more pharmaceutically-acceptable adjuvant, diluent or carrier,
which components (a) and (b) are each provided in a form that is suitable for administration in conjunction with the other.

15. A process for the preparation of a compound as defined in any one of claims 1 to 9, which process comprises:
(i) reaction of a compound of formula II 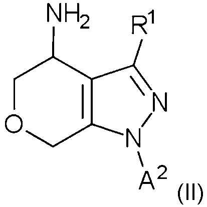
wherein R¹ and A² are as defined in claims 1 to 9, with a compound of formula III
LG₁-L¹-A¹ (III)
wherein A' and L¹ are as defined in claims 1 to 9 and LG₁ represents a suitable leaving group, in the presence of a suitable solvent and optionally in the presence of a suitable base and/or a suitable catalyst;
(ii) where L¹ represents -C(O)N(R²)- wherein R² represents H, reaction of a compound of formula II with a compound of formula IV
A¹-N=C=O (IV)
wherein A¹ is as defined in claims 1 to 9 in the presence of a suitable solvent and optionally in the presence of a suitable base and/or a suitable catalyst; or
(iii) reaction of a compound of formula (V) 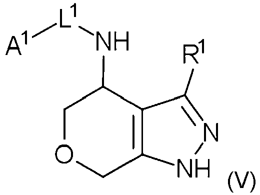
wherein R¹and A¹ and L¹ are as defined in claims 1 to 9, with a compound of formula VI
LG₂-A² (VI)
wherein A² is as defined in claims 1 to 9 and LG₂ represents a suitable leaving group, in the presence of a suitable solvent and optionally in the presence of a suitable base and/or a suitable catalyst.

## Patentansprüche

1. Verbindung der Formel I 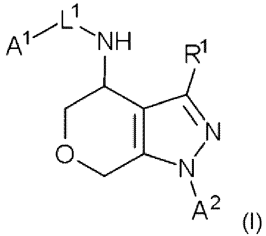
oder ein pharmazeutisch akzeptables Salz davon, wobei
A¹ ein 9-gliedriges bicyclisches Heteroaryl, 6-gliedriges Heteroaryl oder5-gliedriges Heteroaryl ist, optional substituiert mit einer oder mehreren Gruppen, die unabhängig voneinander aus G² ausgewählt sind;
A² Phenyl ist, optional substituiert durch eine oder mehrere Gruppen, die unabhängig voneinander aus G³ ausgewählt sind;
L¹für -C(O)- steht;
R¹ für H steht;
wobei:
jedes G² unabhängig Halogen, R^{a2}, -CN, -Aa2-C(Qa2)Rb2 -A^{b2}-C(Q^{b2})N(R^{c2})R^{d2}, -A^{c2}-C(Q^{c2})OR^{e2}, -A^{d2}-S(O)ₚR^{f2}, -A^{e2}-S(O)_{q}N(R^{g2})R^{h2}, -A^{l2}-S(O)pORⁱ², -N₃, -N(R^{j2})R^{k2}, -N(H)CN, -NO₂, -ONO₂, -OR¹² oder -SR^{m2} darstellt;
jedes Q^{a2} bis Q^{c2} unabhängig voneinander für =0, =S, =NRⁿ² oder =N(OR^{o2}) darstellt;
jedes A^{a2} bis A^{f2} unabhängig voneinander eine Einfachbindung, -N(R^{p2})- oder -O- darstellt;
jedes G³ unabhängig voneinander Halogen, R^{a3} , -CN, -A^{a3} - C(Q^{a3})R^{b3}, -A^{b3}-C(Q^{b3})N(R^{c3})R^{d3}, -A^{c3}-C(Q^{c3})OR^{e3}, -A^{d3}-S(O)pR^{f3}, - A^{e3}-S(O)q N(R^{g3})R^{h3}, -A^{f3}-S(O)ₚORⁱ³, -N₃, -N(R^{j3})R^{k3}, -N(H)CN, -NO₂, -ONO₂, -OR¹³ oder -SR^{m3} darstellt;
jedes Q^{a3} bis Q^{c3} unabhängig voneinander =0, =S, =NRⁿ³ oder =N(OR^{o3}) darstellt;
jedes A^{a3} bis A ^{f3} unabhängig voneinander eine Einfachbindung, -N(R^{p3})- oder -O- darstellt;
jedes R ^{a2} und R ^{f2} unabhängig voneinander C₁₋₆-Alkyl, optional substituiert durch eine oder mehrere Gruppen, unabhängig voneinander ausgewählt aus G^{6a}, Hetero-Cycloalkyl, optional substituiert durch eine oder mehrere Gruppen, die unabhängig voneinander aus G^{6b} ausgewählt sind, Aryl, optional substituiert durch eine oder mehrere Gruppen, die unabhängig voneinander aus G^{6c} ausgewählt sind, oder Heteroaryl, optional substituiert durch eine oder mehrere Gruppen, die unabhängig voneinander aus G^{6d}; ausgewählt sind, darstellt;
jedes R^{p2} unabhängig H oder C₁₋₆-Alkyl, optional substituiert durch ein oder mehrere F, darstellt;
jedes R^{b2}, R^{c2}, R^{d2}, R^{e2}, R^{g2}, R^{h2}, Rⁱ², R^{j2}, R^{k2}, R^{l2}, R^{m2}, Rⁿ² und R^{o2} unabhängig voneinander H, C₁₋₆-Alkyl, optional substituiert durch eine oder mehrere Gruppen, die unabhängig voneinander aus G^{6a} ausgewählt sind, Hetero-Cycloalkyl, optional substituiert durch eine oder mehrere Gruppen, die unabhängig voneinander aus G^{6b} ausgewählt sind, Aryl, optional substituiert durch eine oder mehrere Gruppen, die unabhängig voneinander aus G^{6c} ausgewählt sind, oder Heteroaryl, optional substituiert durch eine oder mehrere Gruppen, die unabhängig voneinander aus G^{6d}; ausgewählt sind, darstellt;
oder
alternativ beliebige R^{c2} und R^{d2}, R^{g2} und R^{h2} und/oder R^{j2} und R^{k2} miteinander verbunden sind, um zusammen mit dem Stickstoffatom, an das sie gebunden sind, einen 3- bis 6-gliedrigen Ring zu bilden, wobei der Ring optional ein weiteres Heteroatom enthält, und der Ring optional durch eine oder mehrere Gruppen substituiert ist, die unabhängig voneinander aus Halogen, C₁₋₃-Alkyl, optional substituiert durch ein oder mehrere Halogenatome, und =O ausgewählt sind,
jedes R^{a3} und R^{f3} unabhängig voneinander C₁₋₆-Alkyl, optional substituiert durch eine oder mehrere Gruppen, die unabhängig voneinander aus G^{7a} ausgewählt sind, Hetero-Cycloalkyl, optional substituiert durch eine oder mehrere Gruppen, die unabhängig voneinander aus G^{7b} ausgewählt sind, Aryl, optional substituiert durch eine oder mehrere Gruppen, die unabhängig voneinander aus G_{7c} ausgewählt sind, oder Heteroaryl, optional substituiert durch eine oder mehrere Gruppen, die unabhängig voneinander aus G^{7d} ausgewählt sind, darstellt;
jedes R^{p3} unabhängig H oder C₁₋₆-Alkyl, das optional durch ein oder mehrere F substituiert ist, darstellt;
jedes R^{b3}, R^{c3}, R^{d3}, R^{e3}, R^{g3}, R^{h3}, Rⁱ³, Rⁱ³, R^{k3}, R^{l3}, Rm3, Rⁿ³ und R^{o3} unabhängig voneinander H, C₁₋₆-Alkyl, optional substituiert durch eine oder mehrere Gruppen, die unabhängig voneinander aus G^{7a} ausgewählt sind, Hetero-Cycloalkyl, optional substituiert durch eine oder mehrere Gruppen, die unabhängig voneinander aus G^{7b} ausgewählt sind, Aryl, optional substituiert durch eine oder mehrere Gruppen, die unabhängig voneinander aus G^{7c} ausgewählt sind, oder Heteroaryl, optional substituiert durch eine oder mehrere Gruppen, die unabhängig voneinander aus G^{7d} ausgewählt sind, darstellt; oder
alternativ beliebige R^{c3} und R^{d3}, R^{g3} und R^{h3} und/oder R^{j3} und R^{k3} miteinander verbunden sind, um zusammen mit dem Stickstoffatom, an das sie gebunden sind, einen 3- bis 6-gliedrigen Ring zu bilden, wobei der Ring optional ein weiteres Heteroatom enthält und wobei der Ring optional durch eine oder mehrere Gruppen substituiert ist, die unabhängig voneinander aus Halogen, C₁₋₃-Alkyl, das optional durch ein oder mehrere Halogen substituiert ist, und =O ausgewählt sind;
jedes G6a, G^{6b}, G^{7a} und G^{7b} unabhängig voneinander Halogen, -CN, -N(R^{b5})R^{c5}, -OR^{d5}, -SR^{e5} oder =O darstellt;
jedes G^{6c}, G^{6d}, G^{7c} und G^{7d} unabhängig voneinander Halogen, R^{a5}, -CN, -N(R^{b5})R^{c5}, -OR^{d5}, -SR^{e5} oder =O darstellt;
jedes R^{a5} unabhängig voneinander C₁₋₆-Alkyl, optional substituiert durch ein oder mehrere F, darstellt;
jedes R^{b5}, R^{c5}, R^{d5} und R^{e5} unabhängig voneinander H oder C₁₋₆-Alkyl, optional substituiert durch ein oder mehrere F oder =O, darstellt; und
oder R^{b5} und R^{c5} miteinander verbunden sind, um zusammen mit dem Stickstoffatom, an das sie gebunden sind, einen 3- bis 6-gliedrigen Ring zu bilden, der optional ein weiteres Heteroatom enthält und der optional durch eine oder mehrere Gruppen substituiert ist, die unabhängig voneinander aus F, C₁₋₃-Alkyl, das optional durch ein oder mehrere F substituiert ist, und =O ausgewählt sind;
und p und q jeweils unabhängig 1 oder 2 bedeuten.

2. Verbindung nach Anspruch 1, bei der
jedes G² unabhängig für -CN, -A^{a2}-C(Q^{a2})R^{b2} -A^{b2}-C(Q^{b2})N(R^{c2})R^{d2}, -A^{c2}-C(Q^{c2})OR^{e2}, -A^{d2}-S(O)pR^{f2}, -A^{e2}-S(O)qN(R^{g2})R^{h2}, -A^{f2}-S(O)ₚORⁱ², -N₃, -N(R^{j2})R^{k2}, -N(H)CN, -NO₂, - ONO₂, Halogen, R^{a2}, -OR12 oder -SR^{m2} steht; jedes Q^{a2} bis Q^{c2} unabhängig voneinander für =0, =S, =NRⁿ² oder = N(OR^{o2}) steht; und/oder jedes A^{a2} bis A^{f2} unabhängig voneinander für eine Einfachbindung, -N(R^{p2})- oder -O- steht; und/oder
jedes G³ unabhängig voneinander für -CN, -A^{a3} -C(Q^{a3})R^{b3}, - A^{b3}-C(Q^{b3})N(R^{e3})R^{d3}, -A^{c3}-C(Q^{e3})OR^{e3}, -A^{d3}-S(O)ₚR^{f3}, -A^{e3}-S(O)_{q}N(R^{g3})R^{h3}, -A^{f3}-S(O)pORⁱ³, -N₃, -N(R^{j3})R^{k3}, -N(H)CN, -NO₂, - ONO₂, Halogen, R^{a3}, -OR¹³ oder -SR^{m3} steht; jedes Q^{a3} bis Q^{c3} unabhängig voneinander für =O, =S, =NRⁿ³ oder =N(OR^{o3}) steht; und/oder jedes A^{a3} bis A^{f3} unabhängig für eine Einfachbindung, -N(Rp3)- oder -O- steht.

3. Verbindung nach Anspruch 1 oder 2, bei der
A¹ durch eine oder zwei Gruppen substituiert ist, die unabhängig voneinander ausgewählt sind aus Halogen, R^{a2}, -C(O)OR^{e2}, -OR^{l2} und -SR^{m2}; oder C₁₋₃-Alkyl, optional substituiert durch ein oder mehrere Fluor, -C(O)OH, -C(O)OC₁₋₃-Alkyl, -OH, - OC₁₋₃-Alkyl, -SH und -SC₁₋₃-Alkyl;

4. Verbindung nach einem der Ansprüche 1 bis 3, bei der A¹ ausgewählt ist aus Tetrahydro-2,1-benzisoxazolyl, Benzoxazoyl, Pyrazinyl, Indazolyl, 5H,6H,7H,8H-imidazo[1,5-a]pyridin-3-yl, 5H,6H,7H,8H-imidazo[1,5-a]pyridin-1-yl, Imidazo[1,5-a]pyridin-3-yl, Pyridinyl, Thiazolyl, Isoxazolyl und Tetrahydro-1,2-benzisoxazolyl.

5. Verbindung nach einem der Ansprüche 1 bis 4, bei der
A² durch eine oder mehrere Gruppen substituiert ist, die unabhängig voneinander ausgewählt sind aus Halogen, -A^{a3}-C(Q^{a3})R^{b3}, -A^{c3}-C(Q^{c3})OR^{e3}, R^{a3} und -OR^{l3}; oder
A² durch eine oder mehrere Gruppen substituiert ist, die unabhängig voneinander ausgewählt sind aus Halogen, -C(O)-Morpholinyl, -C(O)OC₁₋₃-Alkyl und C₁₋₄-Alkyl.

6. Verbindung nach einem der Ansprüche 1 bis 3 oder 5, bei der
A¹ 5H,6H,7H,8H-imidazo[1,5-a]pyridin-3-yl, 5H,6H,7H,8H-imidazo[1,5-a]pyridin-1-yl, 4H,5H,6H,7H-1,2-benzisoxazol-3-yl,4H,5H,6H,7H-2,1-Benzisoxazol-3-yl oder 4H,5H,6H,7H-Indazol-3-yl; Pyridinyl, Pyrazolyl oder Imidazolyl ist.

7. Verbindung nach einem der Ansprüche 1 bis 6, bei der:
jedes G2 unabhängig für R^{a2} steht; und/oder
jedes G3 unabhängig für Halogen, R^{a3}, -A^{b3}-C(Q^{b3})N(R^{c3})R^{d3}, -A^{c3}-C(Q^{c3})OR^{e3}, -A^{d3}-S(O)ₚR^{f3}, -OR^{l3} steht;
optional wobei
Halogen Fluor oder Chlor ist, R^{a3} CH₃ oder CF₃ ist, A^{b3}, A^{c3} und A^{d3} eine Einfachbindung sind, Q^{b3} und Q^{c3} =O sind, p 2 ist,
R^{c3} und R^{d3} CH₃ sind oder miteinander verbunden sind, um zusammen mit dem Stickstoffatom, an das sie gebunden sind, einen 5- oder 6-gliedrigen Ring zu bilden, wobei der Ring optional ein weiteres Heteroatom enthält,
R^{e3} C₁₋₃-Alkyl ist, R^{f3} CH₃ ist,
R^{l3} CH₃, CF₃, C₂-Alkyl substituiert mit N(CH₃)₂, OH, OCH₃ oder N(C=O)CH₃, und/oder C₁-Alkyl, substituiert mit C(=O)N(CH₃)₂ ist.

8. Verbindung nach einem der Ansprüche 1 bis 7, wobei die Verbindung eine Verbindung der Formel 1a 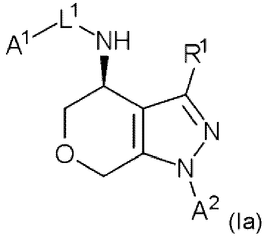
oder ein pharmazeutisch akzeptables Salz davon ist, wobei A¹,
A², L¹ und R¹ wie in einem der Ansprüche 1 bis 7 definiert sind.

9. Verbindung nach Anspruch 1, wobei die Verbindung
N-(1-(2-Fluorphenyl)-1,4,5,7-tetrahydropyrano[3,4-c]pyrazol-4-yl)-5, 6,7,8-tetrahydroimidazo[1,5-a]pyridin-1-carboxamid
(R)-N-(1-(2-Fluorphenyl)-1,4,5,7-tetrahydropyrano[3,4-c]pyrazol-4-yl)-5, 6,7,8-tetrahydroimidazo[1,5-a]pyridin-1-carboxamid
(S)-N-(1-(2-Fluorphenyl)-1,4,5,7-tetrahydropyrano[3,4-c]pyrazol-4-yl)-5, 6,7,8-tetrahydroimidazo[1,5-a]pyridin-1-carboxamid
N-(1-(2-Fluorphenyl)-1,4,5,7-tetrahydropyrano[3,4-c]pyrazol-4-yl)-4,5,6,7-tetrahydrobenzo[d]isoxazol-3-carboxamid
(R)-N-(1-(2-Fluorphenyl)-1,4,5,7-tetrahydropyrano[3,4-c]pyrazol-4-yl)-4,5,6,7-tetrahydrobenzo[d]isoxazol-3-carboxamid
(S)-N-(1-(2-Fluorphenyl)-1,4,5,7-tetrahydropyrano[3,4-c]pyrazol-4-yl)-4,5,6,7-tetrahydrobenzo[d]isoxazol-3-carboxamid
N-(1-(2-Fluorphenyl)-1,4,5,7-tetrahydropyrano[3,4-c]pyrazol-4-yl)-5, 6,7,8-tetrahydroimidazo[1,5-a]pyridin-3-carboxamid
(R)-N-(1-(2-Fluorphenyl)-1,4,5,7-tetrahydropyrano[3,4-c]pyrazol-4-yl)-5, 6,7,8-tetrahydroimidazo[1,5-a]pyridin-3-carboxamid
(S)-N-(1-(2-Fluorphenyl)-1,4,5,7-tetrahydropyrano[3,4-c]pyrazol-4-yl)-5, 6,7,8-tetrahydroimidazo[1,5-a]pyridin-3-carboxamid
N-1-(2-Chlor-6-fluor-3-methoxyphenyl)-1,4,5,7-tetrahydropyrano[3,4-c]pyrazol-4-yl)-5,6,7,8-tetrahydroimidazo[1,5-a]pyridin-1-carboxamid
N-((4S)-1-(2-Chlor-6-fluor-3-methoxyphenyl)-1,4,5,7-tetrahydropyrano[3,4-c]pyrazol-4-yl)-5,6,7,8-tetrahydroimidazo[1,5-a]pyridin-1-carboxamid
N-((4R)-1-(2-Chlor-6-fluor-3-methoxyphenyl)-1,4,5,7-tetrahydropyrano[3,4-c]pyrazol-4-yl)-5,6,7,8-tetrahydroimidazo[1,5-a]pyridin-1-carboxamid
N-1-(2-Chlor-6-fluorphenyl)-1,4,5,7-tetrahydropyrano[3,4-c]pyrazol-4-yl)-5, 6,7,8-tetrahydroimidazo[1,5-a]pyridin-1-carboxamid
N-((4S)-1-(2-Chlor-6-fluorphenyl)-1,4,5,7-tetrahydropyrano[3,4-c]pyrazol-4-yl)-5,6,7,8-tetrahydroimidazo[1,5-a]pyridin-1-carboxamid
N-((4R)-1-(2-Chlor-6-fluorphenyl)-1,4,5,7-tetrahydropyrano[3,4-c]pyrazol-4-yl)-5,6,7,8-tetrahydroimidazo[1,5-a]pyridin-1-carboxamid
N-(1-(2,6-Dichlorphenyl)-1,4,5,7-tetrahydropyrano[3,4-c]pyrazol-4-yl)-5,6,7,8-tetrahydroimidazo[1,5-a]pyridin-1-carboxamid
(S)-N-(1-(2,6-Dichlorphenyl)-1,4,5,7-tetrahydropyrano[3,4-c]pyrazol-4-yl)-5,6,7,8-tetrahydroimidazo[1,5-a]pyridin-1-carboxamid
(R)-N-(1-(2,6-Dichlorphenyl)-1,4,5,7-tetrahydropyrano[3,4-c]pyrazol-4-yl)-5,6,7,8-tetrahydroimidazo[1,5-a]pyridin-1-carboxamid
(S)-N-(1-(2-Chlor-3-methylphenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Cloro-3-methylphenyl)-1,4,5,7-tetrahydropyrano[3,4-c]pyrazol-4-yl)-5,6,7,8-tetrahydroimidazo[1 ,5-a]pyridin-1-carboxamid;
(S)-N-(1-(2-Chlor-3-methylphenyl)-1,4,5,7-tetrahydropyrano[3,4-c]pyrazol-4-yl)-5-ethyl-1-methyl-1H-imidazol-4-carboxamid;
(R)-N-(1-(2-chloro-3-methylphenyl)-1,4,5,7-tetrahydropyrano[3,4-c]pyrazol-4-yl)-5-ethyl-1-methyl-1H-imidazol-4-carboxamid;
(S)-N-(1-(2-Chlor-3-methylphenyl)-1,4,5,7-tetrahydropyrano[3,4-c]pyrazol-4-yl)-5,6,7,8-tetrahydroimidazo [1,5-a]pyridin-3-carboxamid;
(R)-N-(1-(2-Chlor-3-methylphenyl)-1,4,5,7-tetrahydropyrano[3,4-c]pyrazol-4-yl)-5,6,7,8-tetrahydroimidazo [1,5-a]pyridin-3-carboxamid;
(S)-N-(1-(2-Chlor-3-methylphenyl)-1,4,5,7-tetrahydropyrano[3,4-c]pyrazol-4-yl)-4,5,6,7-tetrahydro-1H-indazol-3-carboxamid;
(R)-N-(1-(2-Chlor-3-methylphenyl)-1,4,5,7-tetrahydropyrano[3,4-c]pyrazol-4-yl)-4,5,6,7-tetrahydro-1H-indazol-3-carboxamid;
(S)-N-(1-(2-Chlor-3-methylphenyl)-1,4,5,7-tetrahydropyrano[3,4-c]pyrazol-4-yl)-4,5,6,7-tetrahydrobenzo[d]isoxazol-3-carboxamid;
(R)-N-(1-(2-Chlor-3-methylphenyl)-1,4,5,7-tetrahydropyrano[3,4-c]pyrazol-4-yl)-4,5,6,7-tetrahydrobenzo[d]isoxazol-3-carboxamid;
(S)-N-(1-(2-Chlor-3-methylphenyl)-1,4,5,7-tetrahydropyrano[3,4-c]pyrazol-4-yl)-4-ethyl-5-methyl-1H-pyrazol-3-carboxamid;
(R)-N-(1-(2-Chlor-3-methylphenyl)-1,4,5,7-tetrahydropyrano[3,4-c]pyrazol-4-yl)-4-ethyl-5-methyl-1H-pyrazol-3-carboxamid;
(S)-N-(1-(2-Chlor-3-methylphenyl)-1,4,5,7-tetrahydropyrano[3,4-c]pyrazol-4-yl)-4,5-dimethylpicolinamid;
(R)-N-(1-(2-Chlor-3-methylphenyl)-1,4,5,7-tetrahydropyrano[3,4-c]pyrazol-4-yl)-4,5-dimethylpicolinamid;
(S)-N-(1-(2-Chlor-3-methoxyphenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-3-methoxyphenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-3-methoxyphenyl)-1,4,5,7-tetrahydropyrano[3,4-c]pyrazol-4-yl)-5-ethyl-1-methyl-1H-imidazol-4-carboxamid;
(R)-N-(1-(2-Chlor-3-methoxyphenyl)-1,4,5,7-tetrahydropyrano[3,4-c]pyrazol-4-yl)-5-ethyl-1-methyl-1H-imidazol-4-carboxamid;
(S)-N-(1-(2-Chlor-3-methoxyphenyl)-1,4,5,7-tetrahydropyrano[3,4-c]pyrazol-4-yl)-5,6,7,8-tetrahydroimidazo[1,5-a]pyridin-3-carboxamid;
(R)-N-(1-(2-Chlor-3-methoxyphenyl)-1,4,5,7-tetrahydropyrano[3,4-c]pyrazol-4-yl)-5,6,7,8-tetrahydroimidazo[1,5-a]pyridin-3-carboxamid;
(S)-N-(1-(2-Chlor-3-methoxyphenyl)-1,4,5,7-tetrahydropyrano[3,4-c]pyrazol-4-yl)-4,5,6,7-tetrahydro-1H-indazol-3-carboxamid;
(R)-N-(1-(2-Chlor-3-methoxyphenyl)-1,4,5,7-tetrahydropyrano[3,4-c]pyrazol-4-yl)-4,5,6,7-tetrahydro-1H-indazol-3-carboxamid;
(S)-N-(1-(2-Chlor-3-methoxyphenyl)-1,4,5,7-tetrahydropyrano[3,4-c]pyrazol-4-yl)-4,5,6,7-tetrahydrobenzo[d]isoxazol-3-carboxamid;
(R)-N-(1-(2-Chlor-3-methoxyphenyl)-1,4,5,7-tetrahydropyrano[3,4-c]pyrazol-4-yl)-4,5,6,7-tetrahydrobenzo[d]isoxazol-3-carboxamid;
(S)-N-(1-(2-Chlor-3-methoxyphenyl)-1,4,5,7-tetrahydropyrano[3,4-c]pyrazol-4-yl)-4-ethyl-5-methyl-1H-pyrazol-3-carboxamid;
(R)-N-(1-(2-Chlor-3-methoxyphenyl)-1,4,5,7-tetrahydropyrano[3,4-c]pyrazol-4-yl)-4-ethyl-5-methyl-1H-pyrazol-3-carboxamid;
(S)-N-(1-(2-Chlor-6-fluor-3-methoxyphenyl)-1,4,5,7-tetrahydropyrano[3,4-c]pyrazol-4-yl)-4,5-dimethylpicolinamid;
(R)-N-(1-(2-Chlor-6-fluor-3-methoxyphenyl)-1,4,5,7-tetrahydropyrano[3,4-c]pyrazol-4-yl)-4,5-dimethylpicolinamid;
(S)-N-(1-(2-Chlor-6-fluor-3-methoxyphenyl)-1,4,5,7-tetrahydropyrano[3,4-c]pyrazol-4-yl)-5-ethyl-1-methyl-1H-imidazol-4-carboxamid;
(R)-N-(1-(2-Chlor-6-fluor-3-methoxyphenyl)-1,4,5,7-tetrahydropyrano[3,4-c]pyrazol-4-yl)-5-ethyl-1-methyl-1H-imidazol-4-carboxamid;
(S)-N-(1-(2-Chlor-6-fluor-3-methoxyphenyl)-1,4,5,7-tetrahydropyrano[3,4-c]pyrazol-4-yl)-5,6,7,8-tetrahydroimidazo [1,5-a]pyridine-3-carboxamid;
(R)-N-(1-(2-Chlor-6-fluor-3-methoxyphenyl)-1,4,5,7-tetrahydropyrano[3,4-c]pyrazol-4-yl)-5,6,7,8-tetrahydroimidazo[1,5-a]pyridin-3-carboxamid;
(S)-N-(1-(2-Chlor-6-fluor-3-methoxyphenyl)-1,4,5,7-tetrahydropyrano[3,4-c]pyrazol-4-yl)-4,5,6,7-tetrahydro-1H-indazol-3-carboxamid;
(R)-N-(1-(2-Chlor-6-fluor-3-methoxyphenyl)-1,4,5,7-tetrahydropyrano[3,4-c]pyrazol-4-yl)-4,5,6,7-tetrahydro-1H-indazol-3-carboxamid;
(S)-N-(1-(2-Chlor-6-fluor-3-methoxyphenyl)-1,4,5,7-tetrahydropyrano[3,4-c]pyrazol-4-yl)-4,5,6,7-tetrahydrobenzo[d]isoxazol-3-carboxamid;
(R)-N-(1-(2-Chlor-6-fluor-3-methoxyphenyl)-1,4,5,7-tetrahydropyrano[3,4-c]pyrazol-4-yl)-4,5,6,7-tetrahydrobenzo[d]isoxazol-3-carboxamid;
(S)-N-(1-(2-Chlor-6-fluor-3-methoxyphenyl)-1,4,5,7-tetrahydropyrano[3,4-c]pyrazol-4-yl)-4-ethyl-5-methyl-1H-pyrazol-3-carboxamid;
(R)-N-(1-(2-Chlor-6-fluor-3-methoxyphenyl)-1,4,5,7-tetrahydropyrano[3,4-c]pyrazol-4-y]l)-4-ethyl-5-methyl-1H-pyrazol-3-carboxamid;
(S)-N-(1-(2-Chlor-6-fluor-3-methoxyphenyl)-1,4,5,7-tetrahydropyrano[3,4-c]pyrazol-4-yl)-4,5-dimethylpicolinamid;
(R)-N-(1-(2-Chlor-6-fluor-3-methoxyphenyl)-1,4,5,7-tetrahydropyrano[3,4-c]pyrazol-4-yl)-4,5-dimethylpicolinamid;
(S)-N-(1-(2-Chlor-6-methylphenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-6-methylphenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-6-methylphenyl)-1,4,5,7-tetrahydropyrano[3,4-c]pyrazol-4-yl)-5-ethyl-1-methyl-1H-imidazol-4-carboxamid;
(R)-N-(1-(2-Chlor-6-methylphenyl)-1,4,5,7-tetrahydropyrano[3,4-c]pyrazol-4-yl)-5-ethyl-1-methyl-1H-imidazol-4-carboxamid;
(S)-N-(1-(2-Chlor-6-methylphenyl)-1,4,5,7-tetrahydropyrano[3,4-c]pyrazol-4-yl)-5,6,7,8-tetrahydroimidazo[1,5-a]pyridin-3-carboxamid;
(R)-N-(1-(2-Chlor-6-methylphenyl)-1,4,5,7-tetrahydropyrano[3,4-c]pyrazol-4-yl)-5,6,7,8-tetrahydroimidazo[1,5-a]pyridin-3-carboxamid;
(S)-N-(1-(2-Chlor-6-methylphenyl)-1,4,5,7-tetrahydropyrano[3,4-c]pyrazol-4-yl)-4,5,6,7-tetrahydro-1H-indazol-3-carboxamid;
(R)-N-(1-(2-Chlor-6-methylphenyl)-1,4,5,7-tetrahydropyrano[3,4-c]pyrazol-4-yl)-4,5,6,7-tetrahydro-1H-indazol-3-carboxamid;
(S)-N-(1-(2-Chlor-6-methylphenyl)-1,4,5,7-tetrahydropyrano[3,4-c]pyrazol-4-yl)-4,5,6,7-tetrahydrobenzo[d]isoxazol-3-carboxamid;
(R)-N-(1-(2-Chlor-6-methylphenyl)-1,4,5,7-tetrahydropyrano[3,4-c]pyrazol-4-yl)-4,5,6,7-tetrahydrobenzo[d]isoxazol-3-carboxamid;
(S)-N-(1-(2-Chlor-6-methylphenyl)-1,4,5,7-tetrahydropyrano[3,4-c]pyrazol-4-yl)-4-ethyl-5-methyl-1H-pyrazol-3-carboxamid;
(R)-N-(1-(2-Chlor-6-methylphenyl)-1,4,5,7-tetrahydropyrano[3,4-c]pyrazol-4-yl)-4-ethyl-5-methyl-1H-pyrazol-3-carboxamid;
(S)-N-(1-(2-Chlor-6-methylphenyl)-1,4,5,7-tetrahydropyrano[3,4-c]pyrazol-4-yl)-4,5-dimethylpicolinamid;
(R)-N-(1-(2-Chlor-6-methylphenyl)-1,4,5,7-tetrahydropyrano[3,4-c]pyrazol-4-yl)-4,5-dimethylpicolinamid;
(S)-5-Ethyl-N-(1-(2-fluorphenyl)-1,4,5,7-tetrahydropyrano[3,4-c]pyrazol-4-yl)- 1-methyl- 1H-imidazol-4-carboxamid;
(R)-5-Ethyl-N-(1-(2-fluorphenyl)-1,4,5,7-tetrahydropyrano[3,4-c]pyrazol-4-yl)- 1-methyl- 1H-imidazol-4-carboxamid;
(S)-N-(1-(2-Fluorphenyl)-1,4,5,7-tetrahydropyrano[3,4-c]pyrazol-4-yl)-4,5,6,7-tetrahydro-1H-indazol-3-carboxamid;
(R)-N-(1-(2-Fluorphenyl)-1,4,5,7-tetrahydropyrano[3,4-c]pyrazol-4-yl)-4,5,6,7-tetrahydro-1H-indazol-3-carboxamid;
(S)-N-(1-(2-Fluorphenyl)-1,4,5,7-tetrahydropyrano[3,4-c]pyrazol-4-yl)-4,5,6,7-tetrahydrobenzo[d]isoxazol-3-carboxamid;
(R)-N-(1-(2-Fluorphenyl)-1,4,5,7-tetrahydropyrano[3,4-c]pyrazol-4-yl)-4,5,6,7-tetrahydrobenzo[d]isoxazol-3-carboxamid;
(S)-4-Ethyl-N-(1-(2-fluorphenyl)-1,4,5,7-tetrahydropyrano[3,4-c]pyrazol-4-yl)-5-methyl- 1H-pyrazol-3-carboxamid;
(R)-4-Ethyl-N-(1-(2-fluorphenyl)-1,4,5,7-tetrahydropyrano[3,4-c]pyrazol-4-yl)-5-methyl- 1H-pyrazol-3-carboxamid;
(S)-N-(1-(2-Fluorphenyl)-1,4,5,7-tetrahydropyrano[3,4-c]pyrazol-4-yl)-4,5-dimethylpicolinamid;
(R)-N-(1-(2-Fluorphenyl)-1,4,5,7-tetrahydropyrano[3,4-c]pyrazol-4-yl)-4,5-dimethylpicolinamid;
(S)-N-(1-(2-Chlor-6-fluorphenyl)-1,4,5,7-tetrahydropyrano[3,4-c]pyrazol-4-yl)-4,5,6,7-tetrahydro-1H-indazol-3-carboxamid;
(R)-N-(1-(2-Chlor-6-fluorphenyl)-1,4,5,7-tetrahydropyrano[3,4-c]pyrazol-4-yl)-4,5,6,7-tetrahydro-1H-indazol-3-carboxamid;
(S)-N-(1-(2-Chlor-6-fluorphenyl)-1,4,5,7-tetrahydropyrano[3,4-c]pyrazol-4-yl)-5, 6,7,8-tetrahydroimidazo[1,5-a]pyridin-3-carboxamid;
(R)-N-(1-(2-Chlor-6-fluorphenyl)-1,4,5,7-tetrahydropyrano[3,4-c]pyrazol-4-yl)-5,6,7,8-tetrahydroimidazo[1,5-a]pyridine-3-carboxamid;
(S)-N-(1-(2-Chlor-6-fluorphenyl)-1,4,5,7-tetrahydropyrano[3,4-c]pyrazol-4-yl)-4,5-dimethylpicolinamid;
(R)-N-(1-(2-Chlor-6-fluorphenyl)-1,4,5,7-tetrahydropyrano[3,4-c]pyrazol-4-yl)-4,5-dimethylpicolinamid;
(S)-N-(1-(3-(Methylsulfonyl)phenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(3-(Methylsulfonyl)phenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-3-(trifluormethyl)phenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-3-(trifluormethyl)phenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlorphenyl)-1,4,5,7-tetrahydropyrano[3,4-c]pyrazol-4-yl)-5, 6,7,8-tetrahydroimidazo[1,5-a]pyridin-1-carboxamid;
(R)-N-(1-(2-Chlorphenyl)-1,4,5,7-tetrahydropyrano[3,4-c]pyrazol-4-yl)-5, 6,7,8-tetrahydroimidazo[1,5-a]pyridin-1-carboxamid;
(S)-N-(1-(o-Tolyl)-1,4,5,7-tetrahydropyrano[3,4-c]pyrazol-4-yl)-5,6,7,8-tetrahydroimidazo[1,5-a]pyridin-1-carboxamid;
(R)-N-(1-(o-Tolyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-5-methylphenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-5-methylphenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Methoxyphenyl)-1,4,5,7-tetrahydropyrano[3,4-c]pyrazol-4-yl)-5, 6,7,8-tetrahydroimidazo[1,5-a]pyridin-1-carboxamid;
(R)-N-(1-(2-Methoxyphenyl)-1,4,5,7-tetrahydropyrano[3,4-c]pyrazol-4-yl)-5, 6,7,8-tetrahydroimidazo[1,5-a]pyridin-1-carboxamid;
(S)-N-(1-(2-(Trifluormethyl)phenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-(Trifluormethyl)phenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(3-Methoxyphenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(3-Methoxyphenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(3-(Trifluormethoxy)phenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(3-(Trifluormethoxy)phenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Fluor-6-methylphenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Fluor-6-methylphenyl)-1,4,5,7-tetrahydropyrano[3,4-c]pyrazol-4-yl)-5, 6,7,8-tetrahydroimidazo[1,5-a]pyridin-1-carboxamid;
(S)-N-(1-(2,6-Difluorphenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2,6-Difluorphenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-6-methoxyphenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-6-methoxyphenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-6-methoxyphenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Methoxy-6-methylphenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Methoxy-6-methylphenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-3-hydroxyphenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-3-hydroxyphenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-3-(2-(dimethylamino)ethoxy)phenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-3-(2-(dimethylamino)ethoxy)phenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-3-(2-(dimethylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamid;
(R)-N-(1-(2-Chlor-3-(2-(dimethylamino)-2-oxoethoxy)phenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-3-(2-(methylamino)-2-oxoethoxy)phenyl)-1,4,5,7-tetrahydropyrano[3,4-c]pyrazol-4-yl)-5,6,7,8-tetrahydroimidazo[1,5-a]pyridine-1-carboxamid;
(R)-N-(1-(2-Chlor-3-(2-(methylamino)-2-oxoethoxy)phenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(3-(2-Acetamidoethoxy)-2-chlorphenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(3-(2-Acetamidoethoxy)-2-chlorophenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-3-(2-methoxyethoxy)phenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-3-(2-methoxyethoxy)phenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-3-(2-hydroxyethoxy)phenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-3-(2-hydroxyethoxy)phenyl)-1,4,5,7-tetrahydropyrano[3,4-c]pyrazol-4-yl)-5,6,7,8-tetrahydroimidazo[1,5-a]pyridin-1-carboxamid;
Isopropyl-(S)-2-chlor-3-(4-(5,6,7,8-tetrahydroimidazo[1,5-a]pyridin-1-carboxamido)-4,7-dihydropyrano[3,4-c]pyrazol-1(5H)-yl)benzoat;
Isopropyl-(R)-2-chlor-3-(4-(5,6,7,8-tetrahydroimidazo[1,5-a]pyridin-1-carboxamido)-4,7-dihydropyrano[3,4-c]pyrazol-1(5H)-yl)benzoate;
(S)-N-(1-(2-Chlor-3-(dimethylcarbamoyl)phenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-3-(dimethylcarbamoyl)phenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-3-(pyrrolidin-1-carbonyl)phenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-3-(pyrrolidin-1-carbonyl)phenyl)-1,4,5,7-tetrahydropyrano[3,4-c]pyrazol-4-yl)-5,6,7,8-tetrahydroimidazo[1,5-a]pyridin-1-carboxamide;
(S)-N-(1-(2-Chlor-3-(piperidine-1-carbonyl)phenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-3-(piperidine-1-carbonyl)phenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-3-(morpholine-4-carbonyl)phenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-3-(morpholin-4-carbonyl)phenyl)-1,4,5,7-tetrahydropyrano[3,4-c]pyrazol-4-yl)-5,6,7,8-tetrahydroimidazo[1,5-a]pyridin-1-carboxamid;
(S)-N-(1-(2-Chlor-3-(diethylcarbamoyl)phenyl)-1,4,5,7-tetrahydropyrano[3,4-c]pyrazol-4-yl)-5,6,7,8-tetrahydroimidazo[1,5-a]pyridin-1-carboxamid;
(R)-N-(1-(2-Chlor-3-(diethylcarbamoyl)phenyl)-1,4,5,7-tetrahydropyrano[3,4-c]pyrazol-4-yl)-5,6,7,8-tetrahydroimidazo[1,5-a]pyridin-1-carboxamid;
oder ein pharmazeutisch akzeptables Salz davon ist.

10. Pharmazeutische Zusammensetzung, umfassend eine Verbindung wie in einem der Ansprüche 1 bis 9 definiert, und optional einen oder mehrere pharmazeutisch verträgliche Hilfsstoffe, Verdünnungsmittel und/oder Träger.

11. Verbindung, wie in einem der Ansprüche 1 bis 9 definiert, oder pharmazeutische Zusammensetzung, wie in Anspruch 10 definiert, zur Verwendung in der Medizin.

12. Verbindung nach einem der Ansprüche 1 bis 9 oder pharmazeutische Zusammensetzung nach Anspruch 10 zur Verwendung bei der Behandlung von Krebs und/oder der Behandlung oder Vorbeugung einer Virusinfektion.

13. Kombinationsprodukt, umfassend:
(A) eine Verbindung nach einem der Ansprüche 1 bis 9; und
(B) einen oder mehrere zusätzliche therapeutische Wirkstoffe zur Behandlung von Krebs oder zur Behandlung oder Vorbeugung einer Virusinfektion,
wobei jeder der Bestandteile (A) und (B) in einer Mischung, optional mit einem oder mehreren pharmazeutisch verträglichen Adjuvanzien, Verdünnungsmitteln oder Trägern, formuliert ist.

14. Teile-Kit, umfassend:
(a) eine Verbindung, wie in einem der Ansprüche 1 bis 9 definiert, oder eine pharmazeutische Zusammensetzung, wie in Anspruch 10 definiert; und
(b) ein oder mehrere zusätzliche therapeutische Mittel zur Behandlung von Krebs oder zur Behandlung oder Vorbeugung einer Virusinfektion, optional unter Hinzufügung von einem oder mehreren pharmazeutisch akzeptablen Adjuvanzien, Verdünnungsmitteln oder Trägern,
wobei die Komponenten (a) und (b) jeweils in einer Form bereitgestellt sind, die zur Verabreichung in Verbindung miteinander geeignet ist.

15. Verfahren zur Herstellung einer Verbindung nach einem der Ansprüche 1 bis 9, wobei das Verfahren umfasst:
(i) Umsetzen einer Verbindung der Formel II 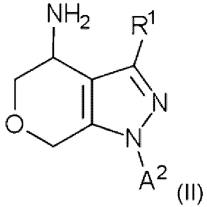 worin R¹ und A² wie in den Ansprüchen 1 bis 9 definiert sind, mit einer Verbindung der Formel III
LG₁-L¹-A¹ (III)
wobei A¹ und L¹ wie in den Ansprüchen 1 bis 9 definiert sind und LG₁ eine geeignete Abgangsgruppe darstellt, in Gegenwart eines geeigneten Lösungsmittels und optional in Gegenwart einer geeigneten Base und/oder eines geeigneten Katalysators;
(ii) wenn L¹ für -C(O)N(R2)- und R² für H steht, Umsetzen einer Verbindung der Formel II mit einer Verbindung der Formel IV
A¹-N=C=O (IV)
wobei A¹ wie in den Ansprüchen 1 bis 9 definiert ist, in Gegenwart eines geeigneten Lösungsmittels und optional in Gegenwart einer geeigneten Base und/oder eines geeigneten Katalysators; oder
(iii) Umsetzen einer Verbindung der Formel (V) 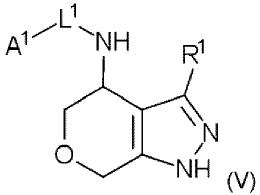 wobei R¹ und A¹ und L¹ wie in den Ansprüchen 1 bis 9 definiert sind, mit einer Verbindung der Formel VI
LG₂-A² (VI)
wobei A² wie in den Ansprüchen 1 bis 9 definiert ist und LG₂ eine geeignete Abgangsgruppe darstellt, in Gegenwart eines geeigneten Lösungsmittels und optional in Gegenwart einer geeigneten Base und/oder eines geeigneten Katalysators.

## Revendications

1. Composé de formule I 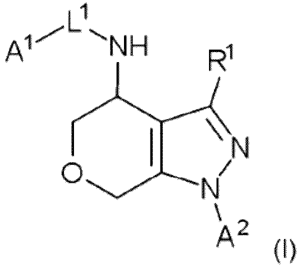 ou un sel pharmaceutiquement acceptable de celui-ci, dans lequel :
A¹ représente un groupe hétéroaryle bicyclique à 9 chaînons, un groupe hétéroaryle à 6 chaînons ou un groupe hétéroaryle à 5 chaînons, éventuellement substitué par un ou plusieurs groupes indépendamment choisis parmi G² ;
A² représente un groupe phényle éventuellement substitué par un ou plusieurs groupes indépendamment choisis parmi G³ ;
L¹ représente -C(O)- ;
R¹ représente H ;
dans lequel :
chaque G² représente indépendamment un atome d'halogène, R^{a2}, -CN, -A^{a2}-C(Q^{a2})R^{b2}-A^{b2}-C(Q^{b2})N(R^{c2})R^{d2}, -A^{c2}-C(Q^{c2})OR^{e2}, -A^{d2}-S (O) ₚR^{f2}, -A^{e2}-S(O)_{q}N(R^{p2})R^{h2}, -A^{f2}-S(O) ₚORⁱ², -N₃, -N (R^{j2})R^{k2}, -N(H)CN, -NO₂, -ONO₂, -OR^{l2} ou -SR^{m2} ;
chaque Q^{a2} à Q^{c2} représente indépendamment =O, =S, =NRⁿ² ou =N (OR^{o2}) ;
chaque A^{a2} à A^{f2} représente indépendamment une liaison simple, -N(R^{p2})- ou -O- ;
chaque G³ représente indépendamment un atome d'halogène, R^{a3} , -CN, -A^{a3}-C(Q^{a3})R^{b3}, A^{b3}-C(Q^{b3})N(R^{c3})R^{e3}, -A^{c3}-C(Q^{c3})OR^{e3}, -A^{d3}-S(O)ₚR^{f3}, -A^{e3}-S(O)_{q}N(R^{p3})R^{h3}, -A^{f3}-S(O)ₚORⁱ³, -N₃, -N(R^{j3})R^{k3}, -N (H) CN, -NO₂, -ONO₂, -OR¹³ ou -SR^{m3} ;
chaque Q^{a3} à Q^{c3} représente indépendamment =O, =S, =NRⁿ³ ou =N(OR^{o3}) ;
chaque A^{a3} à A^{f3} représente indépendamment une liaison simple, -N(R^{p3}) - ou -O- ;
chaque R^{a2} et R^{f2} représente indépendamment un groupe alkyle en C₁₋₆ éventuellement substitué par un ou plusieurs groupes indépendamment choisis parmi G^{6a}, un groupe hétérocycloalkyle éventuellement substitué par un ou plusieurs groupes indépendamment choisis parmi G^{6b}, un groupe aryle éventuellement substitué par un ou plusieurs groupes indépendamment choisis parmi G^{6c}, ou un groupe hétéroaryle éventuellement substitué par un ou plusieurs groupes indépendamment choisis parmi G^{6d} ;
chaque R^{p2} représente indépendamment H ou un groupe alkyle en C₁₋₆ éventuellement substitué par un ou plusieurs F ;
chaque R^{b2}, R^{c2}, R^{d2}, R^{e2}, R^{g2}, R^{h2}, Rⁱ², R^{j2}, R^{k2}, R^{l2}, R^{m2}, Rⁿ² et R^{o2} représente indépendamment H, un groupe alkyle en C₁₋₆ éventuellement substitué par un ou plusieurs groupes indépendamment choisis parmi G^{6a}, un groupe hétérocycloalkyle éventuellement substitué par un ou plusieurs groupes indépendamment choisis parmi G^{6b}, un groupe aryle éventuellement substitué par un ou plusieurs groupes indépendamment choisis parmi G^{6c}, ou un groupe hétéroaryle éventuellement substitué par un ou plusieurs groupes indépendamment choisis parmi G^{6d} ; ou
alternativement des quelconques parmi R^{c2} et R^{e2}, R^{g2} et R^{h2} et/ou R^{j2} et R^{k2} sont liés ensemble pour former, ensemble avec l'atome d'azote auquel ils sont liés, un cycle de 3 à 6 chaînons, lequel cycle contient éventuellement un hétéroatome supplémentaire et lequel cycle est éventuellement substitué par un ou plusieurs groupes indépendamment choisis parmi un atome d'halogène, un groupe alkyle en C₁₋₃ éventuellement substitué par un ou plusieurs atomes d'halogène et =O ;
chaque R^{a3} et R^{f3} représente indépendamment un groupe alkyle en C₁₋₆ éventuellement substitué par un ou plusieurs groupes indépendamment choisis parmi G^{7a}, un groupe hétérocycloalkyle éventuellement substitué par un ou plusieurs groupes indépendamment choisis parmi G^{7b}, un groupe aryle éventuellement substitué par un ou plusieurs groupes indépendamment choisis parmi G^{7c}, ou un groupe hétéroaryle éventuellement substitué par un ou plusieurs groupes indépendamment choisis parmi G^{7d} ;
chaque R^{p3} représente indépendamment H ou un groupe alkyle en C₁₋₆ éventuellement substitué par un ou plusieurs F ;
chaque R^{b3}, R^{c3}, R^{d3}, R^{e3}, R^{g3}, R^{h3}, Rⁱ³, R^{j3}, R^{k3}, R^{l3}, R^{m3}, Rⁿ³ et R^{o3} représente indépendamment H, un groupe alkyle en C₁₋₆ éventuellement substitué par un ou plusieurs groupes indépendamment choisis parmi G^{7a}, un groupe hétérocycloalkyle éventuellement substitué par un ou plusieurs groupes indépendamment choisis parmi G^{7b}, un groupe aryle éventuellement substitué par un ou plusieurs groupes indépendamment choisis parmi G^{7c}, ou un groupe hétéroaryle éventuellement substitué par un ou plusieurs groupes indépendamment choisis parmi G^{7d} ; ou
alternativement des quelconques parmi R^{c3} et R^{d3}, R^{g3} et R^{h3} et/ou R^{j3} et R^{k3} sont liés ensemble pour former, ensemble avec l'atome d'azote auquel ils sont liés, un cycle de 3 à 6 chaînons, lequel cycle contient éventuellement un hétéroatome supplémentaire et lequel cycle est éventuellement substitué par un ou plusieurs groupes indépendamment choisis parmi un atome d'halogène, un groupe alkyle en C₁₋₃ éventuellement substitué par un ou plusieurs atomes d'halogène et =O ;
chaque G^{6a}, G^{6b}, G^{7a} et G^{7b} représente indépendamment un atome d'halogène, -CN, -N(R^{b5})R^{c5}, -OR^{d5}, -SR^{e5} ou =O ;
chaque G^{6c}, G^{6d}, G^{7c} et G^{7d} représente indépendamment un atome d'halogène, R^{a5}, -CN, -N(R^{b5})R^{c5}, -OR^{d5}, -SR^{e5} ou =O ;
chaque R^{a5} représente indépendamment un groupe alkyle en C₁₋₆ éventuellement substitué par un ou plusieurs F ;
chaque R^{b5}, R^{c5}, R^{d5} et R^{e5} représente indépendamment H ou un groupe alkyle en C₁₋₆ éventuellement substitué par un ou plusieurs F ou =O ; et
ou R^{b5} et R^{c5} sont liés ensemble pour former, avec l'atome d'azote auquel ils sont liés, un cycle de 3 à 6 chaînons, lequel cycle contient éventuellement un hétéroatome supplémentaire et lequel cycle est éventuellement substitué par un ou plusieurs groupes indépendamment choisis parmi F, un groupe alkyle en C₁₋₃ éventuellement substitué par un ou plusieurs F et =O ;
et chaque p et q représente indépendamment 1 ou 2.

2. Composé selon la revendication 1, dans lequel :
chaque G² représente indépendamment -CN, -A^{a2}-C(Q^{a2})R^{b2} -A^{b2}-C(Qb²)N(R^{c2})R^{d2}, -A^{c2}-C(Q^{c2})OR^{e2}, -A^{d2}-S(O)ₚR^{f2}, -A^{e2}-S (O)_{q}N(R^{p2})R^{h2}, -A^{f2}-S(O) ₚORⁱ², -N₃, -N (R^{j2})R^{k2}, -N (H) CN, -NO₂, -ONO₂, un atome d'halogène, R^{a2}, -OR^{l2} ou -SR^{m2} ; chaque Q^{a2} à Q^{c2} représente indépendamment =O, =S, =NRⁿ² ou =N(OR^{o2}) ; et/ou chaque A^{a2} à A^{f2} représente indépendamment une liaison simple, -N(R^{p2})- ou -O- ; et/ou
chaque G³ représente indépendamment -CN, -Aa³-C(Q^{a3})R^{b3}, -A^{b3}-C(Q^{b3}) N(R^{c3})R^{d3}, -A^{c3}-C(Q^{c3})OR^{e3}, -Ad3-S(O)ₚR^{f3} , -Ae3-S (O)_{q}N(R^{p3})R^{h3}, -A^{f3}-S(O)ₚORⁱ³, -N₃, -N(R^{j3})R^{k3}, -N (H) CN, -NO₂, -ONO₂, un atome d'halogène, R^{a3}, -OR¹³ ou -SR^{m3} ; chaque Q^{a3} à Q^{c3} représente indépendamment =O, =S, =NRⁿ³ ou =N(OR^{o3}) ; et/ou chaque A^{a3} à A^{f3} représente indépendamment une liaison simple, -N(R^{p3})- ou -O-.

3. Composé selon la revendication 1 ou 2, dans lequel :
A¹ est substitué par un ou deux groupes indépendamment choisis parmi un atome d'halogène, R^{a2}, -C (O) OR^{e2}, -OR^{l2} et -SR^{m2} ; ou un groupe alkyle en C₁₋₃ éventuellement substitué par un ou plusieurs atomes de fluor, -C(O)OH, -C(O)O-alkyle en C₁₋₃, -OH, -O-alkyle en C₁₋₃, -SH et -S-alkyle en C₁₋₃.

4. Composé selon l'une quelconque des revendications 1 à 3, dans lequel
A¹ est choisi parmi un groupe tétrahydro-2,1-benzisoxazolyle, un groupe benzoxazoyle, un groupe pyrazinyle, un groupe indazolyle, un groupe quinoléinyle, un groupe 5H,6H,7H,8H-imidazo[1,5-a]pyridin-3-yle, un groupe 5H,6H,7H,8H-imidazo[1,5-a]pyridin-1-yle, un groupe imidazo[1,5-a]pyridin-3-yle, un groupe pyridinyle, un groupe thiazolyle, un groupe isoxazolyle et un groupe tétrahydro-1,2-benzisoxazolyle.

5. Composé selon l'une quelconque des revendications 1 à 4, dans lequel :
A² est substitué par un ou plusieurs groupes indépendamment choisis parmi un atome d'halogène, -A^{a3}-C (Q^{a3})R^{b3}, -A^{c3}-C(Q^{c3})OR^{e3}, R^{a3} et -OR¹³ ; ou
A² est substitué par un ou plusieurs groupes indépendamment choisis parmi un atome d'halogène, un groupe -C(O)-morpholinyle, un groupe -C(O)O-alkyle en C₁₋₃ et un groupe alkyle en C₁₋₄.

6. Composé selon l'une quelconque des revendications 1 à 3 ou 5, dans lequel
A¹ représente un groupe 5H,6H,7H,8H-imidazo[1,5-a]pyridin-3-yle, un groupe 5H,6H,7H,8H-imidazo[1,5-a]pyridin-1-yle, un groupe 4H,5H,6H,7H-1,2-benzisoxazol-3-yle, un groupe 4H,5H,6H,7H-2,1-benzisoxazol-3-yle ou un groupe 4H,5H,6H,7H-indazol-3-yle ; un groupe pyridinyle, un groupe pyrazolyle ou un groupe imidazolyle.

7. Composé selon l'une quelconque des revendications 1 à 6, dans lequel :
chaque G² représente indépendamment R^{a2} ; et/ou
chaque G³ représente indépendamment un atome d'halogène, R^{a3}, -A^{b3}-C(Q^{b3})N(R^{c3})R^{d3}, -A^{c3}-C(Q^{c3})OR^{e3} ; -A^{d3}-S(O)ₚR^{f3}, -OR¹³ ;
éventuellement dans lequel :
l'atome d'halogène représente un atome de fluor ou un atome de chlore, R^{a3} représente CH₃ ou CF₃, A^{b3}, A^{c3} et A^{d3} représentent une liaison simple, Q^{b3} et Q^{c3} représentent =O, p est égal à 2,
R^{c3} et R^{e3} représentent CH₃ ou sont liés ensemble pour former, ensemble avec l'atome d'azote auquel ils sont liés, un cycle à 5 ou 6 chaînons, lequel cycle contient éventuellement un hétéroatome supplémentaire,
R^{e3} représente un groupe alkyle en C₁₋₃, R^{f3} représente CH₃,
R^{l3} représente CH₃, CF₃, un groupe alkyle en C₂ substitué par N(CH₃)₂, OH, OCH₃ ou N(C=O)CH₃, et/ou un groupe alkyle en C₁ substitué par C(=O)N(CH₃)₂.

8. Composé selon l'une quelconque des revendications 1 à 7, dans lequel le composé est un composé de formule Ia 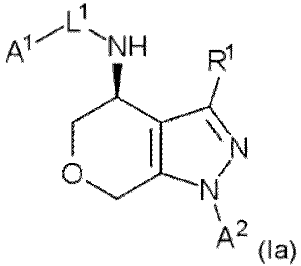 ou un sel pharmaceutiquement acceptable de celui-ci, dans lequel A¹, A², L¹ et R¹ sont tels que définis dans l'une quelconque des revendications 1 à 7.

9. Composé selon la revendication 1, dans lequel le composé est :
le N-(1-(2-fluorophényl)-1,4,5,7-tétrahydropyrano[3,4-c]pyrazol-4-yl)-5,6,7,8-tétrahydroimidazo[1,5-a]pyridine-1-carboxamide
le (R)-N-(1-(2-fluorophényl)-1,4,5,7-tétrahydropyrano[3,4-c]pyrazol-4-yl)-5,6,7,8-tétrahydroimidazo[1,5-a]pyridine-1-carboxamide
le (S)-N-(1-(2-fluorophényl)-1,4,5,7-tétrahydropyrano[3,4-c]pyrazol-4-yl)-5,6,7,8-tétrahydroimidazo[1,5-a]pyridine-1-carboxamide
le N-(1-(2-fluorophényl)-1,4,5,7-tétrahydropyrano[3,4-c]pyrazol-4-yl)-4,5,6,7-tétrahydrobenzo[d]isoxazole-3-carboxamide
le (R)-N-(1-(2-fluorophényl)-1,4,5,7-tétrahydropyrano[3,4-c]pyrazol-4-yl)-4,5,6,7-tétrahydrobenzo[d]isoxazole-3-carboxamide
le (S)-N-(1-(2-fluorophényl)-1,4,5,7-tétrahydropyrano[3,4-c]pyrazol-4-yl)-4,5,6,7-tétrahydrobenzo[d]isoxazole-3-carboxamide
le N-(1-(2-fluorophényl)-1,4,5,7-tétrahydropyrano[3,4-c]pyrazol-4-yl)-5,6,7,8-tétrahydroimidazo[1,5-a]pyridine-3-carboxamide
le (R)-N-(1-(2-fluorophényl)-1,4,5,7-tétrahydropyrano[3,4-c]pyrazol-4-yl)-5,6,7,8-tétrahydroimidazo[1,5-a]pyridine-3-carboxamide
le (S)-N-(1-(2-fluorophényl)-1,4,5,7-tétrahydropyrano[3,4-c]pyrazol-4-yl)-5,6,7,8-tétrahydroimidazo[1,5-a]pyridine-3-carboxamide
le N-1-(2-chloro-6-fluoro-3-méthoxyphényl)-1,4,5,7-tétrahydropyrano[3,4-c]pyrazol-4-yl)-5,6,7,8-tétrahydroimidazo[1,5-a]pyridine-1-carboxamide
le N-((4S)-1-(2-chloro-6-fluoro-3-méthoxyphényl)-1,4,5,7-tétrahydropyrano[3,4-c]pyrazol-4-yl)-5,6,7,8-tétrahydroimidazo[1,5-a]pyridine-1-carboxamide
le N-((4R)-1-(2-chloro-6-fluoro-3-méthoxyphényl)-1,4,5,7-tétrahydropyrano[3,4-c]pyrazol-4-yl)-5,6,7,8-tétrahydroimidazo[1,5-a]pyridine-1-carboxamide
le N-1-(2-chloro-6-fluorophényl)-1,4,5,7-tétrahydropyrano[3,4-c]pyrazol-4-yl)-5,6,7,8-tétrahydroimidazo[1,5-a]pyridine-1-carboxamide
le N-((4S)-1-(2-chloro-6-fluorophényl)-1,4,5,7-tétrahydropyrano[3,4-c]pyrazol-4-yl)-5,6,7,8-tétrahydroimidazo[1,5-a]pyridine-1-carboxamide
le N-((4R)-1-(2-chloro-6-fluorophényl)-1,4,5,7-tétrahydropyrano[3,4-c]pyrazol-4-yl)-5,6,7,8-tétrahydroimidazo[1,5-a]pyridine-1-carboxamide
le N-(1-(2,6-dichlorophényl)-1,4,5,7-tétrahydropyrano[3,4-c]pyrazol-4-yl)-5,6,7,8-tétrahydroimidazo[1,5-a]pyridine-1-carboxamide
le (S)-N-(1-(2,6-dichlorophényl)-1,4,5,7-tétrahydropyrano[3,4-c]pyrazol-4-yl)-5,6,7,8-tétrahydroimidazo[1,5-a]pyridine-1-carboxamide
le (R)-N-(1-(2,6-dichlorophényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-méthylphényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-méthylphényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-méthylphényl)-1,4,5,7-tétrahydropyrano[3,4-c]pyrazol-4-yl)-5-éthyl-1-méthyl-1H-imidazole-4-carboxamide ;
le (R)-N-(1-(2-chloro-3-méthylphényl)-1,4,5,7-tétrahydropyrano[3,4-c]pyrazol-4-yl)-5-éthyl-1-méthyl-1H-imidazole-4-carboxamide ;
le (S)-N-(1-(2-chloro-3-méthylphényl)-1,4,5,7-tétrahydropyrano[3,4-c]pyrazol-4-yl)-5,6,7,8-tétrahydroimidazo[1,5-a]pyridine-3-carboxamide ;
le (R)-N-(1-(2-chloro-3-méthylphényl)-1,4,5,7-tétrahydropyrano[3,4-c]pyrazol-4-yl)-5,6,7,8-tétrahydroimidazo[1,5-a]pyridine-3-carboxamide ;
le (S)-N-(1-(2-chloro-3-méthylphényl)-1,4,5,7-tétrahydropyrano[3,4-c]pyrazol-4-yl)-4,5,6,7-tétrahydro-1H-indazole-3-carboxamide ;
le (R)-N-(1-(2-chloro-3-méthylphényl)-1,4,5,7-tétrahydropyrano[3,4-c]pyrazol-4-yl)-4,5,6,7-tétrahydro-1H-indazole-3-carboxamide ;
le (S)-N-(1-(2-chloro-3-méthylphényl)-1,4,5,7-tétrahydropyrano[3,4-c]pyrazol-4-yl)-4,5,6,7-tétrahydrobenzo[d]isoxazole-3-carboxamide ;
le (R)-N-(1-(2-chloro-3-méthylphényl)-1,4,5,7-tétrahydropyrano[3,4-c]pyrazol-4-yl)-4,5,6,7-tétrahydrobenzo[d]isoxazole-3-carboxamide ;
le (S)-N-(1-(2-chloro-3-méthylphényl)-1,4,5,7-tétrahydropyrano[3,4-c]pyrazol-4-yl)-4-éthyl-5-méthyl-1H-pyrazole-3-carboxamide ;
le (R)-N-(1-(2-chloro-3-méthylphényl)-1,4,5,7-tétrahydropyrano[3,4-c]pyrazol-4-yl)-4-éthyl-5-méthyl-1H-pyrazole-3-carboxamide ;
le (S)-N-(1-(2-chloro-3-méthylphényl)-1,4,5,7-tétrahydropyrano[3,4-c]pyrazol-4-yl)-4,5-diméthylpicolinamide ;
le (R)-N-(1-(2-chloro-3-méthylphényl)-1,4,5,7-tétrahydropyrano[3,4-c]pyrazol-4-yl)-4,5-diméthylpicolinamide ;
le (S)-N-(1-(2-chloro-3-méthoxyphényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-méthoxyphényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-méthoxyphényl)-1,4,5,7-tétrahydropyrano[3,4-c]pyrazol-4-yl)-5-éthyl-1-méthyl-1H-imidazole-4-carboxamide ;
le (R)-N-(1-(2-chloro-3-méthoxyphényl)-1,4,5,7-tétrahydropyrano[3,4-c]pyrazol-4-yl)-5-éthyl-1-méthyl-1H-imidazole-4-carboxamide ;
le (S)-N-(1-(2-chloro-3-méthoxyphényl)-1,4,5,7-tétrahydropyrano[3,4-c]pyrazol-4-yl)-5,6,7,8-tétrahydroimidazo[1,5-a]pyridine-3-carboxamide ;
le (R)-N-(1-(2-chloro-3-méthoxyphényl)-1,4,5,7-tétrahydropyrano[3,4-c]pyrazol-4-yl)-5,6,7,8-tétrahydroimidazo[1,5-a]pyridine-3-carboxamide ;
le (S)-N-(1-(2-chloro-3-méthoxyphényl)-1,4,5,7-tétrahydropyrano[3,4-c]pyrazol-4-yl)-4,5,6,7-tétrahydro-1H-indazole-3-carboxamide ;
le (R)-N-(1-(2-chloro-3-méthoxyphényl)-1,4,5,7-tétrahydropyrano[3,4-c]pyrazol-4-yl)-4,5,6,7-tétrahydro-1H-indazole-3-carboxamide ;
le (S)-N-(1-(2-chloro-3-méthoxyphényl)-1,4,5,7-tétrahydropyrano[3,4-c]pyrazol-4-yl)-4,5,6,7-tétrahydrobenzo[d]isoxazole-3-carboxamide ;
le (R)-N-(1-(2-chloro-3-méthoxyphényl)-1,4,5,7-tétrahydropyrano[3,4-c]pyrazol-4-yl)-4,5,6,7-tétrahydrobenzo[d]isoxazole-3-carboxamide ;
le (S)-N-(1-(2-chloro-3-méthoxyphényl)-1,4,5,7-tétrahydropyrano[3,4-c]pyrazol-4-yl)-4-éthyl-5-méthyl-1H-pyrazole-3-carboxamide ;
le (R)-N-(1-(2-chloro-3-méthoxyphényl)-1,4,5,7-tétrahydropyrano[3,4-c]pyrazol-4-yl)-4-éthyl-5-méthyl-1H-pyrazole-3-carboxamide ;
le (S)-N-(1-(2-chloro-6-fluoro-3-méthoxyphényl)-1,4,5,7-tétrahydropyrano[3,4-c]pyrazol-4-yl)-4,5-diméthylpicolinamide ;
le (R)-N-(1-(2-chloro-6-fluoro-3-méthoxyphényl)-1,4,5,7-tétrahydropyrano[3,4-c]pyrazol-4-yl)-4,5-diméthylpicolinamide ;
le (S)-N-(1-(2-chloro-6-fluoro-3-méthoxyphényl)-1,4,5,7-tétrahydropyrano[3,4-c]pyrazol-4-yl)-5-éthyl-1-méthyl-1H-imidazole-4-carboxamide ;
le (R)-N-(1-(2-chloro-6-fluoro-3-méthoxyphényl)-1,4,5,7-tétrahydropyrano[3,4-c]pyrazol-4-yl)-5-éthyl-1-méthyl-1H-imidazole-4-carboxamide ;
le (S)-N-(1-(2-chloro-6-fluoro-3-méthoxyphényl)-1,4,5,7-tétrahydropyrano[3,4-c]pyrazol-4-yl)-5,6,7,8-tétrahydroimidazo[1,5-a]pyridine-3-carboxamide ;
le (R)-N-(1-(2-chloro-6-fluoro-3-méthoxyphényl)-1,4,5,7-tétrahydropyrano[3,4-c]pyrazol-4-yl)-5,6,7,8-tétrahydroimidazo[1,5-a]pyridine-3-carboxamide ;
le (S)-N-(1-(2-chloro-6-fluoro-3-méthoxyphényl)-1,4,5,7-tétrahydropyrano[3,4-c]pyrazol-4-yl)-4,5,6,7-tétrahydro-1H-indazole-3-carboxamide ;
le (R)-N-(1-(2-chloro-6-fluoro-3-méthoxyphényl)-1,4,5,7-tétrahydropyrano[3,4-c]pyrazol-4-yl)-4,5,6,7-tétrahydro-1H-indazole-3-carboxamide ;
le (S)-N-(1-(2-chloro-6-fluoro-3-méthoxyphényl)-1,4,5,7-tétrahydropyrano[3,4-c]pyrazol-4-yl)-4,5,6,7-tétrahydrobenzo[d]isoxazole-3-carboxamide ;
le (R)-N-(1-(2-chloro-6-fluoro-3-méthoxyphényl)-1,4,5,7-tétrahydropyrano[3,4-c]pyrazol-4-yl)-4,5,6,7-tétrahydrobenzo[d]isoxazole-3-carboxamide ;
le (S)-N-(1-(2-chloro-6-fluoro-3-méthoxyphényl)-1,4,5,7-tétrahydropyrano[3,4-c]pyrazol-4-yl)-4-éthyl-5-méthyl-1H-pyrazole-3-carboxamide ;
le (R)-N-(1-(2-chloro-6-fluoro-3-méthoxyphényl)-1,4,5,7-tétrahydropyrano[3,4-c]pyrazol-4-yl)-4-éthyl-5-méthyl-1H-pyrazole-3-carboxamide ;
le (S)-N-(1-(2-chloro-6-fluoro-3-méthoxyphényl)-1,4,5,7-tétrahydropyrano[3,4-c]pyrazol-4-yl)-4,5-diméthylpicolinamide ;
le (R)-N-(1-(2-chloro-6-fluoro-3-méthoxyphényl)-1,4,5,7-tétrahydropyrano[3,4-c]pyrazol-4-yl)-4,5-diméthylpicolinamide ;
le (S)-N-(1-(2-chloro-6-méthylphényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-6-méthylphényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-6-méthylphényl)-1,4,5,7-tétrahydropyrano[3,4-c]pyrazol-4-yl)-5-éthyl-1-méthyl-1H-imidazole-4-carboxamide ;
le (R)-N-(1-(2-chloro-6-méthylphényl)-1,4,5,7-tétrahydropyrano[3,4-c]pyrazol-4-yl)-5-éthyl-1-méthyl-1H-imidazole-4-carboxamide ;
le (S)-N-(1-(2-chloro-6-méthylphényl)-1,4,5,7-tétrahydropyrano[3,4-c]pyrazol-4-yl)-5,6,7,8-tétrahydroimidazo[1,5-a]pyridine-3-carboxamide ;
le (R)-N-(1-(2-chloro-6-méthylphényl)-1,4,5,7-tétrahydropyrano[3,4-c]pyrazol-4-yl)-5,6,7,8-tétrahydroimidazo[1,5-a]pyridine-3-carboxamide ;
le (S)-N-(1-(2-chloro-6-méthylphényl)-1,4,5,7-tétrahydropyrano[3,4-c]pyrazol-4-yl)-4,5,6,7-tétrahydro-1H-indazole-3-carboxamide ;
le (R)-N-(1-(2-chloro-6-méthylphényl)-1,4,5,7-tétrahydropyrano[3,4-c]pyrazol-4-yl)-4,5,6,7-tétrahydro-1H-indazole-3-carboxamide ;
le (S)-N-(1-(2-chloro-6-méthylphényl)-1,4,5,7-tétrahydropyrano[3,4-c]pyrazol-4-yl)-4,5,6,7-tétrahydrobenzo[d]isoxazole-3-carboxamide ;
le (R)-N-(1-(2-chloro-6-méthylphényl)-1,4,5,7-tétrahydropyrano[3,4-c]pyrazol-4-yl)-4,5,6,7-tétrahydrobenzo[d]isoxazole-3-carboxamide ;
le (S)-N-(1-(2-chloro-6-méthylphényl)-1,4,5,7-tétrahydropyrano[3,4-c]pyrazol-4-yl)-4-éthyl-5-méthyl-1H-pyrazole-3-carboxamide ;
le (R)-N-(1-(2-chloro-6-méthylphényl)-1,4,5,7-tétrahydropyrano[3,4-c]pyrazol-4-yl)-4-éthyl-5-méthyl-1H-pyrazole-3-carboxamide ;
le (S)-N-(1-(2-chloro-6-méthylphényl)-1,4,5,7-tétrahydropyrano[3,4-c]pyrazol-4-yl)-4,5-diméthylpicolinamide ;
le (R)-N-(1-(2-chloro-6-méthylphényl)-1,4,5,7-tétrahydropyrano[3,4-c]pyrazol-4-yl)-4,5-diméthylpicolinamide ;
le (S)-5-éthyl-N-(1-(2-fluorophényl)-1,4,5,7-tétrahydropyrano[3,4-c]pyrazol-4-yl)-1-méthyl-1H-imidazole-4-carboxamide ;
le (R)-5-éthyl-N-(1-(2-fluorophényl)-1,4,5,7-tétrahydropyrano[3,4-c]pyrazol-4-yl)-1-méthyl-1H-imidazole-4-carboxamide ;
le (S)-N-(1-(2-fluorophényl)-1,4,5,7-tétrahydropyrano[3,4-c]pyrazol-4-yl)-4,5,6,7-tétrahydro-1H-indazole-3-carboxamide ;
le (R)-N-(1-(2-fluorophényl)-1,4,5,7-tétrahydropyrano[3,4-c]pyrazol-4-yl)-4,5,6,7-tétrahydro-1H-indazole-3-carboxamide ;
le (S)-N-(1-(2-fluorophényl)-1,4,5,7-tétrahydropyrano[3,4-c]pyrazol-4-yl)-4,5,6,7-tétrahydrobenzo[d]isoxazole-3-carboxamide ;
le (R)-N-(1-(2-fluorophényl)-1,4,5,7-tétrahydropyrano[3,4-c]pyrazol-4-yl)-4,5,6,7-tétrahydrobenzo[d]isoxazole-3-carboxamide ;
le (S)-4-éthyl-N-(1-(2-fluorophényl)-1,4,5,7-tétrahydropyrano[3,4-c]pyrazol-4-yl)-5-méthyl-1H-pyrazole-3-carboxamide ;
le (R)-4-éthyl-N-(1-(2-fluorophényl)-1,4,5,7-tétrahydropyrano[3,4-c]pyrazol-4-yl)-5-méthyl-1H-pyrazole-3-carboxamide ;
le (S)-N-(1-(2-fluorophényl)-1,4,5,7-tétrahydropyrano[3,4-c]pyrazol-4-yl)-4,5-diméthylpicolinamide ;
le (R)-N-(1-(2-fluorophényl)-1,4,5,7-tétrahydropyrano[3,4-c]pyrazol-4-yl)-4,5-diméthylpicolinamide ;
le (S)-N-(1-(2-chloro-6-fluorophényl)-1,4,5,7-tétrahydropyrano[3,4-c]pyrazol-4-yl)-4,5,6,7-tétrahydro-1H-indazole-3-carboxamide ;
le (R)-N-(1-(2-chloro-6-fluorophényl)-1,4,5,7-tétrahydropyrano[3,4-c]pyrazol-4-yl)-4,5,6,7-tétrahydro-1H-indazole-3-carboxamide ;
le (S)-N-(1-(2-chloro-6-fluorophényl)-1,4,5,7-tétrahydropyrano[3,4-c]pyrazol-4-yl)-5,6,7,8-tétrahydroimidazo[1,5-a]pyridine-3-carboxamide ;
le (R)-N-(1-(2-chloro-6-fluorophényl)-1,4,5,7-tétrahydropyrano[3,4-c]pyrazol-4-yl)-5,6,7,8-tétrahydroimidazo[1,5-a]pyridine-3-carboxamide ;
le (S)-N-(1-(2-chloro-6-fluorophényl)-1,4,5,7-tétrahydropyrano[3,4-c]pyrazol-4-yl)-4,5-diméthylpicolinamide ;
le (R)-N-(1-(2-chloro-6-fluorophényl)-1,4,5,7-tétrahydropyrano[3,4-c]pyrazol-4-yl)-4,5-diméthylpicolinamide ;
le (S)-N-(1-(3-(méthylsulfonyl)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(3-(méthylsulfonyl)phényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-(trifluorométhyl)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-(trifluorométhyl)phényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chlorophényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chlorophényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(o-tolyl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(o-tolyl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-5-méthylphényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-5-méthylphényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-méthoxyphényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-méthoxyphényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-(trifluorométhyl)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-(trifluorométhyl)phényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(3-méthoxyphényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(3-méthoxyphényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(3-(trifluorométhoxy)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(3-(trifluorométhoxy)phényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-fluoro-6-méthylphényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-fluoro-6-méthylphényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2,6-difluorophényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2,6-difluorophényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-6-méthoxyphényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-6-méthoxyphényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-méthoxy-6-méthylphényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-méthoxy-6-méthylphényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-hydroxyphényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-hydroxyphényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-(2-(diméthylamino)éthoxy)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-(2-(diméthylamino)éthoxy)phényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-(2-(diméthylamino)-2-oxoéthoxy)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-(2-(diméthylamino)-2-oxoéthoxy)phényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-(2-(méthylamino)-2-oxoéthoxy)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-(2-(méthylamino)-2-oxoéthoxy)phényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(3-(2-acétamidoéthoxy)-2-chlorophényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(3-(2-acétamidoéthoxy)-2-chlorophényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-(2-méthoxyéthoxy)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-(2-méthoxyéthoxy)phényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-(2-hydroxyéthoxy)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-(2-hydroxyéthoxy)phényl)-1,4,5,7-tétrahydropyrano[3,4-c]pyrazol-4-yl)-5,6,7,8-tétrahydroimidazo[1,5-a]pyridine-1-carboxamide ;
le (S)-2-chloro-3-(4-(5,6,7,8-tétrahydroimidazo[1,5-a]pyridine-1-carboxamido)-4,7-dihydropyrano[3,4-c]pyrazol-1(5H)-yl)benzoate d'isopropyle ;
le (R)-2-chloro-3-(4-(5,6,7,8-tétrahydroimidazo[1,5-a]pyridine-1-carboxamido)-4,7-dihydropyrano[3,4-c]pyrazol-1(5H)-yl)benzoate d'isopropyle ;
le (S)-N-(1-(2-chloro-3-(diméthylcarbamoyl)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-(diméthylcarbamoyl)phényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-(pyrrolidine-1-carbonyl)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-(pyrrolidine-1-carbonyl)phényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-(pipéridine-1-carbonyl)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-(pipéridine-1-carbonyl)phényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-(morpholine-4-carbonyl)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-(morpholine-4-carbonyl)phényl)-1,4,5,7-tétrahydropyrano[3,4-c]pyrazol-4-yl)-5,6,7,8-tétrahydroimidazo[1,5-a]pyridine-1-carboxamide ;
le (S)-N-(1-(2-chloro-3-(diéthylcarbamoyl)phényl)-1,4,5,7-tétrahydropyrano[3,4-c]pyrazol-4-yl)-5,6,7,8-tétrahydroimidazo[1,5-a]pyridine-1-carboxamide ;
le (R)-N-(1-(2-chloro-3-(diéthylcarbamoyl)phényl)-1,4,5,7-tétrahydropyrano[3,4-c]pyrazol-4-yl)-5,6,7,8-tétrahydroimidazo[1,5-a]pyridine-1-carboxamide ;
ou des sels pharmaceutiquement acceptables de ceux-ci.

10. Composition pharmaceutique comprenant un composé tel que défini dans l'une quelconque des revendications 1 à 9, et éventuellement un ou plusieurs adjuvants, diluants et/ou véhicules pharmaceutiquement acceptables.

11. Composé tel que défini dans l'une quelconque des revendications 1 à 9 ou composition pharmaceutique telle que définie dans la revendication 10 pour une utilisation en médecine.

12. Composé tel que défini dans l'une quelconque des revendications 1 à 9 ou composition pharmaceutique telle que définie dans la revendication 10 pour une utilisation dans le traitement d'un cancer et/ou le traitement ou la prévention d'une infection virale.

13. Produit combiné comprenant :
(A) un composé tel que défini dans l'une quelconque des revendications 1 à 9 ; et
(B) un ou plusieurs agents thérapeutiques supplémentaires pour le traitement d'un cancer ou pour le traitement ou la prévention d'une infection virale,
dans lequel chacun des composants (A) et (B) est formulé sous forme de mélange, éventuellement avec un ou plusieurs adjuvants, diluants ou véhicules pharmaceutiquement acceptables.

14. Kit de composants comprenant :
(a) un composé tel que défini dans l'une quelconque des revendications 1 à 9, ou une composition pharmaceutique telle que définie dans la revendication 10 ; et
(b) un ou plusieurs agents thérapeutiques supplémentaires pour le traitement d'un cancer ou pour le traitement ou la prévention d'une infection virale, éventuellement sous forme de mélange avec un ou plusieurs adjuvants, diluants ou véhicules pharmaceutiquement acceptables,
lesquels composants (a) et (b) sont chacun fournis sous une forme qui est appropriée pour une administration conjointe.

15. Procédé de préparation d'un composé tel que défini dans l'une quelconque des revendications 1 à 9, lequel procédé comprend :
(i) la réaction d'un composé de formule II 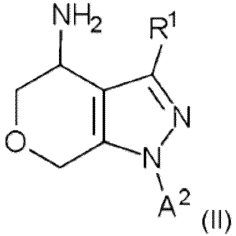 dans lequel R¹ et A² sont tels que définis dans les revendications 1 à 9, avec un composé de formule III
LG₁-L¹-A¹ (III)
dans lequel A¹ et L¹ sont tels que définis dans les revendications 1 à 9 et LG₁ représente un groupe partant approprié, en présence d'un solvant approprié et éventuellement en présence d'une base appropriée et/ou d'un catalyseur approprié ;
(ii) où L¹ représente -C(O)N(R²)- où R² représente H, la réaction d'un composé de formule II avec un composé de formule IV
A¹-N=C=O (IV)
dans lequel A¹ est tel que défini dans les revendications 1 à 9 en présence d'un solvant approprié et éventuellement en présence d'une base appropriée et/ou d'un catalyseur approprié ; ou
(iii) la réaction d'un composé de formule (V) 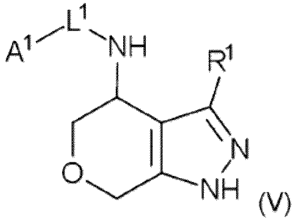 dans lequel R¹ et A¹ et L¹ sont tels que définis dans les revendications 1 à 9, avec un composé de formule VI
LG₂-A² (VI)
dans lequel A² est tel que défini dans les revendications 1 à 9 et LG₂ représente un groupe partant approprié, en présence d'un solvant approprié et éventuellement en présence d'une base appropriée et/ou d'un catalyseur approprié.
